# EUROPEAN PATENT SPECIFICATION

(11) **EP 3 893 913 B1**
(45) Date of publication and mention of the grant of the patent: **26.03.2025**
(21) Application number: 19897452.9
(22) Date of filing: 13.12.2019
(51) Int. Cl.: A61K 38/03, A61P 43/00, A61K 49/00, C07K 7/08, C07K 9/00

(54) **TRANSFERRIN RECEPTOR TARGETING PEPTIDES**
AUF DEN TRANSFERRINREZEPTOR ABZIELENDE PEPTIDE
PEPTIDES CIBLANT UN RÉCEPTEUR DE LA TRANSFERRINE

(30) Priority: 14.12.2018 US 201862779885 P; 19.04.2019 US 201962836520 P
(43) Date of publication of application: 20.10.2021
(73) Proprietor: Fred Hutchinson Cancer Center, Seattle, WA 98109 (US)
(72) Inventor: CROOK, Zachary, Seattle, Washington 98109 (US); BRUSNIAK, Mi-Youn, Seattle, Washington 98109 (US); OLSON, James, Seattle, Washington 98109 (US); MHYRE, Andrew James, Seattle, Washington 98109 (US); YIN, Chunfeng, Seattle, Washington 98109 (US); HOPPING, Gene Gregory, Seattle, Washington 98109 (US); STRONG, Roland, Seattle, Washington 98109 (US)
(74) Representative: MacLean, Martin Robert
(86) International application number: PCT/US2019/066376
(87) International publication number: WO 2020/124032

(56) References cited:
- WO-A1-2018/031424
- WO-A2-2016/077840
- US-A1- 2004 157 330
- US-A1- 2016 355 568
- FERNANDA I. STAQUICINI ET AL: "Systemic combinatorial peptide selection yields a non-canonical iron-mimicry mechanism for targeting tumors in a mouse model of human glioblastoma", THE JOURNAL OF CLINICAL INVESTIGATION, vol. 121, no. 1, 4 January 2011 (2011-01-04), GB, pages 161 - 173, XP055182684, ISSN: 0021-9738, DOI: 10.1172/JCI44798
- ROGER PRADES ET AL: "Applying the Retro-Enantio Approach To Obtain a Peptide Capable of Overcoming the Blood-Brain Barrier", ANGEWANDTE CHEMIE, WILEY - V C H VERLAG GMBH & CO. KGAA, DE, vol. 127, no. 13, 4 February 2015 (2015-02-04), pages 4039 - 4044, XP071363178, ISSN: 0044-8249, DOI: 10.1002/ANGE.201411408
- DATABASE NCBI 2 November 2008 (2008-11-02), "hypothetical protein [Monosiga brevicollis MX 1", Database accession no. XP_001746243 .1
- MU ET AL.: "Lipid vesicles containing transferrin receptor binding peptide TfR-T12 and octa- arginine conjugate stearyl-R8 efficiently treat brain glioma along with glioma stem cells", SCIENTIFIC REPORTS, vol. 7, 14 June 2017 (2017-06-14), pages 3487, XP055715637

## Description

### BACKGROUND

Drug delivery to targets in the central nervous system (CNS) is hampered by the blood-brain barrier (BBB), a term for the particularly impassable vascular endothelial cells in CNS capillaries. The BBB exists to keep toxic metabolites and pathogens out of the brain, but also serves to render diseases of the CNS particularly difficult to treat using conventional medicines. For example, primary CNS tumors (e.g., gliomas) and neuroinflammatory and neurodegenerative diseases (e.g., Multiple Sclerosis and Alzheimer's Disease) respond particularly poorly to therapeutic modalities that would otherwise be more effective in similar diseases affecting peripheral tissues. Thus, approaches for an improved delivery of therapeutic and/or diagnostic molecules into the CNS and other organs and tissues are needed. WO2018031424A1 describes *in vivo* methods for selecting peptides that cross the blood brain barrier, related compositions and methods of use. WO2016077840A2 describes variable new antigen receptors (VNARS) directed against transferrin receptor (TFR) and their use. Staquicini et al (J Clin Invest. 2011 Jan;121(1):161-73) describes systemic combinatorial peptide selection yields a non-canonical iron-mimicry mechanism for targeting tumors in a mouse model of human glioblastoma. Prades et al (Angew Chem Int Ed Engl. 2015 Mar 23;54(13):3967-72) describes applying the retro-enantio approach to obtain a peptide capable of overcoming the blood-brain barrier.

### SUMMARY

The present invention is defined by the claims. Any reference to a method of treatment identified in this specification refers to the composition as defined in the claims for use in the identified method. In various aspects, the present disclosure provides a composition comprising an engineered transferrin receptor (TfR)-binding peptide as defined in the claims.

In various aspects, the present disclosure provides a composition comprising a peptide construct, wherein the peptide construct comprises: a) a transferrin receptor (TfR)-binding peptide as defined in the claims, and b) an active agent, wherein the peptide is conjugated to, linked to, or fused to the active agent.

In some aspects, the peptide penetrates a cellular layer comprising a blood brain barrier (BBB). In some aspects, the peptide penetrates a membrane and wherein the membrane is the membrane of a cell. In some aspects, the cell expresses a TfR. In some aspects, the peptide penetrates the membrane of the cell via TfR-mediated endocytosis.

In some aspects, the peptide is delivered across the blood-brain barrier to the CNS by binding and then dissociating from TfR. In some aspects, the peptide is delivered across the blood-brain barrier by receptor-mediated transcytosis. In some aspects, the cell is a tumor cell. In some aspects, a cell or an organ in which the peptide localizes overexpresses a TfR.

In some aspects, the peptide comprises a surface interface residue corresponding to G5, A7, S8, N14, L17, E18, E21, L38, L42, L45, D46, H47, S50, and Q51, or any combination thereof, with reference to SEQ ID NO: 32. In some aspects, the peptide comprises hydrophilic surface-distal residues corresponding to any one of the positions 3, 4, 9, 11, 15, 16, 19, 23, 26, 28, 29, 30, 31, 32, 33, 35, 36, 37, 39, and 40, or any combination thereof, with reference to SEQ ID NO: 32. In some aspects, the hydrophilic surface-distal residues are selected from the amino acids D, E, H, K, R, N, Q, S, or T. In some aspects, the peptide comprises any one of the hydrophilic surface-distal residues corresponding to R3, E4, R9, K12, D15, E16, K19, R23, S26, S28, N29, T30, E31, E32, D33, E35, Q36, E37, E39, and D40, or any combination thereof, with reference to SEQ ID NO: 32. In some aspects, the peptide comprises hydrophilic residues at a position corresponding to any one of 15, 35, 39, or 49, or any combination thereof, with reference to SEQ ID NO: 32. In some aspects, the hydrophilic residues are selected from the amino acids D, E, H, K, R, N, Q, S, or T. In some aspects, the peptide comprises a hydrophilic residue corresponding to D15, E35, E39, and H49, or any combination thereof, with reference to SEQ ID NO: 32.

In some aspects, the peptide comprises hydrophobic residues at a position corresponding to 11, 25, or 27, or any combination thereof, with reference to SEQ ID NO: 32. In some aspects, the hydrophobic residues are selected from the amino acids A, M, I, L, V, F, W, or Y, or any combination thereof. In some aspects, the peptide comprises any one of the hydrophobic residues corresponding to M11, M25, M27, or any combination thereof, with reference to SEQ ID NO: 32.

In some aspects, the peptide comprises an aliphatic residue corresponding to position 45, with reference to SEQ ID NO: 32. In some aspects, the aliphatic residue is selected from the amino acids A, M, I, L, or V. In some aspects, the peptide comprises an aliphatic residue corresponding to L45, with reference to SEQ ID NO: 32.

In some aspects, the peptide comprises at least one disulfide bond, at least two disulfide bonds, at least three disulfide bonds, or at least five disulfide bonds. The peptide comprises a sequence as defined by the claims.
In some aspects, the peptide comprises the sequence set forth in SEQ ID NO: 1. In some aspects, the peptide comprises the sequence set forth in SEQ ID NO: 2.
In some aspects, the peptide comprises the sequence set forth in SEQ ID NO: 32.

In some aspects, the peptide is further formulated with, fused to, or conjugated to a cell-penetrating moiety. In some aspects, the cell-penetrating moiety comprises polycations, polyorganic acids, endosomal releasing polymers, poly(2-propylacrylic acid), poly(2-ethylacrylic acid), Tat peptide, Arg patch, a knotted peptide, CysTAT, S19-TAT, R8 (SEQ ID NO: 73), pAntp, Pas-TAT, Pas-R8 (SEQ ID NO: 76), Pas-FHV, Pas-pAntP, F2R4 (SEQ ID NO: 79), B55, aurein, IMT-P8, BR2, OMOTAG1, OMOTAG2, pVEC, SynB3, DPV1047, C105Y, Transportan, MTS, hLF, PFVYLI (SEQ ID NO: 93), maurocalcine, imperatoxin, hadrucalin, hemicalcin, opicalcin-1, opicalcin-2, midkine (62-104), MCoTI-II, chlorotoxin, DRI-TAT, cFΦR₄ (SEQ ID NO: 96), R₆W₃ (SEQ ID NO: 421), myristate, yBBR, or a fragment or variant thereof, or any combination thereof. In some aspects, the cell-penetrating moiety is any one of SEQ ID NO: 71 - SEQ ID NO: 96, SEQ ID NO: 98 - SEQ ID NO: 101, SEQ ID NO: 116 - SEQ ID NO: 126, or SEQ ID NO: 403 - SEQ ID NO: 455.

In some aspects, the peptide sequence comprises
at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58 residues, at least 59, at least 60, at least 61, at least 62, at least 63, at least 64, at least 65, at least 66, at least 67, at least 68, at least 69, at least 70, at least 71, at least 72, at least 73, at least 74, at least 75, at least 76, at least 77, at least 78, at least 79, at least 80, or at least 81 amino acid residues.

In some aspects, the active agent is linked via a linker, wherein the linker is selected from **TABLE** 7 or any one of SEQ ID NO: 103 - SEQ ID NO: 115, or SEQ ID NO: 125. In some aspects, the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter. In some aspects, the Kv1.3 inhibitor is Vm24, ShK-170, ShK-186, or ShK-192. In some aspects, the Vm24 has a sequence of SEQ ID NO: 378 or SEQ ID NO: 391. In some aspects, the Shk-185 has a sequence of SEQ ID NO: 379, SEQ ID NO: 390, or SEQ ID NO: 402. In some aspects, the neurotransmitter is neurotensin, a neurotensin peptide variant, or a functional fragment thereof. In some aspects, the neurotensin peptide variant comprises at least 80%, at least 85%, at least 90%, at least 92%, at least 95%, at least 96%, at least 97%, at least 98%, at least 99%, or 100% sequence identity to SEQ ID NO: 350 or SEQ ID NO: 365 - SEQ ID NO: 369.

In some aspects, the composition further comprises a half-life modifying agent coupled to the peptide. In some aspects, the half-life modifying agent comprises a polymer, a polyethylene glycol (PEG), a hydroxyethyl starch, polyvinyl alcohol, a water soluble polymer, a zwitterionic water soluble polymer, a water soluble poly(amino acid), a water soluble polymer of proline, alanine and serine, a water soluble polymer containing glycine, glutamic acid, and serine, an Fc region, a fatty acid, palmitic acid, or a molecule that binds to albumin. In some aspects, the half-life modifying agent comprises SA21. In some aspects, the SA21 has a sequence of SEQ ID NO: 376 or SEQ ID NO: 377.

In some aspects, the composition further comprises a detectable agent coupled to the peptide by a linker. In some aspects, the detectable agent is linked via a linker, wherein the linker is selected from TABLE 7 or any one of SEQ ID NO: 103 - SEQ ID NO: 115, or SEQ ID NO: 125. In some aspects, the detectable agent is a fluorophore, a near-infrared dye, a contrast agent, a nanoparticle, a metal-containing nanoparticle, a metal chelate, an X-ray contrast agent, a PET agent, a radionuclide, or a radionuclide chelator.

In various aspects, the present disclosure provides a pharmaceutical composition comprising any of the compositions described herein, or a salt thereof, and a pharmaceutically acceptable carrier.

Any reference to a method of treatment identified in this specification refers to the composition as defined in the claims for use in the identified method. In various aspects, the present disclosure provides a method for transporting a composition across a cell layer, the method comprising: a) contacting the cell layer with the composition comprising any of the peptides described herein or any of the pharmaceutical compositions described herein; and b) transporting the composition across the cell layer.

In various aspects, the present disclosure provides a method of treating a condition in a subject in need thereof, the method comprising: a) administering to the subject a composition comprising any of the peptides described herein or any of the pharmaceutical compositions described herein, and b) transporting the composition across a cell layer.

In various aspects, the present disclosure provides a method of treating a condition in a subject in need thereof, the method comprising administering to the subject an amount of a composition comprising any of the peptides described herein or any of the pharmaceutical compositions described herein based on the expression of TfR in a tissue of the subject.

In some aspects, the peptide construct is capable of binding and activating a neurotensin receptor. In some aspects, the method further comprises reducing a level of pain upon administration of the peptide to the subject. In some aspects, the administered is inhalation, intranasally, orally, topically, intravenously, subcutaneously, intramuscularly administration, intraperitoneally, intratumoral, intrathecal, or a combination thereof. In some aspects, the composition is administered intravenously as a bolus, infusion, or prolonged infusion.

In some aspects, the method further comprises administering the peptide to a patient with schizophrenia, drug abuse, Parkinson's disease, high blood pressure, an eating disorder, stroke, Alzheimer's disease, cancer, inflammation, pain, epilepsy, a mood disorder, or an endocrine disorder. In some aspects, the subject has a cancer. In some aspects, the subject has a disease of the CNS. In some aspects, the subject has neuropathic pain, migraine, anxiety, or epilepsy.

In various aspects, the present disclosure provides a method for transporting a composition
as claimed across a cell layer, the method comprising contacting the cell layer with the composition, and transporting the composition across the cell layer.

In various aspects, the present disclosure provides a method of treating a condition in a subject in need thereof, the method comprising administering to the subject the composition as claimed.

In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR. In some aspects, the transporting further comprises releasing an active agent from the composition.

In some aspects, the transferrin receptor (TfR) is a human transferrin receptor, or murine transferrin receptor, or a human transferrin receptor and murine transferrin receptor.

In some aspects, the peptide penetrates a cellular layer comprising endothelial cells. In some aspects, the peptide penetrates a cellular layer comprising a blood brain barrier (BBB). In some aspects, the peptide penetrates a cellular layer comprising endothelial cells via TfR-mediated transcytosis. In some aspects, the peptide penetrates a membrane and the membrane is the membrane of a cell. In some aspects, the cell expresses a TfR. In some aspects, the peptide penetrates the membrane of the cell via TfR-mediated endocytosis. In some aspects, the peptide is capable of localizing in or with any cell that expresses a TfR. In some aspects, the peptide remains bound to a cell surface membrane expressing TfR. In some aspects, the peptide is internalized into a cell by binding TfR. In some aspects, the peptide is delivered across the blood-brain barrier to the CNS by binding and then dissociating from TfR. In some aspects, the peptide is delivered across the blood-brain barrier by receptor-mediated transcytosis.

In some aspects, the cell is a CNS cell, an erythrocyte, an erythrocyte precursor cell, an immune cell, a stem cell, a muscle cell, a brain cell, a thyroid cell, a parathyroid cell, an adrenal gland cell, a bone marrow cell, an appendix cell, a lymph node cell, a tonsil cell, a spleen cell, a muscle cell, a liver cell, a gallbladder cell, a pancreas cell, a cell of the gastrointestinal tract, a glandular cell, a kidney cell, a urinary bladder cell, an endothelial cell, an epithelial cell, a choroid plexus epithelial cell, a glial cell, an astrocyte, or a cell associated with a nervous system.

In some aspects, the cell is a tumor cell. In some aspects, the tumor cell is an ovarian cancer cell, a colon cancer cell, a lung cancer cell, a cancer cell located in the bone or bone marrow, a glioblastoma cell, an astrocytoma cell, a glioma cell, a medulloblastoma cell, an ependymoma cell, a choroid plexus carcinoma cell, a midline glioma cell, a diffuse intrinsic pontine glioma (DIPG) cell, a breast cancer cell, a liver cancer cell, a colon cancer cell, a brain cancer cell, a spleen cancer cell, a cancer cell of the salivary gland, a kidney cancer cell, a muscle cancer cell, a bone marrow cell cancer cell, a skin cancer cell, a genitourinary cancer cell, an osteosarcoma cell, a muscle-derived sarcoma cell, a melanoma cell, a head and neck cancer cell, a neuroblastoma cell, a prostate cancer cell, a bladder cancer cell, an acute lymphocytic leukemia cell, an acute myeloid leukemia cell, a chronic lymphocytic leukemia cell, a chronic myeloid leukemia cell, a Hodgkin lymphoma cell, a Non-Hodgkin lymphoma cell, or a CMYC-overexpressing cancer cell. In some aspects, the peptide localizes to an organ or tissue.

In some aspects, the organ or tissue is brain, thyroid, parathyroid, adrenal glands, bone marrow, appendix, lymph node, tonsil, spleen, muscle, liver, gallbladder, pancreas, gastrointestinal tract, kidney, urinary bladder, fallopian tube, breast, vagina, cervix, endometrium, ovary, placenta, and skin. In some aspects, the cell or organ in which the peptide localizes overexpresses a TfR. In some aspects, the peptide localizes to the parenchyma of the organ.

In some aspects, the peptide binds at least one amino acid residues selected from the group of residues 506-510, 523-531, or 611-662 corresponding to SEQ ID NO: 363 (MMDQARSAFSNLFGGEPLSYTRFSLARQVDGDNSHVEMKLAVDEEENADNNTKANVT KPKRCSGSICYGTIAVIVFFLIGFMIGYLGYCKGVEPKTECERLAGTESPVREEPGEDFPA ARRLYWDDLKRKLSEKLDSTDFTGTIKLLNENSYVPREAGSQKDENLALYVENQFREFK LSKVWRDQHFVKIQVKDSAQNSVIIVDKNGRLVYLVENPGGYVAYSKAATVTGKLVHA NFGTKKDFEDLYTPVNGSIVIVRAGKITFAEKVANAESLNAIGVLIYMDQTKFPIVNAELS FFGHAHLGTGDPYTPGFPSFNHTQFPPSRSSGLPNIPVQTISRAAAEKLFGNMEGDCPSD WKTDSTCRMVTSESKNVKLTVSNVLKEIKILNIFGVIKGFVEPDHYVVVGAQRDAWGPG AAKSGVGTALLLKLAQMFSDMVLKDGFQPSRSIIFASWSAGDFGSVGATEWLEGYLSSL HLKAFTYINLDKAVLGTSNFKVSASPLLYTLIEKTMQNVKHPVTGQFLYQDSNWASKVE KLTLDNAAFPFLAYSGIPAVSFCFCEDTDYPYLGTTMDTYKELIERIPELNKVARAAAEV AGQFVIKLTHDVELNLDYERYNSQLLSFVRDLNQYRADIKEMGLSLQWLYSARGDFFR ATSRLTTDFGNAEKTDRFVMKKLNDRVMRVEYHFLSPYVSPKESPFRHVFWGSGSHTLP ALLENLKLRKQNNGAFNETLFRNQLALATWTIQGAANALSGDVWDIDNEF) of human TfR1 (UniProtKB - P02786).
In some aspects, peptide comprises a surface interface residue corresponding to G5, A7, S8, N14, L17, E18, E21, L38, L42, L45, D46, H47, S50, and Q51, or any combination thereof, with reference to SEQ ID NO: 32. In some aspects, the peptide comprises hydrophilic surface-distal residues corresponding to any one of the positions 3, 4, 9, 11, 15, 16, 19, 23, 26, 28, 29, 30, 31, 32, 33, 35, 36, 37, 39, and 40, or any combination thereof, with reference to SEQ ID NO: 32.

In some aspects, the hydrophilic surface-distal residues are selected from the amino acids D, E, H, K, R, N, Q, S, or T. In some aspects, the peptide comprises any one of the hydrophilic surface-distal residues corresponding to R3, E4, R9, K12, D15, E16, K19, R23, S26, S28, N29, T30, E31, E32, D33, E35, Q36, E37, E39, and D40, or any combination thereof, with reference to SEQ ID NO: 32. In some aspects, the peptide comprises hydrophilic residues at a position corresponding to any one of 15, 35, 39, or 49, or any combination thereof, with reference to SEQ ID NO: 32.

In some aspects, the hydrophilic residues are selected from the amino acids D, E, H, K, R, N, Q, S, or T. In some aspects, the peptide comprises a hydrophilic residue corresponding to D15, E35, E39, and H49, or any combination thereof, with reference to SEQ ID NO: 32. In some aspects, the peptide comprises hydrophobic residues at a position corresponding to 11, 25, or 27, or any combination thereof, with reference to SEQ ID NO: 32. In some aspects, the hydrophobic residues are selected from the amino acids A, M, I, L, V, F, W, or Y, or any combination thereof. In some aspects, the peptide comprises any one of the hydrophobic residues corresponding to M11, M25, M27, or any combination thereof, with reference to SEQ ID NO: 32. In some aspects, the peptide comprises an aliphatic residue corresponding to position 45, with reference to SEQ ID NO: 32. In some aspects, the aliphatic residue is selected from the amino acids A, M, I, L, or V.

In some aspects, the peptide comprises an aliphatic residue corresponding to L45, with reference to SEQ ID NO: 32. In some aspects, the peptide comprises at least one disulfide bond, at least two disulfide bonds, at least three disulfide bonds, or at least five disulfide bonds. In some aspects, the peptide comprises three disulfide bonds. In some aspects, the peptide comprises at least two, at least four, or at least six cysteine residues. In some aspects, the peptide comprises six cysteine residues.

In some aspects, the peptide comprises cysteine residues at positions corresponding to C6, C10, C20, C34, C44, and C48, or any combination thereof, with reference to SEQ ID NO: 32. In some aspects, at least one cysteine residue of the peptide participates in a disulfide bond. In some aspects, the six cysteine residues participate in disulfide bonds. In some aspects, a disulfide bond increases the stability of the peptide.

In some aspects, the peptide comprises the sequence set forth in SEQ ID NO: 1. In some aspects, the peptide comprises the sequence set forth in SEQ ID NO: 2.
In some aspects, the peptide comprises the sequence set forth in SEQ ID NO: 32.

In some aspects, the peptide comprising three disulfide bonds is derived from a cystine-dense peptide (CDP). In some aspects, the peptide comprising three disulfide bonds is a CDP. In some aspects, the peptide competes with transferrin, or a homolog, variant, or fragment thereof, for binding to TfR. In some aspects, the peptide sequesters TfR or prevents another protein from binding to TfR. In some aspects, the peptide has equal or greater affinity or specificity for TfR as compared to that of transferrin, or an endogenous derivative thereof. In some aspects, the peptide has equal or lower dissociation constant for TfR as compared to that of transferrin, or an endogenous derivative thereof. In some aspects, the peptide has a K_{D} of less than 40 nM, less than 30 nM, less than 20 nM, less than 10 nM, less than 1 nM, or less than 0.1 nM. In some aspects, the peptide penetrates or localizes into a target cell.

In some aspects, the peptide is further formulated with, fused to, or conjugated to a cell-penetrating moiety. In some aspects, the cell-penetrating moiety comprises polycations, polyorganic acids, endosomal releasing polymers, poly(2-propylacrylic acid), poly(2-ethylacrylic acid), Tat peptide, Arg patch, a knotted peptide, CysTAT, S19-TAT, R8 (SEQ ID NO: 73), pAntp, Pas-TAT, Pas-R8 (SEQ ID NO: 76), Pas-FHV, Pas-pAntP, F2R4 (SEQ ID NO: 79), B55, aurin, IMT-P8, BR2, OMOTAG1, OMOTAG2, pVEC, SynB3, DPV1047, C105Y, Transportan, MTS, hLF, PFVYLI (SEQ ID NO: 93), maurocalcine, imperatoxin, hadrucalin, hemicalcin, opicalcin-1, opicalcin-2, midkine (62-104), MCoTI-II, chlorotoxin, DRI-TAT, cFΦR₄ (SEQ ID NO: 96), myristate, yBBR, or a fragment or variant thereof, or any combination thereof. In some aspects, the cell-penetrating moiety is maurocalcin, imperatoxin, hadrucalcin, hemicalcin, opicalcin-1, opicalcin-2, midkine (62-104), MCoTI-II, or chlorotoxin.

In some aspects, the peptide targets, homes, accumulates in, or selectively penetrates a tissue or cell. In some aspects, the peptide localizes into a target cell's nucleus. In some aspects, the peptide localizes into the target cell's nucleus by formulation with, fusion to, or conjugation to a nuclear localization signal peptide or moiety. In some aspects, the peptide is further conjugated to, linked to, or fused to a moiety or a second peptide that targets, homes, accumulates in, or selectively penetrates a cancerous tissue or cancer cell. In some aspects, the moiety or a second peptide is a knotted peptide or variant or fragment thereof. In some aspects, the target cell is a cancerous cell or a tumor cell. In some aspects, the target cell is a CNS cell, an erythrocyte, an erythrocyte precursor cell, an immune cell, a stem cell, a muscle cell, a brain cell, a thyroid cell, a parathyroid cell, an adrenal gland cell, a bone marrow cell, an appendix cell, a lymph node cell, a tonsil cell, a spleen cell, a muscle cell, a liver cell, a gallbladder cell, a pancreas cell, a cell of the gastrointestinal tract, a glandular cell, a kidney cell, a urinary bladder cell, an endothelial cell, an epithelial cell, a choroid plexus epithelial cell, a glial cell, an astrocyte, or a cell associated with a nervous system.

In some aspects, the peptide comprises a disulfide through disulfide knot. In some aspects, the peptide comprises a plurality of disulfide bridges formed between cysteine residues. In some aspects, the peptide comprises three or more disulfide bridges formed between cysteine residues, wherein one of the disulfide bridges passes through a loop formed by two other disulfide bridges. In some aspects, the peptide comprises no disulfide bond or a disulfide knot. In some aspects, at least one amino acid residue of the peptide is in an L configuration or, wherein at least one amino acid residue is in a D configuration.

In some aspects, the peptide comprises at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 positively charged residues. In some aspects, the peptide comprises at least 2, at least 3, at least 4, at least 5, at least 6, at least 7, at least 8, at least 9, or at least 10 consecutive positively charged residues. In some aspects, the positively charged residues are Arg, Lys, or any combination thereof. In some aspects, the peptide comprises an Arg patch. In some aspects, the peptide comprises two or more consecutive Arg residues at the N-terminus.

In some aspects, the peptide comprises a Tat peptide, or a fragment thereof. In some aspects, the Tat peptide comprises a sequence of YGRKKRRQRRR (SEQ ID NO: 99), GRKKRRQRRR (SEQ ID NO: 100), or a fragment or variant thereof. In some aspects, the Tat peptide is appended to the N-terminus or C-terminus of the peptide using a linker having a sequence of (GS)ₓ (SEQ ID NO: 105) or GₓS_{y} (SEQ ID NO: 104), wherein x and y is independently 1, 2, 3, 4, 5, 6, 7, 8, 9, or 10. In some aspects, the Tat peptide is appended to any residue of the peptide.

In some aspects, the peptide comprises at least 4, at least 5, at least 6, at least 7, at least 8, at least 9, at least 10, at least 11, at least 12, at least 13, at least 14, at least 15, or at least 16 cysteine residues. In some aspects, the peptide comprises one or more regions of negatively charged residues. In some aspects, the peptide comprises one or more regions of positively charged residues.

In some aspects, the peptide sequence comprises
at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58 residues, at least 59, at least 60, at least 61, at least 62, at least 63, at least 64, at least 65, at least 66, at least 67, at least 68, at least 69, at least 70, at least 71, at least 72, at least 73, at least 74, at least 75, at least 76, at least 77, at least 78, at least 79, at least 80, or at least 81 amino acid residues.

In some aspects, the peptide comprises or is derived from a human protein or peptide. In some aspects, the peptide is arranged in a multimeric structure with at least one other peptide. In some aspects, the multimeric structure comprises a dimer, trimer, tetramer, pentamer, hexamer, or heptamer. In some aspects, the peptide comprises an isoelectric point within a range from about 3.0 to about 10.0. In some aspects, the peptide comprises an isoelectric point within a range from about 4.5 to about 8.9. In some aspects, the peptide comprises a non-uniform charge distribution. In some aspects, the peptide is stable at physiological or intracellular pH values.

In some aspects, the peptide is stable at a pH of about 7 or 7.5. In some aspects, the peptide is stable at a pH of about 6.5 to 7.5. In some aspects, the peptide is stable at pH values less than or equal to about 5.0, less than or equal to about 3.0, or within a range from about 3.0 to about 5.0. In some aspects, the peptide is stable at pH values within a range from about 5.0 to about 7.0. In some aspects, the peptide comprises a hydrophobic core.

In some aspects, the peptide is resistant to protease degradation. In some aspects, the protease is any one of pepsin, trypsin, chymotrypsin, serum proteases, intracellular proteases, or any combination thereof. In some aspects, the peptide is resistant to reduction or is stable in a reducing environment. In some aspects, the reduction or reducing environment comprises DTT or GSH. In some aspects, the peptide is stable at an elevated temperature. In some aspects, the peptide exhibits an anti-cancer effect. In some aspects, the peptide comprises one or more chemical modifications. In some aspects, the chemical modification modifies one or more pharmacokinetic properties of the peptide. In some aspects, the chemical modification extends the plasma half-life and/or the biological half-life of the peptide.

In some aspects, the chemical modification is blocking the N-terminus of the peptide. In some aspects, the chemical modification is methylation, acetylation, or acylation. In some aspects, the chemical modification is: methylation of one or more lysine residues or analogue thereof; methylation of the N-terminus; or methylation of one or more lysine residue or analogue thereof and methylation of the N-terminus. In some aspects, the peptide is linked to an acyl adduct. In some aspects, an active sequence is grafted onto the peptide or the peptide is conjugated to, linked to, or fused to or grafted onto an active agent resulting in a peptide construct.

In some aspects, the active agent is conjugated to, linked to, or fused to the peptide at an N-terminus or a C-terminus of the peptide. In some aspects, the active agent is an antibody, antibody fragment, Fc, single chain Fv, polypeptide, peptide, small molecule, or nucleic acid.

In some aspects, the nucleic acid is DNA. In some aspects, the nucleic acid is RNA. In some aspects, 1, 2, 3, 4, 5, 6, 7, 8, 9, or 10 active agents are linked to the peptide by a linker. In some aspects, the peptide is linked to the active agent via a cleavable linker or a pH sensitive linker. In some aspects, the peptide is linked to the active agent at an N-terminus, at the epsilon amine of an internal lysine residue, at the carboxylic acid of an aspartic acid or glutamic acid residue, or a C-terminus of the peptide by a linker. In some aspects, the composition further comprises a non-natural amino acid, wherein the non-natural amino acid is an insertion, appendage, or substitution for another amino acid. In some aspects, the peptide is linked to the active agent at the non-natural amino acid by a linker. In some aspects, the linker comprises an amide bond, an ester bond, an ether bond, a triazole, a macrocycle, an oxime bond, a carbamate bond, a carbonate bond, a hydrazone bond, an azo bond, an oxime bond, a disulfide bond, a thioester bond, a thioether bond, a carbon-carbon single, double, or triple bond, a disulfide bond, a two carbon bridge between two cysteines, a three carbon bridge between two cysteines, or a carbon-nitrogen bond.

In some aspects, the cleavable linker comprises a cleavage site for a protease. In some aspects, the protease is a matrix metalloproteinases, thrombin, cathepsins, or beta-glucuronidase. In some aspects, the linker is a hydrolytically labile linker. In some aspects, the peptide is linked to the active agent via a noncleavable linker. In some aspects, the active agent is linked via a linker, wherein the linker is selected from **TABLE 4.** In some aspects, the active agent is an anti-cancer agent. In some aspects, the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter.

In some aspects, the neurotransmitter is neurotensin, a neurotensin peptide variant, or a functional fragment thereof. In some aspects, the functional fragment is at least at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues.

In some aspects, the neurotensin peptide variant comprises at least 80%, at least 85%, at least 90%, at least 92%, at least 95%, at least 96%, at least 97%, at least 98%, at least 99%, or 100% sequence identity to SEQ ID NO: 365 - SEQ ID NO: 369. In some aspects, the neurotensin has at least 80%, at least 85%, at least 90%, at least 92%, at least 95%, at least 96%, at least 97%, at least 98%, at least 99%, or 100% sequence identity to SEQ ID NO: 350. In some aspects, the peptide construct is capable of binding and activating a neurotensin receptor. In some aspects, the peptide is capable of reducing a level of pain in a subject. In some aspects, the peptide is administered to a patient with schizophrenia, drug abuse, Parkinson's disease, high blood pressure, an eating disorder, stroke, Alzheimer's disease, cancer, inflammation, or an endocrine disorder. In some aspects, the peptide interacts with dopamine signaling neurons. In some aspects, the peptide increases dopamine release in neurons. In some aspects, the composition further comprises a half-life modifying agent coupled to the peptide. In some aspects, the half-life modifying agent comprises a polymer, a polyethylene glycol (PEG), a hydroxyethyl starch, polyvinyl alcohol, a water soluble polymer, a zwitterionic water soluble polymer, a water soluble poly(amino acid), a water soluble polymer of proline, alanine and serine, a water soluble polymer containing glycine, glutamic acid, and serine, an Fc region, a fatty acid, palmitic acid, or a molecule that binds to albumin.

In some aspects, the composition further comprises a detectable agent coupled to the peptide by a linker. In some aspects, the detectable agent is conjugated to, linked to, or fused with the peptide at an N-terminus or a C-terminus of the peptide. In some aspects, 1, 2, 3, 4, 5, 6, 7, 8, 9, or 10 detectable agents are linked to the peptide. In some aspects, the peptide is linked to the detectable agent via a cleavable linker. In some aspects, the peptide is linked to the detectable agent at an N-terminus, at the epsilon amine of an internal lysine residue, or a C-terminus of the peptide by a linker. In some aspects, the composition further comprises a non-natural amino acid, wherein the non-natural amino acid is an insertion, appendage, or substitution for another amino acid. In some aspects, the peptide is linked to the detectable agent at the non-natural amino acid by a linker. In some aspects, the linker comprises an amide bond, an ester bond, an ether bond, a triazole, a macrocycle, an oxime bond, a carbamate bond, a carbonate bond, a hydrazone bond, an azo bond, an oxime bond, a disulfide bond, a thioester bond, a thioether bond, a carbon-carbon single, double, or triple bond, a disulfide bond, a two carbon bridge between two cysteines, a three carbon bridge between two cysteines, or a carbon-nitrogen bond. In some aspects, the cleavable linker comprises a cleavage site for matrix metalloproteinases, thrombin, cathepsins, or beta-glucuronidase. In some aspects, the peptide is linked to the detectable agent via a stable linker. In some aspects, the detectable agent is linked via a linker, wherein the linker is selected from **TABLE 4.**

In some aspects, the detectable agent is a fluorophore, a near-infrared dye, a contrast agent, a nanoparticle, a metal-containing nanoparticle, a metal chelate, an X-ray contrast agent, a PET agent, a radionuclide, or a radionuclide chelator. In some aspects, the detectable agent is a fluorescent dye.

In various aspects, the present disclosure provides a pharmaceutical composition comprising any of the compositions disclosed herein, or a salt thereof, and a pharmaceutically acceptable carrier.

In some aspects, the pharmaceutical composition is formulated for administration to a subject. In some aspects, the pharmaceutical composition is formulated for oral administration, intravenous administration, subcutaneous administration, intramuscular administration, or a combination thereof.

In various aspects, the present disclosure provides a method for transporting a composition across a cell layer, the method comprising: contacting the cell layer with the composition; and transporting the composition across the cell layer.

The
peptide is capable of binding a transferrin receptor (TfR). In some aspects, the peptide comprises a surface interface residues corresponding to G5, A7, S8, N14, L17, E18, E21, L38, L42, L45, D46, H47, S50, and Q51, or any combination thereof, with reference to SEQ ID NO: 32. In some aspects, the peptide comprises a hydrophilic surface-distal residue at a position corresponding to 3, 4, 9, 11, 15, 16, 19, 23, 26, 28, 29, 30, 31, 32, 33, 35, 36, 37, 39, and 40, or any combination thereof, with reference to SEQ ID NO: 32. In some aspects, the hydrophilic surface-distal residues are selected from the amino acids D, E, H, K, R, N, Q, S, or T. In some aspects, the peptide comprises any one of the hydrophilic surface-distal residues corresponding to R3, E4, R9, K12, D15, E16, K19, R23, S26, S28, N29, T30, E31, E32, D33, E35, Q36, E37, E39, and D40, or any combination thereof, with reference to SEQ ID NO: 32. In some aspects, the peptide comprises hydrophilic residues at any one of the positions corresponding to 15, 35, 39, or 49, or any combination thereof, with reference to SEQ ID NO: 32.

In some aspects, the hydrophilic residues are selected from the amino acids D, E, H, K, R, N, Q, S, or T. In some aspects, the peptide comprises any one of the hydrophilic residues corresponding to D15, E35, E39, and H49, or any combination thereof, with reference to SEQ ID NO: 32. In some aspects, the peptide comprises hydrophobic residues at any of the positions corresponding to 11, 25, or 27, or any combination thereof, with reference to SEQ ID NO: 32. In some aspects, the hydrophobic residues are selected from the amino acids A, M, I, L, V, F, W, or Y, or any combination thereof. In some aspects, the peptide comprises any one of the hydrophobic residues corresponding to M11, M25, M27, or any combination thereof, with reference to SEQ ID NO: 32. In some aspects, the peptide comprises an aliphatic residue corresponding to position 45, with reference to SEQ ID NO: 32. In some aspects, the aliphatic residue is selected from the amino acids A, M, I, L, or V. In some aspects, the peptide comprises an aliphatic residue corresponding to L45, with reference to SEQ ID NO: 32.

In some aspects, the peptide penetrates or localizes into or on a target cell. In some aspects, an active sequence is grafted onto the peptide or the peptide is conjugated to, linked to, or fused to or grafted onto an active agent resulting in a peptide construct.

In some aspects, the peptide is conjugated to, linked to, or fused to the active agent. In some aspects, the active agent is linked via a linker, wherein the linker is selected from **TABLE 4.** In some aspects, the active agent is an antibody, antibody fragment, Fc, single chain Fv, polypeptide, peptide, small molecule, or nucleic acid.

In some aspects, the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter. In some aspects, the neurotransmitter is neurotensin, a neurotensin peptide variant, or a functional fragment thereof.

In some aspects, the functional fragment is at least at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues. In some aspects, the neurotensin peptide variant comprises at least 80%, at least 85%, at least 90%, at least 92%, at least 95%, at least 96%, at least 97%, at least 98%, at least 99%, or 100% sequence identity to SEQ ID NO: 365 - SEQ ID NO: 369. In some aspects, the neurotensin has at least 80%, at least 85%, at least 90%, at least 92%, at least 95%, at least 96%, at least 97%, at least 98%, at least 99%, or 100% sequence identity to SEQ ID NO: 350.

In some aspects, the peptide construct is capable of binding and activating a neurotensin receptor. In some aspects, the peptide is capable of reducing a level of pain upon administration to a subject. In some aspects, the peptide is administered to a patient with schizophrenia, drug abuse, Parkinson's disease, high blood pressure, an eating disorder, stroke, Alzheimer's disease, cancer, inflammation, or an endocrine disorder. In some aspects, the peptide interacts with dopamine signaling neurons. In some aspects, the peptide increases dopamine release in neurons. In some aspects, the peptide is further formulated with, fused to, or conjugated to a cell-penetrating moiety. In some aspects, the cell layer is an endothelial cell layer or an epithelial cell layer. In some aspects, the endothelial cell layer is the blood brain barrier. In some aspects, the endothelial cell layer is an intestinal endothelial cell layer. In some aspects, the epithelial cell layer is an intestinal epithelial cell layer. In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR.

In various aspects, the present disclosure provides a method of treating a condition in a subject in need thereof, the method comprising: administering to the subject a composition, and transporting the composition across a cell layer.

In some aspects, the peptide comprises a surface interface residues corresponding to G5, A7, S8, N14, L17, E18, E21, L38, L42, L45, D46, H47, S50, and Q51, or any combination thereof, with reference to SEQ ID NO: 32. In some aspects, the peptide comprises a hydrophilic surface-distal residues at any one of the positions corresponding to 3, 4, 9, 11, 15, 16, 19, 23, 26, 28, 29, 30, 31, 32, 33, 35, 36, 37, 39, and 40, or any combination thereof, with reference to SEQ ID NO: 32. In some aspects, the hydrophilic surface-distal residues are selected from the amino acids D, E, H, K, R, N, Q, S, or T. In some aspects, the peptide comprises any one of the hydrophilic surface-distal residues corresponding to R3, E4, R9, K12, D15, E16, K19, R23, S26, S28, N29, T30, E31, E32, D33, E35, Q36, E37, E39, and D40, or any combination thereof, with reference to SEQ ID NO: 32.

In some aspects, the peptide comprises hydrophilic residues at any one of the positions corresponding to 15, 35, 39, or 49, or any combination thereof, with reference to SEQ ID NO: 32. In some aspects, the hydrophilic residues are selected from the amino acids D, E, H, K, R, N, Q, S, or T. In some aspects, the peptide comprises any one of the hydrophilic residues corresponding to D15, E35, E39, and H49, or any combination thereof, with reference to SEQ ID NO: 32. In some aspects, the peptide comprises hydrophobic residues at any of the positions corresponding to 11, 25, or 27, or any combination thereof, with reference to SEQ ID NO: 32. In some aspects, the hydrophobic residues are selected from the amino acids A, M, I, L, V, F, W, or Y, or any combination thereof. In some aspects, the peptide comprises any one of the hydrophobic residues corresponding to M11, M25, M27, or any combination thereof, with reference to SEQ ID NO: 32.

In some aspects, the peptide comprises an aliphatic residue corresponding to position 45, with reference to SEQ ID NO: 32. In some aspects, the aliphatic residue is selected from the amino acids A, M, I, L, or V. In some aspects, the peptide comprises an aliphatic residue corresponding to L45, with reference to SEQ ID NO: 32. In some aspects, the peptide comprises at least one disulfide bond, at least two disulfide bonds, at least three disulfide bonds, or at least five disulfide bonds.

In some aspects, the peptide penetrates or localizes into a target cell. In some aspects, an active sequence is grafted onto the peptide or the peptide is conjugated to, linked to, or fused to or grafted onto an active agent resulting in a peptide construct. In some aspects, the peptide is conjugated to, linked to, or fused to the active agent. In some aspects, the active agent is linked via a linker, wherein the linker is selected from **TABLE 4.**

In some aspects, the active agent is an antibody, antibody fragment, Fc, single chain Fv, polypeptide, peptide, small molecule, or nucleic acid.

In some aspects, the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter.

In some aspects, the neurotransmitter is neurotensin, a neurotensin peptide variant, or a functional fragment thereof. In some aspects, the functional fragment is at least at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues. In some aspects, the neurotensin peptide variant comprises at least 80%, at least 85%, at least 90%, at least 92%, at least 95%, at least 96%, at least 97%, at least 98%, at least 99%, or 100% sequence identity to SEQ ID NO: 365 - SEQ ID NO: 369. In some aspects, the neurotensin has at least 80%, at least 85%, at least 90%, at least 92%, at least 95%, at least 96%, at least 97%, at least 98%, at least 99%, or 100% sequence identity to SEQ ID NO: 350.

In some aspects, the peptide construct is capable of binding and activating a neurotensin receptor. In some aspects, the peptide is capable of reducing a level of pain upon administration to the subject. In some aspects, the peptide is administered to a patient with schizophrenia, drug abuse, Parkinson's disease, high blood pressure, an eating disorder, stroke, Alzheimer's disease, cancer, inflammation, or an endocrine disorder. In some aspects, the peptide interacts with dopamine signaling neurons. In some aspects, the peptide increases dopamine release in neurons. In some aspects, the peptide is further formulated with, fused to, or conjugated to a cell-penetrating moiety. In some aspects, the cell layer is an endothelial cell layer or an epithelial cell layer. In some aspects, the endothelial cell layer is the blood brain barrier.

In some aspects, the endothelial cell layer is an intestinal endothelial cell layer. In some aspects, the epithelial cell layer is an intestinal epithelial cell layer. In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR. In some aspects, the transporting further comprises releasing the active agent from the composition.

In various aspects, the present disclosure provides a method of treating a condition in a subject in need thereof, the method comprising administering to the subject an amount of a composition based on the expression of TfR in the subject.

In some aspects, the peptide comprises a surface interface residues corresponding to G5, A7, S8, N14, L17, E18, E21, L38, L42, L45, D46, H47, S50, and Q51, or any combination thereof, with reference to SEQ ID NO: 32. In some aspects, the peptide comprises a hydrophilic surface-distal residues at any one of the positions corresponding to 3, 4, 9, 11, 15, 16, 19, 23, 26, 28, 29, 30, 31, 32, 33, 35, 36, 37, 39, and 40, or any combination thereof, with reference to SEQ ID NO: 32. In some aspects, the hydrophilic surface-distal residues are selected from the amino acids D, E, H, K, R, N, Q, S, or T. In some aspects, the peptide comprises any one of the hydrophilic surface-distal residues corresponding to R3, E4, R9, K12, D15, E16, K19, R23, S26, S28, N29, T30, E31, E32, D33, E35, Q36, E37, E39, and D40, or any combination thereof, with reference to SEQ ID NO: 32. In some aspects, the peptide comprises hydrophilic residues at any one of the positions corresponding to 15, 35, 39, or 49, or any combination thereof, with reference to SEQ ID NO: 32. In some aspects, the hydrophilic residues are selected from the amino acids D, E, H, K, R, N, Q, S, or T. In some aspects, the peptide comprises any one of the hydrophilic residues corresponding to D15, E35, E39, and H49, or any combination thereof, with reference to SEQ ID NO: 32. In some aspects, the peptide comprises hydrophobic residues at any of the positions corresponding to11, 25, or 27, or any combination thereof, with reference to SEQ ID NO: 32.

In some aspects, the hydrophobic residues are selected from the amino acids A, M, I, L, V, F, W, or Y, or any combination thereof. In some aspects, the peptide comprises any one of the hydrophobic residues corresponding to M11, M25, M27, or any combination thereof, with reference to SEQ ID NO: 32. In some aspects, the peptide comprises an aliphatic residue corresponding to position 45, with reference to SEQ ID NO: 32. In some aspects, the aliphatic residue is selected from the amino acids A, M, I, L, or V. In some aspects, the peptide comprises an aliphatic residue corresponding to L45, with reference to SEQ ID NO: 32. In some aspects, the peptide comprises at least one disulfide bond, at least two disulfide bonds, at least three disulfide bonds, or at least five disulfide bonds.

In some aspects, the peptide penetrates or localizes into a target cell. In some aspects, an active sequence is grafted onto the peptide or the peptide is conjugated to, linked to, or fused to or grafted onto an active agent resulting in a peptide construct. In some aspects, the peptide is conjugated to, linked to, or fused to the active agent. In some aspects, the active agent is linked via a linker, wherein the linker is selected from **TABLE 4.**

In some aspects, the active agent is an antibody, antibody fragment, Fc, single chain Fv, polypeptide, peptide, small molecule, or nucleic acid.

In some aspects, the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter.

In some aspects, the neurotransmitter is neurotensin, a neurotensin peptide variant, or a functional fragment thereof. In some aspects, the functional fragment is at least at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues. In some aspects, the neurotensin peptide variant comprises at least 80%, at least 85%, at least 90%, at least 92%, at least 95%, at least 96%, at least 97%, at least 98%, at least 99%, or 100% sequence identity to SEQ ID NO: 365 - SEQ ID NO: 369. In some aspects, the neurotensin has at least 80%, at least 85%, at least 90%, at least 92%, at least 95%, at least 96%, at least 97%, at least 98%, at least 99%, or 100% sequence identity to SEQ ID NO: 350.

In some aspects, the peptide construct is capable of binding and activating a neurotensin receptor. In some aspects, the peptide is capable of reducing a level of pain upon administration to the subject. In some aspects, the peptide is administered to a patient with schizophrenia, drug abuse, Parkinson's disease, high blood pressure, an eating disorder, stroke, Alzheimer's disease, cancer, inflammation, or an endocrine disorder. In some aspects, the peptide interacts with dopamine signaling neurons. In some aspects, the peptide increases dopamine release in neurons. In some aspects, the peptide is further formulated with, fused to, or conjugated to a cell-penetrating moiety. In some aspects, the composition is administered by inhalation, intranasally, orally, topically, intravenously, subcutaneously, intramuscularly administration, intraperitoneally, intratumoral, intrathecal, or a combination thereof.

In some aspects, the composition is administered intravenously as a bolus, infusion, or prolonged infusion. In some aspects, the composition or the pharmaceutical composition is administered multiple times a day, twice a day, once a day, twice a week, three times a week, once a week, once every two weeks, or once a month or once every three months. In some aspects, the composition is administered subcutaneously once a day, once a week, or once a month. In some aspects, the condition is a cancer.

In some aspects, the cancer is ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer. In some aspects, the condition is a brain cancer. In some aspects, the brain cancer is glioblastoma, astrocytoma, glioma, medulloblastoma, ependymoma, choroid plexus carcinoma, midline glioma, or diffuse intrinsic pontine glioma.

In some aspects, the condition is a disease of the CNS. In some aspects, the disease of the CNS is a neuroinflammatory or neurodegenerative disease. In some aspects, the neurodegenerative disease is Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia. In some aspects, the condition is neuropathic pain, migraine, anxiety, or epilepsy. In some aspects, the condition is chronic pain, acute pain, nociceptive pain, neuropathic pain, allodynia, phantom pain, visceral pain, or breakthrough pain.

In some aspects, the condition is chronic pain, acute pain, injury, mechanical pain, heat pain, cold pain, ischemic pain, and chemical-induced pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain, nociceptive pain, neuropathic pain, allodynia, phantom pain, visceral pain, breakthrough pain, familial episodic pain syndrome, paroxysmal extreme pain disorder, congenital indifference to pain, pain associated with opioid withdrawal. In some aspects, the condition is muscular dystrophy, or spinal bulbar muscular dystrophy (SBMA). In some aspects, the condition is an inflammatory bowel disease. In some aspects, the inflammatory bowel disease is a Crohn's disease.

In various aspects, the present disclosure provides a method for transporting any of the compositions disclosed herein across a cell layer, the method comprising contacting the cell layer with the composition, and transporting the composition across the cell layer.

In some aspects, the cell layer is an endothelial cell layer or an epithelial cell layer. In some aspects, the endothelial cell layer is the blood brain barrier. In some aspects, the endothelial cell layer is an intestinal endothelial cell layer. In some aspects, the epithelial cell layer is an intestinal epithelial cell layer. In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR. In some aspects, the transporting further comprises releasing an active agent from the composition.

In various aspects, the present disclosure provides a method of treating a condition in a subject in need thereof, the method comprising administering to the subject any of the compositions disclosed herein or any of the pharmaceutical compositions disclosed herein.

In some aspects, the composition is administered by inhalation, intranasally, orally, topically, intravenously, subcutaneously, intramuscularly administration, intraperitoneally, intratumoral, intrathecal, or a combination thereof. In some aspects, the composition is administered intravenously as a bolus, infusion, or prolonged infusion. In some aspects, the composition or the pharmaceutical composition is administered multiple times a day, twice a day, once a day, twice a week, three times a week, once a week, once every two weeks, or once a month or once every three months. In some aspects, the composition is administered subcutaneously once a day, once a week, or once a month. In some aspects, the condition is a cancer. In some aspects, the cancer is ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer. In some aspects, the condition is a brain cancer.

In some aspects, the brain cancer is glioblastoma, astrocytoma, glioma, medulloblastoma, ependymoma, choroid plexus carcinoma, midline glioma, or diffuse intrinsic pontine glioma. In some aspects, the condition is a disease of the CNS. In some aspects, the condition of the CNS is a neuroinflammatory or neurodegenerative disease. In some aspects, the neurodegenerative disease is Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia. In some aspects, the condition is neuropathic pain, migraine, anxiety, or epilepsy.

In some aspects, the condition is chronic pain, acute pain, injury, mechanical pain, heat pain, cold pain, ischemic pain, and chemical-induced pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain, nociceptive pain, neuropathic pain, allodynia, phantom pain, visceral pain, breakthrough pain, familial episodic pain syndrome, paroxysmal extreme pain disorder, congenital indifference to pain, pain associated with opioid withdrawal. In some aspects, the condition is muscular dystrophy, or spinal bulbar muscular dystrophy (SBMA).

In some aspects, the condition is an inflammatory bowel disease. In some aspects, the inflammatory bowel disease is a Crohn's disease. In some aspects, the method further comprises transporting the composition across a cell layer. In some aspects, the cell layer is an endothelial cell layer or an epithelial cell layer. In some aspects, the endothelial cell layer is the blood brain barrier. In some aspects, the endothelial cell layer is an intestinal endothelial cell layer.

In some aspects, the epithelial cell layer is an intestinal epithelial cell layer. In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR. In some aspects, the transporting further comprises releasing an active agent from the composition.

In various aspects, the present disclosure provides a method of treating a condition in a subject in need thereof, the method comprising administering to the subject an amount of any of the compositions disclosed herein or any of the pharmaceutical compositions disclosed herein that is dependent on the expression of TfR in the subject.

In some aspects, the composition is administered by inhalation, intranasally, orally, topically, intravenously, subcutaneously, intramuscularly administration, intraperitoneally, intratumoral, intrathecal, or a combination thereof. In some aspects, the composition is administered intravenously as a bolus, infusion, or prolonged infusion. In some aspects, the composition or the pharmaceutical composition is administered multiple times a day, twice a day, once a day, twice a week, three times a week, once a week, once every two weeks, or once a month or once every three months. In some aspects, the composition is administered subcutaneously once a day, once a week, or once a month. In some aspects, the condition is a cancer.

In some aspects, the cancer is ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer. In some aspects, the condition is a brain cancer. In some aspects, the brain cancer is glioblastoma, astrocytoma, glioma, medulloblastoma, ependymoma, choroid plexus carcinoma, midline glioma, or diffuse intrinsic pontine glioma.

In some aspects, the condition is a disease of the CNS. In some aspects, the disease of the CNS is a neuroinflammatory or neurodegenerative disease. In some aspects, the neurodegenerative disease is Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia.

In some aspects, the condition is neuropathic pain, migraine, anxiety, or epilepsy. In some aspects, the condition is chronic pain, acute pain, injury, mechanical pain, heat pain, cold pain, ischemic pain, and chemical-induced pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain, nociceptive pain, neuropathic pain, allodynia, phantom pain, visceral pain, breakthrough pain, familial episodic pain syndrome, paroxysmal extreme pain disorder, congenital indifference to pain, pain associated with opioid withdrawal. In some aspects, the condition is muscular dystrophy, or spinal bulbar muscular dystrophy (SBMA).

In some aspects, the condition is an inflammatory bowel disease. In some aspects, the inflammatory bowel disease is a Crohn's disease. In some aspects, the method further comprises transporting the composition across a cell layer. In some aspects, the cell layer is an endothelial cell layer or an epithelial cell layer. In some aspects, the endothelial cell layer is the blood brain barrier. In some aspects, the endothelial cell layer is an intestinal endothelial cell layer. In some aspects, the epithelial cell layer is an intestinal epithelial cell layer.

In some aspects, the transporting comprises binding of the composition to TfR. In some aspects, the transporting further comprises transcytosis. In some aspects, the transcytosis is vesicular transcytosis. In some aspects, the transcytosis is TfR-mediated. In some aspects, the transporting further comprises dissociation of the composition from the TfR. In some aspects, the transporting further comprises releasing an active agent from the composition.

In various aspects, the present disclosure provides a method for designing a peptide capable of binding to TfR, the method comprising: using a computer program to identify a peptide from a library of peptides with optimized stability and folding, wherein each peptide of the library of peptides comprises at least three intramolecular disulfide bonds; using a computer program to superimpose amino acid residues of a binding patch from a peptide:TfR complex to determine a stable peptide that binds to TfR after grafting the binding patch onto the peptide; and grafting the binding patch onto the peptide to produce the stable peptide.

In some aspects, the method further comprises performing site saturation mutagenesis to obtain a mutated peptide with a higher binding affinity for TfR than the stable peptide. In some aspects, the peptide from a library of peptides is between 20 and 75 amino acid residues in length.

In various aspects, the present disclosure provides any of the compositions disclosed herein, wherein the neurotensin comprises any one of SEQ ID NO: 350, SEQ ID NO: 365 - SEQ ID NO: 371, or a functional fragment thereof.

In various aspects, the present disclosure provides any of the methods disclosed herein, wherein the neurotensin comprises any one of SEQ ID NO: 350, SEQ ID NO: 365 - SEQ ID NO: 371, or a functional fragment thereof.

In various aspects, the present disclosure provides any of the methods disclosed herein, wherein the neurotensin comprises any one of SEQ ID NO: 350, SEQ ID NO: 365 - SEQ ID NO: 371, or a functional fragment thereof.

In various aspects, the present disclosure provides any of the methods disclosed herein, wherein the neurotensin comprises any one of SEQ ID NO: 350, SEQ ID NO: 365 - SEQ ID NO: 371, or a functional fragment thereof.

### BRIEF DESCRIPTION OF THE DRAWINGS

The patent or application file contains at least one drawing executed in color. Copies of this patent or patent application publication with color drawing(s) will be provided by the Office upon request and payment of the necessary fee. The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** illustrates a Coomassie stained gel of human soluble transferrin receptor (hTfR) ectodomain protein and flow cytometry plots showing successive enrichment of cells that bind to hTfR ectodomain from a pooled, highly diverse peptide library.
**FIG. 1A** illustrates a Coomassie stained gel of transferrin receptor (TfR) protein.
**FIG. 1B** illustrates a flow cytometry plot showing cells that bind to TfR after one flow sort.
**FIG. 1C** illustrates a flow cytometry plot showing cells of a control stain after one flow sort.
**FIG. 1D** illustrates a flow cytometry plot showing cells that bind to TfR after two flow sorts.
**FIG. 1E** illustrates a flow cytometry plot showing cells of a control stain after two flow sorts.
**FIG. 1F** illustrates a flow cytometry plot showing cells that bind to TfR after three flow sorts.
**FIG. 1G** illustrates a flow cytometry plot showing cells of a control stain after three flow sorts.
**FIG. 2** illustrates cells displaying TfR and a single clonal peptide. The control protein used in this experiment has an amino acid sequence set forth in SEQ ID NO: 333 
(MRLAVGALLVCAVLGLCLADYKDEHHHHHHGLNDIFEAQKIEWHEGGGSKTVRWCAVSEHEA
TKCQSFRDHMKSVIPSDGPSVACVKKASYLDCIRAIAANEADAVTLDAGLVYDAYLAPNNLKPV
VAEFYGSKEDPQTFYYAVAVVKKDSGFQMNQLRGKKSCHTGLGRSAGWNIPIGLLYCDLPEPRK
PLEKAVANFFSGSCAPCADGTDFPQLCQLCPGCGCSTLNQYFGYSGAFKCLKDGAGDVAFVKHS
TIFENLANKADRDQYELLCLDNTRKPVDEYKDCHLAQVPSHTVVARSMGGKEDLIWELLNQAQ
EHFGKDKSKEFQLFSSPHGKDLLFKDSAHGFLKVPPRMDAKMYLGYEYVTAIRNLREGTCPEAP
TDECKPVKWCALSHHERLKCDEWSVNSVGKIECVSAETTEDCIAKIMNGEADAMSLDGGFVYIA
GKCGLVPVLAENYDKSDNCEDTPEAGYFAVAVVKKSASDLTWDNLKGKKSCHTAVGRTAGWN
IPMGLLYNKINHCRFDEFFSEGCAPGSKKDSSLCKLCMGSGLNLCEPNNKEGYYGYTGAFRCLVE
KGDVAFVKHQTVPQNTGGKNPDPWAKNLNEKDYELLCLDGTRKPVEEYANCHLARAPNHAVV
TRKDKEACVHKILRQQQHLFGSDVTDCSGNFCLFRSETKDLLFRDDTVCLAKLHDRNTYEKYLG
EEYVKAVGNLRKCSTSSLLEACTFRRPGSSELYENKPRRPYIL).
**FIG. 2A** illustrates a flow cytometry plot of cells expressing a peptide of SEQ ID NO: 1 (x-axis, GFP) and a control protein (y-axis, stained with a fluorescent anti-His antibody).
**FIG. 2B** illustrates a flow cytometry plot of cells expressing a peptide of SEQ ID NO: 1 (x-axis, GFP) and a control protein (y-axis, stained with a fluorescent streptavidin).
**FIG. 2C** illustrates a flow cytometry plot of cells expressing a peptide of SEQ ID NO: 1 (x-axis, GFP) and TfR (y-axis, stained with a fluorescent anti-His antibody).
**FIG. 2D** illustrates a flow cytometry plot of cells expressing a peptide of SEQ ID NO: 1 (x-axis, GFP) and a TfR (y-axis, stained with a fluorescent streptavidin).
**FIG. 3** illustrates TfR-binding for peptide variants arising from permuting enriched variants from site-saturation mutagenesis (SSM). Each graph represents a round of SSM completed and the colored bars within the graphs indicate the number of mutations in the variant peptides in comparison to the respective peptide sequence with which the round of SSM was started. The data represent the last step of SSM, after which the next generation molecule is settled upon.
**FIG. 3A** illustrates the amount of hTfR bound for variants comprising sequences of SEQ ID NO: 3 - SEQ ID NO: 23, respectively, derived from a site-saturation mutagenesis (SSM) for affinity maturation of the peptide having a sequence of SEQ ID NO: 1.
**FIG. 3B** illustrates the amount of hTfR bound for peptide variants having sequences of SEQ ID NO: 24 - SEQ ID NO: 31, respectively derived from a site-saturation mutagenesis (SSM) for affinity maturation of the starting peptide having a sequence of SEQ ID NO: 2.
**FIG. 4** illustrates sodium dodecyl sulfate-polyacrylamide gel electrophoresis (SDS-PAGE) under reducing (DTT) or non-reducing (PBS) conditions and RP-HPLC plots of five soluble TfR-binding peptide variants having a sequence of SEQ ID NO: 1, SEQ ID NO: 2, SEQ ID NO: 4, SEQ ID NO: 30, and SEQ ID NO: 32, respectively. The data show that the TfR-binding peptides exhibit high solubility. Comparison of the gels under reducing conditions (10 mM DTT) and non-reducing (PBS) conditions demonstrates that the peptides form disulfide bonds.
**FIG. 4A** illustrates SDS-PAGE and an RP-HPLC chromatogram of SEQ ID NO: 1.
**FIG. 4B** illustrates SDS-PAGE and an RP-HPLC chromatogram of SEQ ID NO: 2.
**FIG. 4C** illustrates SDS-PAGE and an RP-HPLC chromatogram of SEQ ID NO: 4.
**FIG. 4D** illustrates SDS-PAGE and an RP-HPLC chromatogram of SEQ ID NO: 30.
**FIG. 4E** illustrates SDS-PAGE and an RP-HPLC chromatogram of SEQ ID NO: 32.
**FIG. 5** illustrates that later generation TfR-binding peptides have improved resistance to glutathione (GSH) reduction. **FIG. 5** illustrates overlays (one per peptide tested) of reversed-phase HPLC traces of peptides subjected to either no treatment (red), 10 µM GSH (blue), or 10 µM DTT (green).
**FIG. 5A** illustrates resistance of a TfR-binding peptide having a sequence of SEQ ID NO: 1 to glutathione (GSH) reduction.
**FIG. 5B** illustrates improved TfR-binding peptide resistance to glutathione (GSH) reduction of the peptide having a sequence of SEQ ID NO: 2.
**FIG. 5C** illustrates improved TfR-binding peptide resistance to glutathione (GSH) and dithiothreitol (DTT) reduction of the peptide having a sequence of SEQ ID NO: 32.
**FIG. 5D** illustrates improved TfR-binding peptide resistance to glutathione (GSH) reduction of the peptide having a sequence of SEQ ID NO: 30.
**FIG. 6** illustrates improved resistance of affinity matured TfR-binding peptides to pepsin improves as peptides mature. **FIG. 6** illustrates overlays (one per peptide tested) of reversed-phase HPLC traces of TfR-binding peptides subjected to either no treatment (red), trypsin (blue), or pepsin (green).
**FIG. 6A** illustrates treatment with trypsin (blue) and pepsin (green) of the peptide having a sequence of SEQ ID NO: 1.
**FIG. 6B** illustrates treatment with trypsin (blue) and pepsin (green) of the peptide having a sequence of SEQ ID NO: 2.
**FIG. 6C** illustrates treatment with trypsin (blue) and pepsin (green) of the peptide having a sequence of SEQ ID NO: 32.
**FIG. 6D** illustrates treatment with trypsin (blue) and pepsin (green) of the peptide having a sequence of SEQ ID NO: 30.
**FIG. 7** illustrates surface plasmon resonance (SPR) curves showing binding of SEQ ID NO: 1, SEQ ID NO: 2, SEQ ID NO: 4, and SEQ ID NO: 32 to hTfR.
**FIG. 8** illustrates a surface plasmon resonance (SPR) trace showing hTfR-binding for varying concentrations of the peptide having a sequence of SEQ ID NO: 2.
**FIG. 9** illustrates a surface plasmon resonance (SPR) trace showing hTfR-binding for varying concentrations of the peptide having a sequence of SEQ ID NO: 4.
**FIG. 10** illustrates binding and single cycle kinetics data of SEQ ID NO: 32 binding to captured biotinylated hTfR by surface plasmon resonance (SPR). 5 concentrations of a peptide having a sequence of SEQ ID NO: 32 (0.037 nM, 0.11 nM, 0.33 nM, 1 nM, 3 nM) were injected over 2 densities of captured biotinylated (Bt)-hTfR and analyzed globally.
**FIG. 11** illustrates binding and single cycle kinetics data of SEQ ID NO: 30 binding to captured biotinylated hTfR by SPR. 5 concentrations of a peptide having a sequence of SEQ ID NO: 30 (0.037 nM, 0.11 nM, 0.33 nM, 1 nM, 3 nM) were injected over 2 densities of captured Bt-hTfR and analyzed globally.
**FIG. 12** illustrates a bar graph quantitating flow cytometry data from a competitive binding assay in which peptides having a sequence of SEQ ID NO: 2 and SEQ ID NO: 32 were incubated with hTfR in the presence of 10 µM of iron-loaded human holo-transferrin or empty human apo-transferrin.
**FIG. 13** illustrates co-crystallization of hTfR and the hTfR-binding peptide having a sequence of SEQ ID NO: 32.

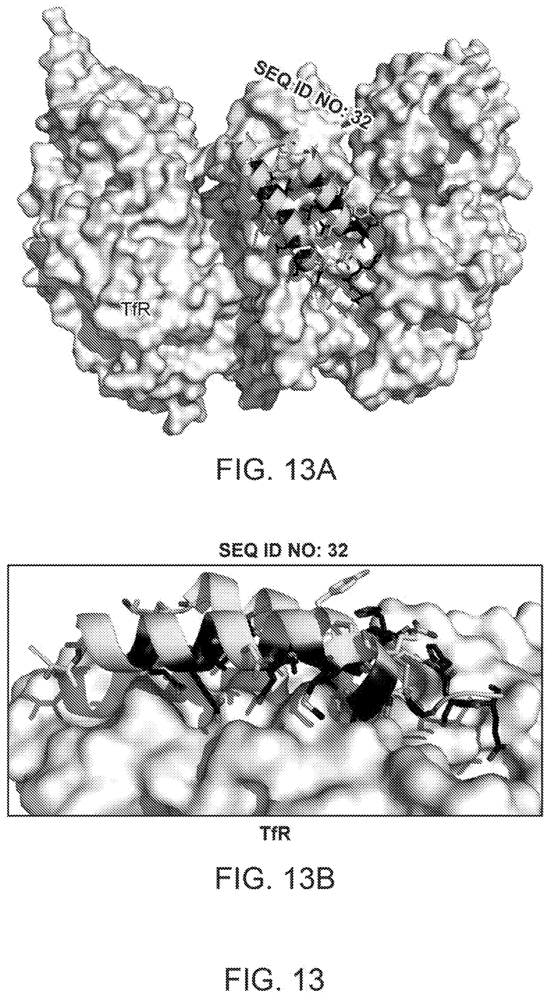

**FIG. 13A** illustrates a first view of hTfR co-crystallized with the peptide having a sequence of SEQ ID NO: 32. The structures show that the binding site of the hTfR-binding peptides overlaps with that of the endogenous binder transferrin. The linking sequence between the two helices, depicted as a solid, black coiled line, was not resolved in crystal structure and is based on structural modeling. The crystal structure of the transferrin receptor (TfR) is displayed as a space-filling electron density map. It appears as a large, globular protein with two prominent lobes. In between the two lobes the crystal structure of a short peptide, corresponding to SEQ ID NO: 32, is shown as interacting with the globular structure of the transferrin receptor. The peptide of SEQ ID NO: 32 is shown as a cartoon model with two anti-parallel α-helices oriented diagonally from upper left to lower right. Stick models corresponding to the amino acid side chains of SEQ ID NO: 32 are shown protruding from the α-helices, some of which are interacting with the globular protein of TfR.
**FIG. 13B** illustrates a zoomed in view of **FIG. 13A** and shows hTfR co-crystallized with a peptide having a sequence of SEQ ID NO: 32. Amino acid side chains either mutated from endogenous CDP or those that are part of the interface are shown as sticks. Interface-adjacent residues are shown in black, whereas interface-distal residues are shown in cyan. A portion of the TfR space-filling electron density map depicted in **FIG. 13A** that interacts with the peptide of SEQ ID NO: 32 is shown. The peptide of SEQ ID NO: 32, as shown in **FIG. 13A**, is displayed as a cartoon model with two anti-parallel α-helices and is positioned directly above the structure of TfR, with the α-helices positioned horizontally. The amino acid side chains of SEQ ID NO: 32 are shown as sticks. The side chains that protrude downward from the α-helices of SEQ ID NO: 32 toward the globular structure of TfR interact with TfR.

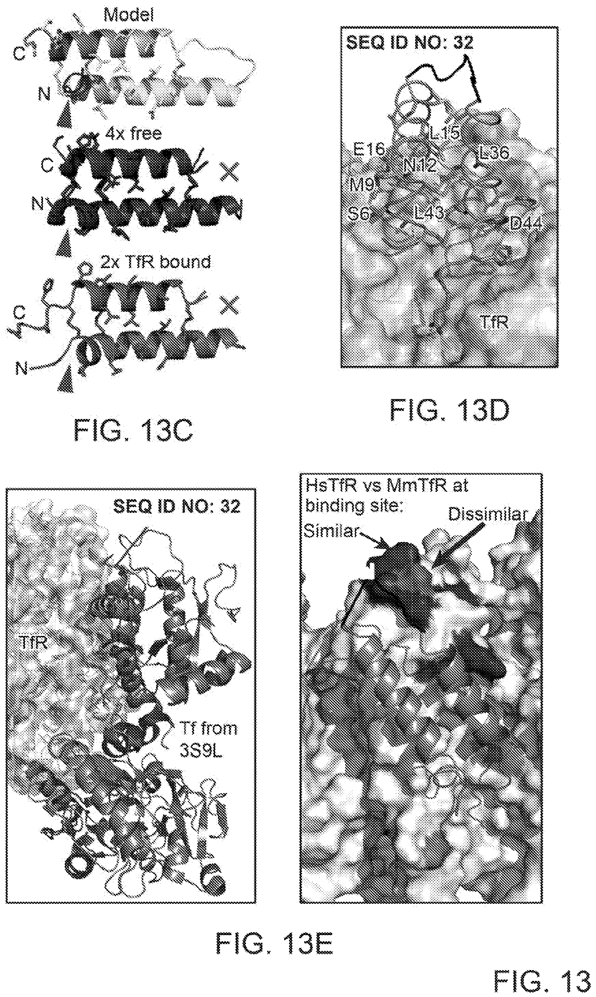

**FIG. 13C** illustrates the cartoon representations of a structural model of SEQ ID NO: 32 (top, N-terminus in blue, C-terminus in red, visible side chains colored by atom type), the crystal structure of free SEQ ID NO: 32 (middle, blue; 2.55 Å resolution, 4-fold superposition), and the crystal structure of SEQ ID NO: 32 when complexed with TfR (bottom, red; 1.85 Å resolution, 2-fold superposition). Side-chains with an atom within 4 Å of TfR in the complex structure are shown as sticks. N- and C-termini are labeled. Red arrows mark the most significant structural differences between the model, unbound, and bound structures of SEQ ID NO: 32 near the N-terminus. Gray crosses mark the location of the disordered inter-helical linker. The top structure, labeled "Model," shows the *in silico* model of the peptide corresponding to SEQ ID NO: 32 displayed as a cartoon model. The anti-parallel α-helices, oriented horizontally, are connected on the right side by a short loop. The N-terminus of the peptide is located at the left side of the bottom helix, and the C-terminus is located at the left side of the upper helix. Amino acid side chains are shown as sticks protruding from the α-helices. The middle structure, labeled "4x free," shows the crystal structure of the peptide corresponding to SEQ ID NO: 32 crystallized in the absence of TfR displayed as a cartoon model. The loop seen in the *in silico* model is not visible in this structure, as denoted by an X to the right of the two helixes. The N-terminus of the peptide is located at the left side of the bottom helix, and the C-terminus is located at the left side of the upper helix. Amino acid side chains are shown as sticks protruding from the α-helices. The bottom structure, labeled "2x TfR bound," shows the crystal structure of the peptide corresponding to SEQ ID NO: 32 crystallized in the presence of TfR displayed as a cartoon model. The loop seen in the *in silico* model is not visible in this structure, as denoted by an X to the right of the two helixes. The N-terminus of the peptide is located at the left side of the bottom helix, and the C-terminus is located at the left side of the upper helix. Amino acid side chains are shown as sticks protruding from the α-helices. Three triangular arrows pointing to the N-terminal region at the left end of the lower helix in each structure indicate the regions of the most significant structural differences between the three structures.
**FIG. 13D** illustrates a zoomed-in view of binding of SEQ ID NO: 32 bound to TfR, showing numerous polar and non-polar interactions driving binding. Non-polar interactions are primarily Leu and Met, while polar interactions are a mix of several charged and non-charged residues. A portion of the crystal structure of TfR is shown as a space-filling electron density map. The peptide corresponding to SEQ ID NO: 2 is shown as a ribbon diagram on top of the TfR structure. The α-helices are shown oriented vertically, and a loop connects the top ends of the two helices. Amino acid side chains are shown as sticks, and side chains facing the structure of TfR are interacting with TfR. Amino acid residues of SEQ ID NO: 32 are labeled as follows (from top to bottom, then from left to right): E16, M9, S6, N12, L43, L15, L36, and D44.
**FIG. 13E** illustrates the alignment of the TfR:SEQ ID NO: 32 co-crystal with that of the published TfR:transferrin complex (PDB 3S9L; TfR hidden) and is zoomed in on the binding interactions shown in **FIG. 14A****.** The structural alignment (left) demonstrates an overlap of the binding sites for SEQ ID NO: 32 (blue) and transferrin
   (MRLAVGALLVCAVLGLCLADYKDEHHHHHHGLNDIFEAQKIEWHEGGGSKTVRWCA VSEHEATKCQSFRDHMKSVIPSDGPSVACVKKASYLDCIRAIAANEADAVTLDAGLVYD AYLAPNNLKPVVAEFYGSKEDPQTFYYAVAVVKKDSGFQMNQLRGKKSCHTGLGRSA GWNIPIGLLYCDLPEPRKPLEKAVANFFSGSCAPCADGTDFPQLCQLCPGCGCSTLNQYF GYSGAFKCLKDGAGDVAFVKHSTIFENLANKADRDQYELLCLDNTRKPVDEYKDCHLA QVPSHTVVARSMGGKEDLIWELLNQAQEHFGKDKSKEFQLFSSPHGKDLLFKDSAHGFL KVPPRMDAKMYLGYEYVTAIRNLREGTCPEAPTDECKPVKWCALSHHERLKCDEWSV NSVGKIECVSAETTEDCIAKIMNGEADAMSLDGGFVYIAGKCGLVPVLAENYDKSDNCE DTPEAGYFAVAVVKKSASDLTWDNLKGKKSCHTAVGRTAGWNIPMGLLYNKINHCRF DEFFSEGCAPGSKKDSSLCKLCMGSGLNLCEPNNKEGYYGYTGAFRCLVEKGDVAFVK HQTVPQNTGGKNPDPWAKNLNEKDYELLCLDGTRKPVEEYANCHLARAPNHAVVTRK DKEACVHKILRQQQHLFGSDVTDCSGNFCLFRSETKDLLFRDDTVCLAKLHDRNTYEKY LGEEYVKAVGNLRKCSTSSLLEACTFRRP, SEQ ID NO: 353, green). The SEQ ID NO: 32 binding site, colored for human (SEQ ID NO: 353) and murine
   (MRLAVGALLVCAVLGLCLADYKDEHHHHHHGLNDIFEAQKIEWHEGGGSVPDKTVK WCAVSEHENTKCISFRDHMKTVLPPDGPRLACVKKTSYPDCIKAISASEADAMTLDGG WVYDAGLTPNNLKPVAAEFYGSVEHPQTYYYAVAVVKKGTDFQLNQLEGKKSCHTGL GRSAGWVIPIGLLFCKLSEPRSPLEKAVSSFFSGSCVPCADPVAFPKLCQLCPGCGCSSTQ PFFGYVGAFKCLKDGGGDVAFVKHTTIFEVLPEKADRDQYELLCLDNTRKPVDQYEDC YLARIPSHAVVARKNNGKEDLIWEILKVAQEHFGKGKSKDFQLFSSPLGKDLLFKDSAF GLLRVPPRMDYRLYLGHNYVTAIRNQQEGVCPEGSIDNSPVKWCALSHLERTKCDEWSI ISEGKIECESAETTEDCIEKIVNGEADAMTLDGGHAYIAGQCGLVPVMAEYYESSNCAIP SQQGIFPKGYYAVAVVKASDTSITWNNLKGKKSCHTGVDRTAGWNIPMGMLYNRINHC KFDEFFSQGCAPGYEKNSTLCDLCIGPLKCAPNNKEEYNGYTGAFRCLVEKGDVAFVKH QTVLDNTEGKNPAEWAKNLKQEDFELLCPDGTRKPVKDFASCHLAQAPNHVVVSRKE KAARVKAVLTSQETLFGGSDCTGNFCLFKSTTKDLLFRDDTKCFVKLPEGTTPEKYLGA EYMQSVGNMRKCSTSRLLEACTFHKH, SEQ ID NO: 354) transferrin receptor homology, shows that most of the binding surface on TfR shares identity or similarity between the two species (right). The left panel shows a portion of the structure of TfR as a space-filling electron density map. The ribbon diagram of transferrin from the 3S9L crystal structure (labeled "Tf from 3S9L) is positioned to the right of TfR and superimposed on the TfR structure. The Tf structure contains numerous α-helices which fit with the TfR structure. The structure also contains a ß-sheet structure in the lower right region, as well as numerous loops protruding from the ends of the α-helices. The structure of SEQ ID NO: 32 crystallized with TfR is shown as a cartoon model interacting with TfR on the upper right side. It consists of two α-helixes positioned vertically that overlap with the superimposed Tf structure. The right panel shows a rotated view of the TfR and SEQ ID NO: 32 structure shown in the left panel. TfR is shown as a space-filling electron density map, and SEQ ID NO: 32 appears as a cartoon model containing two α-helices oriented diagonally from upper left to lower right. Shaded regions on the TfR structure denote regions where the HsTfR and MmTfR binding sites are either similar or dissimilar. Exemplary similar and dissimilar regions are indicated by arrows. The remaining regions of the structure not denoted as similar or dissimilar, making up the majority of the surface of the TfR structure, has identical binding sites. A small region directly above the right helix of SEQ ID NO: 32 and a small region directly beneath the upper end of the right helix are denoted as having a similar binding site. A small region directly above the right helix of SEQ ID NO: 32, a small region directly beneath the lower end of the right helix, and a small region below the lower end of the left helix are denoted as having a dissimilar binding site.

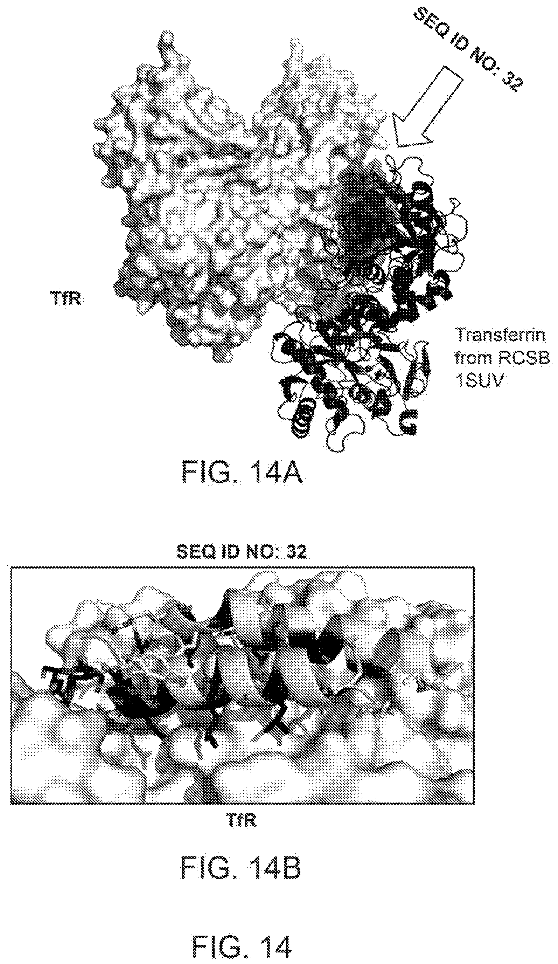

**FIG. 14** illustrates a TfR:transferrin complex and a TfR:peptide complex showing superimposition of each of transferrin (data taken from published literature (RCSB PDB 1SUV)) and the peptide having a sequence of SEQ ID NO: 32 bound to hTfR, illustrating that transferrin and the peptide having a sequence of SEQ ID NO: 32 overlap in their binding sites on hTfR.
**FIG. 14A** illustrates the superimposition of each of transferrin (data taken from published literature (RCSB PDB 1SUV)) and the peptide having a sequence of SEQ ID NO: 32 bound to hTfR. The structure of TfR appears as a space-filling electron density map of a large, globular protein having two lobes. The structure of transferrin from the 1SUV crystal structure (labeled "Transferrin from RCSB 1SUV") appears as a cartoon model with numerous α-helices and connecting loops. The transferrin structure is positioned to the lower right of TfR and fits with the TfR structure. An arrow labeled "SEQ ID NO: 32" points to a site to the right of TfR, directly above transferrin, and indicates the binding site of the peptide corresponding to SEQ ID NO: 32.
**FIG. 14B** illustrates a zoomed in view of the peptide having a sequence of SEQ ID NO: 32 bound to TfR, indicating that, when compared to the binding site of endogenous transferrin in **FIG. 14A**, both the peptide having a sequence of SEQ ID NO: 32 and transferrin have overlapping binding sites on hTfR. Amino acid side chains either mutated from endogenous CDP or that are part of the interface are shown as sticks. Interface-adjacent residues are shown in black, whereas interface-distal residues are shown in cyan. A portion of the TfR space-filling electron density map depicted in **FIG. 13A** that interacts with the peptide of SEQ ID NO: 32 is shown. The peptide of SEQ ID NO: 32, as shown in **FIG. 13A**, is displayed as a cartoon model with two anti-parallel α-helices and is positioned directly above the structure of TfR, with the α-helices positioned horizontally. The amino acid side chains of SEQ ID NO: 32 are shown as sticks. The side chains that protrude downward from the α-helices of SEQ ID NO: 32 toward the globular structure of TfR interact with TfR.

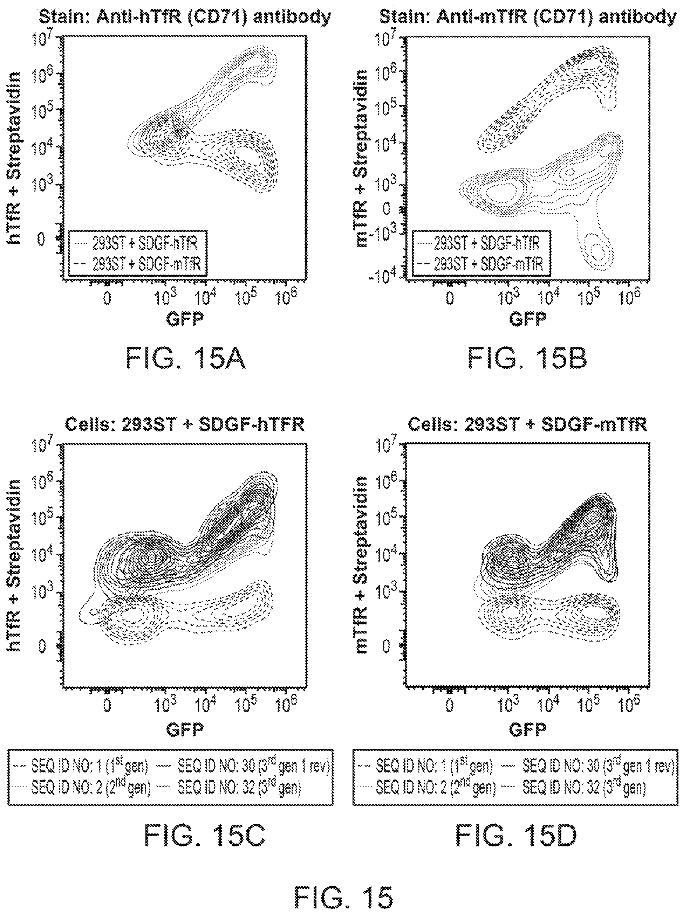

**FIG. 15** illustrates that TfR-binding peptides are cross-reactive with murine TfR (mTfR) in cell surface binding assays. 293F cells expressing either human or mouse TfR from their surface were stained with soluble TfR-binding peptides that were directly labeled with AlexaFluor 647 dye.
**FIG. 15A** illustrates the species specificity of the TfR used in these experiments, in this case human TfR. Data is displayed as two topographical density maps and indicates flow cytometry data of transferrin stained with Anti-hTfR (CD71) antibody. The upper density map, oriented diagonally from lower left to upper right, depicts 293ST+SDGF-hTfR. The lower density map, oriented horizontally, depicts 293ST+SDGF-mTfR. The y-axis shows hTfR + Streptavidin from 0 to 10⁷, in increments of 10 on a log scale. The x-axis shows GFP from 0 to 10⁶, in increments of 10 on a log scale.
**FIG. 15B** illustrates the species specificity of the TfR used in these experiments, in this case murine TfR. Data is displayed as two topographical density maps and indicates flow cytometry data of transferrin stained with Anti-mTfR (CD71) antibody. The upper density map, oriented diagonally from lower left to upper right, depicts 293ST+SDGF-mTfR. The lower density map, having three lobes, depicts 293ST+SDGF-hTfR. The y-axis shows hTfR + Streptavidin from 10⁻⁴ to 10⁷, in increments of 10 on a log scale. The x-axis shows GFP from 0 to 10⁶, in increments of 10 on a log scale.
**FIG. 15C** illustrates that the peptide having a sequence of SEQ ID NO: 1, the peptide having a sequence of SEQ ID NO: 2, the peptide having a sequence of SEQ ID NO: 30, and the peptide having a sequence of SEQ ID NO: 32 bind human TfR. Data is displayed as four topographical density maps and indicates flow cytometry data using 293ST cells + SDGF-hTFR. Three density maps appear nearly superimposed and are oriented above a fourth density map. The lower density map is oriented horizontally and depicts SEQ ID NO: 1 (1^{st} gen). The upper three density maps are oriented diagonally from lower left to upper right. The density map slightly above the other two corresponds to SEQ ID NO: 32 (3^{rd} gen). The density map slightly below the other two corresponds to SEQ ID NO: 2 (2^{nd} gen). The third density map corresponds to SEQ ID NO: 30 (3^{rd} gen). The y-axis shows hTfR + Streptavidin from 0 to 10⁷, in increments of 10 on a log scale. The x-axis shows GFP from 0 to 10⁶, in increments of 10 on a log scale.
**FIG. 15D** illustrates that the peptide having a sequence of SEQ ID NO: 1, the peptide having a sequence of SEQ ID NO: 2, the peptide having a sequence of SEQ ID NO: 30, and the peptide having a sequence of SEQ ID NO: 32 bind murine TfR. Data is displayed as four topographical density maps and indicates flow cytometry data using 293ST cells + SDGF-mTFR. Three density maps appear nearly superimposed and are oriented above a fourth density map. The lower density map is oriented horizontally and depicts SEQ ID NO: 1 (1^{st} gen). The upper three density maps are oriented diagonally from lower left to upper right. The density map slightly above the other two corresponds to SEQ ID NO: 32 (3^{rd} gen). The density map slightly below the other two corresponds to SEQ ID NO: 2 (2^{nd} gen). The third density map corresponds to SEQ ID NO: 30 (3^{rd} gen). The y-axis shows hTfR + Streptavidin from 0 to 10⁷, in increments of 10 on a log scale. The x-axis shows GFP from 0 to 10⁶, in increments of 10 on a log scale.

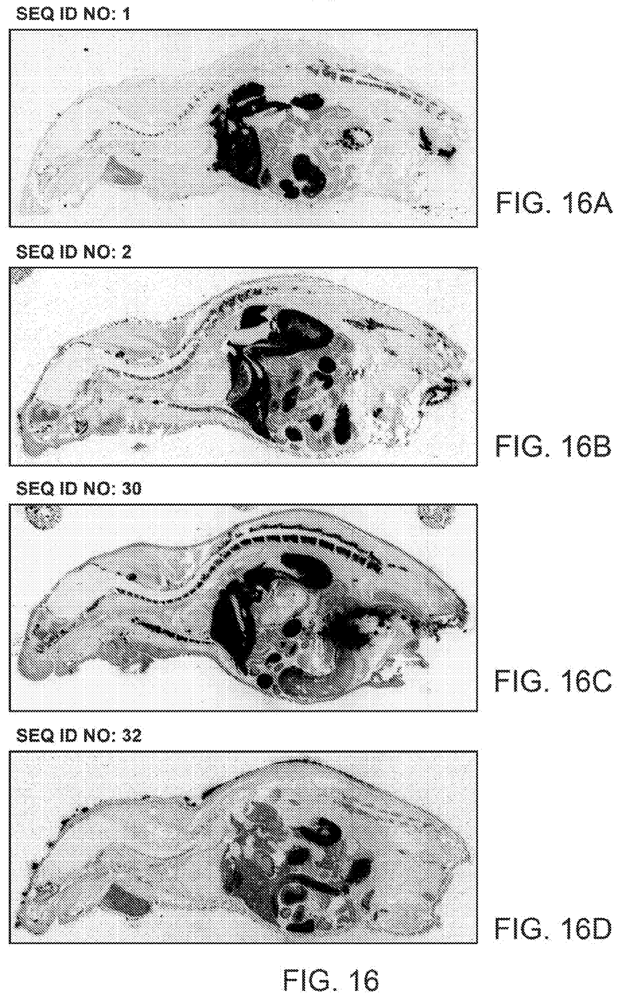

**FIG. 16** illustrates whole body autoradiography of mice injected intravenously with ¹⁴C-labeled TfR-binding peptides (SEQ ID NO: 1, SEQ ID NO: 2, SEQ ID NO: 30, and SEQ ID NO: 32) to assess biodistribution. Images were taken 3 hours after a dose of 100 nmol peptide (20 mg/kg, assuming approximately 25 g body weight) was administered to mice bearing xenograft tumors (subcutaneous human astrocytoma U87 cells). The images demonstrate substantial accumulation in spleen, liver, kidney, and also high accumulation in muscle, bone marrow, and skin. CNS accumulation is less than other tissues but still substantial (>25% of serum) compared to historical values of compounds and biologics that do not penetrate the blood-brain barrier (BBB) - background blood level found in brain is 3% that of cardiac blood signal.
**FIG. 16A** illustrates whole body autoradiography of mice injected intravenously with ¹⁴C-labeled SEQ ID NO: 1.
**FIG. 16B** illustrates whole body autoradiography of mice injected intravenously with ¹⁴C-labeled SEQ ID NO: 2.
**FIG. 16C** illustrates whole body autoradiography of mice injected intravenously with ¹⁴C-labeled SEQ ID NO: 30.
**FIG. 16D** illustrates whole body autoradiography of mice injected intravenously with ¹⁴C-labeled SEQ ID NO: 32.

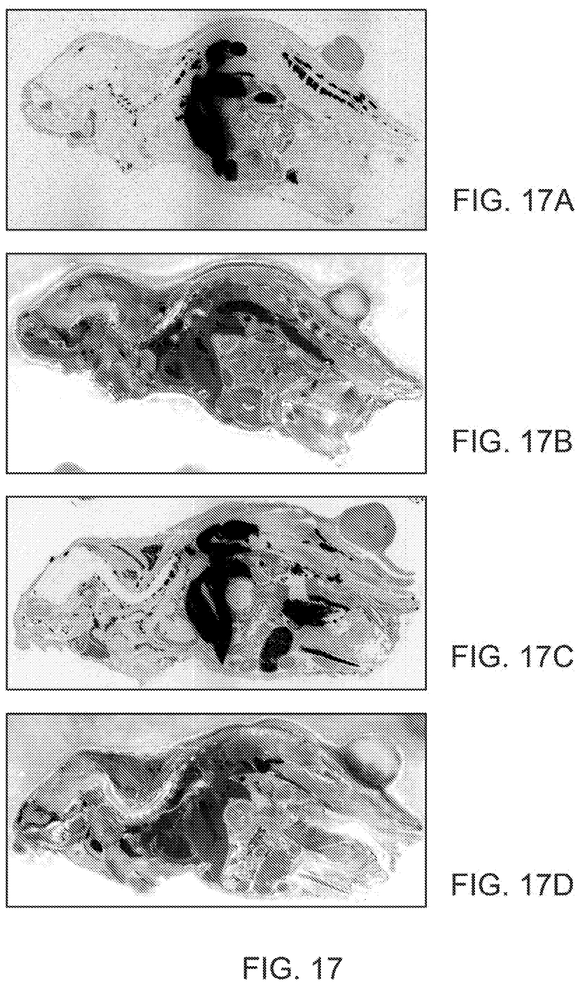

**FIG. 17** illustrates whole body autoradiography of mice injected intravenously with ¹⁴C-labeled peptides (SEQ ID NO: 1 and SEQ ID NO: 32) to assess biodistribution. Substantial accumulation in flank tumors was also seen. Accumulation in tumors is disperse throughout, rather than concentrated at sites rich in blood. This suggests extravasation and dispersal throughout the tumor parenchyma.
**FIG. 17A** illustrates whole body autoradiography of mice injected intravenously with ¹⁴C-labeled SEQ ID NO: 1.
**FIG. 17B** illustrates whole body autoradiography of mice injected intravenously with ¹⁴C-labeled SEQ ID NO: 1.
**FIG. 17C** illustrates whole body autoradiography of mice injected intravenously with ¹⁴C-labeled SEQ ID NO: 32.
**FIG. 17D** illustrates whole body autoradiography of mice injected intravenously with ¹⁴C-labeled SEQ ID NO: 32.

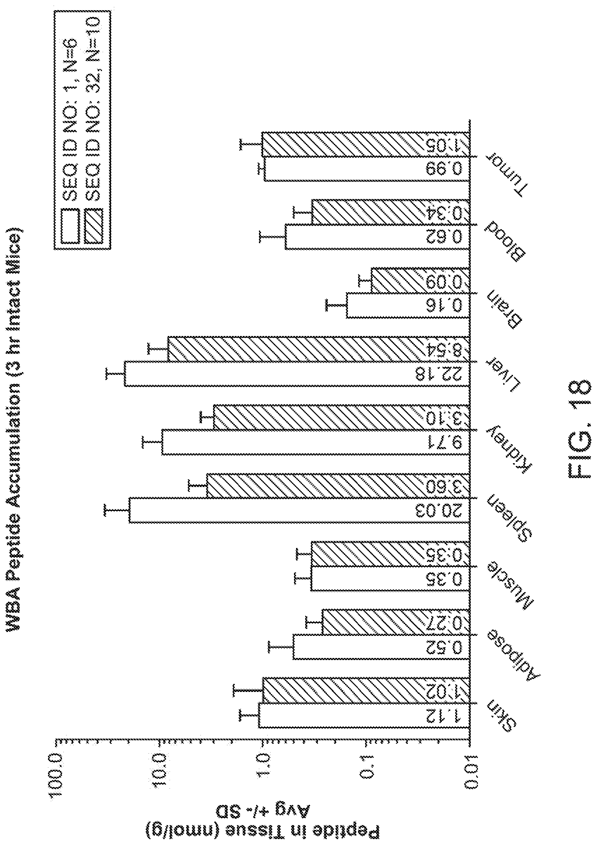

**FIG. 18** illustrates the quantitation of peptide biodistribution and organ accumulation after a single 20 mg/kg IV dose of either a TfR-binding peptide having a sequence of SEQ ID NO: 1 or a peptide having a sequence of SEQ ID NO: 32. This dose achieved levels of >150 nM in the CNS, ~1 µM in tumor after 3 hours. The bar graph quantifies the levels of each ¹⁴C-labeled peptides with known radioactivity in the same image and to peptide specific activity in various tissues from mice injected intravenously with peptides, 3 hours post-administration, as measured by whole body autoradiography.

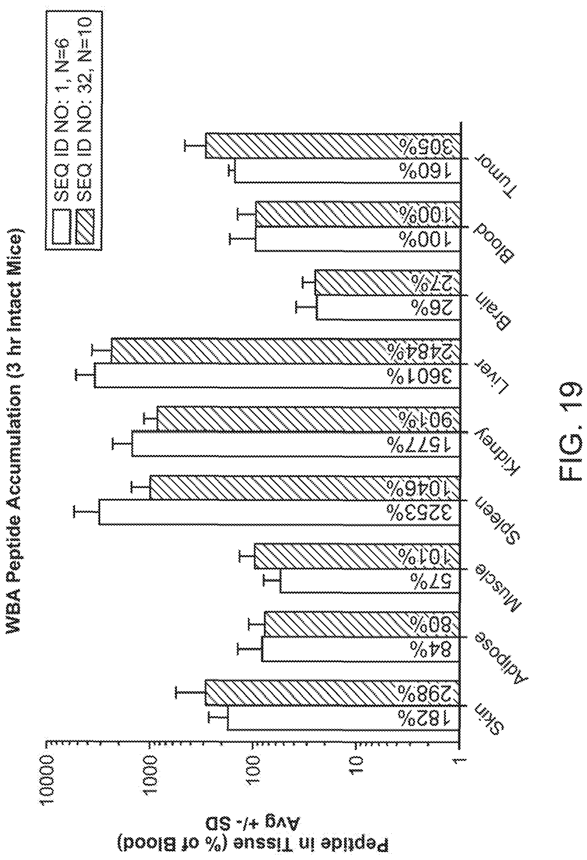

**FIG. 19** illustrates the quantitation of peptide biodistribution and organ accumulation for the peptides having a sequence of SEQ ID NO: 1 and SEQ ID NO: 32. The values are shown as levels in the tissue versus levels in the blood, where no brain penetration would give a level of 3% given that brain is 3% blood. The bar graph quantifies the levels of each ¹⁴C-labeled peptides normalized to controls with known radioactivity in the same image and to peptide specific activity in various tissues from mice injected intravenously with peptides, 3 hours post-administration, as measured by whole body autoradiography.

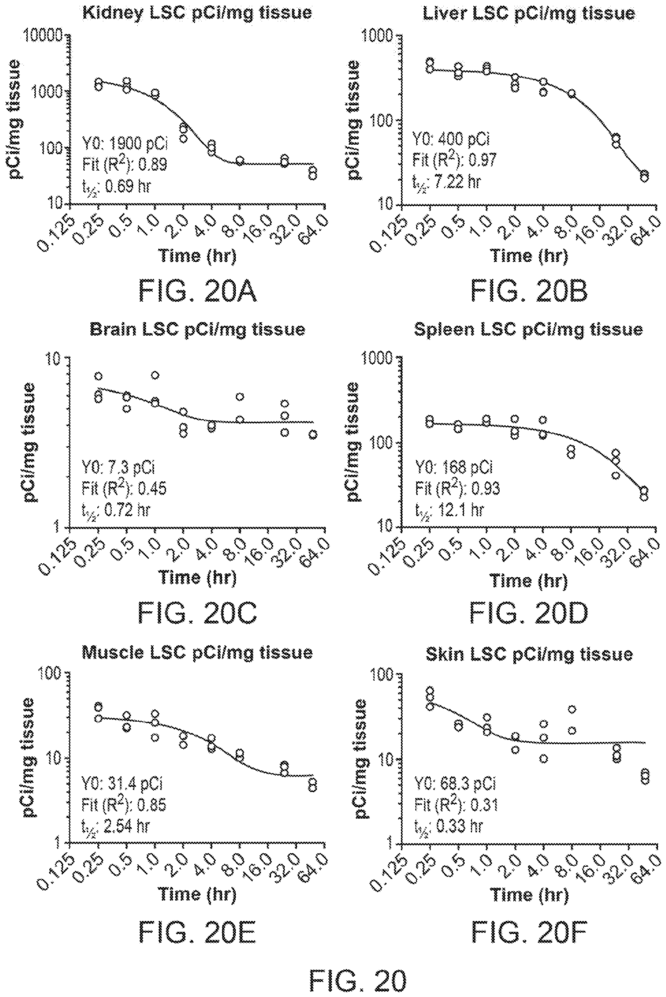

**FIG. 20** illustrates scintillation counting over time in various tissues after administration of 20 mg/Kg of TfR-binding peptide having a sequence of SEQ ID NO: 32 with a specific activity of 147 Ci/mol, validating whole body autoradiography (WBA) tissue accumulation.
**FIG. 20A** illustrates scintillation counting over time in the kidneys. The y-intercept at time 0 (Y0) is 1900 pCi, the half-life (t_{1/2}) is 0.89 hrs, and the R² of the fit is 0.89.
**FIG. 20B** illustrates scintillation counting over time in the liver. The y-intercept at time 0 (Y0) is 400 pCi, the half-life (t_{1/2}) is 7.22 hrs, and the R² of the fit is 0.97.
**FIG. 20C** illustrates scintillation counting over time in the brain. The y-intercept at time 0 (Y0) is 7.3 pCi, the half-life (t_{1/2}) is 0.72 hrs, and the R² of the fit is 0.45.
**FIG. 20D** illustrates scintillation counting over time in the spleen. The y-intercept at time 0 (Y0) is 168 pCi, the half-life (t_{1/2}) is 12.1 hrs, and the R² ofthe fit is 0.93.
**FIG. 20E** illustrates scintillation counting over time in the muscle. The y-intercept at time 0 (Y0) is 31.4 pCi, the half-life (t_{1/2}) is 2.54 hrs, and the R² ofthe fit is 0.85.
**FIG. 20F** illustrates scintillation counting over time in the skin. The y-intercept at time 0 (Y0) is 68.3 pCi, the half-life (t_{1/2}) is 0.33 hrs, and the R² of the fit is 0.31.

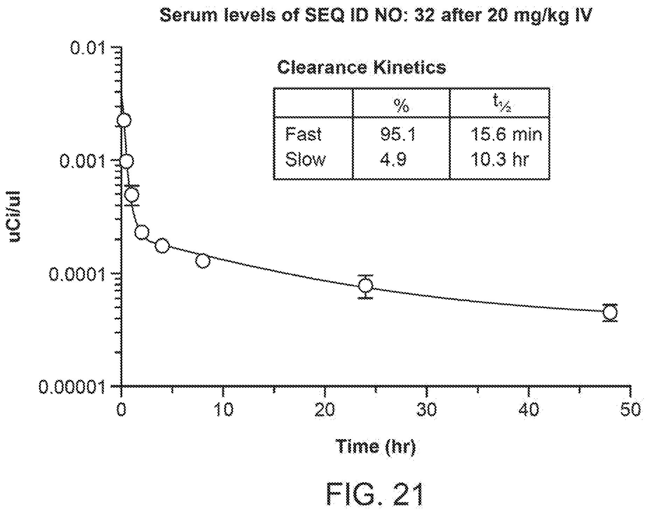

**FIG. 21** illustrates a serum elimination plot, including two-phase elimination regression analysis showing fast (95%, 15.6 min t_{½}) and slow (5%, 10.3 hr t_{½}) phase kinetics in mice intravenously administered 20 mg/kg of peptide having a sequence of SEQ ID NO: 32.

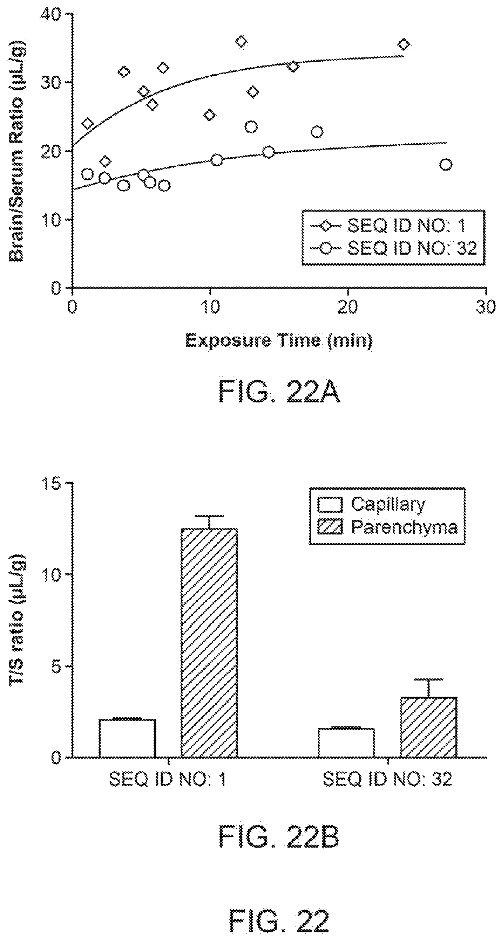

**FIG. 22** illustrates multiple time regression analysis and capillary depletion analysis of TfR-binding peptides having a sequence of SEQ ID NO: 1 and SEQ ID NO: 32.
**FIG. 22A** illustrates multiple regression analysis of SEQ ID NO: 1 and SEQ ID NO: 32 in a single plot, wherein the y-axis indicates the brain to serum ratio.
**FIG. 22B** illustrates parenchyma and capillary distribution of TfR-binding peptides having a sequence of SEQ ID NO: 1 and SEQ ID NO: 32, wherein the y-axis indicates the tissue-to-serum ratio (µL/g).

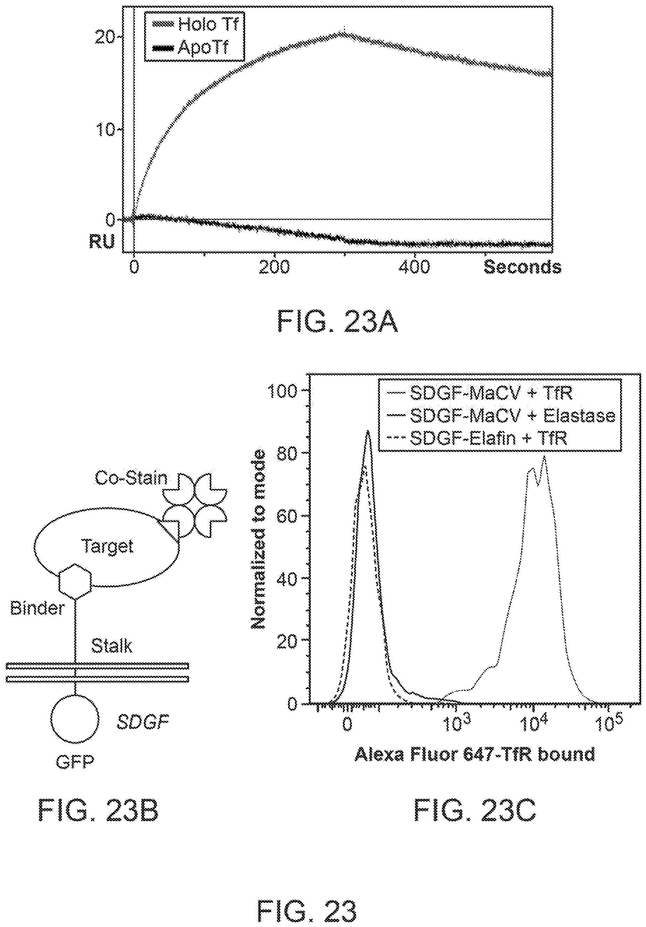

**FIG. 23** illustrates the purification and testing of soluble transferrin receptor (TfR) ectodomain.
**FIG. 23A** illustrates the surface plasmon resonance (SPR) of holo or apo transferrin (Tf) binding to the purified TfR. Holo Tf binds TfR, but apo Tf does not, as seen by the increase in response (RU) over time.
**FIG. 23B** illustrates a schematic of the vector display scaffold and target engagement used to screen for and optimize peptide binding properties. The surface display vector (SDGF) encoding a GFP-tagged construct of the binder (e.g., SEQ ID NO: 1, SEQ ID NO: 2, or SEQ ID NO: 32) is expressed on the cell surface. A target protein (e.g., TfR) labeled with a fluorescent dye ("Co-Stain") bind to the surface-expressed binder.
**FIG. 23C** illustrates flow cytometry to screen for specific TfR binding, as measured by the amount of Alexa Fluor 647-TfR (co-stain in **FIG. 23B****)** bound. Cells transfected with Machupo virus glycoprotein (SDGF-MaCV) are tested with a combination of biotinylated TfR and Alexa Fluor 647-labeled streptavidin (Strep-647), SDGF-MaCV cells and Alexa Fluor 647-labeled elastase, or SDGF-elafin cells and TfR + Strep-647. The elastase and elafin cells conjugates fail to bind cells.

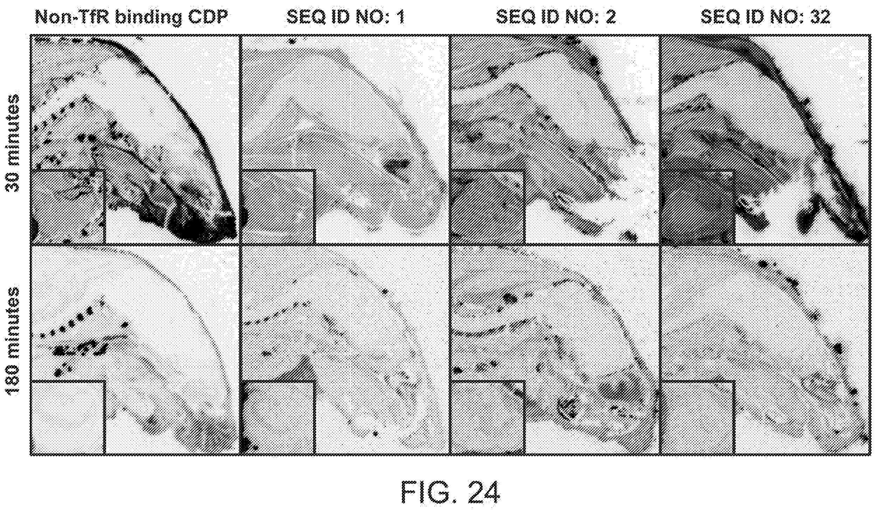

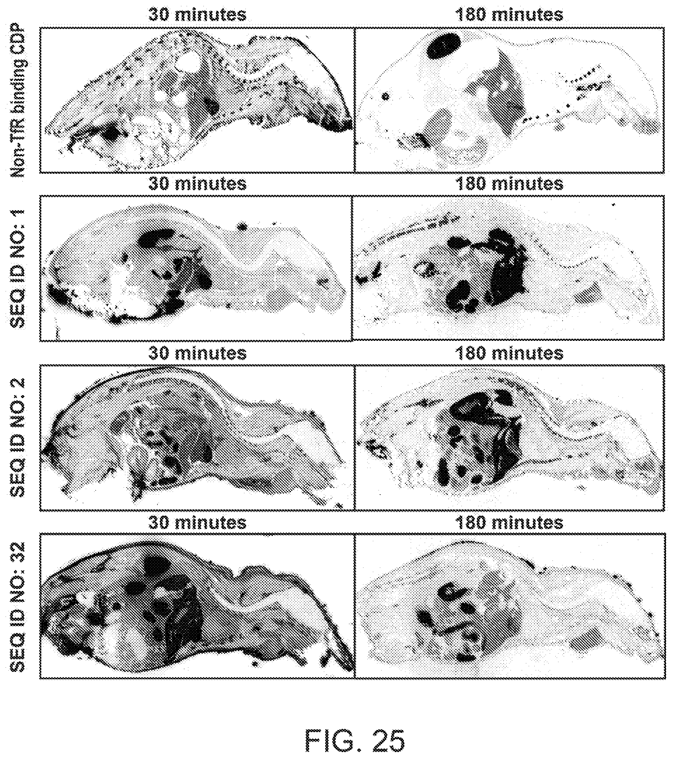

**FIG. 24** illustrates whole body autoradiography (WBA) of ¹⁴C peptide variant distribution. Histological sections of mice treated with ¹⁴C peptide variant are shown 30 minutes and 180 minutes post- administration. Representative brain and heart (inset) WBA images of three peptide generations, plus that of a control, non-TfR binding CDP are shown. Full body images of the same sections are shown in **FIG. 25** and some of the 180 min. sections are also show in **FIG. 16****.**
**FIG. 25** illustrates the full body images of the whole body radiography shown in **FIG. 24,** at 30 minutes (left column) and 180 minutes (right column) post-administration. Some of the sections from 180 min. are also shown in **FIG. 16****.**

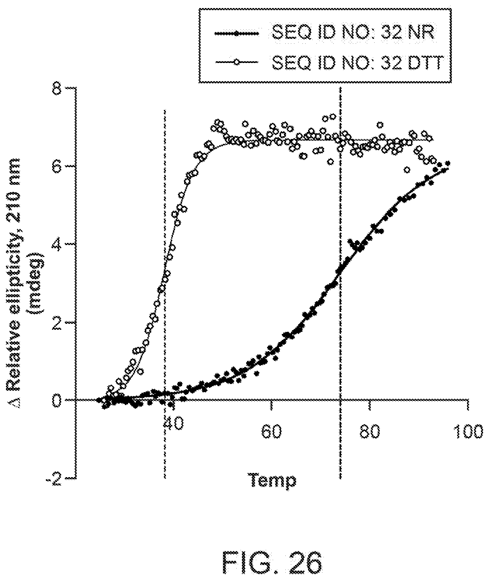

**FIG. 26** illustrates the heat stability of SEQ ID NO: 32 under reduced and non-reduced conditions as measured using circular dichroism. Relative ellipticity at 210 nm (mdeg) is plotted as a function of temperature in degrees C. NR indicates non-reduced. DTT indicates reducing the peptide with DTT. Vertical lines represent calculated melting points (~74°C for NR, ~38°C for 10 mM DTT treated) based on non-linear curve fitting (sigmoidal, variable slope, constrained bottom = 0; R² > 0.98 for both). Note that the NR sample failed to reach equilibrium up to 95°C, so it is likely that the protein is not fully "melted" at high temperature, but simply more disordered, particularly in the extended loop region.

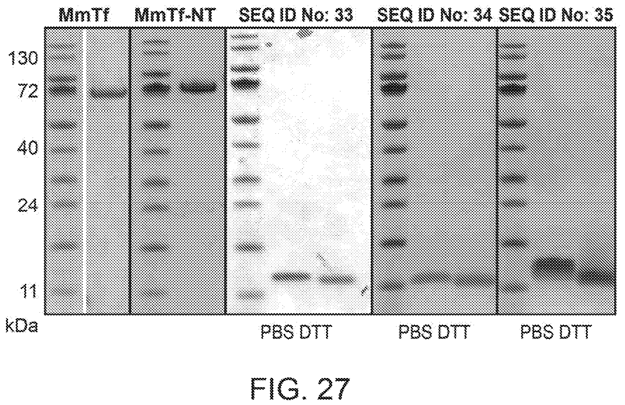

**FIG. 27** illustrates the purification and characterization of murine transferrin, murine transferrin-NT fusion, and various CDP-neurotensin (NT)-complexes of the present disclosure. SDS-PAGE under reducing (DTT) and non-reducing (PBS) conditions and gels stained with Coomassie dye show the purity of the peptides following purification. As shown, are murine endogenous transferrin (denoted MmTf, SEQ ID NO: 354), murine endogenous transferrin conjugated to neurotensin (denoted MmTf-NT, SEQ ID NO: 355), and CDP-NT peptide constructs (SEQ ID NO: 33 - SEQ ID NO: 35). Gels are run under reducing (DTT) or non-reducing (PBS) conditions. Reduction with 10 mM DTT suggested that the peptides form disulfide bonds.

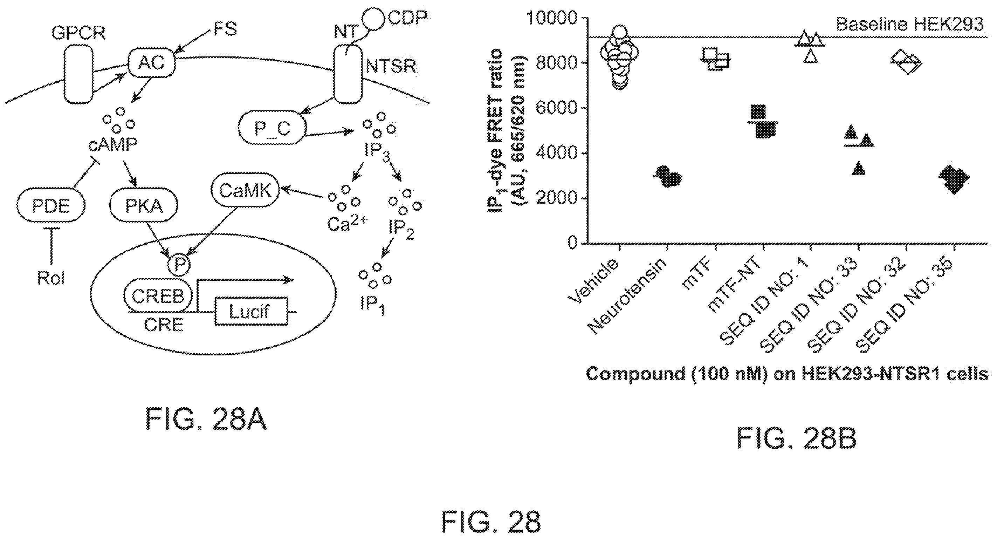

**FIG. 28** illustrates CDP-NT peptide constructs which induce an IP₁ response downstream of the neurotensin receptor (NTSR) both in CRE-Luciferase (CRE-Luc) mice and in mammalian cells.
**FIG. 28A** illustrates the relevant pathways influencing CRE-driven luciferase in the CRE-Luc mice. PLC denotes phospholipase C. AC denotes adenylyl cyclase. CaMK denotes calmodulin-dependent protein kinase. CREB denotes the cAMP response element binding protein. PKA denotes protein kinase A. PDE denotes cAMP phosphodiesterase. FS denotes forskolin. Rol denotes rolipram. GPCR denotes a G-protein-coupled receptor.
**FIG. 28B** illustrates *in vitro* neurotensin (NT) receptor engagement showing IP₁ accumulation only in response to NT or NT peptide constructs in HEK-293 cells expressing *NTSR1.* IP₁ is measured using an assay kit (CisBio 62IPAPEB) with a readout of FRET ratio. N = 3 wells for all except vehicle, which had N = 36. Horizontal bar indicates sample mean. mTF = murine transferrin. Baseline HEK293 = mean assay value for HEK293 cells (N=36 wells) that do not express *NTSR1,* included as a reference.

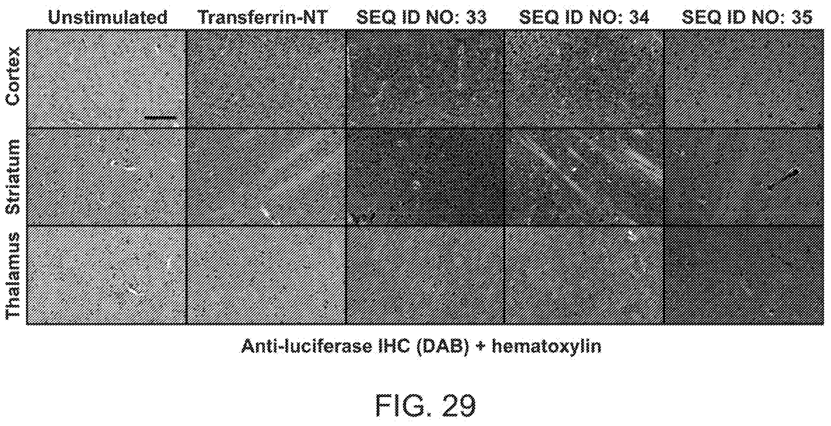

**FIG. 29** illustrates immunohistochemistry stained tissue samples from mice administered NT peptide constructs. Tissue staining shows enhanced luciferase expression in the cortex, striatum, and thalamus of mice 4 hours after CDP-NT administration of SEQ ID NO: 33, SEQ ID NO: 34, and SEQ ID NO: 35. Mice administered Transferrin-NT ("Transferrin-NT," SEQ ID NO: 355) or no peptide ("Unstimulated") are shown as controls. Scale bar (top left panel) is 100 µm; all panels are the same magnification.

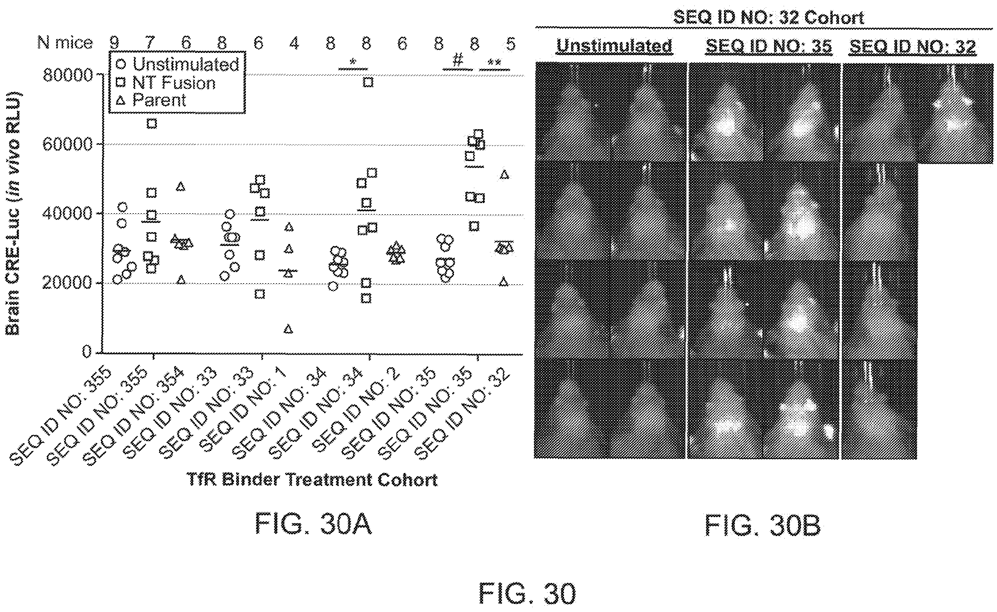

**FIG. 30** illustrates quantitation of the effects of CDP-NT peptide constructs in CRE-luciferase (CRE-Luc) mice.
**FIG. 30A** illustrates luminescence (via intraperitoneal luciferin dosage) either before (unstimulated) or four hours after (denoted "NT fusion" or "Parent") intravenous administration of parent TfR-binding peptides ("Parent") (e.g., SEQ ID NO: 1, SEQ ID NO: 2, or SEQ ID NO: 32, or SEQ ID NO: 354 as denoted) or cystine dense peptide (CDP)-NT constructs or murine transferrin-NT constructs (e.g., comprising SEQ ID NO: 33 - SEQ ID NO: 35 or SEQ ID NO: 355 as denoted) ("NT fusion"), using matched cohorts. Horizontal bar indicates sample mean. Significance was determined using a T-test (unpaired, 2-tailed). *: P < 0.05. **: P < 0.01. #: P < 0.0001. For both the parent (SEQ ID NO: 354) and NT transferrin peptide constructs (SEQ ID NO: 355), murine transferrin was used.
**FIG. 30B** illustrates images from the SEQ ID NO: 32 cohort quantitated in **FIG. 30A** with pseudocolored luminescence intensity. All images were equally background-subtracted and contrast-enhanced for ease of viewing only.

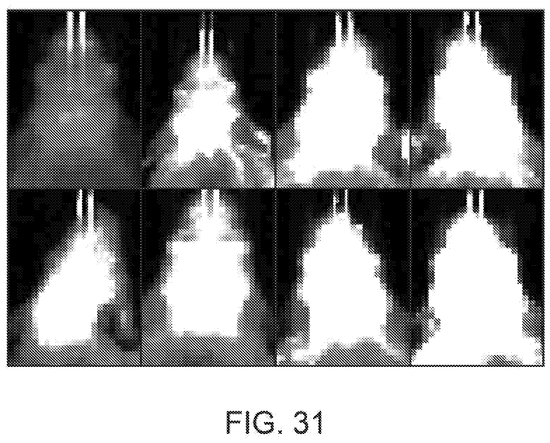

**FIG. 31** illustrates *in vivo* CRE-luciferase luminescence four hours after forskolin and rolipram induction. Images show luciferase fluorescence images of mice and serve as positive controls for the experiments depicted in **FIG. 30****.** Forskolin and rolipram activate CRE-dependent luciferase expression independent of the neurotensin receptor pathway. Forskolin and rolipram induce CRE expression via activation of adenylyl cyclase and inhibition of cAMP phosphodiesterase, respectively. N = 8 mice. Panels show replicate animals treated identically with rolipram and forskolin. One mouse was censored after the luciferin injection failed to hit the peritoneal cavity.

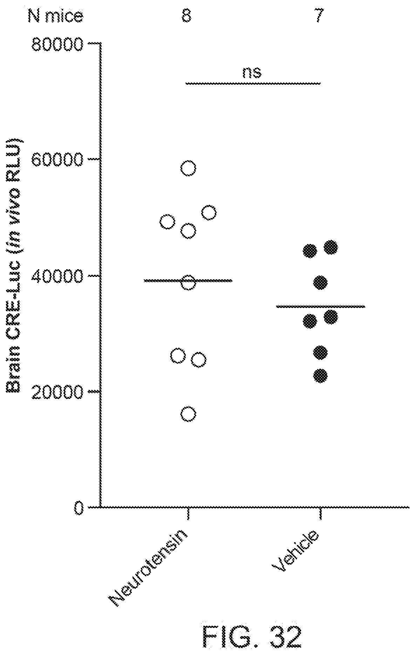

**FIG. 32** illustrates luciferase expression in CRE-Luc mice after intravenous administration of free neurotensin peptide. Intravenous free neurotensin fails to induce luciferase expression in CRE-Luc mice. *In vivo* luminescence of CRE-luciferase mice 4 hours after intravenous administration of high dose free neurotensin peptide (300 nmol) or vehicle (10% DMSO in PBS) was assessed with intraperitoneal luciferin injection and imaged in an IVIS imager. "ns" indicates that the results were not significant (P = 0.48), as calculated by T test (unpaired, 2 tailed).

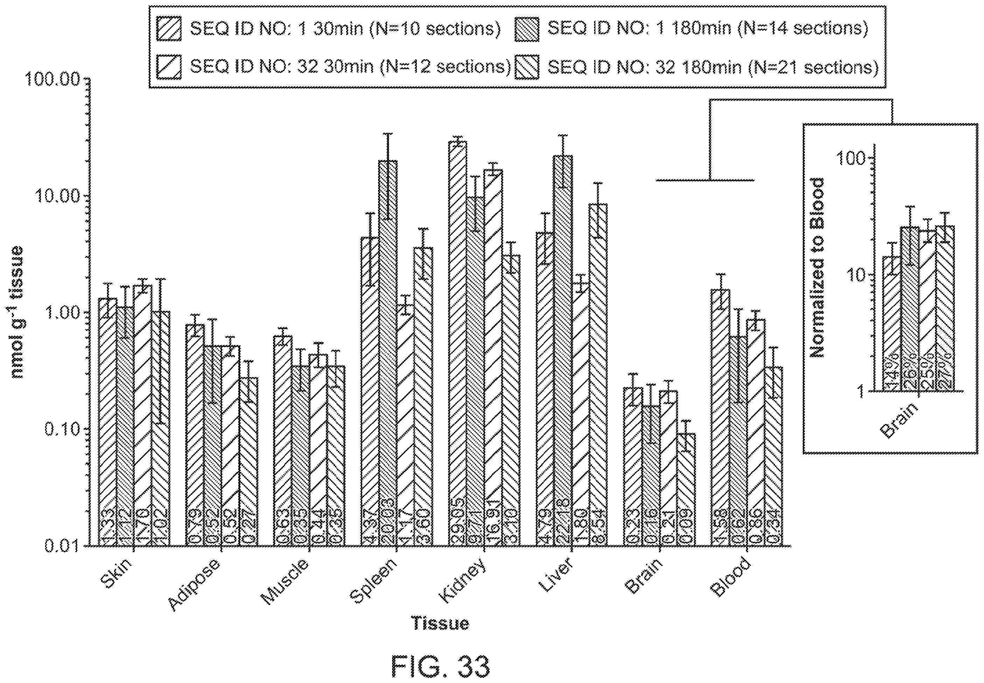

**FIG. 33** illustrates the quantitation of peptide biodistribution and organ accumulation after a single 20 mg/kg IV dose of either a TfR-binding peptide having a sequence of SEQ ID NO: 1 or a peptide having a sequence of SEQ ID NO: 32. Peptide levels are measured at 30 min and 3 hours (3 hour data is also shown in **FIG. 18****).** This dose achieved levels of >150 nM in the CNS, ~1 µM in tumor after 3 hours. The bar graph quantifies the levels of each ¹⁴C-labeled peptides with known radioactivity in the same image and to peptide specific activity in various tissues from mice injected intravenously with peptides, 3 hours post-administration, as measured by whole body autoradiography. Tissue data is plotted as seven clusters of four bars each. The 4-bar clusters correspond to different tissue types, from left to right, of Skin, Adipose, Muscle, Spleen, Kidney, Liver, Brain, and Blood, as shown on the x-axis. The four bars in each cluster correspond to, from left to right, SEQ ID NO: 1 30 min (N=10 sections), SEQ ID NO: 1 180 min (N=14 sections), SEQ ID NO: 32 30 min (N=12 sections), and SEQ ID NO: 32 180 min (N=21 sections). The y-axis shows nmol g⁻¹ tissue of peptide from 0.01 to 100 in increments of 10 on a log scale. The values of each bar, from left to right are, Skin: 1.33, 1.12, 1.70, 1.02, Adipose: 0.79, 0.52, 0.52, 0.27, Muscle: 0.63, 0.35, 0.44, 0.35, Spleen: 4.37, 20.03, 1.17, 3.60, Kidney: 29.05, 9.71, 16.91, 3.10, Liver: 4.79, 22.18, 1.80, 8.54, Brain: 0.23, 0.16, 0.21, 0.09, and Blood: 1.58, 0.62, 0.86, 0.34. The bars corresponding to Spleen, Kidney, and Liver are noticeably higher than the other sets. The inset shows the same data from the brain normalized to blood. The four bars correspond to, from left to right, SEQ ID NO: 1 30 min (N=10 sections), SEQ ID NO: 1 180 min (N=14 sections), SEQ ID NO: 32 30 min (N=12 sections), and SEQ ID NO: 32 180 min (N=21 sections). The y-axis shows peptide normalized to blood from 1 to 100 in increments of 10 on a log scale. The values for each bar, from left to right, are: 14%, 26%, 25%, and 27% of blood peptide levels.

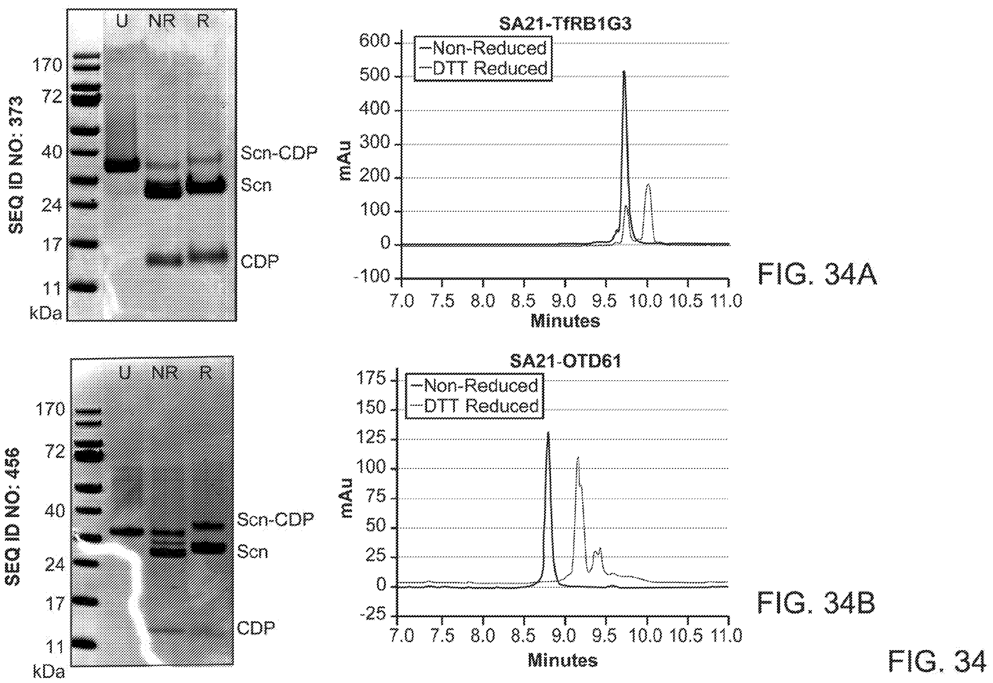

**FIG. 34** illustrates the purification of SA21 fusion peptides.
**FIG. 34A** shows purification of a TfR-binding peptide fused to a serum albumin peptide (SA21) corresponding to SEQ ID NO: 373. Purity was verified by SDS-PAGE (left) and RP-HPLC (right) under DTT reducing ("R") or non-reducing ("NR") conditions. SDS-PAGE was also run on the uncleaved ("U") siderocalin-CDP fusion peptide.
**FIG. 34B** shows purification of a peptide fused to SA21 corresponding to SEQ ID NO: 456
   (GSRLIEDICLPRWGCLWEDDGGGGSGGGGSVRIPVSCKHSGQCLKPCKDAGMRFGKC MNGKCDCTPK). Purity was verified by SDS-PAGE (left) and RP-HPLC (right) under DTT reducing ("R") or non-reducing ("NR") conditions. SDS-PAGE was also run on the uncleaved ("U") siderocalin-CDP fusion peptide.

### DETAILED DESCRIPTION

Drug delivery to targets located in the central nervous system (CNS) can be hindered by the blood-brain barrier (BBB), a term for the vascular endothelial cells in CNS capillaries. This system exists to prevent toxic metabolites and pathogens from entering the CNS (e.g., the brain), but also serves to render diseases of the CNS particularly difficult to treat using conventional medicines. It can be for this reason that primary CNS tumors (e.g., gliomas) and neuroinflammatory and neurodegenerative diseases such as Multiple Sclerosis and Alzheimer's disease respond particularly poorly to therapeutics that can otherwise be more effective in similar diseases affecting peripheral tissues where drug delivery to target cells can be less hindered. The myriad disorders of the CNS, from brain cancer to neurodegeneration to age-associated inflammatory processes, necessitate varied approaches to CNS drug delivery. While the BBB allows osmolytes and nutrients into the brain from serum, and CNS astrocytes provide many of the growth and survival signals required by neurons, several larger hormones and proteins such as transferrin (an iron chaperone), insulin, and leptin can cross the BBB. CNS transport of larger molecules such as transferrin can be accomplished by receptor-mediated transcytosis or "vesicular transcytosis" (e.g., transport of cargo from the apical to the basal side, or vice versa, in intracellular vesicles). For example, variants of the endogenous receptors of insulin, leptin, and transferrin - InsR, ObR, and TfR, respectively - that contain the normal ectodomain can be employed by CNS vascular endothelial cells in order to facilitate transport of these molecules into the CNS. In this highly selective way, certain large molecules like transferrin (molecular weight is approximately 75 kDa) can access the brain parenchyma.

In various embodiments, the present disclosure provides compositions as claimed that enable transport of cargo molecules or active agents (e.g., small molecules, peptides, or proteins) across cell layers or barriers, including endothelial (e.g., the BBB) or epithelial cell layers and methods of using these compositions. In some embodiments, the present disclosure provides compositions and methods that enable delivery of various molecules into the CNS that would otherwise not be able to pass the BBB. In some cases, the present disclosure provides compositions and methods for delivery of therapeutic and/or diagnostic molecules into the CNS, e.g., the brain. Thus, in various embodiments, the present disclosure provides peptides capable of crossing the BBB. These peptides can have an affinity and selectively for a transferrin receptor (TfR). The TfR-binding peptides can be cystine-dense peptides (CDPs). In some cases, the peptides of the present disclosure can cross the BBB via TfR-mediated transcytosis.

In some embodiments, the presently described peptides can be peptide conjugates, peptide constructs, fusion peptides, or fusion molecules such as linked by chemical conjugation of any molecule type, such as small molecules, peptides, or proteins, or by recombinant fusions of peptides or proteins, respectively (e.g., a peptide construct). The terms "fusion peptide" and "peptide fusion" are used interchangeably herein. In some embodiments, the peptide constructs can be produced biologically or synthetically. Thus, in some cases, a TfR-binding peptide can comprise a TfR-binding peptide domain linked to another molecule or group of molecules such as small molecules, peptides, or proteins or other macromolecules such as nanoparticles.

In some embodiments, the present disclosure provides methods and compositions that enable transport across cellular or molecular barriers. In various embodiments, the present disclosure provides methods and compositions that enable TfR-mediated transport across cellular layers (e.g., endothelial cells or epithelial cells) or cell membranes. In some cases, the TfR-binding peptides of the present disclosure enable transport across the blood brain barrier (BBB). In various aspects, the peptides of the present disclosure can be used to target any cell expressing TfR. In addition to the BBB, various other cells, tissues, and organs express TfR. Cells expressing TfR can include hepatocytes, erythrocytes and erythrocyte precursors in bone marrow, immune cells, stem cells, and rapidly dividing cells. Tissues and organs expressing TfR can include the brain (e.g., cerebral cortex, hippocampus, caudate, cerebellum), endocrine tissues (e.g., thyroid, parathyroid, and adrenal glands), bone marrow and immune system (e.g., appendix, lymph node, tonsil, spleen), muscle tissues (e.g., heart, skeletal, and smooth muscle), liver, gallbladder, pancreas, gastrointestinal tract (e.g., oral mucosa, esophagus, stomach, duodenum, small intestine, colon, rectum), kidney, urinary bladder, female tissues (e.g., fallopian tube, breast, vagina, cervix, endometrium, ovary, and placenta), adipose and soft tissue, and skin. Thus, the TfR-binding peptides of the present disclosure can be used to target these cells, tissues, and organs and deliver an active agent to these cells, tissues, and organs via, for example, TfR-mediated transcytosis (e.g., across cellular barrier such as the BBB) or TfR-mediated endocytosis (e.g., across cell membranes into cells).

In various embodiments, the present disclosure provides methods and compositions that enable TfR-mediated transport and delivery to cancer cells expressing TfR or CMYC-overexpressing cancers, as, in some cases, CMYC-overexpression can cause TfR-overexpression. Cancers overexpressing TfR can include ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer.

In some embodiments, the presently described peptides can be peptide constructs comprising one or more TfR-binding peptides as described herein conjugated to, linked to, or fused to one or more active agents. Peptide constructs as described herein can include chemical conjugates and recombinant fusion molecules. In some cases, a chemical conjugate can comprise a TfR-binding peptide as described herein that is chemically conjugated to or linked to another molecule. Molecules can include small molecules, peptides, polypeptides, proteins, or other macromolecules (e.g., nanoparticles) and polymers (e.g., nucleic acids, polylysine, or polyethylene glycol). In some cases, a TfR-binding peptide of the present disclosure is conjugated to another molecule via a linker. Linker moieties can include cleavable (e.g., pH sensitive or enzyme-labile linkers) or stable linkers. In some embodiments, a peptide construct is a fusion molecule (e.g., a fusion peptide or fusion protein) that can be recombinantly expressed, and wherein the fusion molecule can comprise one or more TfR-binding peptides fused to one or more other molecules peptides, polypeptides, proteins, or other macromolecules that can be recombinantly expressed.

In some cases, the TfR-binding peptides of the present disclosure are conjugated to, linked to, or fused to a therapeutic (also referred to as an "active agent") cargo molecule capable of exerting a certain biological effect or diagnostic (also referred to as a "detectable agent") cargo molecule, resulting in peptide constructs. In some cases, the therapeutic or diagnostic cargo molecules used herein can be transported across the BBB via TfR-mediated transcytosis. Once inside the CNS, the cargo molecules can target a specific cell, cell population, or tissue. In some cases, the therapeutic or diagnostic cargo molecules are known compounds that, when used in combination with the compositions and methods disclosed herein, are able to exert a significantly higher potency inside the CNS due to effective transport across the BBB.

The peptides of the present disclosure, or derivatives, fragments, or variants thereof, can have an affinity and selectively for TfR, or a derivative or analog thereof. In some cases, the peptides of the present disclosure can be engineered using site-saturation mutagenesis (SSM) to exhibit improved TfR-binding properties or promote transcytosis more effectively. In some cases, the peptides of the present disclosure are cystine-dense peptides (CDPs), related to knotted peptides or hitchin-derived peptides or knottin-derived peptides. The TfR-binding peptides can be cystine-dense peptides (CDPs). The terms "peptides", "CDPs", "TfR-binding peptides", and ''TfR-binding CDPs" are used interchangeably herein. Hitchins can be a subclass of CDPs wherein six cysteine residues form disulfide bonds according to the connectivity [1-4], 2-5, 3-6 indicating that the first cysteine residue forms a disulfide bond with the fourth residue, the second with the fifth, and the third cysteine residue with the sixth. The brackets in this nomenclature indicate cysteine residues form the knotting disulfide bond. (See e.g., Correnti et al. Screening, large-scale production, and structure-based classification for cystine-dense peptides. Nat Struct Mol Biol. 2018 Mar; 25(3): 270-278). Knottins can be a subclass of CDPs wherein six cysteine residues form disulfide bonds according to the connectivity 1-4, 2-5, [3-6]. Knottins are a class of peptides, usually ranging from about 20 to about 80 amino acids in length that are often folded into a compact structure. Knottins are typically assembled into a complex tertiary structure that is characterized by a number of intramolecular disulfide crosslinks and may contain beta strands and other secondary structures. The presence of the disulfide bonds gives knottins remarkable environmental stability, allowing them to withstand extremes of temperature and pH and to resist the proteolytic enzymes of the blood stream. In some cases, the peptides described herein can be derived from knotted peptides. The amino acid sequences of peptides as disclosed herein can comprise a plurality of cysteine residues. In some cases, at least cysteine residues of the plurality of cysteine residues present within the amino acid sequence of a peptide participate in the formation of disulfide bonds. In some cases, all cysteine residues of the plurality of cysteine residues present within the amino acid sequence of a peptide participate in the formation of disulfide bonds. As described herein, the term "knotted peptide" can be used interchangeably with the terms "cystine-dense peptide", "CDP", or "peptide".

Provide herein are methods of identification, maturation, characterization, and utilization of CDPs that bind the transferrin receptor and allow accumulation of bioactive molecules at therapeutically relevant concentrations in a subject (e.g., a human or non-human animal). This disclosure demonstrates the utility of CDPs as a diverse scaffold family that can be screened for applicability to modem drug discovery strategies. CDPs comprise alternatives to existing biologics, primarily antibodies, which may bypass some of the liabilities of the immunoglobulin scaffold, including poor tissue permeability, immunogenicity, and long serum half-life that can become problematic if toxicities arise. Peptides of the present disclosure in the 20-80 amino acid range represent medically relevant therapeutics that are mid-sized, with many of the favorable binding specificity and affinity characteristics of antibodies but with improved stability, reduced immunogenicity, and simpler manufacturing methods. The intramolecular disulfide architecture of CDPs provides particularly high stability metrics, reducing fragmentation and immunogenicity, while their smaller size could improve tissue penetration or cell penetration and facilitate tunable serum half-life. Disclosed herein are peptides representing candidate peptides that can serve as CNS drug delivery vehicles. A peptide of the present disclosure may be a cell-penetrating peptide (CPP). A CPP may be cell-penetrating, tissue-penetrating, or both.

In some embodiments, a CPP may be a CDP. CPPs may comprise peptides that facilitate cellular intake or uptake of various moieties and agents, and themselves may translocate across a cell membrane. CPPs may contain protein transduction domains, which are a class of short peptide sequences which can translocate across the cell membrane. A cell-penetrating peptide may directly or indirectly enter the cytosol of a cell, the nucleus of a cell, or other subcellular locations of a cell. A cell-penetrating peptide can be used as an appropriate carrier for various cargos including nucleic acid, peptides, proteins, siRNA, dsRNA, nucleic acids with alternative backbone chemistries (e.g., linked nucleic acids (LNAs) or peptide nucleic acids (PNAs)), radionuclides, imaging agents, fluorescent agents, therapeutic agents, nanoparticles, and the like. CPPs can be an artificial or engineered sequence (e.g., a synthetic sequence). CPPs may comprise multiple protein sequences, whether such protein sequences are native, synthetic, or a variant (e.g., chimeric). CPPs can be derived from a single protein sequence, whether such protein sequences are native synthetic or variant (e.g., a protein-derived sequence). CPPs can exhibit a variety of physiochemical properties such as cationic, amphipathic or hydrophobic. Some mechanisms for internalization of CPPs include direct cell penetration, use of the endocytosis pathway, and translocation through the formation of a transitory structure. It is understood that the description and use of a cell-penetrating peptide (CPP) herein is non-limiting. Non-limiting examples of CPPs can be found, for example, in Derakhshankhah and Jafari, "Cell penetrating peptides: A concise review with emphasis on biomedical applications" (Biomedicine & Pharmacotherapy; Volume 108, December 2018, Pages 1090-1096).

Some therapeutic molecules that can be used in combination with the herein disclosed methods and compositions can be able to target one or more specific cells or tissues that are part of the CNS. For example, the neurotensin receptor (NTSR), a G-protein coupled receptor, is targeted with a fusion protein comprising a CDP linked to neurotensin (abbreviated herein as "NT"; ELYENKPRRPYIL (SEQ ID NO: 350)), or a derivative thereof, a neuropeptide that activates the NTSR. In some cases, a CDP-NT peptide construct is used to prevent or treat chronic pain or neuropathic pain in a subject (e.g., a human).

Also described herein are peptides that selectively home, target, are directed to, migrate to, are able to reach, are retained by, or accumulate in and/or bind to specific regions, tissues, structures or cells of the central nervous system (CNS) that are involved in sensing, modulating, managing, decreasing, ablating or reducing pain, including nociceptive pain, or other therapeutic indications as described herein. A peptide that homes, targets, migrates to, is directed to, is retained by, or accumulates in and/or binds to one or more specific regions, tissues, structures or cells of the affected region can have fewer off-target and potentially negative effects, for example, side effects that often limit use and efficacy of pain drugs. In addition, such peptides can deliver active agents to regions, such as the CNS, where those active agents are otherwise unable to reach the CNS at therapeutic levels, such as due to the blood-brain barrier. Such peptides can also reduce need for other pain medication, including opioid medications. In addition, such peptides can reduce dosage and increase the efficacy of existing drugs by directly targeting them to a specific region, tissue, structure or cell of the affected region and helping to contact the affected region or increasing the local concentration of agent. The peptide itself can modulate pain or it can be conjugated to an agent that modulates pain. Such pain modulation may operate by various mechanisms such as modulating inflammation, autoimmune responses, direct or indirect action on pain receptors, cell killing, or programmed cell death (whether via an apoptotic and/or non-apoptotic pathway of diseased cells or tissues, and the like (Tait et al. J Cell Sci 127(Pt 10):2135-44 (2014)).

CDPs may be advantageous for delivery to the CNS, as compared to other molecules such as antibodies due to smaller size, greater tissue or cell penetration, and quicker clearance from serum, and as compared to smaller peptides due to resistance to proteases (both for stability and for immunogenicity reduction). In some embodiments, the TfR-binding peptides of the present disclosure (e.g., CDPs, knotted peptides, or hitchins), TfR-binding peptide conjugates (e.g., comprising one or more TfR-binding peptides and one or more active agents), or engineered TfR-binding fusion peptides (e.g., comprising one or more TfR-binding peptides and one or more peptides) may have properties that are superior to TfR-binding antibodies. For example, the peptides and constructs described herein can provide superior, deeper, and/or faster tissue or cell penetration to cells and targeted tissues (e.g., brain parenchyma penetration, solid tumor penetration) and faster clearance from non-targeted tissues and serum. The TfR-binding peptides, TfR-binding peptide conjugates, or TfR-binding fusion peptides of this disclosure may have lower molecular weights than TfR-binding antibodies. The lower molecular weight may confer advantageous properties on the TfR-binding peptides, TfR-binding peptide conjugates, or TfR-binding fusion peptides of this disclosure as compared to TfR-binding antibodies. For example, the TfR-binding peptides, TfR-binding peptide conjugates, or TfR-binding fusion peptides of this disclosure may penetrate a cell or tissue more readily than an anti-TfR antibody or may have lower molar dose toxicity than an anti-TfR antibody. The TfR-binding peptides, TfR-binding peptide conjugates, or TfR-binding fusion peptides of this disclosure may be advantageous for lacking the Fc function of an antibody. The TfR-binding peptides, TfR-binding peptide conjugates, or TfR-binding fusion peptides of this disclosure may be advantageous for allowing higher concentrations, on a molar basis, of formulations.

The TfR-binding peptides, TfR-binding peptide conjugates, or TfR-binding fusion peptides of this disclosure may have a wider therapeutic window (e.g., the dosage above which a therapeutic pharmacodynamic response is observed but below which toxicity is observed) as compared to TfR-binding antibody-based therapeutics. The TfR-binding peptides, TfR-binding peptide conjugates, or TfR-binding fusion peptides of this disclosure may be used at higher molar dosage with less risk of toxicity as compared to TfR-binding antibody-based therapeutics. The TfR-binding peptides, TfR-binding peptide conjugates, or TfR-binding fusion peptides of this disclosure may have fewer epitopes to trigger an adaptive immune response, resulting in reduced immunogenicity as compared to TfR-binding antibody-based therapeutics. The TfR-binding peptides, TfR-binding peptide conjugates, or TfR-binding fusion peptides of this disclosure may exhibit more facile and less disruptive incorporation of active agents into protein fusion constructs as compared to TfR-binding antibody-based therapeutics. The TfR-binding peptides, TfR-binding peptide conjugates, or TfR-binding fusion peptides of this disclosure may have a smaller surface area, resulting in lower risk for off-target binding, as compared to TfR-binding antibody-based therapeutics. An exemplary comparison is described in **EXAMPLE 54.**

In some embodiments, the TfR-binding peptides, TfR-binding peptide conjugates, or TfR-binding fusion peptides of this disclosure exhibit lower on-target toxicity than an anti-TfR antibody when administered to a subject at the same molar dose or at a similarly effective dose. In some embodiments, the TfR-binding peptides, TfR-binding peptide conjugates, or TfR-binding fusion peptides exhibit lower off-target toxicity than an antibody when administered to a subject at the same molar dose or a similarly effective dose. For example, the TfR-binding peptides, TfR-binding peptide conjugates, or TfR-binding fusion peptides of this disclosure may be administered to a subject at about 1-fold, 2-fold, 3-fold, 4-fold, 5-fold, 10-fold, 15-fold, 20-fold, 25-fold, 30-fold, 40-fold, 50-fold, 60-fold, 70-fold, 80-fold, 90-fold, 100-fold higher molar dose than an antibody while providing similar or lower observed toxicity. In some embodiments, the TfR-binding peptides, TfR-binding peptide conjugates, or TfR-binding fusion peptides of this disclosure exhibit higher efficacy than an anti-TfR antibody when administered to a subject at the same dose by weight as the anti-TfR antibody. The TfR-binding peptides of the present disclosure, when fused to a half-life extending moiety (e.g., Fc, SA21, PEG), can be delivered at even lower doses while preserving activity and efficacy and, thus, is far superior to administering an anti-TfR antibody.

The presently disclosed TfR-binding peptides when fused to an active agent in a peptide construct can exhibit higher efficacy than an engineered anti-TfR bispecific antibody against the same target. For example, the engineered TfR-binding peptides disclosed herein can be further fused to a BACE inhibitor. These engineered TfR-binding peptide-BACE inhibitor constructs can be administered at a higher molar dose than a bispecific anti-TfR/BACE1 antibody while still maintaining the same or higher efficacy. For example, the engineered TfR-binding peptide-BACE inhibitor constructs can cause less reticulocytopenia and effectively inhibitor amyloid beta formation, with low or no toxicity to the subject to which the engineered TfR-binding peptide-BACE inhibitor constructs is administered. In contrast, the bispecific anti-TfR/BACE1 antibody may cause more reticulocytopenia and/or have lower efficacy in preventing amyloid beta formation and can have higher toxicity to subjects, resulting in lethargy, distress, and signs of hemolysis.

In some embodiments, the present disclosure provides peptides (e.g., CDPs, knotted peptides, or hitchins), chemical conjugates (e.g., comprising one or more TfR-binding peptides and one or more active agents), or recombinantly expressed fusion molecules (e.g., comprising one or more TfR-binding peptides and one or more active agents) that bind to TfR. The TfR-binding peptides can be cystine-dense peptides (CDPs). The terms "peptides", "CDPs", "TfR-binding peptides," "TfR-binding CDPs," "TfR-binding peptides," and "engineered TfR-binding peptides" are used interchangeably herein. The binding of peptides described in the present disclosure to TfR can facilitate transcytosis of the peptide or peptide construct (e.g., fusion protein, or peptide conjugated to, linked to, or fused to an agent) across a cell barrier (e.g., the BBB). Also disclosed herein is the use of a mammalian surface display screening platform to screen a diverse library of CDPs and identify CDPs that specifically bind to human TfR. Further affinity maturation can be subsequently implemented to produce an allelic series of TfR-binding CDPs with varying affinities. In some embodiments, TfR-binding CDPs are identified and binding can be determined by crystallography. Peptides of the present disclosure can have cross-reactivity across species. For example, the peptides disclosed herein, in some cases, bind to human and murine TfR. Peptides disclosed herein can accumulate in the CNS and can penetrated the BBB via engagement of the TfR, following intravenous administration, including induction of a cAMP response element signaling cascade using neurotensin peptide constructs. Disclosed herein are TfR-binding CDPs for use as therapeutic delivery agents in oncology, autoimmune disease, acute and chronic neurodegeneration, and pain management. Delivery of active or pharmaceutical agents via TfR-binding CDP can be advantageous over conventional anti-TfR antibodies due to simpler manufacturing (peptides can be made via biologic or synthetic means), improved stability, and smaller size (less potential for steric hindrance of cargo activity). Thus, the methods and compositions of the present disclosure can provide a solution to the problem of effectively transporting cargo molecules (e.g., therapeutic and/or diagnostic small molecules, peptides or proteins) into the CNS (e.g., the brain). For example, the peptides of the present disclosure aid in drug delivery to tumors located in the brain.

In some embodiments of the present disclosure, a diverse library of CDPs, knotted peptides, hitchins, or peptides derived from knotted peptides or hitchins can be used in combination with a mammalian surface display screening platform is used to identify peptides that specifically bind to human TfR. (See e.g., Crook et al. (2017) Mammalian display screening of diverse cystine-dense peptides for difficult to drug targets. Nat Commun 8:2244). In some embodiments, a diverse library of CDPs, knotted peptides, hitchins, or peptides derived from knotted peptides or hitchins is mutagenized from endogenous peptide sequences to provide novel peptide sequences. Once TfR-binding peptides have been identified, affinity maturation (e.g., site-saturation mutagenesis) can be performed to produce an allelic series of binders with varying (e.g., improved) affinities for TfR. These techniques can be used in combination with various other analytical methods (e.g., crystallography or spectroscopy) in order to determine the nature of peptide-receptor interaction (e.g., critical amino acid residues for receptor binding etc.). In some cases, the peptides of the present disclosure are developed to bind human TfR; however, those peptides can show cross-reactivity with TfR derived from other species such as murine TfR.

The peptides of the present disclosure can have varying biodistribution patters *in vivo.* In some cases, mouse biodistribution and pharmacokinetic studies are performed using, for example, radiolabeled peptides (e.g., ¹⁴C-labeled peptides), in order to determine organ distribution, the uptake and residence times of the peptides in target organs (e.g., brain), and their mode of clearance (e.g., renal or hepatic).

In various embodiments, the present disclosure provides peptides that accumulate in the CNS (e.g., the brain). In some cases, CNS accumulation of those peptides is due to penetration across the BBB. In some cases, engineered TfR-binding peptide variants as described herein cross the BBB by TfR-mediated transcytosis. In some cases, the TfR-binding peptides of the present disclosure accumulate in tumor tissue that is located in the CNS. In some cases, the tumor that the peptide accumulates in is a brain tumor (e.g., glioma). As described herein, the terms "accumulates" and "accumulates in" can be used interchangeably and be used to refer to "accumulation in or accumulation on a cell and/or a tissue and can be understood as a gradual increase of concentration of the respective molecule (e.g., a TfR-binding peptide or peptide construct) over time.

In some embodiments, the engineered peptides of the present disclosure (e.g., histidine-containing or histidine-enriched TfR-binding peptides) can have a high TfR binding affinity at physiological pH but a significantly reduced binding affinity at lower pH levels such as endosomal pH of 5.4. In some cases, the TfR-binding peptides of the present disclosure can be optimized for improved intra-vesicular (e.g., intra-endosomal) and/or intracellular delivery function while retaining high TfR binding capabilities. In some cases, histidine scans and comparative binding experiments can be performed to develop and screen for such peptides. In addition, some peptides can comprise (e.g., conjugated to, linked to, or fused to) a motif that facilitates low-pH endosomal escape of the peptide or peptide construct for enhanced delivery functions (e.g., intracellular delivery of a therapeutic agent). In some embodiments, an amino acid residue in a peptide of the present disclosure is substituted with a different amino acid residue to alter a pH-dependent binding affinity to TfR. The amino acid substitution may increase a binding affinity at low pH, increase a binding affinity at high pH, decrease a binding affinity at low pH, decrease a binding affinity at high pH, or a combination thereof.

In various embodiments, the peptides of the present disclosure are part of a peptide construct, such as a peptide construct or a fusion peptide
designed to transport therapeutic and/or diagnostic molecules (e.g., small molecules, peptides, or proteins) across cell layers or cell barriers such as the BBB. Exemplary peptide constructs are shown in **TABLE 2, TABLE 5,** and **TABLE 6.** A peptide construct of the present disclosure can comprise a TfR targeting peptide linked (e.g., chemically conjugated or fused) to a therapeutic and/or diagnostic molecule or compound. The TfR-binding peptide construct can cross the BBB via TfR-mediated transcytosis and hence deliver the therapeutic and/or diagnostic molecule or compound into the CNS. The methods and compositions of the present disclosure therefore can have a profound impact on the diagnosis, prevention, and treatment of various diseases or conditions. Disease areas for which the methods and compositions of the present disclosure can be used include, but are not limited to, oncology, autoimmune diseases, acute and chronic neurodegeneration, muscle disorders, neuropsychological disorders, epilepsy, schizophrenia, depression, anxiety, bipolar disorder, developmental brain disorders (e.g., autism spectrum), pain, epilepsy, mood disorder, and pain management.

Hence, a subject having a disorder of the CNS is otherwise unable to benefit from a drug or drug class whose limited by poor BBB penetration, can benefit from the compositions and methods described herein because therapeutically effective concentrations of the drug in the CNS can be achieved by conjugation to a TfR-binding peptide provided herein. In addition to pharmacological advances, the compositions and methods disclosed herein comprising TfR-binding peptides can be superior over conventional CNS- and TfR-targeting agents (e.g., anti-TfR antibodies) in terms of their production, quality control, and safety. For example, the peptide and peptide constructs of the present disclosure are manufactured in a simpler, less expensive, and more resource-effective manner (e.g., peptides can be synthesized via biologic or synthetic approaches); the peptide and peptide constructs of the present disclosure can show improved ex vivo and in vivo stability, are smaller in size (e.g., less potential for steric hindrance of cargo activity and the ability to penetrate dense tissue such as solid tumors as well as the potential for faster clearance from systemic circulation), exhibit a higher tissue or cell penetration, and have lower immunogenicity. Peptides of this disclosure can be engineered to have lower immunogenicity by combining crystallographic data with major histocompatibility complex (MHC) or human leukocyte antigen (HLA) peptide fragment binding experiments or computational predictions thereof, or a combination of the foregoing.

The identification of therapeutic or diagnostic agents that have the ability to cross cellular layers or barriers such as the BBB have been challenging, as such methods involve demand for high specificity or targeting, high affinity binders for receptors that can promote transcytosis (e.g., TfR), and the ability to target cells and act on target proteins (low off-target adverse effects) after transcytosis. With few exceptions, high throughput screening campaigns with small molecule libraries failed to provide specific compounds capable of crossing endothelial or epithelial layers and/or transporting cargo across those layers.

Described herein are, in some embodiments, peptides and peptide constructs and methods of screening for peptides and peptide constructs that target a protein of interest, such as TfR. Compared to wildtype or endogenous molecules such as transferrin, the methods and compositions as described herein can provide peptides with improved TfR-binding capabilities, or peptides that exhibit improved transport capabilities across the BBB, or any combination thereof. In some cases, the presently described peptides efficiently transport cargo molecules (e.g., therapeutic or diagnostic small molecules or proteins) across endothelial cell layers (e.g., the BBB) or epithelial layers. In some embodiments, the TfR-binding peptides of the present disclosure bind to a TfR and promote vesicular transcytosis. In some cases, the TfR-binding peptides of the present disclosure bind to a cell that overexpress a TfR (e.g., a cancer cell) and promotes uptake of the peptide by the cell. In some aspects, a TfR binding peptide or peptide constructs as described herein promotes vesicular transcytosis and uptake by a TfR-overexpressing cell such as a cancer, or a combination thereof.

The TfR-binding peptides of the present disclosure can bind TfR of different species including human, monkey, mouse, and rat TfR. In some cases, variations or mutations in any of the amino acid residues of a TfR-binding peptide may influence cross-reactivity. In some cases, variations or mutations in any of the amino acid residues of a TfR-binding peptide that interact with the bindings site of TfR may influence cross-reactivity.

Described herein are peptides, including, but not limited to, designed or engineered peptides, recombinant peptides, and cystine-dense peptides (CDPs)/small disulfide-knotted peptides (e.g., knotted peptides, hitchins, and peptides derived therefrom), that can be large enough to carry a cargo molecule while retaining the ability to bind a target protein with high affinity (e.g., TfR), but yet small enough to access cellular compartments, such as the cytosol or the nucleus, or tissues, such as the center of cell agglomerates (e.g., solid tumors). In some cases, the peptides as described herein carry cargo molecules across the BBB into the CNS (e.g., the parenchyma) via vascular transcytosis. In some cases, the transcytosis is TfR-mediated.

Further described herein are methods and compositions for determining the nature of peptide-receptor interactions (e.g., using X-ray crystallography) as well as their pharmacodynamic and pharmacokinetic properties in vivo, including accumulation in the CNS (e.g., brain). Some of the peptides described herein have the ability to target and accumulate in tumor cells. In some cases, the tumor cells overexpress TfR. In some aspects, the peptides of the present disclosure have high in vivo stabilities, e.g., high protease stability, high tolerability of reducing agents such as glutathione (GSH), and tolerate elevated temperatures (e.g., up to 95 °C).

The present disclosure provides, in some embodiments, a peptide or protein design approach based on the 3D protein or receptor structure for identifying peptides or proteins capable of binding such receptor. In some cases, the receptor is a transferrin receptor.

Additional aspects and advantages of the present disclosure will become apparent to those skilled in this art from the following detailed description, wherein illustrative embodiments of the present disclosure are shown and described. As will be realized, the present disclosure is capable of other and different embodiments, and its several details are capable of modifications in various respects, all without departing from the disclosure. Accordingly, the drawings and description are to be regarded as illustrative in nature, and not as restrictive.

As used herein, the abbreviations for the natural L-enantiomeric amino acids are conventional and are as follows: alanine (A, Ala); arginine (R, Arg); asparagine (N, Asn); aspartic acid (D, Asp); cysteine (C, Cys); glutamic acid (E, Glu); glutamine (Q, Gln); glycine (G, Gly); histidine (H, His); isoleucine (I, Ile); leucine (L, Leu); lysine (K, Lys); methionine (M, Met); phenylalanine (F, Phe); proline (P, Pro); serine (S, Ser); threonine (T, Thr); tryptophan (W, Trp); tyrosine (Y, Tyr); valine (V, Val). Typically, Xaa can indicate any amino acid. In some embodiments, X can be asparagine (N), glutamine (Q), histidine (H), lysine (K), or arginine (R).

Some embodiments of the disclosure contemplate D-amino acid residues of any standard or non-standard amino acid or analogue thereof. When an amino acid sequence is represented as a series of three-letter or one-letter amino acid abbreviations, the left-hand direction is the amino terminal direction and the right-hand direction is the carboxy terminal direction, in accordance with standard usage and convention.

The terms "peptide", "polypeptide", "protein", "hitchin", "cystine-dense peptide", "knotted peptides" or "CDP" can be used interchangeably herein to refer to a polymer of amino acid residues. In various embodiments, "peptides", "polypeptides", and "proteins" can be chains of amino acids whose alpha carbons are linked through peptide bonds. The terminal amino acid at one end of the chain (e.g., amino terminal) therefore can have a free amino group, while the terminal amino acid at the other end of the chain (e.g., carboxy terminal) can have a free carboxyl group. As used herein, the term "amino terminus" (e.g., abbreviated N-terminus) can refer to the free α-amino group on an amino acid at the amino terminal of a peptide or to the α-amino group (e.g., imino group when participating in a peptide bond) of an amino acid at any other location within the peptide. Similarly, the term "carboxy terminus" can refer to the free carboxyl group on the carboxy terminus of a peptide or the carboxyl group of an amino acid at any other location within the peptide. Peptides also include essentially any polyamino acid including, but not limited to, peptide mimetics such as amino acids joined by an ether or thioether as opposed to an amide bond.

As used herein, the term "peptide construct" can refer to a molecule comprising one or more peptides of the present disclosure that can be conjugated to, linked to, or fused to one or more cargo molecules. In some cases, cargo molecules are active agents. The term "active agent" can refer to any molecule, e.g., any molecule that is capable of eliciting a biological effect and/or a physical effect (e.g., emission of radiation) which can allow the localization, detection, or visualization of the respective peptide construct. In various embodiments, the term "active agent" refers to a therapeutic and/or diagnostic agent. A peptide construct of the present disclosure can comprise a TfR-binding peptide that is linked to one or more active agents via one or more linker moieties (e.g., cleavable or stable linker) as described herein.

As used herein, the terms "comprising" and "having" can be used interchangeably. For example, the terms "a peptide comprising an amino acid sequence of SEQ ID NO: 32" and "a peptide having an amino acid sequence of SEQ ID NO: 32" can be used interchangeably.

As used herein, and unless otherwise stated, the term "TfR" or "transferrin receptor" is a class of protein used herein and can refer to a transferrin receptor from any species (e.g., human or murine TfR or any human or non-human animal TfR). In some cases, and as used herein, the term "TfR" or "transferrin receptor" refers to human TfR (hTfR) and can include TfR or any of the known TfR homologs or orthologs, including TfR1, TfR2, soluble TfR, or any combination or fragment (e.g., ectodomain) thereof.

The term "engineered," when applied to a polynucleotide, denotes that the polynucleotide has been removed from its natural genetic milieu and is thus free of other extraneous or unwanted coding sequences, and is in a form suitable for use within genetically engineered protein production systems. Such engineered molecules are those that are separated from their natural environment and include cDNA and genomic clones (i.e., a prokaryotic or eukaryotic cell with a vector containing a fragment of DNA from a different organism). Engineered DNA molecules of the present invention are free of other genes with which they are ordinarily associated but may include naturally occurring or non-naturally occurring 5' and 3' untranslated regions such as enhancers, promoters and terminators.

An "engineered" polypeptide or protein is a polypeptide or protein that is found in a condition other than its native environment, such as apart from blood and animal tissue. In a preferred form, the engineered polypeptide is substantially free of other polypeptides, particularly other polypeptides of animal origin. It is preferred to provide the polypeptides in a highly purified form, e.g., greater than 95% pure, more preferably greater than 98% pure or greater than 99% pure. When used in this context, the term "engineered" does not exclude the presence of the same polypeptide in alternative physical forms, such as dimers, heterodimers and multimers, or alternatively glycosylated, caboxylated, modified, or derivatized forms.

An "engineered" peptide or protein is a polypeptide that is distinct from a naturally occurring polypeptide structure, sequence, or composition. Engineered peptides include non-naturally occurring, artificial, isolated, synthetic, designed, modified, or recombinantly expressed peptides. Provided herein are engineered TfR-binding peptides, variants, or fragments thereof. These engineered TfR-binding peptides can be further linked to an active agent or a detectable agent. The active agent can be a half-life extending moiety.

Polypeptides of the disclosure include polypeptides that have been modified in any way, for example, to: (1) reduce susceptibility to proteolysis, (2) reduce susceptibility to oxidation, (3) alter binding affinity for forming protein complexes, (4) alter binding affinities, and (5) confer or modify other physicochemical or functional properties. For example, single or multiple amino acid substitutions (e.g., conservative amino acid substitutions) are made in the naturally occurring sequence (e.g., in the portion of the polypeptide outside the domain(s) forming intermolecular contacts). A "conservative amino acid substitution" can refer to the substitution in a polypeptide of an amino acid with a functionally similar amino acid. The following six groups each contain amino acids that can be conservative substitutions for one another: i) Alanine (A), Serine (S), and Threonine (T); ii) Aspartic acid (D) and Glutamic acid (E); iii) Asparagine (N) and Glutamine (Q); iv) Arginine (R) and Lysine (K); v) Isoleucine (I), Leucine (L), Methionine (M), and Valine (V); vi) Phenylalanine (F), Tyrosine (Y), and Tryptophan (W).

The terms "polypeptide fragment" and "truncated polypeptide" as used herein can refer to a polypeptide that has an amino-terminal and/or carboxy-terminal deletion as compared to a corresponding full-length peptide or protein. In various embodiments, fragments are at least 5, at least 10, at least 25, at least 50, at least 100, at least 150, at least 200, at least 250, at least 300, at least 350, at least 400, at least 450, at least 500, at least 600, at least 700, at least 800, at least 900 or at least 1000 amino acids in length. In various embodiments, fragments can also be, *e.g.,* at most 1000, at most 900, at most 800, at most 700, at most 600, at most 500, at most 450, at most 400, at most 350, at most 300, at most 250, at most 200, at most 150, at most 100, at most 50, at most 25, at most 10, or at most 5 amino acids in length. A fragment can further comprise, at either or both of its ends, one or more additional amino acids, for example, a sequence of amino acids from a different naturally-occurring protein (e.g., an Fc or leucine zipper domain) or an artificial amino acid sequence (e.g., an artificial linker sequence).

In various embodiments, the interface residues of the TfR-binding peptides of the present disclosure (e.g., those amino acid residues that interact with TfR for receptor binding) can be divided between two largely helical domains of the peptide. In some cases, the interface residues can comprise residues corresponding to residues 5-25 (e.g., and comprising corresponding residues G5, A7, S8, M11, N14, L17, E18, and E21), with reference to SEQ ID NO: 32, or corresponding to residues 35-51 (e.g., and comprising corresponding residues L38, L41, L42, L45, D46, H47, H49, S50, and Q51), with reference to SEQ ID NO: 32, or both. For example, the interface residues can comprise residues corresponding to residues 5-25 (e.g., and comprising corresponding residues G5, A7, S8, M11, N14, L17, E18, and E21), with reference to SEQ ID NO: 32, or corresponding to residues 35-51 (e.g., and comprising corresponding residues L38, L41, L42, L45, D46, H47, H49, S50, and Q51), with reference to SEQ ID NO: 32. In some embodiments, a TfR-binding peptide can comprise a fragment of a peptide provided herein, wherein the fragment comprises the minimum interface residues for binding, for example residues corresponding to residues 5-25 (e.g., and comprising corresponding residues G5, A7, S8, M11, N14, L17, E18, and E21), with reference to SEQ ID NO: 32, or corresponding to residues 35-51 (e.g., and comprising corresponding residues L38, L41, L42, L45, D46, H47, H49, S50, and Q51), with reference to SEQ ID NO: 32. In some cases, the TfR-binding peptide is a peptide having the sequence set forth in SEQ ID NO: 32 comprising the TfR-binding residues corresponding to residues G5, A7, S8, M11, N14, L17, E18, and E21 of the domain and corresponding to residues L38, L41, L42, L45, D46, H47, H49, S50, and Q51 of the second domain, with reference to SEQ ID NO: 32.

As used herein, the terms "peptide" or "polypeptide" in conjunction with "variant" "mutant" or "enriched mutant" or "permuted enriched mutant" can refer to a peptide or polypeptide that can comprise an amino acid sequence wherein one or more amino acid residues are inserted into, deleted from and/or substituted into the amino acid sequence relative to another polypeptide sequence. In various embodiments, the number of amino acid residues to be inserted, deleted, or substituted is at least 1, at least 2, at least 3, at least 4, at least 5, at least 10, at least 25, at least 50, at least 75, at least 100, at least 125, at least 150, at least 175, at least 200, at least 225, at least 250, at least 275, at least 300, at least 350, at least 400, at least 450 or at least 500 amino acids in length. Variants of the present disclosure include peptide conjugates or fusion molecules (e.g., peptide constructs).

A "derivative" of a peptide or polypeptide can be a peptide or polypeptide that can have been chemically modified, e.g., conjugation to another chemical moiety such as, for example, polyethylene glycol, albumin (e.g., human serum albumin), phosphorylation, and glycosylation.

The term "% sequence identity" can be used interchangeably herein with the term "% identity" and can refer to the level of amino acid sequence identity between two or more peptide sequences or the level of nucleotide sequence identity between two or more nucleotide sequences, when aligned using a sequence alignment program. For example, as used herein, 80% identity means the same thing as 80% sequence identity determined by a defined algorithm, and means that a given sequence is at least 80% identical to another length of another sequence. In various embodiments, the % identity is selected from, *e.g.,* at least 60%, at least 65%, at least 70%, at least 75%, at least 80%, at least 85%, at least 90%, at least 95%, or at least 99% or more sequence identity to a given sequence. In various embodiments, the % identity is in the range of, e.g., about 60% to about 70%, about 70% to about 80%, about 80% to about 85%, about 85% to about 90%, about 90% to about 95%, or about 95% to about 99%.

The terms "% sequence homology" or "percent sequence homology" or "percent sequence identity" can be used interchangeably herein with the terms "% homology," "% sequence identity," or "% identity" and can refer to the level of amino acid sequence homology between two or more peptide sequences or the level of nucleotide sequence homology between two or more nucleotide sequences, when aligned using a sequence alignment program. For example, as used herein, 80% homology means the same thing as 80% sequence homology determined by a defined algorithm, and accordingly a homologue of a given sequence has greater than 80% sequence homology over a length of the given sequence. In various embodiments, the % homology is selected from, e.g., at least 60%, at least 65%, at least 70%, at least 75%, at least 80%, at least 85%, at least 90%, at least 95%, or at least 99% or more sequence homology to a given sequence. In various embodiments, the % homology is in the range of, e.g., about 60% to about 70%, about 70% to about 80%, about 80% to about 85%, about 85% to about 90%, about 90% to about 95%, or about 95% to about 99%.

A protein or polypeptide can be "substantially pure," "substantially homogeneous", or "substantially purified" when at least about 60% to 75% of a sample exhibits a single species of polypeptide. The polypeptide or protein can be monomeric or multimeric. A substantially pure polypeptide or protein can typically comprise about 50%, 60%, 70%, 80% or 90% W/W of a protein sample, more usually about 95%, and e.g., will be over 99% pure. Protein purity or homogeneity can be indicated by a number of means well known in the art, such as polyacrylamide gel electrophoresis of a protein sample, followed by visualizing a single polypeptide band upon staining the gel with a stain well known in the art. For certain purposes, higher resolution is provided by using high-pressure liquid chromatography (e.g., HPLC) or other high-resolution analytical techniques (e.g., LC-mass spectrometry).

As used herein, the term "pharmaceutical composition" can generally refer to a composition suitable for pharmaceutical use in a subject such as an animal (e.g., human or mouse). A pharmaceutical composition can comprise a pharmacologically effective amount of an active agent and a pharmaceutically acceptable carrier. The term "pharmacologically effective amount" can refer to that amount of an agent effective to produce the intended biological or pharmacological result.

As used herein, the term "pharmaceutically acceptable carrier" can refer to any of the standard pharmaceutical carriers, vehicles, buffers, and excipients, such as a phosphate buffered saline solution, 5% aqueous solution of dextrose, and emulsions, such as an oil/water or water/oil emulsion, and various types of wetting agents and/or adjuvants. Suitable pharmaceutical carriers and formulations are described in Remington's Pharmaceutical Sciences, 21st Ed. 2005, Mack Publishing Co, Easton. A "pharmaceutically acceptable salt" can be a salt that can be formulated into a compound for pharmaceutical use including, e.g., metal salts (sodium, potassium, magnesium, calcium, etc.) and salts of ammonia or organic amines.

As used herein, the terms "treat", "treating" and "treatment" can refer to a method of alleviating or abrogating a biological disorder and/or at least one of its attendant symptoms. As used herein, to "alleviate" a disease, disorder or condition, for example, means reducing the severity and/or occurrence frequency of the symptoms of the disease, disorder, or condition. Further, references herein to "treatment" can include references to curative, palliative, and prophylactic or diagnostic treatment.

Generally, a cell of the present disclosure can be a eukaryotic cell or a prokaryotic cell. A cell can be an epithelial cell. A cell can be an animal cell or a plant cell. An animal cell can include a cell from a marine invertebrate, fish, insects, amphibian, reptile, or mammal. A mammalian cell can be obtained from a primate, ape, equine, bovine, porcine, canine, feline, or rodent. A mammal can be a primate, ape, dog, cat, rabbit, ferret, or the like. A rodent can be a mouse, rat, hamster, gerbil, hamster, chinchilla, or guinea pig. A bird cell can be from a canary, parakeet or parrots. A reptile cell can be from a turtles, lizard or snake. A fish cell can be from a tropical fish. For example, the fish cell can be from a zebrafish (e.g., Danino rerio). A worm cell can be from a nematode (e.g., C. elegans). An amphibian cell can be from a frog. An arthropod cell can be from a tarantula or hermit crab.

A mammalian cell can also include cells obtained from a primate (e.g., a human or a non-human primate). A mammalian cell can include a blood cell, a stem cell, an epithelial cell, connective tissue cell, hormone secreting cell, a nerve cell, a skeletal muscle cell, or an immune system cell. In preferred embodiments, the methods and compositions of the present disclosure are used in combination with one or more mammalian blood cells.

As used herein, the term "vector," generally refers to a DNA molecule capable of replication in a host cell and/or to which another DNA segment can be operatively linked so as to bring about replication of the attached segment. A plasmid is an exemplary vector.

As used herein, the term "subject," generally refers to a human or to another animal. A subject can be of any age, for example, a subject can be an infant, a toddler, a child, a pre-adolescent, an adolescent, an adult, or an elderly individual.

Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are in relation to the other endpoint, and independently of the other endpoint. The term "about" as used herein refers to a range that is 15% plus or minus from a stated numerical value within the context of the particular usage. For example, about 10 can include a range from 8.5 to 11.5.

### Peptides

Disclosed herein are peptide sequences, such as those listed in **TABLE 1,** capable of binding to TfR or any of the known TfR homologs, including TfR1, TfR2, soluble TfR, or any combination or fragment (e.g., ectodomain) thereof. A peptide capable of binding a transferrin receptor or a TfR homolog may be referred to herein as a transferrin receptor-binding peptide or a TfR-binding peptide. In some embodiments, peptides disclosed herein can penetrate, cross, or enter target cells in a TfR-mediated manner. These cell layers or cells can include TfR-expressing endothelial cells, epithelial cells, and TfR-expressing cells of various tissues or organs such as tumor cells, brain cells, cancerous or tumor cells, liver cells, pancreas cells, colon cells, ovarian cells, breast cells, and/or lung cells, or any combination thereof.

In various embodiments, the present disclosure provides methods and compositions that enable TfR-mediated transport across cellular layers (e.g., endothelial cells or epithelial cells) or cell membranes. In addition to the BBB, various other cells, tissues, and organs express TfR. Single cells expressing TfR can include hepatocytes, erythrocytes and erythrocyte precursors in bone marrow, immune cells, stem cells, and rapidly dividing cells. Tissues and organs expressing TfR can include the brain (e.g., cerebral cortex, hippocampus, caudate, cerebellum), endocrine tissues (e.g., thyroid, parathyroid, and adrenal glands), bone marrow and immune system (e.g., appendix, lymph node, tonsil, spleen), muscle tissues (e.g., heart, skeletal, and smooth muscle), liver, gallbladder, pancreas, gastrointestinal tract (e.g., oral mucosa, esophagus, stomach, duodenum, small intestine, colon, rectum), kidney, urinary bladder, female tissues (e.g., fallopian tube, breast, vagina, cervix, endometrium, ovary, and placenta), adipose and soft tissue, and skin. Thus, the TfR-binding peptides of the present disclosure can be used to target these cells, tissues, and organs and deliver an active agent to these cells, tissues, and organs via, for example, TfR-mediated transcytosis (e.g., across cellular barrier such as the BBB) or TfR-mediated endocytosis (e.g., across cell membranes into cells) or TfR-mediated accumulation in tissues to treat and/or prevent a disease or condition in one or more of these cells, tissues, or organs.

In various embodiments, the present disclosure provides methods and compositions that enable TfR-mediated transport and delivery to cancer cells expressing TfR. Cancers overexpressing TfR can include ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer.

TfR-binding peptide constructs that can be used to prevent and/or treat a cancer are those comprising a TfR binding peptide and an active agent with anti-tumor activity such as a fused IL15/IL15Ra complex, IFNgamma, and anti-CD3 agents.
In some embodiments, TfR-binding peptide constructs are fused to IL-15 to recruit and stimulated the immune T cells or NK cells. In some embodiments, TfR-binding peptide constructs are fused to INFg to activate macrophages and upregulated MHC in tumor cells. In some embodiments, TfR-binding peptide constructs are fused to CD3 to bind T cells in addition to binding TfR on cancer cells to create an immune synapse and activate T cells to target the cancer.

Generally, the TfR-binding peptides of the present disclosure can be used in combination with various classes of active agent. The TfR-binding peptides of the present disclosure can be conjugated to, linked to, or fused to one or more of those active agents. In various aspects, an active agent is an immunotherapeutic agent, a CTLA-4 targeting agent, a PD-1 targeting agent, a PDL-1 targeting agent, an IL15 agent, a fused IL15/IL15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter such as neurotensin.

In some embodiments, the peptides as discloses herein can cross cellular layers or barriers (e.g., BBB) or cell membranes via, for example, TfR-mediated vesicular transcytosis and TfR-mediated endocytosis, respectively. In addition to binding TfR and promote transcytosis and/or endocytosis, the peptides of the present disclosure can also bind to additional target proteins on cells such as cancer cells. In some cases, a peptide is a peptide or peptide construct comprising a TfR-binding peptide conjugated to, linked to, or fused to a targeting moiety or an active agent (e.g., a therapeutic or diagnostic agent) such as a small molecule or a peptide that has an affinity for an additional target protein (e.g., receptor or enzyme). In some cases, the TfR-binding peptide is linked to a cargo molecule and enables or promotes TfR-mediated transcytosis of the cargo molecule across the BBB or TfR-mediated endocytosis into a cell. In some instances, and subsequent to transcytosis, a peptide construct comprising the TfR-binding peptide and a cargo moiety can target a specific cell or tissue in the CNS and exert a biological effect (e.g., binding a target protein) upon reaching said cell or tissue. In some cases, a peptide construct of the present disclosure exerts a biological effect that is mediated by the TfR-binding peptide, the cargo molecule or active agent, or a combination thereof. In some cases, a TfR-binding peptide construct of the present disclosure comprising one or more active agents (e.g., therapeutic agents) can transport and/or deliver the one or more active agents into cells that express TfR. In some cases, the TfR-binding peptide accumulates in tissues in the CNS. In some cases, off-target effects are reduced due to CNS-specific accumulation. In some cases, the TfR-binding peptide accumulates in tissue outside of the CNS (e.g., liver, kidney, spleen, or skin). In some cases, the cells expressing TfR are tumor cells and the TfR-binding peptide construct delivers anti-tumor agents to these tumor cells. In some cases, the anti-tumor agents alone show no or only very limited therapeutic efficacy against the tumor cells; however, when the anti-tumor agents are combined with the TfR-binding peptides of the present disclosure as, for example, a peptide construct, the therapeutic efficacy of these anti-tumor agents is significantly improved.

In some embodiments, the TfR-binding peptides of the present disclosure can induce a biologically relevant response. In some embodiments, the biologically relevant response can be induced after intravenous dose, and in some embodiments, after a single intravenous dose. In some embodiments, the TfR-binding peptides can be used in combination with various other classes of therapeutic compounds used to treat and/or prevent pain, neuropathic pain or other neurological disorders such as neurodegenerative disorders, infectious diseases, immunological disorders (e.g., autoimmune diseases). Binding of the herein described peptides and peptide constructs (e.g., peptide conjugates, fusion peptides, or recombinantly produced peptide constructs) to TfR and subsequent transport across a cell layer or barrier such as the BBB (e.g., via TfR-mediated vesicular transcytosis) or a cell membrane (e.g., via TfR-mediated endocytosis) can have implications in a number of diseases, conditions, or disorders associated with chronic pain (e.g., headaches or migraine), neuropathic pain, obesity, insulin resistance, opioid addiction, or other neurologic or psychiatric disorders in a subject (e.g., a human).

Binding of the herein described peptides and peptide constructs (e.g., peptide conjugates, fusion peptides, or recombinantly produced peptide constructs) to TfR and subsequent transport across a cell layer or barrier such as the BBB (e.g., via vesicular transcytosis) or a cell membrane (e.g., via endocytosis) can have implications in a number of diseases, conditions, or disorders associated with neurodegeneration. Neurodegenerative diseases that can treated, prevented, or diagnosed with the herein described TfR-binding peptides can include Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia.

Binding of the herein described peptides and peptide constructs (e.g., peptide conjugates, fusion peptides, or recombinantly produced peptide constructs) to TfR and subsequent transport across a cell layer or barrier such as the BBB (e.g., via vesicular transcytosis) or a cell membrane (e.g., via endocytosis) can have implications in a number of autoimmune diseases to therapeutically address auto-immune or inflammatory disorders in the brain. In some case, an autoimmune disease can be treated and/or prevented by conjugating, linking, or fusing the TfR-binding peptide to an active agent that can be an ion channel modulator such as Kv1.3 potassium channel inhibitor. Additional diseases that can be treated and/or prevented using ion channel modulator such as Kv1.3 potassium channel inhibitors can include psoriasis and other non-brain autoimmune diseases due to its effect on effector T cells, and for neuroinflammatory and neurodegenerative diseases, for example, multiple sclerosis, Alzheimer's, Parkinson's, traumatic brain injury, or radiation therapy toxicity. In some cases, Kv1.3 potassium channel inhibitors can be a small molecule (e.g., domatinostat tosylate), or a peptide (e.g., Vm24 or an ShK peptide, such as ShK-170, ShK-186, or ShK-192, or any fragments or derivatives thereof) or any combinations thereof. (See e.g., Bartok et al. An engineered scorpion toxin analogue with improved Kv1.3 selectivity displays reduced conformational flexibility, Sci Rep. 2015; 5: 18397).

In some embodiments, the TfR-binding peptides of the present disclosure can be used for the treatment and prevention of various neurological diseases including but not limited to epilepsy, schizophrenia, depression, anxiety, bipolar disorder, developmental brain disorders (e.g., autism spectrum), or mood disorder.

In some embodiments, the TfR-binding peptides of the present disclosure can be used for the treatment and prevention of Crohn's disease or, more generally, inflammatory bowel diseases. In some cases, the TfR-binding peptides of the present disclosure show high uptake and retention in glandular cells of the intestine, which can express high amounts of TfR.

In various embodiments, the therapeutic efficacy of these drugs can be significantly improved when used in combination with the TfR-binding peptides of the present disclosure compared to administration without conjugation to the TfR-binding peptides as described herein. In various embodiments, the efficacy can be improved due to higher delivery and achieved levels of drug in the tissue or cell of interest. In various embodiments, the efficacy can be improved by increasing the relative level of the drug in the tissue or cell or interest and reducing the level of the drug in other tissues or compartments, thereby improving the therapeutic window or reducing toxic side effects.

In other embodiments, peptides as described herein compete with endogenous molecules for binding to TfR. As described herein, "compete" or peptide competition for binding to target protein such as TfR encompasses, but is not limited to, steric hindrance, occupying binding sites of the target protein, non-covalent interactions, such as salt bridges or hydrophobic interactions, crosslinking, covalent interactions, sequestration, allosteric modulation, or any combination thereof.

In some embodiments, peptides of the present disclosure can bind to any of the known TfR homologs, including TfR1, TfR2, soluble TfR, or any combination or fragment (e.g., ectodomain) thereof. Thus, as used herein, "TfR" can refer to any known homolog, derivative, fragment, or member of the TfR family including TfR1, TfR2, and a soluble TfR. In other embodiments, peptides are capable of binding to one, one or more, or all TfR homologs. In some embodiments, peptides of the present disclosure can bind to a TfR and promote a particular biological effect such as vesicular transcytosis. In some embodiments, peptides of the present disclosure, including peptides and peptide constructs prevent or decrease the binding of endogenous TfR binders (e.g., transferrin or any derivatives such as apo-transferrin or holo-transferrin) to TfR.

In some embodiments, peptides bind to TfR with equal, similar, or greater affinity (e.g., lower dissociation constant K_{D}) as compared to endogenous molecules (e.g., transferrin, holotransferrin (iron-bound transferrin), apotransferrin (transferrin not bound to iron), or any other endogenous TfR ligands) or other exogenous molecules. In some embodiments, the peptide can have a K_{D} of less than 50 µM, less than 5 µM, less than 500 nM, less than 100 nM, less than 40 nM, less than 30 nM, less than 20 nM, less than 10 nM, less than 1 nM, or less than 0.1 nM. In some embodiments, peptide transport by TfR is improved by having a lower affinity (e.g., a higher dissociation constant K_{D}) as compared to endogenous molecules. In some embodiments, peptide transport by TfR is improved by having a faster off rate or higher k_{off} than endogenous molecules. In some embodiments, the off rate or k_{off} is similar to that of transferrin. In some embodiments, peptide transport is improved by having a faster on rate or a higher kₒₙ, optionally such as higher than that of transferrin. In other embodiments, one or more conserved residues at the transferrin (Tf)-TfR-binding interface are also present in the amino acid sequences of the peptides described herein.

In some embodiments, peptides that exhibit an improved TfR receptor binding show improved transcytosis function. In some embodiments, peptides that exhibit an improved TfR receptor binding show no or small changes in transcytosis function. In some embodiments, peptides that exhibit an improved TfR receptor binding show reduced transcytosis function. In some embodiments, the peptide binds at a site of high homology between human and murine TfR, including one or more, or all, of the amino acid domains corresponding to residues 506-510, 523-531, and 611-662 of the human TfR (SEQ ID NO: 363). In some embodiments, the regions of TfR to which the peptides disclosed herein or variants thereof bind all or in part to such TfR domains. In some embodiments, the peptides disclosed herein bind to any one, any two, or all three of the TfR regions of high homology including the amino acid domains corresponding to residues 506-510, 523-531, and 611-662 of the human TfR (SEQ ID NO: 363). In some embodiments the peptides disclosed herein bind at least to the domain corresponding to residues 611-662 of the human TfR.

In some embodiments, the K_{A} and K_{D} values of a TfR-binding peptide can be modulated and optimized (e.g., via amino acid substitutions) to provide an optimal ratio of TfR-binding affinity and efficient transcytosis function.

In some embodiments, peptides disclosed herein or variants thereof bind to TfR at residues found in the binding interface (e.g., the binding domain or the binding pocket) of TfR with other exogenous or endogenous ligands (e.g., transferrin (Tf), Tf derivatives, or Tf-like peptides or proteins). In some embodiments, a peptide disclosed herein or a variant thereof, which binds to TfR, comprises at least 70% homology, at least 75% homology, at least 80% homology, at least 85% homology, at least 90% homology, at least 95% homology, at least 96% homology, at least 97% homology, at least 98% homology, or at least 99% homology or at least 100% homology to a sequence that binds residues of TfR, which makeup the binding pocket. In some embodiments, a peptide disclosed herein or a variant thereof, which binds to TfR, comprises at least 70% homology, at least 75% homology, at least 80% homology, at least 85% homology, at least 90% homology, at least 95% homology, at least 96% homology, at least 97% homology, at least 98% homology, or at least 99% homology or at least 100% homology to an endogenous or exogenous polypeptide known to bind TfR, for example, endogenous Transferrin or any one of the peptides listed in **TABLE 1.** In other embodiments, a peptide described herein binds to a protein of interest, which comprises at least 70% homology, at least 75% homology, at least 80% homology, at least 85% homology, at least 90% homology, at least 95% homology, at least 96% homology, at least 97% homology, at least 98% homology, or at least 99% homology or at least 100% homology to TfR, a fragment, homolog, or a variant thereof.

In some embodiments, peptides disclosed herein or variants thereof bind regions of TfR that comprise the amino acid residues corresponding to residues 506-510, 523-531, and 611-662 (the numbering of these amino acid residues is based on the following Uniprot reference protein sequence of endogenous human TFRC UniProtKB - P02786 (SEQ ID NO: 363, TFR1_HUMAN)). In some embodiments, the regions of TfR to which the peptides disclosed herein or variants thereof bind overlap with those of Tf, a fragment, homolog, or a variant thereof.

In other embodiments, a nucleic acid, vector, plasmid, or donor DNA comprises a sequence that encodes a peptide, peptide construct, or variant or functional fragment thereof, as described in the present disclosure. In further embodiments, certain parts or fragments of TfR-binding motifs (e.g., conserved binding motifs) can be grafted onto a peptide or peptide construct

In some embodiments, peptides inhibit binding between TfR and Tf, or between TfR and any other protein. In some embodiments, peptides prevent TfR from protein-protein interaction and/or prevent TfR localization to a cell's nucleus. In some cases, peptides deactivate TfR. In some embodiments, peptides can cause TfR to be degraded, or prevent TfR from localization to a cell's nucleus, or prevent TfR from interacting with Tf or Tf-like proteins.

In some embodiments, peptides competitively bind to TfR as compared to endogenous Tf or any other endogenous or exogenous TfR binder by binding to a certain amino acid residue or motif of amino acid residues in TfR. Furthermore, a peptide can be selected for further testing or use based upon its ability to bind to the certain amino acid residue or motif of amino acid residues. The certain amino acid residue or motif of amino acid residues in TfR can be identified an amino acid residue or sequence of amino acid residues that are involved in the binding of TfR to Tf. A certain amino acid residue or motif of amino acid residues can be identified from a crystal structure of the TfR:Tf complex. In some embodiments, peptides (e.g., CDPs) demonstrate the resistance to heat, protease (pepsin), and reduction.

The peptides and peptide constructs (e.g., peptide conjugates or fusion peptides)
can bind to a protein of interest. In some embodiments, the protein of interest is a TfR.

**In** some embodiments, a peptide or a library of peptides is designed *in silico* without derivation from a naturally occurring scaffold of a knotted peptide. In other embodiments, a peptide or a library of peptides is designed *in silico* by derivation, grafting relevant protein-binding residues, or conserved residues in the protein-binding interface a naturally occurring peptide or protein known to bind to a protein or receptor of interest. In some embodiments, the peptide
is a simple helix-turn-helix. In some embodiments, the helix-turn-helix can be used for pharmacophore transfer onto other scaffolds, for example engraftment of the required TfR-engaging surface onto the helix-turn-helix scaffold using fusion tagging.

**In** some embodiments, a peptide comprising SEQ ID NO: 1 is used as a scaffold or base sequence for further modifications, including addition, deletion, or amino acid substitution. In some embodiments, short sequences of amino acid residues such as GS are added at the N-terminus of a peptide. In some embodiments, peptides lack GS at the N-terminus. In some instances, peptides undergo one or more post-translational modifications.

**TABLE 1** lists exemplary peptide sequences according to the methods and compositions of the present disclosure.

**TABLE 1 - Exemplary Peptide Sequences**

| **SEQ ID NO** | **Amino Acid Sequence** |
|---|---|
| SEQ ID NO: 1 | GSREGCASRCTKYNAELEKCEARVSSMSNTEETCVQELFDLLHCVDHCVSQ |
| SEQ ID NO: 2 | GSREGCASRCTKYNAELEKCEARVMSMSNTEEDCEQELEDLLHCLDHCHSQ |
| SEQ ID NO: 3 | GSREGCASRCTKYNAELEKCEARVVSMSNTEEDCEQELEDLLHCLDHCHSQ |
| SEQ ID NO: 4 | GSREGCASRCTKYNAELEKCEARVMSMSNTEETCEQELEDLLHCLDHCHSQ |
| SEQ ID NO: 5 | GSREGCASRCTKYNAELEKCEARVMSMSNTEEDCVQELEDLLHCLDHCHSQ |
| SEQ ID NO: 6 | GSREGCASRCTKYNAELEKCEARVMSMSNTEEDCEQELFDLLHCLDHCHSQ |
| SEQ ID NO: 7 | GSREGCASRCTKYNAELEKCEARVMSMSNTEEDCEQELEDLLHCVDHCHSQ |
| SEQ ID NO: 8 | GSREGCASRCTKYNAELEKCEARVMSMSNTEEDCEQELEDLLHCLDHCVSQ |
| SEQ ID NO: 9 | GSREGCASRCTKYNAELEKCEARVVSMSNTEETCEQELEDLLHCLDHCHSQ |
| SEQ ID NO: 10 | GSREGCASRCTKYNAELEKCEARVVSMSNTEEDCVQELEDLLHCLDHCHSQ |
| SEQ ID NO: 11 | GSREGCASRCTKYNAELEKCEARVVSMSNTEEDCEQELFDLLHCLDHCHSQ |
| SEQ ID NO: 12 | GSREGCASRCTKYNAELEKCEARVVSMSNTEEDCEQELEDLLHCVDHCHSQ |
| SEQ ID NO: 13 | GSREGCASRCTKYNAELEKCEARVVSMSNTEEDCEQELEDLLHCLDHCVSQ |
| SEQ ID NO: 14 | GSREGCASRCTKYNAELEKCEARVMSMSNTEETCVQELEDLLHCLDHCHSQ |
| SEQ ID NO: 15 | GSREGCASRCTKYNAELEKCEARVMSMSNTEETCEQELFDLLHCLDHCHSQ |
| SEQ ID NO: 16 | GSREGCASRCTKYNAELEKCEARVMSMSNTEETCEQELEDLLHCVDHCHSQ |
| SEQ ID NO: 17 | GSREGCASRCTKYNAELEKCEARVMSMSNTEETCEQELEDLLHCLDHCVSQ |
| SEQ ID NO: 18 | GSREGCASRCTKYNAELEKCEARVMSMSNTEEDCVQELFDLLHCLDHCHSQ |
| SEQ ID NO: 19 | GSREGCASRCTKYNAELEKCEARVMSMSNTEEDCVQELEDLLHCVDHCHSQ |
| SEQ ID NO: 20 | GSREGCASRCTKYNAELEKCEARVMSMSNTEEDCVQELEDLLHCLDHCVSQ |
| SEQ ID NO: 21 | GSREGCASRCTKYNAELEKCEARVMSMSNTEEDCEQELFDLLHCVDHCHSQ |
| SEQ ID NO: 22 | GSREGCASRCTKYNAELEKCEARVMSMSNTEEDCEQELFDLLHCLDHCVSQ |
| SEQ ID NO: 23 | GSREGCASRCTKYNAELEKCEARVMSMSNTEEDCEQELEDLLHCVDHCVSQ |
| SEQ ID NO: 24 | GSREGCASRCMKYNAELEKCEARVMSMSNTEEDCEQELEDLLHCLDHCHSQ |
| SEQ ID NO: 25 | GSREGCASRCTKYNDELEKCEARVMSMSNTEEDCEQELEDLLHCLDHCHSQ |
| SEQ ID NO: 26 | GSREGCASRCTKYNAELEKCEARMMSMSNTEEDCEQELEDLLHCLDHCHSQ |
| SEQ ID NO: 27 | GSREGCASRCTKYNAELEKCEARVMSMSNTEEDCEQELEDLLYCLDHCHSQ |
| SEQ ID NO: 28 | GSREGCASRCMKYNDELEKCEARMMSMSNTEEDCEQELEDLLHCLDHCHSQ |
| SEQ ID NO: 29 | GSREGCASRCMKYNDELEKCEARVMSMSNTEEDCEQELEDLLYCLDHCHSQ |
| SEQ ID NO: 30 | GSREGCASRCMKYNAELEKCEARMMSMSNTEEDCEQELEDLLYCLDHCHSQ |
| SEQ ID NO: 31 | GSREGCASRCTKYNDELEKCEARMMSMSNTEEDCEQELEDLLYCLDHCHSQ |
| SEQ ID NO: 32 | GSREGCASRCMKYNDELEKCEARMMSMSNTEEDCEQELEDLLYCLDHCHSQ |
| SEQ ID NO: 36 | REGCASRCTKYNAELEKCEARVSSMSNTEETCVQELFDLLHCVDHCVSQ |
| SEQ ID NO: 37 | REGCASRCTKYNAELEKCEARVMSMSNTEEDCEQELEDLLHCLDHCHSQ |
| SEQ ID NO: 38 | REGCASRCTKYNAELEKCEARVVSMSNTEEDCEQELEDLLHCLDHCHSQ |
| SEQ ID NO: 39 | REGCASRCTKYNAELEKCEARVMSMSNTEETCEQELEDLLHCLDHCHSQ |
| SEQ ID NO: 40 | REGCASRCTKYNAELEKCEARVMSMSNTEEDCVQELEDLLHCLDHCHSQ |
| SEQ ID NO: 41 | REGCASRCTKYNAELEKCEARVMSMSNTEEDCEQELFDLLHCLDHCHSQ |
| SEQ ID NO: 42 | REGCASRCTKYNAELEKCEARVMSMSNTEEDCEQELEDLLHCVDHCHSQ |
| SEQ ID NO: 43 | REGCASRCTKYNAELEKCEARVMSMSNTEEDCEQELEDLLHCLDHCVSQ |
| SEQ ID NO: 44 | REGCASRCTKYNAELEKCEARVVSMSNTEETCEQELEDLLHCLDHCHSQ |
| SEQ ID NO: 45 | REGCASRCTKYNAELEKCEARVVSMSNTEEDCVQELEDLLHCLDHCHSQ |
| SEQ ID NO: 46 | REGCASRCTKYNAELEKCEARVVSMSNTEEDCEQELFDLLHCLDHCHSQ |
| SEQ ID NO: 47 | REGCASRCTKYNAELEKCEARVVSMSNTEEDCEQELEDLLHCVDHCHSQ |
| SEQ ID NO: 48 | REGCASRCTKYNAELEKCEARVVSMSNTEEDCEQELEDLLHCLDHCVSQ |
| SEQ ID NO: 49 | REGCASRCTKYNAELEKCEARVMSMSNTEETCVQELEDLLHCLDHCHSQ |
| SEQ ID NO: 50 | REGCASRCTKYNAELEKCEARVMSMSNTEETCEQELFDLLHCLDHCHSQ |
| SEQ ID NO: 51 | REGCASRCTKYNAELEKCEARVMSMSNTEETCEQELEDLLHCVDHCHSQ |
| SEQ ID NO: 52 | REGCASRCTKYNAELEKCEARVMSMSNTEETCEQELEDLLHCLDHCVSQ |
| SEQ ID NO: 53 | REGCASRCTKYNAELEKCEARVMSMSNTEEDCVQELFDLLHCLDHCHSQ |
| SEQ ID NO: 54 | REGCASRCTKYNAELEKCEARVMSMSNTEEDCVQELEDLLHCVDHCHSQ |
| SEQ ID NO: 55 | REGCASRCTKYNAELEKCEARVMSMSNTEEDCVQELEDLLHCLDHCVSQ |
| SEQ ID NO: 56 | REGCASRCTKYNAELEKCEARVMSMSNTEEDCEQELFDLLHCVDHCHSQ |
| SEQ ID NO: 57 | REGCASRCTKYNAELEKCEARVMSMSNTEEDCEQELFDLLHCLDHCVSQ |
| SEQ ID NO: 58 | REGCASRCTKYNAELEKCEARVMSMSNTEEDCEQELEDLLHCVDHCVSQ |
| SEQ ID NO: 59 | REGCASRCMKYNAELEKCEARVMSMSNTEEDCEQELEDLLHCLDHCHSQ |
| SEQ ID NO: 60 | REGCASRCTKYNDELEKCEARVMSMSNTEEDCEQELEDLLHCLDHCHSQ |
| SEQ ID NO: 61 | REGCASRCTKYNAELEKCEARMMSMSNTEEDCEQELEDLLHCLDHCHSQ |
| SEQ ID NO: 62 | REGCASRCTKYNAELEKCEARVMSMSNTEEDCEQELEDLLYCLDHCHSQ |
| SEQ ID NO: 63 | REGCASRCMKYNDELEKCEARMMSMSNTEEDCEQELEDLLHCLDHCHSQ |
| SEQ ID NO: 64 | REGCASRCMKYNDELEKCEARVMSMSNTEEDCEQELEDLLYCLDHCHSQ |
| SEQ ID NO: 65 | REGCASRCMKYNAELEKCEARMMSMSNTEEDCEQELEDLLYCLDHCHSQ |
| SEQ ID NO: 66 | REGCASRCTKYNDELEKCEARMMSMSNTEEDCEQELEDLLYCLDHCHSQ |
| SEQ ID NO: 67 | REGCASRCMKYNDELEKCEARMMSMSNTEEDCEQELEDLLYCLDHCHSQ |
| SEQ ID NO: 136 | GSREGCASRCTRYNAELERCEARVSSMSNTEETCVQELFDLLHCVDHCVSQ |
| SEQ ID NO: 137 | GSREGCASRCTRYNAELERCEARVMSMSNTEEDCEQELEDLLHCLDHCHSQ |
| SEQ ID NO: 138 | GSREGCASRCTRYNAELERCEARVVSMSNTEEDCEQELEDLLHCLDHCHSQ |
| SEQ ID NO: 139 | GSREGCASRCTRYNAELERCEARVMSMSNTEETCEQELEDLLHCLDHCHSQ |
| SEQ ID NO: 140 | GSREGCASRCTRYNAELERCEARVMSMSNTEEDCVQELEDLLHCLDHCHSQ |
| SEQ ID NO: 141 | GSREGCASRCTRYNAELERCEARVMSMSNTEEDCEQELFDLLHCLDHCHSQ |
| SEQ ID NO: 142 | GSREGCASRCTRYNAELERCEARVMSMSNTEEDCEQELEDLLHCVDHCHSQ |
| SEQ ID NO: 143 | GSREGCASRCTRYNAELERCEARVMSMSNTEEDCEQELEDLLHCLDHCVSQ |
| SEQ ID NO: 144 | GSREGCASRCTRYNAELERCEARVVSMSNTEETCEQELEDLLHCLDHCHSQ |
| SEQ ID NO: 145 | GSREGCASRCTRYNAELERCEARVVSMSNTEEDCVQELEDLLHCLDHCHSQ |
| SEQ ID NO: 146 | GSREGCASRCTRYNAELERCEARVVSMSNTEEDCEQELFDLLHCLDHCHSQ |
| SEQ ID NO: 147 | GSREGCASRCTRYNAELERCEARVVSMSNTEEDCEQELEDLLHCVDHCHSQ |
| SEQ ID NO: 148 | GSREGCASRCTRYNAELERCEARVVSMSNTEEDCEQELEDLLHCLDHCVSQ |
| SEQ ID NO: 149 | GSREGCASRCTRYNAELERCEARVMSMSNTEETCVQELEDLLHCLDHCHSQ |
| SEQ ID NO: 150 | GSREGCASRCTRYNAELERCEARVMSMSNTEETCEQELFDLLHCLDHCHSQ |
| SEQ ID NO: 151 | GSREGCASRCTRYNAELERCEARVMSMSNTEETCEQELEDLLHCVDHCHSQ |
| SEQ ID NO: 152 | GSREGCASRCTRYNAELERCEARVMSMSNTEETCEQELEDLLHCLDHCVSQ |
| SEQ ID NO: 153 | GSREGCASRCTRYNAELERCEARVMSMSNTEEDCVQELFDLLHCLDHCHSQ |
| SEQ ID NO: 154 | GSREGCASRCTRYNAELERCEARVMSMSNTEEDCVQELEDLLHCVDHCHSQ |
| SEQ ID NO: 155 | GSREGCASRCTRYNAELERCEARVMSMSNTEEDCVQELEDLLHCLDHCVSQ |
| SEQ ID NO: 156 | GSREGCASRCTRYNAELERCEARVMSMSNTEEDCEQELFDLLHCVDHCHSQ |
| SEQ ID NO: 157 | GSREGCASRCTRYNAELERCEARVMSMSNTEEDCEQELFDLLHCLDHCVSQ |
| SEQ ID NO: 158 | GSREGCASRCTRYNAELERCEARVMSMSNTEEDCEQELEDLLHCVDHCVSQ |
| SEQ ID NO: 159 | GSREGCASRCMRYNAELERCEARVMSMSNTEEDCEQELEDLLHCLDHCHSQ |
| SEQ ID NO: 160 | GSREGCASRCTRYNDELERCEARVMSMSNTEEDCEQELEDLLHCLDHCHSQ |
| SEQ ID NO: 161 | GSREGCASRCTRYNAELERCEARMMSMSNTEEDCEQELEDLLHCLDHCHSQ |
| SEQ ID NO: 162 | GSREGCASRCTRYNAELERCEARVMSMSNTEEDCEQELEDLLYCLDHCHSQ |
| SEQ ID NO: 163 | GSREGCASRCMRYNDELERCEARMMSMSNTEEDCEQELEDLLHCLDHCHSQ |
| SEQ ID NO: 164 | GSREGCASRCMRYNDELERCEARVMSMSNTEEDCEQELEDLLYCLDHCHSQ |
| SEQ ID NO: 165 | GSREGCASRCMRYNAELERCEARMMSMSNTEEDCEQELEDLLYCLDHCHSQ |
| SEQ ID NO: 166 | GSREGCASRCTRYNDELERCEARMMSMSNTEEDCEQELEDLLYCLDHCHSQ |
| SEQ ID NO: 167 | GSREGCASRCMRYNDELERCEARMMSMSNTEEDCEQELEDLLYCLDHCHSQ |
| SEQ ID NO: 171 | REGCASRCTRYNAELERCEARVSSMSNTEETCVQELFDLLHCVDHCVSQ |
| SEQ ID NO: 172 | REGCASRCTRYNAELERCEARVMSMSNTEEDCEQELEDLLHCLDHCHSQ |
| SEQ ID NO: 173 | REGCASRCTRYNAELERCEARVVSMSNTEEDCEQELEDLLHCLDHCHSQ |
| SEQ ID NO: 174 | REGCASRCTRYNAELERCEARVMSMSNTEETCEQELEDLLHCLDHCHSQ |
| SEQ ID NO: 175 | REGCASRCTRYNAELERCEARVMSMSNTEEDCVQELEDLLHCLDHCHSQ |
| SEQ ID NO: 176 | REGCASRCTRYNAELERCEARVMSMSNTEEDCEQELFDLLHCLDHCHSQ |
| SEQ ID NO: 177 | REGCASRCTRYNAELERCEARVMSMSNTEEDCEQELEDLLHCVDHCHSQ |
| SEQ ID NO: 178 | REGCASRCTRYNAELERCEARVMSMSNTEEDCEQELEDLLHCLDHCVSQ |
| SEQ ID NO: 179 | REGCASRCTRYNAELERCEARVVSMSNTEETCEQELEDLLHCLDHCHSQ |
| SEQ ID NO: 180 | REGCASRCTRYNAELERCEARVVSMSNTEEDCVQELEDLLHCLDHCHSQ |
| SEQ ID NO: 181 | REGCASRCTRYNAELERCEARVVSMSNTEEDCEQELFDLLHCLDHCHSQ |
| SEQ ID NO: 182 | REGCASRCTRYNAELERCEARVVSMSNTEEDCEQELEDLLHCVDHCHSQ |
| SEQ ID NO: 183 | REGCASRCTRYNAELERCEARVVSMSNTEEDCEQELEDLLHCLDHCVSQ |
| SEQ ID NO: 184 | REGCASRCTRYNAELERCEARVMSMSNTEETCVQELEDLLHCLDHCHSQ |
| SEQ ID NO: 185 | REGCASRCTRYNAELERCEARVMSMSNTEETCEQELFDLLHCLDHCHSQ |
| SEQ ID NO: 186 | REGCASRCTRYNAELERCEARVMSMSNTEETCEQELEDLLHCVDHCHSQ |
| SEQ ID NO: 187 | REGCASRCTRYNAELERCEARVMSMSNTEETCEQELEDLLHCLDHCVSQ |
| SEQ ID NO: 188 | REGCASRCTRYNAELERCEARVMSMSNTEEDCVQELFDLLHCLDHCHSQ |
| SEQ ID NO: 189 | REGCASRCTRYNAELERCEARVMSMSNTEEDCVQELEDLLHCVDHCHSQ |
| SEQ ID NO: 190 | REGCASRCTRYNAELERCEARVMSMSNTEEDCVQELEDLLHCLDHCVSQ |
| SEQ ID NO: 191 | REGCASRCTRYNAELERCEARVMSMSNTEEDCEQELFDLLHCVDHCHSQ |
| SEQ ID NO: 192 | REGCASRCTRYNAELERCEARVMSMSNTEEDCEQELFDLLHCLDHCVSQ |
| SEQ ID NO: 193 | REGCASRCTRYNAELERCEARVMSMSNTEEDCEQELEDLLHCVDHCVSQ |
| SEQ ID NO: 194 | REGCASRCMRYNAELERCEARVMSMSNTEEDCEQELEDLLHCLDHCHSQ |
| SEQ ID NO: 195 | REGCASRCTRYNDELERCEARVMSMSNTEEDCEQELEDLLHCLDHCHSQ |
| SEQ ID NO: 196 | REGCASRCTRYNAELERCEARMMSMSNTEEDCEQELEDLLHCLDHCHSQ |
| SEQ ID NO: 197 | REGCASRCTRYNAELERCEARVMSMSNTEEDCEQELEDLLYCLDHCHSQ |
| SEQ ID NO: 198 | REGCASRCMRYNDELERCEARMMSMSNTEEDCEQELEDLLHCLDHCHSQ |
| SEQ ID NO: 199 | REGCASRCMRYNDELERCEARVMSMSNTEEDCEQELEDLLYCLDHCHSQ |
| SEQ ID NO: 200 | REGCASRCMRYNAELERCEARMMSMSNTEEDCEQELEDLLYCLDHCHSQ |
| SEQ ID NO: 201 | REGCASRCTRYNDELERCEARMMSMSNTEEDCEQELEDLLYCLDHCHSQ |
| SEQ ID NO: 202 | REGCASRCMRYNDELERCEARMMSMSNTEEDCEQELEDLLYCLDHCHSQ |

In some embodiments, a peptide that binds to a TfR comprises the amino acid sequence set forth in SEQ ID NO: 32.

In some embodiments, a TfR-binding peptide comprises canonical amino acid residues as surface interface residues at any one of the corresponding positions 5, 7, 8, 14, 17, 18, 21, 38, 42, 45, 46, 47, 50, 51, with reference to SEQ ID NO: 32 or a combination thereof. In some embodiments, a TfR-binding peptide comprises canonical amino acid residues as surface interface residues at any one of the corresponding positions G5, A7, S8, N14, L17, E18, E21, L38, L42, L45, D46, H47, S50, Q51, with reference to SEQ ID NO: 32 or a combination thereof.

In some embodiments, surface-distal hydrophilic amino acid residues (e.g., D, E, H, K, R, N, Q, S, or T) present in the amino acid sequence of a peptide contribute to peptide solubility. In some embodiments, a peptide as disclosed herein comprises a hydrophilic amino acid residue at any one of the corresponding positions 3, 4, 9, 11, 15, 16, 19, 23, 26, 28, 29, 30, 31, 32, 33, 35, 36, 37, 39, 40, with reference to SEQ ID NO: 32, or any combination thereof. In some instances, a peptide of the present disclosure comprises hydrophilic amino acid residues at the following corresponding positions: R3, E4, R9, K12, D15, E16, K19, R23, S26, S28, N29, T30, E31, E32, D33, E35, Q36, E37, E39, D40, with reference to SEQ ID NO: 32, or any combination thereof. In some embodiments, any one of or any combination of corresponding positions R3, E4, R9, K12, D15, E16, K19, R23, S26, S28, N29, T30, E31, E32, D33, E35, Q36, E37, E39, D40 with reference to SEQ ID NO: 32, can be mutated to another hydrophilic residue without significantly impacting solubility or TfR-binding.

In some embodiments, a peptide of the present disclosure comprises hydrophilic residues (e.g., D, E, H, K, R, N, Q, S, or T) at corresponding positions 15, 35, 39, 49, with reference to SEQ ID NO: 32, or any combination thereof. In some instances, a peptide of the present disclosure comprises hydrophilic amino acid residues at the following corresponding positions: D15, E35, E39, H49, with reference to SEQ ID NO: 32, or any combination thereof. In some embodiments, any one of or any combination of corresponding positions D15, E35, E39, H49 with reference to SEQ ID NO: 32, can be mutated to another hydrophilic residue without significantly impacting solubility or TfR-binding.

In some embodiments, a peptide of the present disclosure comprises hydrophobic residues (e.g., A, M, I, L, V, F, W, or Y) at corresponding positions 15, 35, 39, 49, with reference to SEQ ID NO: 32, or any combination thereof.
In some embodiments, hydrophilic amino acid residues at any one of the corresponding positions 15, 35, 39, and 49, with reference to SEQ ID NO: 32, are associated with higher binding affinity for TfR (e.g., target engagement) and higher solubility. In some embodiments, mutation of an amino acid residue at any one of the corresponding positions 15, 35, 39, and 49, with reference to SEQ ID NO: 32, from a hydrophobic to a hydrophilic residue can lead to higher binding affinity for TfR (e.g., target engagement) and higher solubility.

In some embodiments, a peptide of the present disclosure comprises hydrophobic residues (e.g., A, M, I, L, V, F, W, or Y) at corresponding positions 11, 25, 27, with reference to SEQ ID NO: 32, or any combination thereof. In some embodiments, a peptide of the present disclosure comprises hydrophilic residues (e.g., D, E, H, K, R, N, Q, S, or T) at corresponding positions 11, 25, 27, with reference to SEQ ID NO: 32, or any combination thereof. In some embodiments, hydrophobic amino acid residues at any one of the corresponding positions 11, 25, and 27, with reference to SEQ ID NO: 32, are associated with higher binding affinity for TfR (e.g., target engagement) and higher solubility. In some embodiments, mutation of an amino acid residue at any one of the corresponding positions 11, 25, and 27, with reference to SEQ ID NO: 32, from a hydrophilic residue to a hydrophobic residue can lead to higher binding affinity for TfR (e.g., target engagement) and higher solubility. In some embodiments, a peptide of the present disclosure comprises hydrophobic amino acid residues at the corresponding positions M11, M25, M27, with reference to SEQ ID NO: 32, or any combination thereof. In some instances, a peptide comprises the hydrophobic amino acid residues at the corresponding positions M11, M25, and M27, with reference to SEQ ID NO: 32. In some embodiments, any combination of the corresponding positions M11, M25, and M27, with reference to SEQ ID NO: 32, can be mutated to another hydrophobic residue without significantly impacting solubility or TfR-binding.
In some embodiments, a peptide of the present disclosure comprises an aliphatic amino acid residue (e.g., A, M, I, L, or V) at corresponding position 45, with reference to SEQ ID NO: 32. In some embodiments, a peptide of the present disclosure comprises an aromatic amino acid residue (e.g., F, W, or Y) at corresponding position 45. In some embodiments, an aliphatic amino acid residue at corresponding position 45 is associated with higher binding affinity to TfR. In some instances, a peptide comprises the aliphatic amino acid residue corresponding to L45, with reference to SEQ ID NO: 32. In some embodiments, mutation of an amino acid residue at corresponding position 45 from an aromatic residue to an aliphatic reside can lead to higher binding affinity for TfR (e.g., target engagement) and higher solubility. In some embodiments, mutating corresponding position L45 to another aliphatic residue may not significantly impact solubility or TfR-binding.

In some embodiments, these residues at corresponding position 12 and 19, with reference to SEQ ID NO: 32, can be used for chemical conjugation to another molecule (e.g., an active or a detectable agent). In some embodiments, X₁ and X₂ are both R and chemical conjugation occurs at the N-terminus of the peptide.

In some embodiments, mutations in any one or more of the amino acid residues of a peptide of the present disclosure can improve binding affinity of the peptide to TfR. In some embodiments, mutations in 5-80% of amino acid residues of a peptide of the present disclosure improve the binding affinity of the peptide to TfR. In some embodiments, mutations in 1-100%, 5-100%, or 5-50% of amino acid residues of a peptide of the present disclosure improve binding affinity of the peptide to TfR. In some embodiments, mutations in 15-50% of amino acid residues of a peptide of the present disclosure improve binding affinity of the peptide to TfR. In some embodiments, mutations in 15-30% of amino acid residues of a peptide of the present disclosure improve binding affinity of the peptide to TfR. In some embodiments, mutations in 25-30% of amino acid residues of a peptide of the present disclosure improve binding affinity of the peptide to TfR. For example, mutations in 14 of the 51 amino acid residues (27.5%) of a peptide having a sequence of SEQ ID NO: 32 can improve binding affinity of the peptide to TfR.

In some embodiments, mutations in any one or more of the amino acid residues of a peptide of the present disclosure can lie at the binding interface of TfR. In some embodiments, a mutation to a peptide can improve binding affinity, which can be beneficial to binding and transcytosis of a peptide or peptide construct disclosed herein. In some embodiments, the peptides provided herein can have many mutations or few mutations to obtain optimal activity, wherein optimal activity is sufficient binding for engagement of the TfR, but not necessarily binding that is so strong as to preclude release of the peptide and/or peptide construct after transcytosis. Thus, peptides of the present disclosure can comprise a number of mutations (also referred to as % mutated amino acid residues) that tune binding affinity and off rate to obtain optimal binding, function (e.g., transcytosis, BBB-penetration, cell membrane penetration, transport across a biological barrier), and release of the peptide or peptide construct. Thus, mutations that result in the highest possible affinity may not necessarily correlate to a superior peptide having optimal binding and transcytosis.

In some embodiments, 1-100% or 5-100% of amino acid residues of a peptide of the present disclosure lie at the binding interface of TfR. In some embodiments, 10-90% of amino acid residues of a peptide of the present disclosure lie at the binding interface of TfR. In some embodiments, 20-80% of amino acid residues of a peptide of the present disclosure lie at the binding interface of TfR. In some embodiments, 30-70% of amino acid residues of a peptide of the present disclosure lie at the binding interface of TfR. In some embodiments, 40-60% of amino acid residues of a peptide of the present disclosure lie at the binding interface of TfR. In some embodiments, 30-35% of amino acid residues of a peptide of the present disclosure lie at the binding interface of TfR. For example, 17 of the 51 amino acid residues (33%) of a peptide having a sequence of SEQ ID NO: 32 can lie at the binding interface of TfR.

In some embodiments, mutations in any one or more of the amino acid residues of a peptide of the present disclosure that lie at the binding interface of TfR can improve binding affinity of the peptide to TfR. In some embodiments, mutations in 1-100% or 5-100% of amino acid residues of a peptide of the present disclosure that lie at the binding interface of TfR improve binding affinity of the peptide to TfR. In some embodiments, mutations in 5-80% of amino acid residues of a peptide of the present disclosure that lie at the binding interface of TfR improve binding affinity of the peptide to TfR. In some embodiments, mutations in 10-70% of amino acid residues of a peptide of the present disclosure that lie at the binding interface of TfR improve binding affinity of the peptide to TfR. In some embodiments, mutations in 15-60% of amino acid residues of a peptide of the present disclosure that lie at the binding interface of TfR improve binding affinity of the peptide to TfR. In some embodiments, mutations in 20-50% of amino acid residues of a peptide of the present disclosure that lie at the binding interface of TfR improve binding affinity of the peptide to TfR. In some embodiments, mutations in 25-30% of amino acid residues of a peptide of the present disclosure that lie at the binding interface of TfR improve binding affinity of the peptide to TfR. For example, mutations in 5 of the 17 amino acid residues (29%) of a peptide having a sequence of SEQ ID NO: 32 that lie at the binding interface of TfR and can improve binding affinity of the peptide to TfR.

In some embodiments, mutations in any one or more of the amino acid residues of a peptide of the present disclosure are distal to the binding interface of TfR. In some embodiments, 1-100% or 5-100% of amino acid residues of a peptide of the present disclosure are distal to the binding interface of TfR. In some embodiments, 10-90% of amino acid residues of a peptide of the present disclosure are distal to the binding interface of TfR. In some embodiments, 20-80% of amino acid residues of a peptide of the present disclosure are distal to the binding interface of TfR. In some embodiments, 30-70% of amino acid residues of a peptide of the present disclosure are distal to the binding interface of TfR. In some embodiments, 40-60% of amino acid residues of a peptide of the present disclosure are distal to the binding interface of TfR. In some embodiments, 65-70% of amino acid residues of a peptide of the present disclosure are distal to the binding interface of TfR. For example, 34 of the 51 amino acid residues (66%) of a peptide having a sequence of SEQ ID NO: 32 can lie at the binding interface of TfR.

In some embodiments, mutations in any one or more of the amino acid residues of a peptide of the present disclosure are distal to the binding interface of TfR improve binding affinity of the peptide to TfR. In some embodiments, mutations in 1-100% or 5-100% of amino acid residues of a peptide of the present disclosure that are distal to the binding interface of TfR improve binding affinity of the peptide to TfR. In some embodiments, mutations in 5-80% of amino acid residues of a peptide of the present disclosure that are distal to the binding interface of TfR improve binding affinity of the peptide to TfR. In some embodiments, mutations in 10-70% of amino acid residues of a peptide of the present disclosure that are distal to the binding interface of TfR improve binding affinity of the peptide to TfR. In some embodiments, mutations in 15-60% of amino acid residues of a peptide of the present disclosure that are distal to the binding interface of TfR improve binding affinity of the peptide to TfR. In some embodiments, mutations in 20-50% of amino acid residues of a peptide of the present disclosure that are distal to the binding interface of TfR improve binding affinity of the peptide to TfR. In some embodiments, mutations in 25-30% of amino acid residues of a peptide of the present disclosure that are distal to the binding interface of TfR improve binding affinity of the peptide to TfR. For example, mutations in 5 of the 17 amino acid residues that are distal to the binding interface of TfR can improve binding affinity of the peptide to TfR. For example, mutations in 9 of the 34 amino acid residues (26.5%) of a peptide having a sequence of SEQ ID NO: 32 that are distal to the binding interface of TfR can improve binding affinity of the peptide to TfR. In some embodiments, and without being bound to any theory, one or more mutations in the amino acid residues of the peptide that are distal to the binding interface of TfR can improve protein folding, enhance protein solubility, and/or alter the backbone geometry that can improve binding through an optimized interface shape complementarity.

In some embodiments, a peptide of the present disclosure can comprise a sequence having cysteine residues at one or more of positions 11, 12, 13, 14, 19, 20, 21, 22, 36, 38, 39, 41. In some embodiments, a peptide comprises Cys at positions 11, 12, 19, 20, 36, 39, or any combination thereof. For example, in certain embodiments, a peptide can comprise a sequence having a cysteine residue at position 11. In certain embodiments, a peptide can comprise a sequence having a cysteine residue at position 12. In certain embodiments, a peptide can comprise a sequence having a cysteine residue at position 13. In certain embodiments, a peptide can comprise a sequence having a cysteine residue at position 14. In certain embodiments, a peptide can comprise a sequence having a cysteine residue at position 19. In certain embodiments, a peptide can comprise a sequence having a cysteine residue at position 20. In certain embodiments, a peptide can comprise a sequence having a cysteine residue at position 21. In certain embodiments, a peptide can comprise a sequence having a cysteine residue at position 22. In certain embodiments, a peptide can comprise a sequence having a cysteine residue at position 36. In certain embodiments, a peptide can comprise a sequence having a cysteine residue at position 38. In certain embodiments, a peptide can comprise a sequence having a cysteine residue at position 39. In certain embodiments, a peptide can comprise a sequence having a cysteine residue at position 41. In some embodiments, the first cysteine residue in the sequence can be disulfide bonded with the 4th cysteine residue in the sequence, the 2nd cysteine residue in the sequence can be disulfide bonded to the 5th cysteine residue in the sequence, and the 3rd cysteine residue in the sequence can be disulfide bonded to the 6th cysteine residue in the sequence. Optionally, a peptide can comprise one disulfide bridge that passes through a ring formed by two other disulfide bridges, also known as a "two-and-through" structure system. In some embodiments, the peptides disclosed herein can have one or more cysteines mutated to serine.

In some embodiments, peptides of the present disclosure comprise at least one cysteine residue. In some embodiments, peptides of the present disclosure comprise at least two cysteine residues. In some embodiments, peptides of the present disclosure comprise at least three cysteine residues. In some embodiments, peptides of the present disclosure comprise at least four cysteine residues. In some embodiments, peptides of the present disclosure comprise at least five cysteine residues. In some embodiments, peptides of the present disclosure comprise at least six cysteine residues. In some embodiments, peptides of the present disclosure comprise at least ten cysteine residues. In some embodiments, a peptide of the present disclosure comprises six cysteine residues.

In some embodiments, a peptide of the present disclosure comprises an amino acid sequence having cysteine residues at one or more positions. In some embodiments, the one or more cysteine residues are located at any one of the amino acid positions 6, 10, 20, 34, 44, 48, or any combination thereof. In some aspects of the present disclosure, the one or more cysteine (C) residues participate in disulfide bonds with various pairing patterns (e.g., C10-C20). In some embodiments, the pairing patterns are C6-C48, C10-C44, and C20-C34. In some embodiments, the peptides as described herein comprise at least one, at least two, or at least three disulfide bonds. In some embodiments, at least one, at least two, or at least three disulfide bonds are arranges according to the C6-C48, C10-C44, and C20-C34 pairing patterns, or a combination thereof. In some embodiments, peptides as described herein comprise three disulfide bonds with the pairing patterns C6-C48, C10-C44, and C20-C34.

In certain embodiments, a peptide comprises a sequence having a cysteine residue at position 6. In certain embodiments, a peptide comprises a sequence having a cysteine residue at position 10. In certain embodiments, a peptide comprises a sequence having a cysteine residue at position 20. In certain embodiments, a peptide comprises a sequence having a cysteine residue at position 34. In certain embodiments, a peptide comprises a sequence having a cysteine residue at position 44. In certain embodiments, a peptide comprises a sequence having a cysteine residue at position 50. In some embodiments, the first cysteine residue in the sequence is disulfide bonded with the last cysteine residue in the sequence. In some embodiments, the second cysteine residue in the sequence is disulfide bonded with the second to the last cysteine residue in the sequence. In some embodiments, the third cysteine residue in the sequence is disulfide bonded with the third to the last cysteine residue in the sequence and so forth.

In some embodiments, the first cysteine residue in the sequence is disulfide bonded with the 6th cysteine residue in the sequence, the 2nd cysteine residue in the sequence is disulfide bonded to the 5th cysteine residue in the sequence, and the 3rd cysteine residue in the sequence is disulfide bonded to the 4th cysteine residue in the sequence. Optionally, a peptide can comprise one disulfide bridge that passes through a ring formed by two other disulfide bridges, also known as a "two-and-through" structure system. In some embodiments, the peptides disclosed herein have one or more cysteines mutated to serine.

In some embodiments, a peptide comprises no cysteine or disulfides. In some embodiments, a peptide comprises 1 or more, 2 or more, 3 or more, 4 or more, 5 or more, 6 or more, 7 or more, 8 or more, 9 or more, 10 or more, 11 or more, 12 or more, 13 or more, 14 or more, or 15 or more cysteine or disulfides. In other embodiments, 1 or more, 2 or more, 3 or more, 4 or more, 5 or more, 6 or more, 7 or more, 8 or more, 9 or more, 10 or more cysteine residues have been replaced with serine residues. In some embodiments, 1 or more, 2 or more, 3 or more, 4 or more, 5 or more, 6 or more, 7 or more, 8 or more, 9 or more, 10 or more cysteine residues have been replaced with threonine residues.

In some embodiments, a peptide comprises no Cys or disulfides. In some embodiments, a peptide comprises 1 or more, 2 or more, 3 or more, 4 or more, 5 or more, 6 or more, 7 or more, 8 or more, 9 or more, 10 or more, 11 or more, 12 or more, 13 or more, 14 or more, or 15 or more Cys or disulfides. In other embodiments, 1 or more, 2 or more, 3 or more, 4 or more, 5 or more, 6 or more, 7 or more, 8 or more, 9 or more, 10 or more Cys residues have been replaced with Ser residues. In some embodiments, 1 or more, 2 or more, 3 or more, 4 or more, 5 or more, 6 or more, 7 or more, 8 or more, 9 or more, 10 or more Cys residues have been replaced with Thr residues.

In some instances, one or more or all of the methionine residues in the peptide are replaced by leucine or isoleucine. In some instances, one or more or all of the tryptophan residues in the peptide are replaced by phenylalanine or tyrosine. In some instances, one or more or all of the asparagine residues in the peptide are replaced by glutamine. In some embodiments, the N-terminus of the peptide is blocked, such as by an acetyl group. Alternatively or in combination, in some instances, the C-terminus of the peptide is blocked, such as by an amide group. In some embodiments, the peptide is modified by methylation on free amines.

For example, full methylation can be accomplished through the use of reductive methylation with formaldehyde and sodium cyanoborohydride.

In some embodiments, the peptides or peptide constructs as described herein target and/or penetrate a TfR-expressing cellular layer or barrier and/or the membrane of a TfR-expressing cell. In some embodiments, a peptide targets and/or penetrates a cell membrane of a cell, wherein said cell is located in the CNS such as the brain. For example, a peptide construct comprising a TfR-binding peptide and one or more active agents (e.g., a therapeutic or diagnostic compound) crosses a cellular barrier (e.g., BBB) via vesicular transcytosis, and subsequently targets and/or penetrates the cell membrane of a cell located within the CNS to deliver said one or more active agents to that cell.

In various embodiments, a peptide construct comprising a TfR-binding peptide as described herein and one or more active agents (e.g., a therapeutic or diagnostic compound) targets and/or penetrates the cell membrane of a TfR-expressing cell located in the gastrointestinal tract, spleen, liver, kidney, muscle, bone marrow, brain, or skin. In some cases, the TfR-expressing cell is a tumor cell, an immune cell, an erythrocyte, an erythrocyte precursor cell, a stem cell, a bone marrow cell, or stem cell. In some cases, the TfR-binding peptide is responsible for targeting the cell, e.g., in cases where the cell is overexpressing a TfR. In various embodiments, a peptide construct as described herein comprising a TfR-binding peptide conjugated to, linked to, or fused to one or more cargo molecules (e.g., an active and/or detectable agent) targets and/or penetrates the cell membrane of a cell located within various organs such as the spleen, brain, liver, kidney, muscle, bone marrow, gastrointestinal tract, or skin.

In some cases, the cargo molecule promotes the targeting of a specific cell, cell population, or tissue. In some cases, it is a combination of TfR-mediated cell targeting and cargo molecule promoted cell targeting. In some aspects, a peptide or peptide construct (e.g., peptide conjugate or fusion peptide) of the present disclosure is used to target said cell, cell population, or tissue in order to exert a certain biological (e.g., therapeutic) effect. In some aspects, a peptide or peptide construct (e.g., peptide conjugate or fusion peptide) of the present disclosure is used to deliver the cargo molecule into said cell to exert a certain biological effect.

In some embodiments, peptides can comprise at least one or more tag peptide sequences for improved cell penetration. For example, peptides can comprise at least one or multiple Arg residues or residues from Tat protein for improved cell penetration property. Additional tag peptide sequences can include CysTat (CYRKKRRQRRR; SEQ ID NO: 71), S19-TAT (PFVIGAGVLGALGTGIGGIGRKKRRQRRR; SEQ ID NO: 72), R8 (RRRRRRRR; SEQ ID NO: 73), pAntp (RQIKIWFQNRRMKWKK; SEQ ID NO: 74), Pas-TAT (FFLIPKGGRKKRRQRRR; SEQ ID NO: 75), Pas-R8 (FFLIPKGRRRRRRRR; SEQ ID NO: 76), PasFHV (FFLIPKGRRRRNRTRRNRRRVR; SEQ ID NO: 77), Pas-pAntP (FFLIPKGRQIKIWFQNRRMKWKK; SEQ ID NO: 78), F2R4 (FFRRRR; SEQ ID NO: 79), B55 (KAVLGATKIDLPVDINDPYDLGLLLRHLRHHSNLLANIGDPAVREQVLSAMQEEE; SEQ ID NO: 80), auzurin (LSTAADMQGVVTDGMASGLDKDYLKPDD; SEQ ID NO: 81), IMT-P8 (RRWRRWNRFNRRRCR; SEQ ID NO: 82), BR2 (RAGLQFPVGRLLRRLLR; SEQ ID NO: 83), OMOTAG1 (KRAHHNALERKRR; SEQ ID NO: 84), OMOTAG2 (RRMKANARERNRM; SEQ ID NO: 85), pVEC (LLIILRRRIRKQAHAHSK; SEQ ID NO: 86), SynB3 (RRLSYSRRRF; SEQ ID NO: 87), DPV1047 (VKRGLKLRHVRPRVTRMDV; SEQ ID NO: 88), CY105Y (CSIPPEVKFNKPFVYLI; SEQ ID NO: 89), Transportan (GWTLNSAGYLLGKINLKALAALAKKIL; SEQ ID NO: 90), MTS (KGEGAAVLLPVLLAAPG; SEQ ID NO: 91), hLF (KCFQWQRNMRKVRGPPVSCIKR; SEQ ID NO: 92), PFVYLI (PFVYLI; SEQ ID NO: 93), yBBR (VLDSLEFIASKL, SEQ ID NO: 94), DRI-TAT31 (rrrqrrkkrgy, wherein the lowercase notation indicates D-amino acids; SEQ ID NO: 95), cyclic heptapeptide cyclo (cFΦR₄) (FΦRRRRQ, where Φ is l-2-naphthylalanine, the entire moiety is cyclized, and Gln serves as a conjugation handle and can be substituted for other functional groups such as Lys (for amine coupling) or Cys (for sulfhydryl coupling); SEQ ID NO: 96), DPV3 (RKKRRRESRKKRRRES; SEQ ID NO: 403), C10H (RKGFYKRKQCKPSRGRKR; SEQ ID NO: 404), VP22 (NAATATRGRSAASRPTQRPRAPARSASRPRRPVQ; SEQ ID NO: 405), TP10 (AGYLLGKINLKALAALAKKIL; SEQ ID NO: 406), MAP (KLALKLALKALKAALKLA; SEQ ID NO: 407), BPrPp (MVKSKIGSWILVLFVAMWSDVGLCKKRP; SEQ ID NO: 408), ARF (MVRRFLVTLRIRRACGPPRVRV; SEQ ID NO: 409), GALA (WEAALAEALAEALAEHLAEALAEALEALAA; SEQ ID NO: 410), SAP (VRLPPPVRLPPPVRLPPP; SEQ ID NO: 411), MPG (GLAFLGFLGAAGSTMGAWSQPKKKKRKV; SEQ ID NO: 412), Pep-1 (KETWWETWWTEWSQPKKKRKV; SEQ ID NO: 413), [WR]4 (WRWRWRWR; SEQ ID NO: 414), Ig(v) (MGLGLHLLVLAAALQGAKKKRKV; SEQ ID NO: 415), K-FGF (AAVALLPAVLLAHLLAP; SEQ ID NO: 416), Melittin (GIGAVLKVLTTGLPALISWIKRKRQQ; SEQ ID NO: 417), gH625 (HGLASTLTRWAHYNALIRAF; SEQ ID NO: 418), HIV-1 TAT protein (48-60) (GRKKRRQRRRPPQ; SEQ ID NO: 419), MPG HIV-gp41/SV40 T-antigen (GALFLGFLGAAGSTMGAWSQPKKKRKV; SEQ ID NO: 420), R6W3 (RRWWRRWRR; SEQ ID NO: 421), NLS (CGYGPKKKRKVGG; SEQ ID NO: 422), 8-lysines (KKKKKKKK; SEQ ID NO: 423), HRSV (RRIPNRRPRR; SEQ ID NO: 424), AIP6 (RLRWR; SEQ ID NO: 425), Pep-1 (KETWWETWWTEWSQPKKRKV; SEQ ID NO: 426), MAP17 (QLALQLALQALQAALQLA; SEQ ID NO: 427), VT5 (DPKGDPKGVTVTVTVTVTGKGDPKPD; SEQ ID NO: 428), Bac7 (RRIRPRPPRLPRPRPRPLPFPRPG; SEQ ID NO: 429), (PPR)n ((PPRPPRPPR; SEQ ID NO: 430), (PPRPPRPPRPPR; SEQ ID NO: 431), (PPRPPRPPRPPRPPR; SEQ ID NO: 432), (PPRPPRPPRPPRPPRPPR; SEQ ID NO: 433)), INF7 (GLFEAIEGFIENGWEGMIDGWYGC; SEQ ID NO: 434), CADY (GLWRALWRLLRSLWRLLWRA; SEQ ID NO: 435), Pep-7 (SDLWEMMMVSLACQY; SEQ ID NO: 436), TGN (TGNYKALHPHNG ; SEQ ID NO: 437), Ku-70 (VPMLK ; SEQ ID NO: 438), CPP (RRRRRGGRRRRRG; SEQ ID NO: 452) (RRRRRRGGRRRRRG ; SEQ ID NO: 439), SVS-1 (KVKVKVKVDPPTKVKVKVK ; SEQ ID NO: 440), L-CPP (LAGRRRRRRRRRK; SEQ ID NO: 441), RLW (RLWMRWYSPRTRAYG; SEQ ID NO: 442), K16ApoE (KKKKKKKKKKKKKKKKLRVRLASHLRKLRKRLLRDA ; SEQ ID NO: 443), Angiopep-2 (TFFYGGSRGKRNNFKTEEY; SEQ ID NO: 444), ACPP (EEEEEEEEPLGLAGRRRRRRRRN; SEQ ID NO: 445), KAFAK (KAFAKLAARLYRKALARQLGVAA; SEQ ID NO: 446), hCT (9-32) (LGTYTQDFNKFHTFPQTAIGVGAP; SEQ ID NO: 447), VP22(version2) (DAATATRGRSAASRPTQRPRAPARSASRPRRPVE; SEQ ID NO: 448), MPG (GALFLGFLGAAGSTMGAWSQPKSKRKV ; SEQ ID NO: 449), hPP3 (KPKRKRRKKKGHGWSR; SEQ ID NO: 450), PepNeg (SGTQEEY; SEQ ID NO: 451), CM18-TAT (KWKLFKKIGAVLKVLTTG; SEQ ID NO: 453), PTD4 (YARAAARQARA; SEQ ID NO: 454), or WaTx (MKYFTLALTLLFLLLINPCKDMNFAWAESSEKVERASPQQAKYCYEQCNVNKVPFDQC YQMCSPLERS; SEQ ID NO: 455). For example, in some embodiments, the peptide can comprise an Arginine patch (Arg patch), for example, an RRRRRRRR (SEQ ID NO: 73), or a variant or fragment thereof, sequence can be appended to either the N-terminus or the C-terminus of a peptide. In some embodiments, the Arg patch comprises two or more Arg residues, or Argₙ wherein n is a whole number and can be 2, 3, 4, 5, 6, 7, 8, 9, or 10 (SEQ ID NO: 98). In other embodiments, the peptide can comprise a Tat peptide (Tat proteins are reviewed in Gump et al. TAT transduction: the molecular mechanism and therapeutic prospects. Trends Mol Med. 2007 Oct;13(10):443-8 and Harada et al. Antitumor protein therapy; application of the protein transduction domain to the development of a protein drug for cancer treatment. Breast Cancer. 2006;13(1):16-26). The Tat peptide can have a sequence of, for example, YGRKKRRQRRR (SEQ ID NO: 99), GRKKRRQRRR (SEQ ID NO: 100), or any modification, variant, or fragment thereof, can be appended to the N-terminus or C-terminus of any TfR-binding peptide of the present disclosure. In some embodiments, the Tat peptide sequence can be GRKKRRQRRRPQ (SEQ ID NO: 101), GRKKRRQRRR (SEQ ID NO: 100), or a fragment or variant thereof. In some embodiments, the Tat peptide can be appended to the N-terminus of any TfR-binding peptide of the present disclosure following an N-terminal GS dipeptide and preceding, for example, a GGGS (SEQ ID NO: 103) spacer. In some embodiments, a cell-penetrating tag peptides, such as any one of SEQ ID NO: 71 - SEQ ID NO: 96, SEQ ID NO: 98 - SEQ ID NO: 101, SEQ ID NO: 116 - SEQ ID NO: 124, SEQ ID NO: 126, or SEQ ID NO: 403 - SEQ ID NO: 455 can be appended to either the N-terminus or C-terminus of any peptide disclosed herein using a peptide linker such as GₓS_{y} (SEQ ID NO: 104) peptide linker, wherein x and y can be any whole number, such as 1, 2, 3, 4, 5, 6, 7, 8, 9, and 10. In other embodiments, a cell-penetrating peptide, such as a Tat peptide or an Arg patch, or any other moiety, can be appended to either the N-terminus or C-terminus of any peptide disclosed herein using a peptide linker such as GₓS_{y} (SEQ ID NO: 104) peptide linker, wherein x and y can be any whole number, such as 1, 2, 3, 4, 5, 6, 7, 8, 9, and 10. In some embodiments, the peptide linker comprises (GS)x (SEQ ID NO: 105), wherein x can be any whole number, such as 1, 2, 3, 4, 5, 6, 7, 8, 9, and 10. In some embodiments, the peptide linker comprises GGSSG (SEQ ID NO: 106), GGGGG (SEQ ID NO: 107), GSGSGSGS (SEQ ID NO: 108), GSGG (SEQ ID NO: 109), GGGGS (SEQ ID NO: 110), GGGS (SEQ ID NO: 103), GGS (SEQ ID NO: 112), GGGSGGGSGGGS (SEQ ID NO: 113), or a variant or fragment thereof. Additionally, KKYKPYVPVTTN (SEQ ID NO: 114) from DkTx, and EPKSSDKTHT (SEQ ID NO: 115) from human IgG3 can be used as a peptide linker. In other embodiments, the tag peptide can be appended to the peptide at any amino acid residue. In further embodiments, the tag peptide can be appended to the peptide at any amino acid residue without interfering with TfR-binding activity. In some embodiments, the tag peptide is appended via conjugation, linking, or fusion techniques. In other embodiments, the Tat peptide can be appended to the peptide at any amino acid residue. In further embodiments, the Tat peptide can be appended to the peptide at any amino acid residue without interfering with TfR-binding activity. In some embodiments, the Tat peptide is appended via conjugation, linking, or fusion techniques to a TfR-binding peptide to obtain a TfR-binding Cell Penetrating Peptide fusion (CPP fusion).

Cell-penetrating peptides include, but are not limited to, short amphipathic or cationic short peptides with a positive net charge and are capable of penetrating cellular membrane and transferring a molecular or cargo either covalently or non-covalently attached to the peptides into a cell. Such cell-penetrating peptides can be synthesized or derived from known proteins, such as penetratin, Tat peptide, pVEC, or chimeric peptides, such as transportan, MPG, Pep-1, or synthetic peptides, such as polyarginines, MAP, and R₆W₃.

In some embodiments, peptides can comprise at least one or more cell penetrating peptide sequences for improved cell penetration. For example, a cell penetrating peptide can include maurocaline (GDCLPHLKLCKENKDCCSKKCKRRGTNIEKRCR; SEQ ID NO: 116), imperatoxin (GDCLPHLKRCKADNDCCGKKCKRRGTNAEKRCR; SEQ ID NO: 117), hadrucalcin (SEKDCIKHLQRCRENKDCCSKKCSRRGTNPEKRCR; SEQ ID NO: 118), hemicalcin (GDCLPHLKLCKADKDCCSKKCKRRGTNPEKRCR; SEQ ID NO: 119), opicalcin-1 (GDCLPHLKRCKENNDCCSKKCKRRGTNPEKRCR; SEQ ID NO: 120), opicalcin-2 (GDCLPHLKRCKENNDCCSKKCKRRGANPEKRCR; SEQ ID NO: 121, midkine (62-104) (CKYKFENWGACDGGTGTKVRQGTLKKARYNAQCQETIRVTKPC; SEQ ID NO: 122), MCoTI-II (SGSDGGVCPKILKKCRRDSDCPGACICRGNGYCG; SEQ ID NO: 123), or chlorotoxin (MCMPCFTTDHQMARKCDDCCGGKGRGKCYGPQCLCR; SEQ ID NO: 124). In some embodiments, the cell penetrating peptide can have at least 80%, 90%, 95%, or 99% sequence identity with any sequence of SEQ ID NO: 71 - SEQ ID NO: 96, SEQ ID NO: 98 - SEQ ID NO: 101, SEQ ID NO: 116 - SEQ ID NO: 124, SEQ ID NO: 126, or SEQ ID NO: 403 - SEQ ID NO: 455.

In some embodiments, a peptide is conjugated to, linked to, or fused to one or more cell-penetrating peptides, such as arginine-rich, amphipathic and lysine-rich, and hydrophobic residues or peptides capable of penetrating plasma membrane or nucleus for in vivo delivery of a protein or macromolecular cargo. Conjugation or fusion can be direct or with a spacer in between (chemical or peptide-based). A spacer can be any peptide linker. For example, a spacer can be GGGSGGSGGGS (SEQ ID NO: 125), KKYKPYVPVTTN (SEQ ID NO: 114) from DkTx, EPKSSDKTHT (SEQ ID NO: 115) from human IgG3 or any variant or fragment thereof. In some embodiments, the cell penetrating peptide sequence can be appended to either the N-terminus or the C-terminus of a peptide. In some embodiments, the cell penetrating peptide can be appended to the N-terminus of any TfR-binding peptide of the present disclosure following an N-terminal GS dipeptide and preceding, for example, a GGGS (SEQ ID NO: 103) spacer. In some embodiments, a cell-penetrating tag peptide, such as any one of SEQ ID NO: 71 - SEQ ID NO: 96, SEQ ID NO: 98 - SEQ ID NO: 101, SEQ ID NO: 116 - SEQ ID NO: 124, SEQ ID NO: 126, or SEQ ID NO: 403 - SEQ ID NO: 455, can be appended to either the N-terminus or C-terminus of any peptide disclosed herein using a peptide linker such as GₓS_{y} (SEQ ID NO: 104) peptide linker, wherein x and y can be any whole number, such as 1, 2, 3, 4, 5, 6, 7, 8, 9, and 10. In some embodiments, the peptide linker comprises GGSSG (SEQ ID NO: 106), GGGGG (SEQ ID NO: 107), GSGSGSGS (SEQ ID NO: 108), GSGG (SEQ ID NO: 109), GGGGS (SEQ ID NO: 110), GGGS (SEQ ID NO: 103), GGS (SEQ ID NO: 112), GGGSGGGSGGGS (SEQ ID NO: 113), GGGSGGSGGGS (SEQ ID NO: 225), or a variant or fragment thereof. Additionally, KKYKPYVPVTTN (SEQ ID NO: 114) from DkTx, and EPKSSDKTHT (SEQ ID NO: 115) from human IgG3 can be used as a peptide linker. In other embodiments, the cell penetrating peptide can be appended to the peptide at any amino acid residue. In further embodiments, the cell penetrating peptide can be appended to the peptide at any amino acid residue without interfering with TfR-binding activity. In some embodiments, the cell penetrating peptide is appended via conjugation, linking, or fusion techniques. In other embodiments, the cell penetrating peptide can be appended to the peptide at any amino acid residue.

In other embodiments, cell penetration can be increased by using high dosage of a peptide described here, such as up to 10 µM, or 10 µM or more of the peptide. In some cases, an Arg patch can be fused, conjugated to, linked to, or co-delivered with a peptide. Up 10 µM, or 10 µM or more Arg patch can be co-delivered with a peptide to facilitate cell penetration. Protein transfection agents can also be used to increase cell penetration of a peptide. In some embodiments, direct cytosolic expression of a peptide can be used. In other embodiments, physical disruption methods such as electroporation can be used to improve delivery of a peptide into a cell.

In some embodiments, cell penetrance of a peptide described herein can be improved. For example, the binding interface of a peptide described herein can be grafted on a scaffold that is known to be cell-penetrant, such as a calcine. Similarly, sequences that are known to be cell penetrant can be grafted onto peptides of this disclosure. Some non-limiting examples of calcines can be imperatoxin-A, maurocalcine, hemicalcin, opiclacin 1, opicalcin 2, and hadrucalcin. The scaffold can comprise at least 60%, 70%, 80%, 90%, 95%, or 98% with any one of SEQ ID NO: 116 - SEQ ID NO: 124. In some embodiments, the cell penetrance peptide can be calcines, modified calcines, derivatives of calcines, or fragments thereof, which can be used to increase cell penetration. Modified calcines, derivatives of calcines, or fragments can be screened for cell penetration activity such as activation of sarcoplasmic reticulum ryanodine receptors, activity on ryanodine-sensitive Ca²⁺ channels RyR1, Ryr2, or both, or as a selective agonist of the foregoing. Moreover, modified calcines can include substitution, addition or reduction of Lysine residues, or other charged residues, within a calcine in order to modify activity and optimize such calcine cell-penetration activity or activity on the RyR1 or RyR2 receptors. As an example, the six amino acid portion of helix 3 (MLICLF; SEQ ID NO: 126) can be transplanted onto a calcine or modified calcine scaffold to produce a bi-functional peptide that retains the cell penetration of the calcine with the novel TfR-binding function of the TfR-binding peptides. As another example, a peptide as described herein can have improved cell penetrating capabilities using cis-acting elements, including inclusion of K/R-rich sequences like TAT or octa-arginine, intra-helical arginine patches, or fusion to larger fragments of proteins identified in cell penetration screening like penetratin or melittin.

In some embodiments, nuclear localization signals can be couple to, conjugated to, linked to, or fused to a peptide described herein to promote nuclear localization. In some embodiments, TfR-binding peptides are conjugated to, linked to, or fused to a nuclear localization signal, such as a four-residue sequence of K-K/R-X-K/R (SEQ ID NO: 129), wherein X can be any amino acid, or a variant thereof. In some embodiments, TfR-binding peptides are conjugated to, linked to, or fused to a nuclear localization signal as described in Lange et al, J Biol Chem. 2007 Feb 23;282(8):5101-5, such as PKKKRRV (SEQ ID NO: 130) or KRPAATKKAGQAKKKK (SEQ ID NO: 131). In some embodiments, a peptide described herein is conjugated to, linked to, or fused to a nuclear localization signal comprising KxRy (SEQ ID NO: 132), wherein x and y independently can be 0, 1, 2, 3, 4, 5, 6, 7, 8, 9, or 10, such as KKRR (SEQ ID NO: 133), KKKRR (SEQ ID NO: 134), or KKKK (SEQ ID NO: 135). Other cell penetrating moieties can also be linked to, conjugated to, linked to, or fused to the peptides described herein, including, but not limited to, polycations, polyorganic acids, endosomal releasing polymers, poly(2-propylacrylic acid), poly(2-ethylacrylic acid), or any combination thereof.

In some embodiments, the peptides of the present disclosure (e.g., histidine-containing or histidine-enriched TfR-binding peptides) can have a high TfR binding affinity at physiological pH but a significantly reduced binding affinity at lower pH levels such as endosomal pH of 5.4. In some cases, the TfR-binding peptides of the present disclosure can be optimized for improved intra-vesicular (e.g., intra-endosomal) and/or intracellular delivery function while retaining high TfR binding capabilities. In some cases, histidine scans and comparative binding experiments can be performed to develop and screen for such peptides. In addition, some peptides can comprise (e.g., conjugated to, linked to, or fused to) a motif that facilitates low-pH endosomal escape of the peptide or peptide construct for enhanced delivery functions (e.g., intracellular delivery of a therapeutic agent). In some embodiments, an amino acid residue in a peptide of the present disclosure is substituted with a different amino acid residue to alter a pH-dependent binding affinity to TfR. The amino acid substitution may increase a binding affinity at low pH, increase a binding affinity at high pH, decrease a binding affinity at low pH, decrease a binding affinity at high pH, or a combination thereof.

In some embodiments, peptides of the present disclosure are capable of vesicular transcytosis across a cell layer or cell barrier such as the BBB. In some embodiments, the peptides target and/or penetrate into a cell or a nucleus of a cell (e.g., a brain cell). Examples of cells or tissues that can be targeted include cells or tissues associated with a disease or condition such as cells or tissues of the CNS, brain cells, cancerous cells, and other cell types, wherein certain biological pathway can be dysregulated in said cells. Further examples of cells or tissues that can be targeted include cells or tissues of the spleen, liver, kidney, muscle, bone marrow, or skin. A cell that can be targeted with the peptides of the present disclosure can be a human cell, a mammalian cell, a human or mammalian cell line, a cancer cell line, a cell extracted from a subject, in vivo, or in vitro.

In some instances, a peptide as disclosed herein can contain only one lysine residue, or no lysine residues. In some instances, one or more or all of the lysine residues in the peptide are replaced with arginine residues. In some instances, one or more or all of the methionine residues in the peptide are replaced by leucine or isoleucine. One or more or all of the tryptophan residues in the peptide can be replaced by phenylalanine or tyrosine. In some instances, one or more or all of the asparagine residues in the peptide are replaced by glutamine. In some embodiments, one or more or all of the aspartic acid residues can be replaced by glutamic acid residues. In some instances, one or more or all of the lysine residues in the peptide are replaced by alanine or arginine. In some embodiments, the N-terminus of the peptide is blocked or protected, such as by an acetyl group or a tert-butyloxycarbonyl group. Alterly or in combination, the C-terminus of the peptide can be blocked or protected, such as by an amide group or by the formation of an ester (e.g., a butyl or a benzyl ester). In some embodiments, the peptide is modified by methylation on free amines. For example, full methylation is accomplished through the use of reductive methylation with formaldehyde and sodium cyanoborohydride.

In some embodiments, the dipeptide GS can be added as the first two N-terminal amino acids, as shown in SEQ ID NO: 1 - SEQ ID NO: 35, or SEQ ID NO: 136 - SEQ ID NO: 170, or such N-terminal dipeptide GS can be absent as shown in SEQ ID NO: 36 - SEQ ID NO: 70, or SEQ ID NO: 171 - SEQ ID NO: 205, or can be substituted by any other one or two amino acids. In some embodiments, the dipeptide GS is used as a linker or used to couple to a linker to form a peptide conjugate or fusion molecules such as a peptide construct. In some embodiments, the linker comprises a GₓS_{y} (SEQ ID NO: 104) peptide, wherein x and y independently are any whole number, such as 1, 2, 3, 4, 5, 6, 7, 8, 9, and 10. In some embodiments, the peptide linker comprises (GS)x (SEQ ID NO: 105), wherein x can be any whole number, such as 1, 2, 3, 4, 5, 6, 7, 8, 9, and 10. In some embodiments, the peptide linker comprises GGSSG (SEQ ID NO: 106), GGGGG (SEQ ID NO: 107), GSGSGSGS (SEQ ID NO: 108), GGGGS (SEQ ID NO: 110), GGGS (SEQ ID NO: 103), or a variant or fragment thereof.

A peptide as disclosed herein can be a fragment comprising a contiguous fragment
at least 29, at least 30, at least 31, at least 32, at least 33, at least 34, at least 35, at least 36 at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, at least 64, at least 65 residues long, wherein the peptide fragment is selected from any portion of the peptide. In some embodiments, the peptide sequence is flanked by additional amino acids. One or more additional amino acids, for example, confer a particular *in vivo* charge, isoelectric point, chemical conjugation site, stability, or physiologic property to a peptide.

In some instances, the peptides as described herein that are capable of targeting and binding to a TfR comprise no more than 80 amino acids in length, or no more than 70, no more than 60, no more than 40, no more than 35, no more than 30, no more than 25, no more than 20, no more than 15, or no more than 10 amino acids in length.

In other embodiments, peptides can be conjugated to, linked to, or fused to a carrier or a molecule with targeting or homing function for a cell of interest or a target cell. In other embodiments, peptides can be conjugated to, linked to, or fused to a molecule that extends half-life or modifies the pharmacodynamic and/or pharmacokinetic properties of the peptides, or any combination thereof.

In some instances, a peptide comprises at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, or at least 20 positively charged residues, such as Arg or Lys, or any combination thereof. In some instances, one or more lysine residues in the peptide are replaced with arginine residues. In some embodiments, peptides comprise one or more Arg patches. In some embodiments, an Arg patch is positioned in the N-terminus of a peptide. In other aspects, an Arg patch is positioned in the C-terminus of a peptide. In some embodiments, an Arg patch comprises 8 consecutive Arg residues (SEQ ID NO: 73). In some embodiments, 1 or more, 2 or more, 3 or more, 4 or more, 5 or more, 6 or more, 7 or more, 8 or more, 9 or more, or 10 or more Arg or Lys residues are solvent exposed on a peptide. In some embodiments, an Arg patch can be two or more consecutive Arg residues. In some embodiments, an Arg patch comprises one or more Arg substituted with Lys.

The peptides of the present disclosure can further comprise neutral amino acid residues. In some embodiments, the peptide has 35 or fewer neutral amino acid residues. In other embodiments, the peptide has 81 or fewer neutral amino acid residues, 70 or fewer neutral amino acid residues, 60 or fewer neutral amino acid residues, 50 or fewer neutral amino acid residues, 40 or fewer neutral amino acid residues, 36 or fewer neutral amino acid residues, 33 or fewer neutral amino acid residues, 30 or fewer neutral amino acid residues, 25 or fewer neutral amino acid residues, or 10 or fewer neutral amino acid residues.

The peptides of the present disclosure can further comprise negative amino acid residues. In some embodiments the peptide has 6 or fewer negative amino acid residues, 5 or fewer negative amino acid residues, 4 or fewer negative amino acid residues, 3 or fewer negative amino acid residues, 2 or fewer negative amino acid residues, or 1 or fewer negative amino acid residues. While negative amino acid residues can be selected from any negatively charged amino acid residues, in some embodiments, the negative amino acid residues are either E, or D, or a combination of both E and D.

In some embodiments of the present disclosure, a three-dimensional or tertiary structure of a peptide is primarily comprised of beta-sheets and/or alpha-helix structures. In some embodiments, designed or engineered TfR-binding peptides of the present disclosure are small, compact peptides or polypeptides stabilized by intra-chain disulfide bonds (e.g., mediated by cysteines) to form cystine and a hydrophobic core. In some embodiments, engineered TfR-binding peptides have structures comprising helical bundles with at least one disulfide bridge between each of the alpha helices, thereby stabilizing the peptides. In other embodiments, the engineered TfR-binding peptides comprise structures with three alpha helices and three intra-chain disulfide bonds, one between each of the three alpha helices in the bundle of alpha helices.

In other embodiments, peptides can be conjugated to, linked to, or fused to a molecule (e.g., small molecule, peptide, or protein) with targeting or homing function for a cell of interest or a target protein located on the surface or inside said cell. In other embodiments, peptides can be conjugated to, linked to, or fused to a molecule that extends the plasma and/or biological half-life, or modifies the pharmacodynamic (e.g., enhanced binding to a target protein) and/or pharmacokinetic properties (e.g., rate and mode of clearance) of the peptides, or any combination thereof.

Generally, the nuclear magnetic resonance (NMR) solution structures or X-ray crystallography structures of related structural homologs can be used to inform mutational strategies that can improve the folding, stability, and manufacturability of the peptides as described herein, while maintaining a particular biological function (e.g., binding to TfR). These techniques can be used to predict the 3D pharmacophore of a group of structurally homologous scaffolds, as wells as to predict possible graft regions of related proteins to create chimeras with improved properties (e.g., binding properties). For example, this strategy is used to identify critical amino acid positions and loops that are used to design peptides with improved TfR receptor binding and transcytosis properties, high expression, high stability *in vivo,* or any combination of these properties.

Two or more peptides can share a degree of sequence identity or homology and share similar properties *in vivo.* For instance, a peptide can share a degree of sequence identity or homology with any one of the peptides of SEQ ID NO: 1 - SEQ ID NO: 32, SEQ ID NO: 36 - SEQ ID NO: 67, SEQ ID NO: 136 - SEQ ID NO: 167, or SEQ ID NO: 171 - SEQ ID NO: 202. In some embodiments, one or more peptides of the present disclosure have up to about 20% pairwise sequence identity or homology, up to about 25% pairwise sequence identity or homology, up to about 30% pairwise sequence identity or homology, up to about 35% pairwise sequence identity or homology, up to about 40% pairwise sequence identity or homology, up to about 45% pairwise sequence identity or homology, up to about 50% pairwise sequence identity or homology, up to about 55% pairwise sequence identity or homology, up to about 60% pairwise sequence identity or homology, up to about 65% pairwise sequence identity or homology, up to about 70% pairwise sequence identity or homology, up to about 75% pairwise sequence identity or homology, up to about 80% pairwise sequence identity or homology, up to about 85% pairwise sequence identity or homology, up to about 90% pairwise sequence identity or homology, up to about 95% pairwise sequence identity or homology, up to about 96% pairwise sequence identity or homology, up to about 97% pairwise sequence identity or homology, up to about 98% pairwise sequence identity or homology, up to about 99% pairwise sequence identity or homology, up to about 99.5% pairwise sequence identity or homology, or up to about 99.9% pairwise sequence identity or homology. In some embodiments, one or more peptides of the disclosure have at least about 20% pairwise sequence identity or homology, at least about 25% pairwise sequence identity or homology, at least about 30% pairwise sequence identity or homology, at least about 35% pairwise sequence identity or homology, at least about 40% pairwise sequence identity or homology, at least about 45% pairwise sequence identity or homology, at least about 50% pairwise sequence identity or homology, at least about 55% pairwise sequence identity or homology, at least about 60% pairwise sequence identity or homology, at least about 65% pairwise sequence identity or homology, at least about 70% pairwise sequence identity or homology, at least about 75% pairwise sequence identity or homology, at least about 80% pairwise sequence identity or homology, at least about 85% pairwise sequence identity or homology, at least about 90% pairwise sequence identity or homology, at least about 95% pairwise sequence identity or homology, at least about 96% pairwise sequence identity or homology, at least about 97% pairwise sequence identity or homology, at least about 98% pairwise sequence identity or homology, at least about 99% pairwise sequence identity or homology, at least about 99.5% pairwise sequence identity or homology, at least about 99.9% pairwise sequence identity or homology with a second peptide.

In some embodiments, peptides that exhibit an improved TfR receptor binding show improved transcytosis function. In some cases, peptides that exhibit an improved TfR receptor binding show no or small changes in transcytosis function. In some cases, peptides that exhibit an improved TfR receptor binding show reduced transcytosis function. In some embodiments, the K_{A} and K_{D} values of a TfR-binding peptide can be modulated and optimized (e.g., via amino acid substitutions) to provide an optimal ratio of TfR-binding affinity and efficient transcytosis function.

Various methods and software programs can be used to determine the homology between two or more peptides, such as NCBI BLAST, Clustal W, MAFFT, Clustal Omega, AlignMe, Praline, or another suitable method or algorithm. Pairwise sequence alignment can be used to identify regions of similarity that can indicate functional, structural and/or evolutionary relationships between two biological sequences (e.g., amino acid or nucleic acid sequences). In addition, multiple sequence alignment (MSA) is the alignment of three or more biological sequences. From the output of MSA applications, homology can be inferred and the evolutionary relationship between the sequences assessed. As used herein, "sequence homology" and "sequence identity" and "percent (%) sequence identity" and "percent (%) sequence homology" are used interchangeably to mean the sequence relatedness or variation, as appropriate, to a reference polynucleotide or amino acid sequence.

In still other instances, the nucleic acid molecules that encode a peptide or peptide construct of the claims
can be identified by either a determination of the sequence identity or homology of the encoded peptide amino acid sequence with the amino acid sequence of the claims
, or by a nucleic acid hybridization assay. Such peptide variants or peptide construct variants
can be characterized as nucleic acid molecules (1) that remain hybridized with a nucleic acid molecule having the nucleotide sequence
under highly stringent washing conditions, in which the wash stringency is equivalent to 0.1×-0.2×SSC with 0.1% SDS at 50-65° C., and (2) that encode a peptide having at least 70%, at least 80%, at least 90%, at least 95% or greater than 95% sequence identity or homology to the amino acid sequence of the claims.

### Knotted Peptides

In some embodiments, TfR-binding peptides of the present disclosure comprise one or more Cys, or one or more disulfide bond. In some embodiments, the TfR-binding peptides are derived from cystine-dense peptides (CDPs), knotted peptides, or hitchins. As used herein, the term "peptide" is considered to be interchangeable with the terms "knotted peptide", "cystine-dense peptide", "CDP", and "hitchin". (See e.g., Correnti et al. Screening, large-scale production, and structure-based classification for cystine-dense peptides. Nat Struct Mol Biol. 2018 Mar; 25(3): 270-278).

In some embodiments, the TfR-binding peptides of the present disclosure can bind TfR, thereby preventing TfR interactions such as interactions of TfR with other exogenous or endogenous ligands (e.g., Tf or homologs or fragments thereof). In some embodiments, the TfR-binding peptides of the present disclosure can bind TfR without impacting the binding of other exogenous or endogenous ligands (e.g., Tf or homologs or fragments thereof) with TfR. In some embodiments, TfR-binding peptides may be engineered peptides. An engineered peptide may be a peptide that is non-naturally occurring, artificial, isolated, synthetic, designed, or recombinantly expressed. In some embodiments, the TfR-binding peptides of the present disclosure comprise one or more properties of CDPs, knotted peptides, or hitchins, such as stability, resistance to proteolysis, resistance to reducing conditions, and/or ability to cross the blood brain barrier.

In some embodiments, CDPs or knotted peptides, including engineered, non-naturally occurring CDPs and those found in nature, can be conjugated to, linked to, or fused to the TfR-binding peptides of the present disclosure, such as those described in **TABLE 1,** to provide additional homing or targeting function to a target cell, such as a cancer cell, pancreatic cell, liver cell, colon cell, ovarian cell, breast cell, lung cell, or any combination thereof. In some embodiments, CDPs or knotted peptides, including engineered CDPs, isolated CDPs, and CDPs found in nature, can be conjugated to, linked to, or fused to the TfR-binding peptides of the present disclosure, such as those described in **TABLE 1,** to provide additional homing or targeting function to a target cell, such as a cancer cell, pancreatic cell, liver cell, colon cell, ovarian cell, breast cell, lung cell, or any combination thereof. An engineered peptide may be a peptide that is non-naturally occurring, artificial, synthetic, designed, or recombinantly expressed. In some embodiments, a TfR-binding peptide of the present disclosure enables TfR-mediated transcytosis and/or cellular endocytosis, and the additional CDP or knotted peptide that is conjugated to, linked to, or fused to TfR-binding peptide can selectively target an enzyme or other protein of interest in a cell associated with a disease or condition. In some cases, the cell is a cancer cell. Cancers can include breast cancer, liver cancer, colon cancer, brain cancer, spleen cancer, cancers of the salivary gland, kidney cancer, muscle cancers, ovarian cancer, glioblastoma, astrocytoma, glioma, medulloblastoma, ependymoma, choroid plexus carcinoma, midline glioma, diffuse intrinsic pontine glioma, lung cancer, bone marrow cell cancers, or skin cancer, genitourinary cancer, osteosarcoma, muscle-derived sarcoma, melanoma, head and neck cancer, a neuroblastoma, glioblastoma, astrocytoma, glioma, medulloblastoma, ependymoma, choroid plexus carcinoma, midline glioma, and diffuse intrinsic pontine glioma (DIPG), or a CMYC-overexpressing cancer. In some cases, other CDP or knotted peptides (e.g., those found in nature) are conjugated to, linked to, or fused to TfR-binding peptides and are capable of localizing TfR-binding peptides across the blood brain barrier to deliver TfR-binding peptides to target cells in the central nervous system.

CDPs (e.g., knotted peptides) are a class of peptides, usually ranging from about 11 to about 81 amino acids in length that are often folded into a compact structure. Knotted peptides are typically assembled into a complex tertiary structure that is characterized by a number of intramolecular disulfide crosslinks and may contain beta strands, alpha helices, and other secondary structures. The presence of the disulfide bonds gives knotted peptides remarkable environmental stability, allowing them to withstand extremes of temperature and pH and to resist the proteolytic enzymes of the blood stream. The presence of a disulfide knot may provide resistance to reduction by reducing agents. The rigidity of knotted peptides also allows them to bind to targets without paying the "entropic penalty" that a floppy peptide accrues upon binding a target. For example, binding is adversely affected by the loss of entropy that occurs when a peptide binds a target to form a complex. Therefore, "entropic penalty" is the adverse effect on binding, and the greater the entropic loss that occurs upon this binding, the greater the "entropic penalty." Furthermore, unbound molecules that are flexible lose more entropy when forming a complex than molecules that are rigidly structured, because of the loss of flexibility when bound up in a complex. However, rigidity in the unbound molecule also generally increases specificity by limiting the number of complexes that molecule can form. The peptides can bind targets with antibody-like affinity, or with nanomolar or picomolar affinity. A wider examination of the sequence structure and sequence identity or homology of knotted peptides reveals that they have arisen by convergent evolution in all kinds of animals and plants. In animals, they are often found in venoms, for example, the venoms of spiders and scorpions and have been implicated in the modulation of ion channels. The knotted proteins of plants can inhibit the proteolytic enzymes of animals or have antimicrobial activity, suggesting that knotted peptides can function in molecular defense systems found in plants.

The peptides of the present disclosure comprise cysteine amino acid residues. In some embodiments, the peptide has at least 1, at least 2, at least 3, at least 4, at least 5, at least 6, at least 7, at least 8, at least 9, or at least 10 cysteine amino acid residues. In some embodiments, the peptide has at least 8 cysteine amino acid residues. In other embodiments, the peptide has at least 10 cysteine amino acid residues, at least 12 cysteine amino acid residues, at least 14 cysteine amino acid residues or at least 16 cysteine amino acid residues.

A knotted peptide can comprise disulfide bridges. A knotted peptide can be a peptide wherein 5% or more of the residues are cysteines forming intramolecular disulfide bonds. A disulfide-linked peptide can be a drug scaffold. In some embodiments, the disulfide bridges form a knot. A disulfide bridge can be formed between cysteine residues, for example, between cysteines 1 and 4, 2 and 5, or, 3 and 6. In some embodiments, one disulfide bridge passes through a loop formed by the other two disulfide bridges, for example, to form the knot. In other embodiments, the disulfide bridges can be formed between any two cysteine residues.

The present disclosure further includes peptide scaffolds that, e.g., can be used as a starting point for generating additional peptides. In some embodiments, these scaffolds can be derived from a variety of knotted peptides (such as CDPs or knotted peptides). In certain embodiments, CDPs (e.g., knotted peptides) are assembled into a complex tertiary structure that is characterized by a number of intramolecular disulfide crosslinks, and optionally contain beta strands and other secondary structures such as an alpha helix. For example, CDPs (e.g., knotted peptides) include, in some embodiments, small disulfide-rich proteins characterized by a disulfide through disulfide knot. This knot can be, e.g., obtained when one disulfide bridge crosses the macrocycle formed by two other disulfides and the interconnecting backbone. In some embodiments, the knotted peptides can include growth factor cysteine knots or inhibitor cysteine knots. Other possible peptide structures include peptide having two parallel helices linked by two disulfide bridges without β-sheets (e.g., hefutoxin).

Some peptides of the present disclosure can comprise at least one amino acid residue in an L configuration. A peptide can comprise at least one amino acid residue in D configuration. In some embodiments, a peptide is 15-75 amino acid residues long. In other embodiments, a peptide is 11-55 amino acid residues long. In still other embodiments, a peptide is 11-65 amino acid residues long. In further embodiments, a peptide is at least 20 amino acid residues long.

Some CDPs (e.g., knotted peptides) can be derived or isolated from a class of proteins known to be present or associated with toxins or venoms. In some cases, the peptide can be derived from toxins or venoms associated with scorpions or spiders. The peptide can be derived from venoms and toxins of spiders and scorpions of various genus and species. For example, the peptide can be derived from a venom or toxin of the Leiurus quinquestriatus hebraeus, Buthus occitanus tunetanus, Hottentotta judaicus, Mesobuthus eupeus, Buthus occitanus israelis, Hadrurus gertschi, Androctonus australis, Centruroides noxius, Heterometrus laoticus, Opistophthalmus carinatus, Haplopelma schmidti, Isometrus maculatus, Haplopelma huwenum, Haplopelma hainanum, Haplopelma schmidti, Agelenopsis aperta, Haydronyche versuta, Selenocosmia huwena, Heteropoda venatoria, Grammostola rosea, Ornithoctonus huwena, Hadronyche versuta, Atrax robustus, Angelenopsis aperta, Psalmopoeus cambridgei, Hadronyche infensa, Paracoelotes luctosus, and Chilobrachys jingzhaoor another suitable genus or species of scorpion or spider. In some cases, a peptide can be derived from a Buthus martensii Karsh (scorpion) toxin.

### Sequence Identity and Homology

Percent (%) sequence identity or homology is determined by conventional methods. (See e.g., Altschul et al. (1986), Bull. Math. Bio. 48:603 (1986), and Henikoff and Henikoff (1992), Proc. Natl. Acad. Sci. USA 89:10915). Briefly, two amino acid sequences can be aligned to optimize the alignment scores using a gap opening penalty of 10, a gap extension penalty of 1, and the "BLOSUM62" scoring matrix of Henikoff and Henikoff (Id.). The sequence identity or homology is then calculated as: ([Total number of identical matches]/[length of the longer sequence plus the number of gaps introduced into the longer sequence in order to align the two sequences])(100).

Additionally, there are several established algorithms available to align two amino acid sequences. For example, the "FASTA" similarity search algorithm of Pearson and Lipman can be a suitable protein alignment method for examining the level of sequence identity or homology shared by an amino acid sequence of a peptide disclosed herein and the amino acid sequence of a peptide variant. The FASTA algorithm is described, for example, by Pearson and Lipman, Proc. Nat'l Acad. Sci. USA 85:2444 (1988), and by Pearson, Meth. Enzymol. 183:63 (1990). Briefly, FASTA first characterizes sequence similarity by identifying regions shared by the query sequence (e.g., SEQ ID NO: 1) and a test sequence that has either the highest density of identities (if the ktup variable is 1) or pairs of identities (if ktup=2), without considering conservative amino acid substitutions, insertions, or deletions. The ten regions with the highest density of identities are then rescored by comparing the similarity of all paired amino acids using an amino acid substitution matrix, and the ends of the regions are "trimmed" to include only those residues that contribute to the highest score. If there are several regions with scores greater than the "cutoff" value (calculated by a predetermined formula based upon the length of the sequence and the ktup value), then the trimmed initial regions are examined to determine whether the regions can be joined to form an approximate alignment with gaps. Finally, the highest scoring regions of the two amino acid sequences are aligned using a modification of the Needleman-Wunsch-Sellers algorithm (Needleman and Wunsch, J. Mol. Biol. 48:444 (1970); Sellers, Siam J. Appl. Math. 26:787 (1974)), which allows for amino acid insertions and deletions. For example, illustrative parameters for FASTA analysis are: ktup=1, gap opening penalty=10, gap extension penalty=1, and substitution matrix=BLOSUM62. These parameters can be introduced into a FASTA program by modifying the scoring matrix file ("SMATRIX"), as explained in Appendix 2 of Pearson, Meth. Enzymol.183:63 (1990).

FASTA can also be used to determine the sequence identity or homology of nucleic acid sequences or molecules using a ratio as disclosed above. For nucleic acid sequence comparisons, the ktup value can range between one to six, preferably from three to six, most preferably three, with other parameters set as described herein.

Some examples of common amino acids that are a "conservative amino acid substitution" are illustrated by a substitution among amino acids within each of the following groups: (1) glycine, alanine, valine, leucine, and isoleucine, (2) phenylalanine, tyrosine, and tryptophan, (3) serine and threonine, (4) aspartate and glutamate, (5) glutamine and asparagine, and (6) lysine, arginine and histidine. The BLOSUM62 table is an amino acid substitution matrix derived from about 2,000 local multiple alignments of protein sequence segments, representing highly conserved regions of more than 500 groups of related proteins (Henikoff and Henikoff, Proc. Nat'l Acad. Sci. USA 89:10915 (1992)). Accordingly, the BLOSUM62 substitution frequencies can be used to define conservative amino acid substitutions that can be introduced into the amino acid sequences of the present invention. Although it is possible to design amino acid substitutions based solely upon chemical properties (as discussed above), the language "conservative amino acid substitution" preferably refers to a substitution represented by a BLOSUM62 value of greater than -1. For example, an amino acid substitution is conservative if the substitution is characterized by a BLOSUM62 value of 0, 1, 2, or 3. According to this system, preferred conservative amino acid substitutions are characterized by a BLOSUM62 value of at least 1 (e.g., 1, 2 or 3), while more preferred conservative amino acid substitutions are characterized by a BLOSUM62 value of at least 2 (e.g., 2 or 3).

Determination of amino acid residues that are within regions or domains that are critical to maintaining structural integrity can be determined. Within these regions one can determine specific residues that can be more or less tolerant of change and maintain the overall tertiary structure of the molecule. Methods for analyzing sequence structure include, but are not limited to, alignment of multiple sequences with high amino acid or nucleotide identity or homology and computer analysis using available software (e.g., the Insight II.RTM. viewer and homology modeling tools; MSI, San Diego, Calif.), secondary structure propensities, binary patterns, complementary packing and buried polar interactions (Barton, G.J., Current Opin. Struct. Biol. 5:372-6 (1995) and Cordes, M.H. et al., Current Opin. Struct. Biol. 6:3-10 (1996)). In general, when designing modifications to molecules or identifying specific fragments, determination of structure can typically be accompanied by evaluating activity of modified molecules.

### Physicochemical Properties of Peptides

In some embodiments, the TfR-binding peptides of the present disclosure can comprise a wide range of physicochemical properties such as molecular size and structure, pH, isoelectric point, and overall molecular net charge. These parameters can have an effect on the peptides ability to bind TfR, promote transcytosis, and transport of cargo molecules across cell barrier such as the BBB.

In some embodiments, a peptide is at least 30 amino acid residues long. In some embodiments, a peptide is at least 35 amino acid residues long. In some embodiments, a peptide is at least 40 amino acid residues long. In some embodiments, a peptide is at least 45 amino acid residues long. In some embodiments, a peptide is at least 50 amino acid residues long. In some embodiments, a peptide is at least 55 amino acid residues long. In some embodiments, a peptide is at least 60 amino acid residues long. In some embodiments, a peptide is at least 65 amino acid residues long. In some embodiments, a peptide is at least 70 amino acid residues long. In some embodiments, a peptide is at least 75 amino acid residues long.

In some embodiments, an amino acid sequence of a peptide as described herein comprises
at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58 residues, at least 59, at least 60, at least 61, at least 62, at least 63, at least 64, at least 65, at least 66, at least 67, at least 68, at least 69, at least 70, at least 71, at least 72, at least 73, at least 74, at least 75, at least 76, at least 77, at least 78, at least 79, at least 80, or at least 81 amino acid residues.

In some embodiments of the present disclosure, a three-dimensional or tertiary structure of a peptide is primarily comprised of beta-sheets and/or alpha-helix structures. In some embodiments, designed or engineered TfR-binding peptides of the present disclosure are small, compact peptides or polypeptides stabilized by intra-chain disulfide bonds (e.g., mediated by cysteines) and a hydrophobic core. In some embodiments, engineered TfR-binding peptides have structures comprising helical bundles with at least one disulfide bridge between each of the alpha helices, thereby stabilizing the peptides. In other embodiments, the engineered TfR-binding peptides comprise structures with three alpha helices and three intra-chain disulfide bonds, one between each of the three alpha helices in the bundle of alpha helices.

At physiological pH, peptides as described herein can have an overall molecular net charge, for example, of -5, -4, -3, -2, -1, 0, +1, +2, +3, +4, or +5. When the net charge is zero, the peptide can be uncharged or zwitterionic. In some embodiments, a peptide contains one or more disulfide bonds and has a positive net charge at physiological pH where the net charge can be +0.5 or less than +0.5, +1 or less than +1, + 1.5 or less than +1.5, +2 or less than +2, +2.5 or less than +2.5, +3 or less than +3, +3.5 or less than +3.5, +4 or less than +4, +4.5 or less than +4.5, +5 or less than +5, +5.5 or less than +5.5, +6 or less than +6, +6.5 or less than +6.5, +7 or less than +7, +7.5 or less than +7.5, +8 or less than +8, +8.5 or less than +8.5, +9 or less than +9.5, +10 or less than +10. In some embodiments, a peptide has a negative net charge at physiological pH where the net charge can be -0.5 or less than -0.5, -1 or less than -1, -1.5 or less than -1.5, -2 or less than -2, -2.5 or less than -2.5, -3 or less than -3, -3.5 or less than -3.5, -4 or less than -4, -4.5 or less than -4.5, -5 or less than -5, -5.5 or less than -5.5, -6 or less than -6, -6.5 or less than -6.5, - 7 or less than -7, -7.5 or less than -7.5, -8 or less than -8, -8.5 or less than -8.5, -9 or less than - 9.5, -10 or less than -10.

In some embodiments, peptides of the present disclosure can have an isoelectric point (pI) value from 3 and 10. In other embodiments, peptides of the present disclosure can have a pI value from 4.3 and 8.9. In some embodiments, peptides of the present disclosure can have a pI value from 3-4. In some embodiments, peptides of the present disclosure can have a pI value from 3-5. In some embodiments, peptides of the present disclosure can have a pI value from 3-6. In some embodiments, peptides of the present disclosure can have a pI value from 3-7. In some embodiments, peptides of the present disclosure can have a pI value from 3-8. In some embodiments, peptides of the present disclosure can have a pI value from 3-9. In some embodiments, peptides of the present disclosure can have a pI value from 4-5. In some embodiments, peptides of the present disclosure can have a pI value from 4-6. In some embodiments, peptides of the present disclosure can have a pI value from 4-7. In some embodiments, peptides of the present disclosure can have a pI value from 4-8. In some embodiments, peptides of the present disclosure can have a pI value from 4-9. In some embodiments, peptides of the present disclosure can have a pI value from 4-10. In some embodiments, peptides of the present disclosure can have a pI value from 5-6. In some embodiments, peptides of the present disclosure can have a pI value from 5-7. In some embodiments, peptides of the present disclosure can have a pI value from 5-8. In some embodiments, peptides of the present disclosure can have a pI value from 5-9. In some embodiments, peptides of the present disclosure can have a pI value from 5-10. In some embodiments, peptides of the present disclosure can have a pI value from 6-7. In some embodiments, peptides of the present disclosure can have a pI value from 6-8. In some embodiments, peptides of the present disclosure can have a pI value from 6-9. In some embodiments, peptides of the present disclosure can have a pI value from 6-10. In some embodiments, peptides of the present disclosure can have a pI value from 7-8. In some embodiments, peptides of the present disclosure can have a pI value from 7-9. In some embodiments, peptides of the present disclosure can have a pI value from 7-10. In some embodiments, peptides of the present disclosure can have a pI value from 8-9. In some embodiments, peptides of the present disclosure can have a pI value from 8-10. In some embodiments, peptides of the present disclosure can have a pI value from 9-10.

In some cases, the engineering of one or more mutations within a peptide of the present disclosure (e.g., a TfR-binding peptide) yields a peptide with an altered isoelectric point, charge, surface charge, or rheology at physiological pH. Such engineering of a mutation to a peptide that can be derived from a scorpion or spider complex can change the net charge of the peptide, for example, by decreasing the net charge by 1, 2, 3, 4, or 5, or by increasing the net charge by 1, 2, 3, 4, or 5. In such cases, the engineered mutation can facilitate the ability of the peptide to bind a target protein, promote transcytosis, and penetrate a cell, an endosome, or the nucleus. Suitable amino acid modifications for improving the rheology and potency of a peptide can include conservative or non-conservative mutations.

A peptide can comprise at most 1 amino acid mutation, at most 2 amino acid mutations, at most 3 amino acid mutations, at most 4 amino acid mutations, at most 5 amino acid mutations, at most 6 amino acid mutations, at most 7 amino acid mutations, at most 8 amino acid mutations, at most 9 amino acid mutations, at most 10 amino acid mutations, or another suitable number as compared to the sequence of the venom or toxin component that the peptide is derived from. In other embodiments, a peptide, or a functional fragment thereof, comprises at least 1 amino acid mutation, at least 2 amino acid mutations, at least 3 amino acid mutations, at least 4 amino acid mutations, at least 5 amino acid mutations, at least 6 amino acid mutations, at least 7 amino acid mutations, at least 8 amino acid mutations, at least 9 amino acid mutations, at least 10 amino acid mutations, or another suitable number as compared to the sequence of the venom or toxin component that the peptide is derived from. In some embodiments, mutations can be engineered within a peptide to provide a peptide that has a desired charge or stability at physiological pH.

Generally, the NMR solution structures, the X-ray crystal structures, as well as the primary structure sequence alignment of related structural peptide or protein homologs or *in silico* design can be used to generate mutational strategies that can improve the folding, stability, and/or manufacturability, while maintaining a particular biological function (e.g., TfR affinity/binding). A general strategy for producing homologs or *in silico* designed peptides or polypeptides can include identification of a charged surface patch or conserved residues of a protein, mutation of critical amino acid positions and loops, followed by *in vitro* and *in vivo* testing of the peptides. The overall peptide optimization process can be of iterative nature to the extent that, for example, information obtained during *in vitro* or *in vivo* testing is used for the design of the next generation of peptides. Hence, the herein disclosed methods can be used to design peptides with improved properties or to correct deleterious mutations that complicate folding and manufacturability. Key amino acid positions and loops can be retained while other residues in the peptide sequences can be mutated to improve, change, remove, or otherwise modify function, such as binding, transcytosis, or the ability to penetrate a cell, endosome, or nucleus in a cell, homing, or another activity of the peptide.

The present disclosure also encompasses multimers of the various peptides described herein. Examples of multimers include dimers, trimers, tetramers, pentamers, hexamers, heptamers, and so on. A multimer may be a homomer formed from a plurality of identical subunits or a heteromer formed from a plurality of different subunits. In some embodiments, a peptide of the present disclosure is arranged in a multimeric structure with at least one other peptide, or two, three, four, five, six, seven, eight, nine, ten, or more other peptides. In certain embodiments, the peptides of a multimeric structure each have the same sequence. In other embodiments, one or more or all of the peptides of a multimeric structure have different sequences.

In some embodiments, the present disclosure provides peptide scaffolds that can be used as a starting point for generating additional, next-generation peptides with more specific or improved properties. In some embodiments, these scaffolds are derived from a variety of CDPs or knotted peptides. Some suitable peptides for scaffolds can include, but are not limited to, chlorotoxin, brazzein, circulin, stecrisp, hanatoxin, midkine, hefutoxin, potato carboxypeptidase inhibitor, bubble protein, attractin, α-GI, α-GID, µ-PIIIA, ω-MVIIA, ω-CVID, χ-MrIA, ρ-TIA, conantokin G, contulakin G, GsMTx4, margatoxin, shK, toxin K, chymotrypsin inhibitor (CTI), and EGF epiregulin core. In some embodiments, the peptide sequence is flanked by additional amino acids. One or more additional amino acids can confer a desired *in vivo* charge, isoelectric point, chemical conjugation site, stability, or physiologic property to a peptide.

### Chemical Modification of Peptides

A peptide can be chemically modified one or more of a variety of ways. In some embodiments, the peptide can be mutated to add function, delete function, or modify the in vivo behavior. For example, in some embodiments, peptides of the presenting disclosure may be chemically modified with a molecule that would lead to proteasomal degradation of TfR (e.g., ubiquitin ligase engaging conjugate or fusion or cereblon-binding molecule). One or more loops between the disulfide linkages can be modified or replaced to include active elements from other peptides (such as described in Moore and Cochran, Methods in Enzymology, 503, p. 223-251, 2012). Amino acids can also be mutated, such as to increase half-life, modify, add or delete binding behavior in vivo, add new targeting function, modify surface charge and hydrophobicity, or allow conjugation sites. N-methylation is one example of methylation that can occur in a peptide of the disclosure. In some embodiments, the peptide is modified by methylation on free amines. For example, full methylation may be accomplished through the use of reductive methylation with formaldehyde and sodium cyanoborohydride.

The peptides can be modified to add function, such as to graft loops or sequences from other proteins or peptides onto peptides of this disclosure. Likewise, domains, loops, or sequences from this disclosure can be grafted onto other peptides or proteins such as antibodies that have additional function.

A chemical modification can, for instance, extend the half-life of a peptide or change the biodistribution or pharmacokinetic profile. A chemical modification can comprise a polymer, a polyether, polyethylene glycol, a biopolymer, a polyamino acid, a fatty acid, a dendrimer, an Fc region, a simple saturated carbon chain such as palmitate or myristolate, or albumin. A polyamino acid can include, for example, a poly amino acid sequence with repeated single amino acids (e.g., poly glycine), and a poly amino acid sequence with mixed poly amino acid sequences (e.g., gly-ala-gly-ala) that may or may not follow a pattern, or any combination of the foregoing.

The peptides of the present disclosure can be modified such that the modification increases the stability and/or the half-life of the peptides. The attachment of a hydrophobic moiety, such as to the N-terminus, the C-terminus, or an internal amino acid, can be used to extend half-life of a peptide of the present disclosure. The peptides can also be modified to increase or decrease the gut permeability or cellular permeability of the peptide. In some cases, the peptides of the present disclosure show high accumulation in glandular cells of the intestine, demonstrating applicability in the treatment and-or prevention of diseases or conditions of the intestines, such as Crohn's disease or more generally inflammatory bowel diseases. The peptide of the present disclosure can include post-translational modifications (e.g., methylation and/or amidation and/or glycosylation), which can affect, e.g., serum half-life. In some embodiments, simple carbon chains (e.g., by myristoylation and/or palmitylation) can be conjugated to, linked to, the fusion proteins or peptides. The simple carbon chains can render the fusion proteins or peptides easily separable from the unconjugated material. For example, methods that can be used to separate the fusion proteins or peptides from the unconjugated material include, but are not limited to, solvent extraction and reverse phase chromatography. Lipophilic moieties can extend half-life through reversible binding to serum albumin. Conjugated moieties can, e.g., be lipophilic moieties that extend half-life of the peptides through reversible binding to serum albumin. In some embodiments, the lipophilic moiety can be cholesterol or a cholesterol derivative including cholestenes, cholestanes, cholestadienes and oxysterols. In some embodiments, the peptides can be conjugated to, linked to, myristic acid (tetradecanoic acid) or a derivative thereof. In other embodiments, the peptides of the present disclosure can be coupled (e.g., conjugated, linked, or fused) to a half-life modifying agent. Examples of half-life modifying agents can include, but is not limited to: a polymer, a polyethylene glycol (PEG), a hydroxyethyl starch, polyvinyl alcohol, a water soluble polymer, a zwitterionic water soluble polymer, a water soluble poly(amino acid), a water soluble polymer of proline, alanine and serine, a water soluble polymer containing glycine, glutamic acid, and serine, an Fc region, a fatty acid, palmitic acid, or a molecule that binds to albumin. In some embodiments, the half-life modifying agent may be a serum albumin binding peptide, for example SA21 (SEQ ID NO: 376, RLIEDICLPRWGCLWEDD). In some embodiments, a SA21 peptide may be conjugated or fused to the CDPs of the present disclosure (e.g., any of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). A SA21 fusion peptide may include the SA21 TfR-binding peptide constructs disclosed herein (e.g., SEQ ID NO: 373 or SEQ ID NO: 375). The SA21 peptide may comprise a linker sequence for conjugation to, or fusion between, one or more peptides (e.g., SEQ ID NO: 377, GGGGSGGGGSRLIEDICLPRWGCLWEDDGGGGSGGGGS). Exemplary SA21 peptides, fusion peptides, and linkers are provided in **TABLE 2.** A control SA21 fusion peptide may comprise a control peptide fused to SA21 (e.g., SEQ ID NO 372 (GSRLIEDICLPRWGCLWEDDGGGGSGGGGSKCLPPGKPCYGATQKIPCCGVCSHNNCT) , SEQ ID NO: 374 (RLIEDICLPRWGCLWEDDGGGGSGGGGSKCLPPGKPCYGATQKIPCCGVCSHNNCT), SEQ ID NO: 456 (GSRLIEDICLPRWGCLWEDDGGGGSGGGGSVRIPVSCKHSGQCLKPCKDAGMRFGKC MNGKCDCTPK), or SEQ ID NO: 457 (RLIEDICLPRWGCLWEDDGGGGSGGGGSVRIPVSCKHSGQCLKPCKDAGMRFGKCMN GKCDCTPK)). Additionally, conjugation of the peptide to a near infrared dye, such as Cy5.5, or to an albumin binder such as Albu-tag can extend serum half-life of any peptide as described herein. In some embodiments, immunogenicity is reduced by using minimal non-human protein sequences to extend serum half-life of the peptide.

**TABLE 2 - Exemplary TfR-Binding peptide constructs: TfR-binding peptides fused with Serum Albumin binding peptides for half-life extension**

| **SEQ ID NO** | **Amino Acid Sequence** |
|---|---|
| SEQ ID NO: 373 | GSRLIEDICLPRWGCLWEDDGGGGSGGGGSREGCASRCMKYNDELE KCEARMMSMSNTEEDCEQELEDLLYCLDHCHSQ |
| SEQ ID NO: 375 | RLIEDICLPRWGCLWEDDGGGGSGGGGSREGCASRCMKYNDELEKC EARMMSMSNTEEDCEQELEDLLYCLDHCHSQ |

In some embodiments, the first two N-terminal amino acids (GS) of SEQ ID NO: 1 - SEQ ID NO: 35 or SEQ ID NO: 136 - SEQ ID NO: 170 serve as a spacer or linker in order to facilitate conjugation or fusion to another molecule, as well as to facilitate cleavage of the peptide from such conjugated to, linked to, or fused molecules. In some embodiments, the fusion proteins or peptides of the present disclosure can be conjugated to, linked to, or fused to other moieties that, e.g., can modify or effect changes to the properties of the peptides.

### Peptide-Active Agent Conjugates (also referred to as "Peptide Constructs")

In some embodiments, the peptides of the present disclosure can themselves be used to bind TfR. In other embodiments, peptides of the present disclosure can also be used to deliver another active agent. Peptides according to the present disclosure can be conjugated to, linked to, or fused to an agent for use in the treatment of tumors and cancers. For example, in certain embodiments, the peptides described herein are fused to another molecule, such as an active agent that provides an additional functional capability. A peptide can be fused with an active agent through expression of a vector containing the sequence of the peptide with the sequence of the active agent. In various embodiments, the sequence of the peptide and the sequence of the active agent can be expressed from the same Open Reading Frame (ORF). In various embodiments, the sequence of the peptide and the sequence of the active agent can comprise a contiguous sequence. The peptide and the active agent can each retain similar functional capabilities in the peptide construct compared with their functional capabilities when expressed separately. In certain embodiments, examples of active agents can include other peptides.

In certain embodiments, examples of active agents include other peptides such as neurotensin peptide. Neurotensin is a 13 amino acid neuropeptide that can be involved in the regulation of luteinizing hormone and prolactin release, and can interact with the dopaminergic system, but does not cross the blood brain barrier and is also rapidly metabolized by peptidases (Wang, et al., Curr. Pharm. Des. 21(7); 840-848 (2015)).

Neurotensin is a 13-amino acid peptide found in the central nervous system and the gastrointestinal tract and plays a role in a wide range of physiologic and pathologic processes. Various activities of neurotensin and potential therapeutic roles of neurotensin agonists are described in the following: Mustain, Rychahou , and Evers, Curr Opin Endocrinol Diabetes Obes (2011) and Boules, Li, Smith, Fredrickson, and Richelson, Front Endocrinol (Lausanne) (2013*).* NT plays a role in gut motility, modulation of the cardiovascular system, naloxone-independent antinociception, hypothermia, controls of anterior pituary hormone secretion, muscle relaxation, central blood pressure, and inflammation. NT mediates its effects through three receptors NTS1, NTS2, an NTS3. NTS1 is a high affinity receptor and is expressed broadly throughout the CNS, including medial septal nucleus, nucleus basalis magnocellularis, suprachiasmatic nucleus, SN, and VTA, small dorsal root ganglion neurons of the spinal cord, in both neurons and glial cells. NTS2 is localized mainly in the olfactory system, the cerebral and cerebellar cortices, the hippocampal formation, and selective hypothalamic nuclei, VTA, and SN. NTS3 is also expressed at various locations throughout the brain. NT is involved in modulating dopamine neurotransmission, may play a role in the serotonergic system including antinociception, sleep-wake cycle, and stress, a role in glutamate release and may have antipsychotic effects. NT is involved in neuroendocrine regulation, including paracrine and autocrine roles, CRH, GnRH, GHRH, prolactin, ACTH, gonadotropic hormones, growth hormones, prolactin, thyroid hormone modulation, as well as gut motility, intestinal inflammation, lipid metabolism, appetite, and food intake. NT has been implicated in the pathophysiology of several CNS disorders such as schizophrenia, drug abuse, Parkinson's disease (PD), pain, central control of blood pressure, eating disorders, stroke, Alzheimer's, as well as, cancer and inflammation and other disorders of endocrine function . Disruption in the normal mechanisms can lead to diseases ranging from schizophrenia to colorectal cancer. By selective targeting or blockade of specific neurotensin receptors, investigators have identified potential drugs for use in the treatment of schizophrenia, alcoholism, chronic pain, or cancer. NT can provide opioid-independent analgesia and can treat thermal, visceral, and persistent inflammatory pain, somatic pain and visceral pain, and plays a role in stress-induced antinociception. NT exerts a mu-opioid-independent, anti-nociceptive effects. NT agonists can block effects of psychostimulants such as cocaine and amphetamine and nicotine. Plasma NT levels are higher in Parkinson's disease patients, and administration of NT can reduce muscular rigidity and tremors. NT can also reduce body temperature and reduce blood pressure.

**TABLE 3** discloses exemplary neurotensin (NT) peptide variants (Boules, et al., Diverse roles of neurotensin agonists in the central nervous system, Frontiers in Endocrinology, 2013)*.* Any peptides of the present disclosure (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) can be fused or otherwise conjugated to any of the NT peptide variants disclosed herein (e.g., any of the NT peptide variants disclosed in **TABLE 3**), or fragments thereof. In some embodiments, the peptides of the disclosure are fused or otherwise conjugated a neurotensin (NT) peptide variant comprising a at least 53%, at least 60%, at least 69%, at least 75%, at least 77%, at least 85%, least 90%, at least 92% sequence identity with SEQ ID NO: 350. In other embodiments, the peptides of the disclosure are fused or otherwise conjugated a neurotensin (NT) peptide variant comprising the sequence ELYENKP (SEQ ID NO: 370) or ELYENKP-X₁-X₂ -P- X₃ -X₄-L (SEQ ID NO: 371), wherein X₁ and X₂ can be Lys or Arg, X₃ can be Trp or Tyr, and X₄ can be Ile or Leu, or wherein none, or one or more of X₁-X₄ and Leu comprises a modified amino acid residue or non-natural amino acid residue.

**TABLE 3 - Neurotensin Peptide Variants**

| **SEQ ID NO** | **Amino Acid Sequence** |
|---|---|
| SEQ ID NO: 350 | ELYENKPRRPYIL |
| SEQ ID NO: 365 | ELYENKP-[N-methyl-Arg]-KP-[1-neo-Trp]-[*tert*-Leu]-L |
| SEQ ID NO: 366 | ELYENKPR-[d-Lys]-P-[1-neo-Trp]-[*tert*-1le]-L |
| SEQ ID NO: 367 | ELYENKPR-[d-Orn]-P-[1-neo-Trp]-[*tert*-Leu]-L |
| SEQ ID NO: 368 | ELYENKP-[N-methyl-Arg]-RP-[d-3,1-Nal]-[*tert*-Leu]-L |
| SEQ ID NO: 369 | ELYENKP-[H-Lys-psi[CH2NH]]-KPY-[Tle]-[Leu-Oet] |
| SEQ ID NO: 370 | ELYENKP |
| SEQ ID NO: 371 | ELYENKPX₁X₂PX₃X₄L |

Therefore, the fusion of neurotensin peptide and one of the peptides described herein that can cross the blood brain barrier can produce a fusion peptide capable of crossing the blood barrier which can retain the functional capabilities of neurotensin peptide. For example, the DNA sequence of a peptide of the present disclosure is inserted into the gene of neurotensin to manufacture peptide-neurotensin fusions. In some embodiments, the peptides of this disclosure are fused to neurotensin or a functional fragment thereof. A functional fragment of neurotensin can be at least 5 amino acid residues, at least 6 amino acid residues, at least 7 amino acid residues, at least 8 amino acid residues, at least 9 amino acid residues, at least 10 amino acid residues, at least 11 amino acid residues, at least 12 amino acid residues, or at least 13 amino acid residues long.

As another example, in certain embodiments, the peptides described herein are attached to another molecule, such as an active agent that provides a functional capability. In some embodiments, an active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter such as neurotensin.

In some embodiments, TfR-binding peptides can direct the active agent into the cell. In further embodiments, TfR-binding peptides can direct the active agent into the nucleus. In some embodiments, the active agent has intrinsic tumor-homing properties or the active agent can be engineering to have tumor-homing properties. In some embodiments, 1, 2, 3, 4, 5, 6, 7, 8, 9, or 10 active agents can be linked to a peptide. Multiple active agents can be attached by methods such as conjugating to multiple lysine residues and/or the N-terminus, or by linking the multiple active agents to a scaffold, such as a polymer or dendrimer and then attaching that agent-scaffold to the peptide (such as described in Yurkovetskiy, A. V., Cancer Res 75(16): 3365-72 (2015)). Examples of active agents include but are not limited to: a peptide, an oligopeptide, a polypeptide, a peptidomimetic, a polynucleotide, a polyribonucleotide, a DNA, a cDNA, a ssDNA, a RNA, a dsRNA, a micro RNA, an oligonucleotide, an antibody, a single chain variable fragment (scFv), an antibody fragment, an aptamer, a cytokine, an interferon, a hormone, an enzyme, a growth factor, a checkpoint inhibitor, a PD-1 inhibitor, a PD-L1 inhibitor, a CD47 inhibitor, a CTLA4 inhibitor, a CD antigen, a chemokine, a neurotransmitter, an ion channel inhibitor, an ion channel activator, a G-protein coupled receptor inhibitor, a G-protein coupled receptor activator, a chemical agent, a radiosensitizer, a radioprotectant, a radionuclide, a therapeutic small molecule, a steroid, a corticosteroid, an anti-inflammatory agent, an immune modulator, a complement fixing peptide or protein, a tumor necrosis factor inhibitor, a tumor necrosis factor activator, a tumor necrosis factor receptor family agonist, a tumor necrosis receptor antagonist, a Tim-3 inhibitor, a protease inhibitor, an amino sugar, a chemotherapeutic, a cytotoxic molecule, a toxin, a tyrosine kinase inhibitor, an anti-infective agent, an antibiotic, an anti-viral agent, an anti-fungal agent, an aminoglycoside, a nonsteroidal anti-inflammatory drug (NSAID), a statin, a nanoparticle, a liposome, a polymer, a biopolymer, a polysaccharide, a proteoglycan, a glycosaminoglycan, polyethylene glycol, a lipid, a dendrimer, a fatty acid, or an Fc region, or an active fragment or a modification thereof.

In some embodiments, the peptide is covalently or non-covalently linked to an active agent, e.g., directly or via a linker. For example, cytotoxic molecules that can be used include auristatins, MMAE, MMAF, dolostatin, auristatin F, monomethylaurstatin D, DM1, DM4, maytansinoids, maytansine, calicheamicins, N-acetyl-γ-calicheamicin, pyrrolobenzodiazepines, PBD dimers, doxorubicin, vinca alkaloids (4-deacetylvinblastine), duocarmycins, cyclic octapeptide analogs of mushroom amatoxins, epothilones, and anthracylines, CC-1065, taxanes, paclitaxel, cabazitaxel, docetaxel, SN-38, irinotecan, vincristine, vinblastine, platinum compounds, cisplatin, methotrexate, BACE (beta-secretase 1) inhibitors such as verubecestat, chlorambucil, mitomycin C. Additional examples of active agents are described in McCombs, J. R., AAPS J, 17(2): 339-51 (2015), Ducry, L., Antibody Drug Conjugates (2013), and Singh, S. K., Pharm Res. 32(11): 3541-3571 (2015). Additional examples of therapeutic payloads which therapeutic efficacy can be significantly improved when used in combination with the compositions and methods of the present disclosure include Carmustine, Cisplatin, Cyclophosphamide, Etoposide, Irinotecan, Lomustine, Procarbazine, Temozolomide, Vincristine, and Bevacizumab. Additional examples of therapeutic payloads are compounds that have therapeutic benefit in neurodegenerative diseases such as BACE inhibitors or auto-immunity diseases, and effective treatments for pain and migraine such as CGRP Receptor antagonists. Active agents used to treat pain are also consistent with the present disclosure. For example, in some embodiments, the active agent is a neurotransmitter, such as neurotensin. Thus, peptide constructs disclosed herein are peptide-neurotensin constructs. Exemplary linkers suitable for use with the embodiments herein are discussed in further detail below.

Only a small fraction of currently available drug molecules have applicability in CNS diseases due to their poor BBB penetration capabilities. About 98% of small molecule drugs do not or do only to a very limited degree cross the BBB. In addition, nearly 100% of macromolecular drug molecules (e.g., antibodies) do not exhibit significant BBB penetration capabilities. (See e.g., Mikitsh et al. Pathways for Small Molecule Delivery to the Central Nervous System Across the Blood-Brain Barrier, Perspect Medicin Chem. 2014; 6: 11-24). Thus, drug molecules available or currently in preclinical or clinical development can be used in combination with the presently described methods and compositions to provide improved therapeutic activity of these drugs in the cells, tissues, or organs expressing TfR (e.g., cancer cells or immune cells) and thus provide superior clinical outcomes compared to conventional therapies. For example, a drug molecule that shows activity against a specific biological target (e.g., a protein or a nucleic acid) but is prevented from reaching said target in vivo due to cellular barriers or cell membranes, said drug molecule can be conjugated to, linked to, or fused to a TfR-binding peptide of the present disclosure for enhanced delivery via TfR-mediated transport across those cellular barriers (e.g., BBB) or cell membranes (e.g., a cell membrane of a cancer cell). The peptide-drug conjugate or fusion molecule can cross the BBB via TfR-mediated transcytosis and thus provide significantly higher drug concentrations (e.g., therapeutic concentrations in the CNS to treat or prevent a CNS disorder (e.g., a brain cancer). For example, palbociclib has been shown efficacy in the treatment of breast cancer, but activity against CNS tumors has been limited. Thus, conjugating the drug palbociclib to a TfR-binding peptide of the present disclosure can enable its use in the treatment and/or prevention of tumors that are not accessible for the drug molecule itself, for instance those located in the CNS (e.g., brain tumors). Brain tumors that can be treated or prevented using conjugates or fusion molecules comprising one or more TfR-binding peptides of the present disclosure can include glioblastoma, astrocytoma, glioma, medulloblastoma, ependymoma, choroid plexus carcinoma, midline glioma, and diffuse intrinsic pontine glioma.

In some embodiments, the TfR-binding peptides as described herein can be used to treat and/or prevent diseases in non-CNS and/or peripheral cells, tissues, or organs. For example, the TfR-binding peptides as described herein can be used to treat and/or prevent muscle diseases. Drug or drug candidates that may be used in combination with the TfR-binding peptides to treat or prevent muscle diseases can include drugs or drug candidates for muscular dystrophy, spinal bulbar muscular dystrophy (SBMA), myotonic dystrophy, cachexia, or sarcopenia. In some embodiments, drugs can be senolytics or other anti-aging molecules (e.g., caspases), or gene therapies for diseases of the muscle.

In some embodiments, the TfR-binding peptides as described herein can be used to treat pain. The TfR binding peptides can be linked to neurotensin (NT) to generate CDP-NT peptide constructs (e.g., SEQ ID NO: 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361). These peptide constructs can target neurotensin receptors in the CNS to suppress pain. In some embodiments, the peptide is capable of reducing a level of pain in a subject upon administration.

Kinds of pain include chronic pain, acute pain, nociceptive pain, neuropathic pain, allodynia, phantom pain, visceral pain, breakthrough pain. Neurotensin is a non-opioid but can provide analgesia. Opioid can cause addiction and neurotensin delivery to the CNS offers the possibility of non-addictive pain relief. Neurotensin peptide fusions can also be used to treat dopaminergic, serotonergic, GABAergic, glutamatergic, and cholinergic systems, schizophrenia, psychosis, drug abuse, Parkinson's disease (PD), pain, central control of blood pressure, eating disorders, as well as, cancer and inflammation. Neurotensin peptide fusions can be used to treat pain including chronic pain, acute pain, injury, mechanical pain, heat pain, cold pain, ischemic pain, and chemical-induced pain, inflammatory pain, migraine-related pain, headache-related pain, irritable bowel syndrome-related pain, fibromyalgia-related pain, arthritic pain, skeletal pain, joint pain, gastrointestinal pain, muscle pain, angina pain, facial pain, pelvic pain, claudication, postoperative pain, post traumatic pain, tension-type headache, obstetric pain, gynecological pain, or chemotherapy-induced pain, nociceptive pain, neuropathic pain, allodynia, phantom pain, visceral pain, breakthrough pain, familial episodic pain syndrome, paroxysmal extreme pain disorder, congenital indifference to pain, pain associated with opioid withdrawal. Neurotensin peptide fusions can be used in neuroendocrine regulation, such as of the ACTH axis, the HPA axis, hormone regulation, and ion channel regulation, such as in diseases and disorders including Bartter's syndrome, Andersen's syndrome, congenital hyperinsulinism, neonatal diabetes, dilated cardiomyopathy, episodic ataxia type 1, long QT syndrome, short QT syndrome, benign neonatal febrile convulsions, nonsyndromic deafness, long QT syndrome, short QT syndrome, polycystic kidney disease, familial episodic pain syndrome, focal segmental glomerulosclerosis, retinitis pigmentosa, epilepsy, severe myoclonic epilepsy, long QT syndrome, cerebellar ataxia, erythromelalgia, paroxysmal extreme pain disorder, congenital indifference to pain, benign familial neonatal seizures, timothy syndrome, episodic ataxia type 2, stiff baby syndrome, juvenile myoclonic epilepsy, and autosomal dominant nocturnal frontal lobe epilepsy. In some embodiments, peptide constructs (e.g., peptide-NT constructs) interact with dopamine-signaling neurons to reduce pain.

In some embodiments, the TfR-binding peptides of the present disclosure can be used for the treatment and prevention of various neurological diseases including but not limited to epilepsy, schizophrenia, depression, anxiety, bipolar disorder, developmental brain disorders (e.g., autism spectrum), or mood disorder.

Binding of the herein described engineered peptides and peptide constructs (e.g., peptide conjugates, fusion peptides, or recombinantly produced peptide constructs) to TfR and subsequent transport across a cell layer or barrier such as the BBB (e.g., via vesicular transcytosis) or a cell membrane (e.g., via endocytosis) can have implications in a number of diseases, conditions, or disorders associated with neurodegeneration. Neurodegenerative diseases that can treated, prevented, or diagnosed with the herein described TfR-binding peptides can include Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia. In some embodiments, the TfR-binding peptide can be used in combination with BACE inhibitors, galantamine, amantadine, benztropine, biperiden, bromocriptin, carbidopa, donepezil, entacapone, levodopa, pergolie, pramipexole, procyclidine, rivastigmine, ropinirole, selegiline, tacrine, tolcapone, or trihexyphenidyl to treat and/or prevent a neurodegenerative disease.

In some embodiments, modulation (e.g., inhibition) of ion channels such as Kv1.3 potassium channels using TfR-binding peptides and ion channel modulator such as Kv1.3 potassium channel inhibitor conjugates or peptide constructs can be used for treating or preventing inflammation in the brain. Kv1.3 potassium channels, for example, can be highly expressed on microglia in the brain. Thus, neuroinflammatory and neurodegenerative diseases such as multiple sclerosis, Alzheimer's, Parkinson's, traumatic brain injury, radiation therapy toxicity and other neurodegenerative and neuroinflammatory diseases can be treated with TfR-binding peptides that are conjugated to, linked to, or fused to a Kv1.3 inhibitor. These diseases can be marked by upregulation of Kv1.3. In some cases, Kv1.3 inhibition can be used for treatment of psoriasis and other non-brain autoimmune diseases due to its effect on effector T cells. In some embodiments, a Kv1.3 inhibitor peptide may be Vm24 (SEQ ID NO: 378 or SEQ ID NO: 391, GSAAAISCVGSPECPPKCRAQGCKNGKCMNRKCKCYYC) or ShK-186 (SEQ ID NO: 379). A Kv1.3 inhibitor peptide may be conjugated to any of the peptides disclosed herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). Exemplary Kv1.3 inhibitor peptides are provided in TABLE 4.

**TABLE 4 - Exemplary Kv1.3 Inhibitor Peptides**

| **SEQ ID NO** | **Amino Acid Sequence** |
|---|---|
| SEQ ID NO: 378 | AAAISCVGSPECPPKCRAQGCKNGKCMNRKCKCYYC |
| SEQ ID NO: 379 | RSCIDTIPKSRCTAFQCKHSMKYRLSFCRKTCGTC |
| SEQ ID NO: 390 | GSEWSSRSCIDTIPKSRCTAFQCKHSMKYRLSFCRKTCGTC |
| SEQ ID NO: 391 | GSAAAISCVGSPECPPKCRAQGCKNGKCMNRKCKCYYC |
| SEQ ID NO: 402 | EWSSRSCIDTIPKSRCTAFQCKHSMKYRLSFCRKTCGTC |

In some embodiments, a Kv1.3 inhibitor, for example a Kv1.3 peptide inhibitor, may be conjugated to the TfR-binding peptides of the present disclosure. Exemplary Kv1.3 inhibitor fusion peptides are provided in **TABLE 5.** A control for a Kv1.3 inhibitor fusion peptide of the present disclosure may comprise a control peptide fused to a Kv1.3 inhibitor (e.g., SEQ ID NO: 380 
(GSAAAISCVGSPECPPKCRAQGCKNGKCMNRKCKCYYCGGGGSGGGGSRLIEDICLPR
WGCLWEDDGGGGSGGGGSKCLPPGKPCYGATQKIPCCGVCSHNNCT), SEQ ID NO: 382
(GSEWSSRSCIDTIPKSRCTAFQCKHSMKYRLSFCRKTCGTCGGGGSGGGGSGGGGSKCL
PPGKPCYGATQKIPCCGVCSHNNCT), SEQ ID NO: 383
 
(GSEWSSRSCIDTIPKSRCTAFQCKHSMKYRLSFCRKTCGTCKKYKPYVPVTTNKCLPPG
KPCYGATQKIPCCGVCSHNNCT), SEQ ID NO: 386
 
(GSAAAISCVGSPECPPKCRAQGCKNGKCMNRKCKCYYCGGGGSGGGGSGGGGSKCLP
PGKPCYGATQKIPCCGVCSHNNCT), SEQ ID NO: 387
 
(GSAAAISCVGSPECPPKCRAQGCKNGKCMNRKCKCYYCKKYKPYVPVTTNKCLPPGK
PCYGATQKIPCCGVCSHNNCT), SEQ ID NO: 392
 
(AAAISCVGSPECPPKCRAQGCKNGKCMNRKCKCYYCGGGGSGGGGSRLIEDICLPRWG
CLWEDDGGGGSGGGGSKCLPPGKPCYGATQKIPCCGVCSHNNCT), SEQ ID NO: 394
 
(EWSSRSCIDTIPKSRCTAFQCKHSMKYRLSFCRKTCGTCGGGGSGGGGSGGGGSKCLPP
GKPCYGATQKIPCCGVCSHNNCT), SEQ ID NO: 395
 
(EWSSRSCIDTIPKSRCTAFQCKHSMKYRLSFCRKTCGTCKKYKPYVPVTTNKCLPPGKP
CYGATQKIPCCGVCSHNNCT), SEQ ID NO: 398
 
(AAAISCVGSPECPPKCRAQGCKNGKCMNRKCKCYYCGGGGSGGGGSGGGGSKCLPPG
KPCYGATQKIPCCGVCSHNNCT), and SEQ ID NO: 399
 (AAAISCVGSPECPPKCRAQGCKNGKCMNRKCKCYYCKKYKPYVPVTTNKCLPPGKPC YGATQKIPCCGVCSHNNCT)). In some embodiments, a Kv1.3 peptide inhibitor may comprise a linker for fusing or conjugating to a TfR-binding peptide. In some embodiments, a Kv1.3 peptide inhibitor fused to a TfR-binding peptide may comprise a peptide with a sequence of SEQ ID NO: 390 or SEQ ID NO: 402. In some embodiments, a linker may have a sequence of any one of SEQ ID NO: 103 - SEQ ID NO: 115 or SEQ ID NO: 125. A Kv1.3 binding peptide (e.g., SEQ ID NO: 378, SEQ ID NO: 379, SEQ ID NO: 391, or SEQ ID NO: 402) may be conjugated to a TfR-binding peptide with a sequence of any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358.

**TABLE 5 - Peptide-Kv1.3 Inhibitor Fusions**

| **SEQ ID NO** | **Amino Acid Sequence** |
|---|---|
| SEQ ID NO: 381 | GSAAAISCVGSPECPPKCRAQGCKNGKCMNRKCKCYYCGGGGSGGG GSRLIEDICLPRWGCLWEDDGGGGSGGGGSREGCASRCMKYNDELE KCEARMMSMSNTEEDCEQELEDLLYCLDHCHSQ |
| SEQ ID NO: 384 | GSEWSSRSCIDTIPKSRCTAFQCKHSMKYRLSFCRKTCGTCGGGGSGG GGSGGGGSREGCASRCMKYNDELEKCEARMMSMSNTEEDCEQELE DLLYCLDHCHSQ |
| SEQ ID NO: 385 | GSEWSSRSCIDTIPKSRCTAFQCKHSMKYRLSFCRKTCGTCKKYKPYV PVTTNREGCASRCMKYNDELEKCEARMMSMSNTEEDCEQELEDLLY CLDHCHSQ |
| SEQ ID NO: 388 | GSAAAISCVGSPECPPKCRAQGCKNGKCMNRKCKCYYCGGGGSGGG GSGGGGSREGCASRCMKYNDELEKCEARMMSMSNTEEDCEQELEDL LYCLDHCHSQ |
| SEQ ID NO: 389 | GSAAAISCVGSPECPPKCRAQGCKNGKCMNRKCKCYYCKKYKPYVP VTTNREGCASRCMKYNDELEKCEARMMSMSNTEEDCEQELEDLLYC LDHCHSQ |
| SEQ ID NO: 393 | AAAISCVGSPECPPKCRAQGCKNGKCMNRKCKCYYCGGGGSGGGGS RLIEDICLPRWGCLWEDDGGGGSGGGGSREGCASRCMKYNDELEKC EARMMSMSNTEEDCEQELEDLLYCLDHCHSQ |
| SEQ ID NO: 396 | EWSSRSCIDTIPKSRCTAFQCKHSMKYRLSFCRKTCGTCGGGGSGGG GSGGGGSREGCASRCMKYNDELEKCEARMMSMSNTEEDCEQELEDL LYCLDHCHSQ |
| SEQ ID NO: 397 | EWSSRSCIDTIPKSRCTAFQCKHSMKYRLSFCRKTCGTCKKYKPYVPV TTNREGCASRCMKYNDELEKCEARMMSMSNTEEDCEQELEDLLYCL DHCHSQ |
| SEQ ID NO: 400 | AAAISCVGSPECPPKCRAQGCKNGKCMNRKCKCYYCGGGGSGGGGS GGGGSREGCASRCMKYNDELEKCEARMMSMSNTEEDCEQELEDLL YCLDHCHSQ |
| SEQ ID NO: 401 | AAAISCVGSPECPPKCRAQGCKNGKCMNRKCKCYYCKKYKPYVPVT TNREGCASRCMKYNDELEKCEARMMSMSNTEEDCEQELEDLLYCLD HCHSQ |

In some embodiments, the TfR-binding peptides of the present disclosure can be used for the treatment and prevention of Crohn's disease or, more generally, inflammatory bowel diseases. In some cases, the TfR-binding peptides of the present disclosure show high uptake and retention in glandular cells of the intestine, which can express high amounts of TfR.

In some embodiments, the TfR-binding peptides as described herein can be used to treat and/or prevent cancers located both in and outside the CNS, including but not limited to ovarian cancer, colon cancer, glioblastoma, astrocytoma, glioma, medulloblastoma, ependymoma, choroid plexus carcinoma, midline glioma, diffuse intrinsic pontine glioma, and lung cancer, cancer located in the bone or bone marrow, glioblastoma, astrocytoma, glioma, medulloblastoma, ependymoma, choroid plexus carcinoma, midline glioma, and diffuse intrinsic pontine glioma (DIPG), breast cancer, liver cancer, colon cancer, brain cancer, spleen cancer, cancers of the salivary gland, kidney cancer, muscle cancers, bone marrow cell cancers, or skin cancer, genitourinary cancer, osteosarcoma, muscle-derived sarcoma, melanoma, head and neck cancer, a neuroblastoma, or a CMYC-overexpressing cancer.

In some embodiments, TfR-binding peptide constructs that can be used to prevent and/or treat a cancer are those comprising a TfR binding peptide and an active agent with anti-tumor activity such as an IL15, a fused IL15/IL15Ra, IFNgamma, and anti-CD3 agents. Examples of such peptide constructs (e.g., fusion peptides) are those having an amino acid sequence set forth in any one of SEQ ID NO: 225 - SEQ ID NO: 332. In some embodiments, a peptide construct of the present disclosure can have at least 80%, at least 90%, at least 92%, at least 93%, at least 94%, at least 95%, at least 96%, at least 97%, at least 98%, at least 99%, or 100% sequence identity with any one of SEQ ID NO: 225 - SEQ ID NO: 332. Exemplary peptide constructs of the present disclosure are shown below in **TABLE 6.** These exemplary peptide constructs can comprise secretion murine IgKappa leader sequences, an anti-CD3 scFv, lysine to arginine mutants of the scFv, shorty-Flag sequences, His-tags, IL-15Ra, IL-15, IFN gamma, any linker disclosed herein, any peptides disclosed herein, and any fragments or derivatives thereof. Also shown in **TABLE 6** are peptide-neurotensin constructs (e.g., peptide constructs with a peptide of this disclosure complexed to neurotensin), for example those shown in SEQ ID NO: 33 - SEQ ID NO: 35, SEQ ID NO: 68 - SEQ ID NO: 70, SEQ ID NO: 168 - SEQ ID NO: 170, and SEQ ID NO: 359 - SEQ ID NO: 361. In some embodiments, a peptide construct of the present disclosure can have at least 80%, at least 90%, at least 92%, at least 93%, at least 94%, at least 95%, at least 96%, at least 97%, at least 98%, at least 99%, or 100% sequence identity with any one of SEQ ID NO: 33 - SEQ ID NO 35, SEQ ID NO: 68 - SEQ ID NO: 70, SEQ ID NO: 168 - SEQ ID NO: 170, and SEQ ID NO: 359 - SEQ ID NO: 361.

**TABLE 6 - Exemplary Peptide Construct Sequences**

| **SEQ ID NO** | **Amino Acid Sequence** |
|---|---|
| SEQ ID NO: 225 | METDTLLLWVLLLWVPGSTGDIKLQQSGAELARPGASVKMSCKTSGYTFTRY TMHWVKQRPGQGLEWIGYINPSRGYTNYNQKFKDKATLTTDKSSSTAYMQLS SLTSEDSAVYYCARYYDDHYCLDYWGQGTTLTVSSVEGGSGHHHHHHGSGG VDDIQLTQSPAIMSASPGEKVTMTCRASSSVSYMNWYQQKSGTSPKRWIYDTS KVASGVPYRFSGSGSGTSYSLTISSMEAEDAATYYCQQWSSNPLTFGAGTKLE LKGGGSREGCASRCTKYNAELEKCEARVSSMSNTEETCVQELFDLLHCVDHC VSQ |
| SEQ ID NO: 226 | METDTLLLWVLLLWVPGSTGDIKLQQSGAELARPGASVKMSCKTSGYTFTRY TMHWVKQRPGQGLEWIGYINPSRGYTNYNQKFKDKATLTTDKSSSTAYMQLS SLTSEDSAVYYCARYYDDHYCLDYWGQGTTLTVSSVEGGSGHHHHHHGSGG VDDIQLTQSPAIMSASPGEKVTMTCRASSSVSYMNWYQQKSGTSPKRWIYDTS KVASGVPYRFSGSGSGTSYSLTISSMEAEDAATYYCQQWSSNPLTFGAGTKLE LKGGGSREGCASRCTKYNAELEKCEARVMSMSNTEEDCEQELEDLLHCLDHC HSQ |
| SEQ ID NO: 227 | METDTLLLWVLLLWVPGSTGDIKLQQSGAELARPGASVKMSCKTSGYTFTRY TMHWVKQRPGQGLEWIGYINPSRGYTNYNQKFKDKATLTTDKSSSTAYMQLS SLTSEDSAVYYCARYYDDHYCLDYWGQGTTLTVSSVEGGSGHHHHHHGSGG VDDIQLTQSPAIMSASPGEKVTMTCRASSSVSYMNWYQQKSGTSPKRWIYDTS KVASGVPYRFSGSGSGTSYSLTISSMEAEDAATYYCQQWSSNPLTFGAGTKLE LKGGGSREGCASRCMKYNDELEKCEARMMSMSNTEEDCEQELEDLLYCLDH CHSQ |
| SEQ ID NO: 228 | DIKLQQSGAELARPGASVKMSCKTSGYTFTRYTMHWVKQRPGQGLEWIGYIN PSRGYTNYNQKFKDKATLTTDKSSSTAYMQLSSLTSEDSAVYYCARYYDDHY CLDYWGQGTTLTVSSVEGGSGHHHHHHGSGGVDDIQLTQSPAIMSASPGEKV TMTCRASSSVSYMNWYQQKSGTSPKRWIYDTSKVASGVPYRFSGSGSGTSYS LTISSMEAEDAATYYCQQWSSNPLTFGAGTKLELKGGGSREGCASRCTKYNA ELEKCEARVSSMSNTEETCVQELFDLLHCVDHCVSQ |
| SEQ ID NO: 229 | DIKLQQSGAELARPGASVKMSCKTSGYTFTRYTMHWVKQRPGQGLEWIGYIN PSRGYTNYNQKFKDKATLTTDKSSSTAYMQLSSLTSEDSAVYYCARYYDDHY CLDYWGQGTTLTVSSVEGGSGHHHHHHGSGGVDDIQLTQSPAIMSASPGEKV TMTCRASSSVSYMNWYQQKSGTSPKRWIYDTSKVASGVPYRFSGSGSGTSYS LTISSMEAEDAATYYCQQWSSNPLTFGAGTKLELKGGGSREGCASRCTKYNA ELEKCEARVMSMSNTEEDCEQELEDLLHCLDHCHSQ |
| SEQ ID NO: 230 | DIKLQQSGAELARPGASVKMSCKTSGYTFTRYTMHWVKQRPGQGLEWIGYIN PSRGYTNYNQKFKDKATLTTDKSSSTAYMQLSSLTSEDSAVYYCARYYDDHY CLDYWGQGTTLTVSSVEGGSGHHHHHHGSGGVDDIQLTQSPAIMSASPGEKV TMTCRASSSVSYMNWYQQKSGTSPKRWIYDTSKVASGVPYRFSGSGSGTSYS LTISSMEAEDAATYYCQQWSSNPLTFGAGTKLELKGGGSREGCASRCMKYND ELEKCEARMMSMSNTEEDCEQELEDLLYCLDHCHSQ |
| SEQ ID NO: 231 | METDTLLLWVLLLWVPGSTGDIRLQQSGAELARPGASVRMSCRTSGYTFTRY TMHWVRQRPGQGLEWIGYINPSRGYTNYNQRFRDRATLTTDRSSSTAYMQLS SLTSEDSAVYYCARYYDDHYCLDYWGQGTTLTVSSVEGGSGHHHHHHGSGG VDDIQLTQSPAIMSASPGERVTMTCRASSSVSYMNWYQQRSGTSPRRWIYDTS RVASGVPYRFSGSGSGTSYSLTISSMEAEDAATYYCQQWSSNPLTFGAGTRLE LRGGGSREGCASRCTRYNAELERCEARVSSMSNTEETCVQELFDLLHCVDHC VSQ |
| SEQ ID NO: 232 | METDTLLLWVLLLWVPGSTGDIRLQQSGAELARPGASVRMSCRTSGYTFTRY TMHWVRQRPGQGLEWIGYINPSRGYTNYNQRFRDRATLTTDRSSSTAYMQLS SLTSEDSAVYYCARYYDDHYCLDYWGQGTTLTVSSVEGGSGHHHHHHGSGG VDDIQLTQSPAIMSASPGERVTMTCRASSSVSYMNWYQQRSGTSPRRWIYDTS RVASGVPYRFSGSGSGTSYSLTISSMEAEDAATYYCQQWSSNPLTFGAGTRLE LRGGGSREGCASRCTRYNAELERCEARVMSMSNTEEDCEQELEDLLHCLDHC HSQ |
| SEQ ID NO: 233 | METDTLLLWVLLLWVPGSTGDIRLQQSGAELARPGASVRMSCRTSGYTFTRY TMHWVRQRPGQGLEWIGYINPSRGYTNYNQRFRDRATLTTDRSSSTAYMQLS SLTSEDSAVYYCARYYDDHYCLDYWGQGTTLTVSSVEGGSGHHHHHHGSGG VDDIQLTQSPAIMSASPGERVTMTCRASSSVSYMNWYQQRSGTSPRRWIYDTS RVASGVPYRFSGSGSGTSYSLTISSMEAEDAATYYCQQWSSNPLTFGAGTRLE LRGGGSREGCASRCMRYNDELERCEARMMSMSNTEEDCEQELEDLLYCLDH CHSQ |
| SEQ ID NO: 234 | DIRLQQSGAELARPGASVRMSCRTSGYTFTRYTMHWVRQRPGQGLEWIGYIN PSRGYTNYNQRFRDRATLTTDRSSSTAYMQLSSLTSEDSAVYYCARYYDDHY CLDYWGQGTTLTVSSVEGGSGHHHHHHGSGGVDDIQLTQSPAIMSASPGERV TMTCRASSSVSYMNWYQQRSGTSPRRWIYDTSRVASGVPYRFSGSGSGTSYSL TISSMEAEDAATYYCQQWSSNPLTFGAGTRLELRGGGSREGCASRCTRYNAEL ERCEARVSSMSNTEETCVQELFDLLHCVDHCVSQ |
| SEQ ID NO: 235 | DIRLQQSGAELARPGASVRMSCRTSGYTFTRYTMHWVRQRPGQGLEWIGYIN PSRGYTNYNQRFRDRATLTTDRSSSTAYMQLSSLTSEDSAVYYCARYYDDHY CLDYWGQGTTLTVSSVEGGSGHHHHHHGSGGVDDIQLTQSPAIMSASPGERV TMTCRASSSVSYMNWYQQRSGTSPRRWIYDTSRVASGVPYRFSGSGSGTSYSL TISSMEAEDAATYYCQQWSSNPLTFGAGTRLELRGGGSREGCASRCTRYNAEL ERCEARVMSMSNTEEDCEQELEDLLHCLDHCHSQ |
| SEQ ID NO: 236 | DIRLQQSGAELARPGASVRMSCRTSGYTFTRYTMHWVRQRPGQGLEWIGYIN PSRGYTNYNQRFRDRATLTTDRSSSTAYMQLSSLTSEDSAVYYCARYYDDHY CLDYWGQGTTLTVSSVEGGSGHHHHHHGSGGVDDIQLTQSPAIMSASPGERV TMTCRASSSVSYMNWYQQRSGTSPRRWIYDTSRVASGVPYRFSGSGSGTSYSL TISSMEAEDAATYYCQQWSSNPLTFGAGTRLELRGGGSREGCASRCMRYNDE LERCEARMMSMSNTEEDCEQELEDLLYCLDHCHSQ |
| SEQ ID NO: 237 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSREGCASRCTKYNAELEKCEARVSS MSNTEETCVQELFDLLHCVDHCVSQ |
| SEQ ID NO: 238 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSREGCASRCTKYNAELEKCEARVM SMSNTEEDCEQELEDLLHCLDHCHSQ |
| SEQ ID NO: 239 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSREGCASRCMKYNDELEKCEARM MSMSNTEEDCEQELEDLLYCLDHCHSQ |
| SEQ ID NO: 240 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSREGCASRCTKYNAELEKCEARVSSMSNTEETCVQELFDLLHCVDHC VSQ |
| SEQ ID NO: 241 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSREGCASRCTKYNAELEKCEARVMSMSNTEEDCEQELEDLLHCLDHC HSQ |
| SEQ ID NO: 242 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSREGCASRCMKYNDELEKCEARMMSMSNTEEDCEQELEDLLYCLDH CHSQ |
| SEQ ID NO: 243 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSREGCASRCTRYNAELERCEARVSSMS NTEETCVQELFDLLHCVDHCVSQ |
| SEQ ID NO: 244 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSREGCASRCTRYNAELERCEARVMSM SNTEEDCEQELEDLLHCLDHCHSQ |
| SEQ ID NO: 245 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSREGCASRCMRYNDELERCEARMMS MSNTEEDCEQELEDLLYCLDHCHSQ |
| SEQ ID NO: 246 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSREGCASRCTRYNAELERCEARVSSMSNTEETCVQELFDLLHCVDHCV SQ |
| SEQ ID NO: 247 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSREGCASRCTRYNAELERCEARVMSMSNTEEDCEQELEDLLHCLDHC HSQ |
| SEQ ID NO: 248 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSREGCASRCMRYNDELERCEARMMSMSNTEEDCEQELEDLLYCLDHC HSQ |
| SEQ ID NO: 249 | METDTLLLWVLLLWVPGSTGDYKDEHHHHHHGSHGTVIESLESLNNYFNSSGI DVEEKSLFLDIWRNWQKDGDMKILQSQIISFYLRLFEVLKDNQAISNNISVIESH LITTFFSNSKAKKDAFMSIAKFEVNNPQVQRQAFNELIRVVHQLLPESSLRKRK RSRGGGSHGTVIESLESLNNYFNSSGIDVEEKSLFLDIWRNWQKDGDMKILQS QIISFYLRLFEVLKDNQAISNNISVIESHLITTFFSNSKAKKDAFMSIAKFEVNNP QVQRQAFNELIRVVHQLLPESSLRKRKRSRGGGSREGCASRCTKYNAELEKCE ARVSSMSNTEETCVQELFDLLHCVDHCVSQ |
| SEQ ID NO: 250 | METDTLLLWVLLLWVPGSTGDYKDEHHHHHHGSHGTVIESLESLNNYFNSSGI DVEEKSLFLDIWRNWQKDGDMKILQSQIISFYLRLFEVLKDNQAISNNISVIESH LITTFFSNSKAKKDAFMSIAKFEVNNPQVQRQAFNELIRVVHQLLPESSLRKRK RSRGGGSHGTVIESLESLNNYFNSSGIDVEEKSLFLDIWRNWQKDGDMKILQS QIISFYLRLFEVLKDNQAISNNISVIESHLITTFFSNSKAKKDAFMSIAKFEVNNP QVQRQAFNELIRVVHQLLPESSLRKRKRSRGGGSREGCASRCTKYNAELEKCE ARVMSMSNTEEDCEQELEDLLHCLDHCHSQ |
| SEQ ID NO: 251 | METDTLLLWVLLLWVPGSTGDYKDEHHHHHHGSHGTVIESLESLNNYFNSSGI DVEEKSLFLDIWRNWQKDGDMKILQSQIISFYLRLFEVLKDNQAISNNISVIESH LITTFFSNSKAKKDAFMSIAKFEVNNPQVQRQAFNELIRVVHQLLPESSLRKRK RSRGGGSHGTVIESLESLNNYFNSSGIDVEEKSLFLDIWRNWQKDGDMKILQS QIISFYLRLFEVLKDNQAISNNISVIESHLITTFFSNSKAKKDAFMSIAKFEVNNP QVQRQAFNELIRVVHQLLPESSLRKRKRSRGGGSREGCASRCMKYNDELEKC EARMMSMSNTEEDCEQELEDLLYCLDHCHSQ |
| SEQ ID NO: 252 | DYKDEHHHHHHGSHGTVIESLESLNNYFNSSGIDVEEKSLFLDIWRNWQKDG DMKILQSQIISFYLRLFEVLKDNQAISNNISVIESHLITTFFSNSKAKKDAFMSIA KFEVNNPQVQRQAFNELIRVVHQLLPESSLRKRKRSRGGGSHGTVIESLESLNN YFNSSGIDVEEKSLFLDIWRNWQKDGDMKILQSQIISFYLRLFEVLKDNQAISN NISVIESHLITTFFSNSKAKKDAFMSIAKFEVNNPQVQRQAFNELIRVVHQLLPE SSLRKRKRSRGGGSREGCASRCTKYNAELEKCEARVSSMSNTEETCVQELFDL LHCVDHCVSQ |
| SEQ ID NO: 253 | DYKDEHHHHHHGSHGTVIESLESLNNYFNSSGIDVEEKSLFLDIWRNWQKDG DMKILQSQIISFYLRLFEVLKDNQAISNNISVIESHLITTFFSNSKAKKDAFMSIA KFEVNNPQVQRQAFNELIRVVHQLLPESSLRKRKRSRGGGSHGTVIESLESLNN YFNSSGIDVEEKSLFLDIWRNWQKDGDMKILQSQIISFYLRLFEVLKDNQAISN NISVIESHLITTFFSNSKAKKDAFMSIAKFEVNNPQVQRQAFNELIRVVHQLLPE SSLRKRKRSRGGGSREGCASRCTKYNAELEKCEARVMSMSNTEEDCEQELED LLHCLDHCHSQ |
| SEQ ID NO: 254 | DYKDEHHHHHHGSHGTVIESLESLNNYFNSSGIDVEEKSLFLDIWRNWQKDG DMKILQSQIISFYLRLFEVLKDNQAISNNISVIESHLITTFFSNSKAKKDAFMSIA KFEVNNPQVQRQAFNELIRVVHQLLPESSLRKRKRSRGGGSHGTVIESLESLNN YFNSSGIDVEEKSLFLDIWRNWQKDGDMKILQSQIISFYLRLFEVLKDNQAISN NISVIESHLITTFFSNSKAKKDAFMSIAKFEVNNPQVQRQAFNELIRVVHQLLPE SSLRKRKRSRGGGSREGCASRCMKYNDELEKCEARMMSMSNTEEDCEQELED LLYCLDHCHSQ |
| SEQ ID NO: 255 | METDTLLLWVLLLWVPGSTGDYRDEHHHHHHGSHGTVIESLESLNNYFNSSGI DVEERSLFLDIWRNWQRDGDMRILQSQIISFYLRLFEVLRDNQAISNNISVIESH LITTFFSNSRARRDAFMSIARFEVNNPQVQRQAFNELIRVVHQLLPESSLRRRRR SRGGGSHGTVIESLESLNNYFNSSGIDVEERSLFLDIWRNWQRDGDMRILQSQII SFYLRLFEVLRDNQAISNNISVIESHLITTFFSNSRARRDAFMSIARFEVNNPQVQ RQAFNELIRVVHQLLPESSLRRRRRSRGGGSREGCASRCTRYNAELERCEARV SSMSNTEETCVQELFDLLHCVDHCVSQ |
| SEQ ID NO: 256 | METDTLLLWVLLLWVPGSTGDYRDEHHHHHHGSHGTVIESLESLNNYFNSSGI DVEERSLFLDIWRNWQRDGDMRILQSQIISFYLRLFEVLRDNQAISNNISVIESH LITTFFSNSRARRDAFMSIARFEVNNPQVQRQAFNELIRVVHQLLPESSLRRRRR SRGGGSHGTVIESLESLNNYFNSSGIDVEERSLFLDIWRNWQRDGDMRILQSQII SFYLRLFEVLRDNQAISNNISVIESHLITTFFSNSRARRDAFMSIARFEVNNPQVQ RQAFNELIRVVHQLLPESSLRRRRRSRGGGSREGCASRCTRYNAELERCEARV MSMSNTEEDCEQELEDLLHCLDHCHSQ |
| SEQ ID NO: 257 | METDTLLLWVLLLWVPGSTGDYRDEHHHHHHGSHGTVIESLESLNNYFNSSGI DVEERSLFLDIWRNWQRDGDMRILQSQIISFYLRLFEVLRDNQAISNNISVIESH LITTFFSNSRARRDAFMSIARFEVNNPQVQRQAFNELIRVVHQLLPESSLRRRRR SRGGGSHGTVIESLESLNNYFNSSGIDVEERSLFLDIWRNWQRDGDMRILQSQII SFYLRLFEVLRDNQAISNNISVIESHLITTFFSNSRARRDAFMSIARFEVNNPQVQ RQAFNELIRVVHQLLPESSLRRRRRSRGGGSREGCASRCMRYNDELERCEARM MSMSNTEEDCEQELEDLLYCLDHCHSQ |
| SEQ ID NO: 258 | DYRDEHHHHHHGSHGTVIESLESLNNYFNSSGIDVEERSLFLDIWRNWQRDGD MRILQSQIISFYLRLFEVLRDNQAISNNISVIESHLITTFFSNSRARRDAFMSIARF EVNNPQVQRQAFNELIRVVHQLLPESSLRRRRRSRGGGSHGTVIESLESLNNYF NSSGIDVEERSLFLDIWRNWQRDGDMRILQSQIISFYLRLFEVLRDNQAISNNIS VIESHLITTFFSNSRARRDAFMSIARFEVNNPQVQRQAFNELIRVVHQLLPESSL RRRRRSRGGGSREGCASRCTRYNAELERCEARVSSMSNTEETCVQELFDLLHC VDHCVSQ |
| SEQ ID NO: 259 | DYRDEHHHHHHGSHGTVIESLESLNNYFNSSGIDVEERSLFLDIWRNWQRDGD MRILQSQIISFYLRLFEVLRDNQAISNNISVIESHLITTFFSNSRARRDAFMSIARF EVNNPQVQRQAFNELIRVVHQLLPESSLRRRRRSRGGGSHGTVIESLESLNNYF NSSGIDVEERSLFLDIWRNWQRDGDMRILQSQIISFYLRLFEVLRDNQAISNNIS VIESHLITTFFSNSRARRDAFMSIARFEVNNPQVQRQAFNELIRVVHQLLPESSL RRRRRSRGGGSREGCASRCTRYNAELERCEARVMSMSNTEEDCEQELEDLLH CLDHCHSQ |
| SEQ ID NO: 260 | DYRDEHHHHHHGSHGTVIESLESLNNYFNSSGIDVEERSLFLDIWRNWQRDGD MRILQSQIISFYLRLFEVLRDNQAISNNISVIESHLITTFFSNSRARRDAFMSIARF EVNNPQVQRQAFNELIRVVHQLLPESSLRRRRRSRGGGSHGTVIESLESLNNYF NSSGIDVEERSLFLDIWRNWQRDGDMRILQSQIISFYLRLFEVLRDNQAISNNIS VIESHLITTFFSNSRARRDAFMSIARFEVNNPQVQRQAFNELIRVVHQLLPESSL RRRRRSRGGGSREGCASRCMRYNDELERCEARMMSMSNTEEDCEQELEDLLY CLDHCHSQ |
| SEQ ID NO: 261 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSREGCASRCTKYNAELEKCE ARVSSMSNTEETCVQELFDLLHCVDHCVSQ |
| SEQ ID NO: 262 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSREGCASRCTKYNAELEKCE ARVMSMSNTEEDCEQELEDLLHCLDHCHSQ |
| SEQ ID NO: 263 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSREGCASRCMKYNDELEKCE ARMMSMSNTEEDCEQELEDLLYCLDHCHSQ |
| SEQ ID NO: 264 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSREGCASRCTKYNAELEKCEARVSSMSNTEETCVQELFDLLHCV DHCVSQ |
| SEQ ID NO: 265 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSREGCASRCTKYNAELEKCEARVMSMSNTEEDCEQELEDLLHC LDHCHSQ |
| SEQ ID NO: 266 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSREGCASRCMKYNDELEKCEARMMSMSNTEEDCEQELEDLLY CLDHCHSQ |
| SEQ ID NO: 267 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSREGCASRCTRYNAELERCEARV SSMSNTEETCVQELFDLLHCVDHCVSQ |
| SEQ ID NO: 268 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSREGCASRCTRYNAELERCEARV MSMSNTEEDCEQELEDLLHCLDHCHSQ |
| SEQ ID NO: 269 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSREGCASRCMRYNDELERCEAR MMSMSNTEEDCEQELEDLLYCLDHCHSQ |
| SEQ ID NO: 270 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW lDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSREGCASRCTRYNAELERCEARVSSMSNTEETCVQELFDLLHCV DHCVSQ |
| SEQ ID NO: 271 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW lDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSREGCASRCTRYNAELERCEARVMSMSNTEEDCEQELEDLLHC LDHCHSQ |
| SEQ ID NO: 272 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSREGCASRCMRYNDELERCEARMMSMSNTEEDCEQELEDLLYC LDHCHSQ |
| SEQ ID NO: 273 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSGGGSREGCASRCTKYNAEL EKCEARVSSMSNTEETCVQELFDLLHCVDHCVSQ |
| SEQ ID NO: 274 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSGGGSREGCASRCTKYNAEL EKCEARVMSMSNTEEDCEQELEDLLHCLDHCHSQ |
| SEQ ID NO: 275 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSGGGSGGGSGGGSREGCASRCMKYNDEL EKCEARMMSMSNTEEDCEQELEDLLYCLDHCHSQ |
| SEQ ID NO: 276 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSGGGSREGCASRCTKYNAELEKCEARVSSMSNTEETCVQELFDL LHCVDHCVSQ |
| SEQ ID NO: 277 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSGGGSREGCASRCTKYNAELEKCEARVMSMSNTEEDCEQELED LLHCLDHCHSQ |
| SEQ ID NO: 278 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSGGGSGGGSGGGSREGCASRCMKYNDELEKCEARMMSMSNTEEDCEQELE DLLYCLDHCHSQ |
| SEQ ID NO: 279 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSGGGSREGCASRCTRYNAELERC EARVSSMSNTEETCVQELFDLLHCVDHCVSQ |
| SEQ ID NO: 280 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSGGGSREGCASRCTRYNAELERC EARVMSMSNTEEDCEQELEDLLHCLDHCHSQ |
| SEQ ID NO: 281 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSGGGSGGGSGGGSREGCASRCMRYNDELER CEARMMSMSNTEEDCEQELEDLLYCLDHCHSQ |
| SEQ ID NO: 282 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSGGGSREGCASRCTRYNAELERCEARVSSMSNTEETCVQELFDL LHCVDHCVSQ |
| SEQ ID NO: 283 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSGGGSREGCASRCTRYNAELERCEARVMSMSNTEEDCEQELED LLHCLDHCHSQ |
| SEQ ID NO: 284 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSGGGSGGGSGGGSREGCASRCMRYNDELERCEARMMSMSNTEEDCEQELE DLLYCLDHCHSQ |
| SEQ ID NO: 285 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRVPITQNREGCASRCTKYNAELEKCE ARVSSMSNTEETCVQELFDLLHCVDHCVSQ |
| SEQ ID NO: 286 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRVPITQNREGCASRCTKYNAELEKCE ARVMSMSNTEEDCEQELEDLLHCLDHCHSQ |
| SEQ ID NO: 287 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRVPITQNREGCASRCMKYNDELEKCE ARMMSMSNTEEDCEQELEDLLYCLDHCHSQ |
| SEQ ID NO: 288 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRVPITQNREGCASRCTKYNAELEKCEARVSSMSNTEETCVQELFDLLHC VDHCVSQ |
| SEQ ID NO: 289 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRVPITQNREGCASRCTKYNAELEKCEARVMSMSNTEEDCEQELEDLLHC LDHCHSQ |
| SEQ ID NO: 290 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRVPITQNREGCASRCMKYNDELEKCEARMMSMSNTEEDCEQELEDLLY CLDHCHSQ |
| SEQ ID NO: 291 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRVPITQNREGCASRCTRYNAELERCEAR VSSMSNTEETCVQELFDLLHCVDHCVSQ |
| SEQ ID NO: 292 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRVPITQNREGCASRCTRYNAELERCEAR VMSMSNTEEDCEQELEDLLHCLDHCHSQ |
| SEQ ID NO: 293 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRVPITQNREGCASRCMRYNDELERCEAR MMSMSNTEEDCEQELEDLLYCLDHCHSQ |
| SEQ ID NO: 294 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRVPITQNREGCASRCTRYNAELERCEARVSSMSNTEETCVQELFDLLHCV DHCVSQ |
| SEQ ID NO: 295 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRVPITQNREGCASRCTRYNAELERCEARVMSMSNTEEDCEQELEDLLHC LDHCHSQ |
| SEQ ID NO: 296 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRVPITQNREGCASRCMRYNDELERCEARMMSMSNTEEDCEQELEDLLY CLDHCHSQ |
| SEQ ID NO: 297 | METDTLLLWVLLLWVPGSTGDYKDEHHHHHHENLYFQGSREGCASRCTKYN AELEKCEARVSSMSNTEETCVQELFDLLHCVDHCVSQGGGGSGTTCPPPVSIE HADIRVKNYSVNSRERYVCNSGFKRKAGTSTLIECVINKNTNVAHWTTPSLKC IRDPSLAGGGGSGGGGSGGGGSGGGGSNWIDVRYDLEKIESLIQSIHIDTTLYT DSDFHPSCKVTAMNCFLLELQVILHEYSNMTLNETVRNVLYLANSTLSSNKNV AESGCKECEELEEKTFTEFLQSFIRIVQMFINTS |
| SEQ ID NO: 298 | METDTLLLWVLLLWVPGSTGDYKDEHHHHHHENLYFQGSREGCASRCTKYN AELEKCEARVMSMSNTEEDCEQELEDLLHCLDHCHSQGGGGSGTTCPPPVSIE HADIRVKNYSVNSRERYVCNSGFKRKAGTSTLIECVINKNTNVAHWTTPSLKC IRDPSLAGGGGSGGGGSGGGGSGGGGSNWIDVRYDLEKIESLIQSIHIDTTLYT DSDFHPSCKVTAMNCFLLELQVILHEYSNMTLNETVRNVLYLANSTLSSNKNV AESGCKECEELEEKTFTEFLQSFIRIVQMFINTS |
| SEQ ID NO: 299 | METDTLLLWVLLLWVPGSTGDYKDEHHHHHHENLYFQGSREGCASRCMKYN DELEKCEARMMSMSNTEEDCEQELEDLLYCLDHCHSQGGGGSGTTCPPPVSIE HADIRVKNYSVNSRERYVCNSGFKRKAGTSTLIECVINKNTNVAHWTTPSLKC IRDPSLAGGGGSGGGGSGGGGSGGGGSNWIDVRYDLEKIESLIQSIHIDTTLYT DSDFHPSCKVTAMNCFLLELQVILHEYSNMTLNETVRNVLYLANSTLSSNKNV AESGCKECEELEEKTFTEFLQSFIRIVQMFINTS |
| SEQ ID NO: 300 | DYKDEHHHHHHENLYFQGSREGCASRCTKYNAELEKCEARVSSMSNTEETCV QELFDLLHCVDHCVSQGGGGSGTTCPPPVSIEHADIRVKNYSVNSRERYVCNS GFKRKAGTSTLIECVINKNTNVAHWTTPSLKCIRDPSLAGGGGSGGGGSGGGG SGGGGSNWIDVRYDLEKIESLIQSIHIDTTLYTDSDFHPSCKVTAMNCFLLELQ VILHEYSNMTLNETVRNVLYLANSTLSSNKNVAESGCKECEELEEKTFTEFLQS FIRIVQMFINTS |
| SEQ ID NO: 301 | DYKDEHHHHHHENLYFQGSREGCASRCTKYNAELEKCEARVMSMSNTEEDC EQELEDLLHCLDHCHSQGGGGSGTTCPPPVSIEHADIRVKNYSVNSRERYVCN SGFKRKAGTSTLIECVINKNTNVAHWTTPSLKCIRDPSLAGGGGSGGGGSGGG GSGGGGSNWIDVRYDLEKIESLIQSIHIDTTLYTDSDFHPSCKVTAMNCFLLEL QVILHEYSNMTLNETVRNVLYLANSTLSSNKNVAESGCKECEELEEKTFTEFL QSFIRIVQMFINTS |
| SEQ ID NO: 302 | DYKDEHHHHHHENLYFQGSREGCASRCMKYNDELEKCEARMMSMSNTEED CEQELEDLLYCLDHCHSQGGGGSGTTCPPPVSIEHADIRVKNYSVNSRERYVC NSGFKRKAGTSTLIECVINKNTNVAHWTTPSLKCIRDPSLAGGGGSGGGGSGG GGSGGGGSNWIDVRYDLEKIESLIQSIHIDTTLYTDSDFHPSCKVTAMNCFLLE LQVILHEYSNMTLNETVRNVLYLANSTLSSNKNVAESGCKECEELEEKTFTEFL QSFIRIVQMFINTS |
| SEQ ID NO: 303 | METDTLLLWVLLLWVPGSTGDYRDEHHHHHHENLYFQGSREGCASRCTRYN AELERCEARVSSMSNTEETCVQELFDLLHCVDHCVSQGGGGSGTTCPPPVSIE HADIRVRNYSVNSRERYVCNSGFRRRAGTSTLIECVINRNTNVAHWTTPSLRCI RDPSLAGGGGSGGGGSGGGGSGGGGSNWIDVRYDLERIESLIQSIHIDTTLYTD SDFHPSCRVTAMNCFLLELQVILHEYSNMTLNETVRNVLYLANSTLSSNRNVA ESGCRECEELEERTFTEFLQSFIRIVQMFINTS |
| SEQ ID NO: 304 | METDTLLLWVLLLWVPGSTGDYRDEHHHHHHENLYFQGSREGCASRCTRYN AELERCEARVMSMSNTEEDCEQELEDLLHCLDHCHSQGGGGSGTTCPPPVSIE HADIRVRNYSVNSRERYVCNSGFRRRAGTSTLIECVINRNTNVAHWTTPSLRCI RDPSLAGGGGSGGGGSGGGGSGGGGSNWIDVRYDLERIESLIQSIHIDTTLYTD SDFHPSCRVTAMNCFLLELQVILHEYSNMTLNETVRNVLYLANSTLSSNRNVA ESGCRECEELEERTFTEFLQSFIRIVQMFINTS |
| SEQ ID NO: 305 | METDTLLLWVLLLWVPGSTGDYRDEHHHHHHENLYFQGSREGCASRCMRYN DELERCEARMMSMSNTEEDCEQELEDLLYCLDHCHSQGGGGSGTTCPPPVSIE HADIRVRNYSVNSRERYVCNSGFRRRAGTSTLIECVINRNTNVAHWTTPSLRCI RDPSLAGGGGSGGGGSGGGGSGGGGSNWIDVRYDLERIESLIQSIHIDTTLYTD SDFHPSCRVTAMNCFLLELQVILHEYSNMTLNETVRNVLYLANSTLSSNRNVA ESGCRECEELEERTFTEFLQSFIRIVQMFINTS |
| SEQ ID NO: 306 | DYRDEHHHHHHENLYFQGSREGCASRCTRYNAELERCEARVSSMSNTEETCV QELFDLLHCVDHCVSQGGGGSGTTCPPPVSIEHADIRVRNYSVNSRERYVCNS GFRRRAGTSTLIECVINRNTNVAHWTTPSLRCIRDPSLAGGGGSGGGGSGGGG SGGGGSNWIDVRYDLERIESLIQSIHIDTTLYTDSDFHPSCRVTAMNCFLLELQV ILHEYSNMTLNETVRNVLYLANSTLSSNRNVAESGCRECEELEERTFTEFLQSFI RIVQMFINTS |
| SEQ ID NO: 307 | DYRDEHHHHHHENLYFQGSREGCASRCTRYNAELERCEARVMSMSNTEEDC EQELEDLLHCLDHCHSQGGGGSGTTCPPPVSIEHADIRVRNYSVNSRERYVCN SGFRRRAGTSTLIECVINRNTNVAHWTTPSLRCIRDPSLAGGGGSGGGGSGGG GSGGGGSNWIDVRYDLERIESLIQSIHIDTTLYTDSDFHPSCRVTAMNCFLLELQ VILHEYSNMTLNETVRNVLYLANSTLSSNRNVAESGCRECEELEERTFTEFLQS FIRIVQMFINTS |
| SEQ ID NO: 308 | DYRDEHHHHHHENLYFQGSREGCASRCMRYNDELERCEARMMSMSNTEEDC EQELEDLLYCLDHCHSQGGGGSGTTCPPPVSIEHADIRVRNYSVNSRERYVCN SGFRRRAGTSTLIECVINRNTNVAHWTTPSLRCIRDPSLAGGGGSGGGGSGGG GSGGGGSNWIDVRYDLERIESLIQSIHIDTTLYTDSDFHPSCRVTAMNCFLLELQ VILHEYSNMTLNETVRNVLYLANSTLSSNRNVAESGCRECEELEERTFTEFLQS FIRIVQMFINTS |
| SEQ ID NO: 309 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRIPKPSTPPGSSREGCASRCTKYNAELE KCEARVSSMSNTEETCVQELFDLLHCVDHCVSQ |
| SEQ ID NO: 310 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRIPKPSTPPGSSREGCASRCTKYNAELE KCEARVMSMSNTEEDCEQELEDLLHCLDHCHSQ |
| SEQ ID NO: 311 | METDTLLLWVLLLWVPGSTGDYKDEHHHHHHGSGTTCPPPVSIEHADIRVKN YSVNSRERYVCNSGFKRKAGTSTLIECVINKNTNVAHWTTPSLKCIRDPSLAG GGGSGGGGSGGGGSGGGGSNWIDVRYDLEKIESLIQSIHIDTTLYTDSDFHPSC KVTAMNCFLLELQVILHEYSNMTLNETVRNVLYLANSTLSSNKNVAESGCKE CEELEEKTFTEFLQSFIRIVQMFINTSEPRIPKPSTPPGSSREGCASRCMKYNDEL EKCEARMMSMSNTEEDCEQELEDLLYCLDHCHSQ |
| SEQ ID NO: 312 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRIPKPSTPPGSSREGCASRCTKYNAELEKCEARVSSMSNTEETCVQELFDL LHCVDHCVSQ |
| SEQ ID NO: 313 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRIPKPSTPPGSSREGCASRCTKYNAELEKCEARVMSMSNTEEDCEQELED LLHCLDHCHSQ |
| SEQ ID NO: 314 | DYKDEHHHHHHGSGTTCPPPVSIEHADIRVKNYSVNSRERYVCNSGFKRKAGT STLIECVINKNTNVAHWTTPSLKCIRDPSLAGGGGSGGGGSGGGGSGGGGSNW IDVRYDLEKIESLIQSIHIDTTLYTDSDFHPSCKVTAMNCFLLELQVILHEYSNM TLNETVRNVLYLANSTLSSNKNVAESGCKECEELEEKTFTEFLQSFIRIVQMFIN TSEPRIPKPSTPPGSSREGCASRCMKYNDELEKCEARMMSMSNTEEDCEQELE DLLYCLDHCHSQ |
| SEQ ID NO: 315 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRIPRPSTPPGSSREGCASRCTRYNAELERC EARVSSMSNTEETCVQELFDLLHCVDHCVSQ |
| SEQ ID NO: 316 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRIPRPSTPPGSSREGCASRCTRYNAELERC EARVMSMSNTEEDCEQELEDLLHCLDHCHSQ |
| SEQ ID NO: 317 | METDTLLLWVLLLWVPGSTGDYRDEHHHHHHGSGTTCPPPVSIEHADIRVRN YSVNSRERYVCNSGFRRRAGTSTLIECVINRNTNVAHWTTPSLRCIRDPSLAGG GGSGGGGSGGGGSGGGGSNWIDVRYDLERIESLIQSIHIDTTLYTDSDFHPSCR VTAMNCFLLELQVILHEYSNMTLNETVRNVLYLANSTLSSNRNVAESGCRECE ELEERTFTEFLQSFIRIVQMFINTSEPRIPRPSTPPGSSREGCASRCMRYNDELER CEARMMSMSNTEEDCEQELEDLLYCLDHCHSQ |
| SEQ ID NO: 318 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRIPRPSTPPGSSREGCASRCTRYNAELERCEARVSSMSNTEETCVQELFDL LHCVDHCVSQ |
| SEQ ID NO: 319 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRIPRPSTPPGSSREGCASRCTRYNAELERCEARVMSMSNTEEDCEQELED LLHCLDHCHSQ |
| SEQ ID NO: 320 | DYRDEHHHHHHGSGTTCPPPVSIEHADIRVRNYSVNSRERYVCNSGFRRRAGT STLIECVINRNTNVAHWTTPSLRCIRDPSLAGGGGSGGGGSGGGGSGGGGSNW IDVRYDLERIESLIQSIHIDTTLYTDSDFHPSCRVTAMNCFLLELQVILHEYSNM TLNETVRNVLYLANSTLSSNRNVAESGCRECEELEERTFTEFLQSFIRIVQMFIN TSEPRIPRPSTPPGSSREGCASRCMRYNDELERCEARMMSMSNTEEDCEQELED LLYCLDHCHSQ |
| SEQ ID NO: 321 | METDTLLLWVLLLWVPGSTGDYKDEHHHHHHENLYFQGSREGCASRCTKYN AELEKCEARVSSMSNTEETCVQELFDLLHCVDHCVSQGGGGSGGGGSGTTCP PPVSIEHADIRVKNYSVNSRERYVCNSGFKRKAGTSTLIECVINKNTNVAHWTT PSLKCIRDPSLAGGGGSGGGGSGGGGSGGGGSNWIDVRYDLEKIESLIQSIHIDT TLYTDSDFHPSCKVTAMNCFLLELQVILHEYSNMTLNETVRNVLYLANSTLSS NKNVAESGCKECEELEEKTFTEFLQSFIRIVQMFINTS |
| SEQ ID NO: 322 | METDTLLLWVLLLWVPGSTGDYKDEHHHHHHENLYFQGSREGCASRCTKYN AELEKCEARVMSMSNTEEDCEQELEDLLHCLDHCHSQGGGGSGGGGSGTTCP PPVSIEHADIRVKNYSVNSRERYVCNSGFKRKAGTSTLIECVINKNTNVAHWTT PSLKCIRDPSLAGGGGSGGGGSGGGGSGGGGSNWIDVRYDLEKIESLIQSIHIDT TLYTDSDFHPSCKVTAMNCFLLELQVILHEYSNMTLNETVRNVLYLANSTLSS NKNVAESGCKECEELEEKTFTEFLQSFIRIVQMFINTS |
| SEQ ID NO: 323 | METDTLLLWVLLLWVPGSTGDYKDEHHHHHHENLYFQGSREGCASRCMKYN DELEKCEARMMSMSNTEEDCEQELEDLLYCLDHCHSQGGGGSGGGGSGTTCP PPVSIEHADIRVKNYSVNSRERYVCNSGFKRKAGTSTLIECVINKNTNVAHWTT PSLKCIRDPSLAGGGGSGGGGSGGGGSGGGGSNWIDVRYDLEKIESLIQSIHIDT TLYTDSDFHPSCKVTAMNCFLLELQVILHEYSNMTLNETVRNVLYLANSTLSS NKNVAESGCKECEELEEKTFTEFLQSFIRIVQMFINTS |
| SEQ ID NO: 324 | DYKDEHHHHHHENLYFQGSREGCASRCTKYNAELEKCEARVSSMSNTEETCV QELFDLLHCVDHCVSQGGGGSGGGGSGTTCPPPVSIEHADIRVKNYSVNSRER YVCNSGFKRKAGTSTLIECVINKNTNVAHWTTPSLKCIRDPSLAGGGGSGGGG SGGGGSGGGGSNWIDVRYDLEKIESLIQSIHIDTTLYTDSDFHPSCKVTAMNCF LLELQVILHEYSNMTLNETVRNVLYLANSTLSSNKNVAESGCKECEELEEKTF TEFLQSFIRIVQMFINTS |
| SEQ ID NO: 325 | DYKDEHHHHHHENLYFQGSREGCASRCTKYNAELEKCEARVMSMSNTEEDC EQELEDLLHCLDHCHSQGGGGSGGGGSGTTCPPPVSIEHADIRVKNYSVNSRE RYVCNSGFKRKAGTSTLIECVINKNTNVAHWTTPSLKCIRDPSLAGGGGSGGG GSGGGGSGGGGSNWIDVRYDLEKIESLIQSIHIDTTLYTDSDFHPSCKVTAMNC FLLELQVILHEYSNMTLNETVRNVLYLANSTLSSNKNVAESGCKECEELEEKTF TEFLQSFIRIVQMFINTS |
| SEQ ID NO: 326 | DYKDEHHHHHHENLYFQGSREGCASRCMKYNDELEKCEARMMSMSNTEED CEQELEDLLYCLDHCHSQGGGGSGGGGSGTTCPPPVSIEHADIRVKNYSVNSR ERYVCNSGFKRKAGTSTLIECVINKNTNVAHWTTPSLKCIRDPSLAGGGGSGG GGSGGGGSGGGGSNWIDVRYDLEKIESLIQSIHIDTTLYTDSDFHPSCKVTAMN CFLLELQVILHEYSNMTLNETVRNVLYLANSTLSSNKNVAESGCKECEELEEK TFTEFLQSFIRIVQMFINTS |
| SEQ ID NO: 327 | METDTLLLWVLLLWVPGSTGDYRDEHHHHHHENLYFQGSREGCASRCTRYN AELERCEARVSSMSNTEETCVQELFDLLHCVDHCVSQGGGGSGGGGSGTTCPP PVSIEHADIRVRNYSVNSRERYVCNSGFRRRAGTSTLIECVINRNTNVAHWTTP SLRCIRDPSLAGGGGSGGGGSGGGGSGGGGSNWIDVRYDLERIESLIQSIHIDTT LYTDSDFHPSCRVTAMNCFLLELQVILHEYSNMTLNETVRNVLYLANSTLSSN RNVAESGCRECEELEERTFTEFLQSFIRIVQMFINTS |
| SEQ ID NO: 328 | METDTLLLWVLLLWVPGSTGDYRDEHHHHHHENLYFQGSREGCASRCTRYN AELERCEARVMSMSNTEEDCEQELEDLLHCLDHCHSQGGGGSGGGGSGTTCP PPVSIEHADIRVRNYSVNSRERYVCNSGFRRRAGTSTLIECVINRNTNVAHWTT PSLRCIRDPSLAGGGGSGGGGSGGGGSGGGGSNWIDVRYDLERIESLIQSIHIDT TLYTDSDFHPSCRVTAMNCFLLELQVILHEYSNMTLNETVRNVLYLANSTLSS NRNVAESGCRECEELEERTFTEFLQSFIRIVQMFINTS |
| SEQ ID NO: 329 | METDTLLLWVLLLWVPGSTGDYRDEHHHHHHENLYFQGSREGCASRCMRYN DELERCEARMMSMSNTEEDCEQELEDLLYCLDHCHSQGGGGSGGGGSGTTCP PPVSIEHADIRVRNYSVNSRERYVCNSGFRRRAGTSTLIECVINRNTNVAHWTT PSLRCIRDPSLAGGGGSGGGGSGGGGSGGGGSNWIDVRYDLERIESLIQSIHIDT TLYTDSDFHPSCRVTAMNCFLLELQVILHEYSNMTLNETVRNVLYLANSTLSS NRNVAESGCRECEELEERTFTEFLQSFIRIVQMFINTS |
| SEQ ID NO: 330 | DYRDEHHHHHHENLYFQGSREGCASRCTRYNAELERCEARVSSMSNTEETCV QELFDLLHCVDHCVSQGGGGSGGGGSGTTCPPPVSIEHADIRVRNYSVNSRER YVCNSGFRRRAGTSTLIECVINRNTNVAHWTTPSLRCIRDPSLAGGGGSGGGG SGGGGSGGGGSNWIDVRYDLERIESLIQSIHIDTTLYTDSDFHPSCRVTAMNCF LLELQVILHEYSNMTLNETVRNVLYLANSTLSSNRNVAESGCRECEELEERTFT EFLQSFIRIVQMFINTS |
| SEQ ID NO: 331 | DYRDEHHHHHHENLYFQGSREGCASRCTRYNAELERCEARVMSMSNTEEDC EQELEDLLHCLDHCHSQGGGGSGGGGSGTTCPPPVSIEHADIRVRNYSVNSRE RYVCNSGFRRRAGTSTLIECVINRNTNVAHWTTPSLRCIRDPSLAGGGGSGGG GSGGGGSGGGGSNWIDVRYDLERIESLIQSIHIDTTLYTDSDFHPSCRVTAMNC FLLELQVILHEYSNMTLNETVRNVLYLANSTLSSNRNVAESGCRECEELEERTF TEFLQSFIRIVQMFINTS |
| SEQ ID NO: 332 | DYRDEHHHHHHENLYFQGSREGCASRCMRYNDELERCEARMMSMSNTEEDC EQELEDLLYCLDHCHSQGGGGSGGGGSGTTCPPPVSIEHADIRVRNYSVNSRE RYVCNSGFRRRAGTSTLIECVINRNTNVAHWTTPSLRCIRDPSLAGGGGSGGG GSGGGGSGGGGSNWIDVRYDLERIESLIQSIHIDTTLYTDSDFHPSCRVTAMNC FLLELQVILHEYSNMTLNETVRNVLYLANSTLSSNRNVAESGCRECEELEERTF TEFLQSFIRIVQMFINTS |
| SEQ ID NO: 33 | GSREGCASRCTKYNAELEKCEARVSSMSNTEETCVQELFDLLHCVDHCVSQG SSELYENKPRRPYIL |
| SEQ ID NO: 34 | GSREGCASRCTKYNAELEKCEARVMSMSNTEEDCEQELEDLLHCLDHCHSQG SSELYENKPRRPYIL |
| SEQ ID NO: 35 | GSREGCASRCMKYNDELEKCEARMMSMSNTEEDCEQELEDLLYCLDHCHSQ GSSELYENKPRRPYIL |
| SEQ ID NO: 68 | REGCASRCTKYNAELEKCEARVSSMSNTEETCVQELFDLLHCVDHCVSQGSSE LYENKPRRPYIL |
| SEQ ID NO: 69 | REGCASRCTKYNAELEKCEARVMSMSNTEEDCEQELEDLLHCLDHCHSQGSS ELYENKPRRPYIL |
| SEQ ID NO: 70 | REGCASRCMKYNDELEKCEARMMSMNTEEDCEQLEDLLYCLDHCHSQGS SELYENKPRRPYIL |
| SEQ ID NO: 168 | GSREGCASRCTRYNAELERCEARVSSMSNTEETCVQELFDLLHCVDHCVSQGS SELYENRPRRPYIL |
| SEQ ID NO: 169 | GSREGCASRCTRYNAELERCEARVMSMSNTEEDCEQELEDLLHCLDHCHSQG SSELYENRPRRPYIL |
| SEQ ID NO: 170 | GSREGCASRCMRYNDELERCEARMMSMSNTEEDCEQELEDLLYCLDHCHSQ GSSELYENRPRRPYIL |
| SEQ ID NO: 203 | REGCASRCTRYNAELERCEARVSSMSNTEETCVQELFDLLHCVDHCVSQGSSE LYENRPRRPYIL |
| SEQ ID NO: 204 | REGCASRCTRYNAELERCEARVMSMSNTEEDCEQELEDLLHCLDHCHSQGSS ELYENRPRRPYIL |
| SEQ ID NO: 205 | REGCASRCMRYNDELERCEARMMSMSNTEEDCEQELEDLLYCLDHCHSQGSS ELYENRPRRPYIL |
| SEQ ID NO: 359 | METDTLLLWVLLLWVPGSTGDYKDEHHHHHHGGSQDSTSDLIPAPPLSKVPL QQNFQDNQFQGKWYVVGLAGNAILREDKDPQKMYATIYELKEDKSYNVTSV LFRKKKCDYWIRTFVPGSQPGEFTLGNIKSYPGLTSYLVRVVSTNYNQHAMVF FKKVSQNREYFKITLYGRTKELTSELKENFIRFSKSLGLPENHIVFPVPIDQCIDG GGSENLYFQGSREGCASRCTKYNAELEKCEARVSSMSNTEETCVQELFDLLHC VDHCVSQGSSELYENKPRRPYIL |
| SEQ ID NO: 360 | METDTLLLWVLLLWVPGSTGDYKDEHHHHHHGGSQDSTSDLIPAPPLSKVPL QQNFQDNQFQGKWYVVGLAGNAILREDKDPQKMYATIYELKEDKSYNVTSV LFRKKKCDYWIRTFVPGSQPGEFTLGNIKSYPGLTSYLVRVVSTNYNQHAMVF FKKVSQNREYFKITLYGRTKELTSELKENFIRFSKSLGLPENHIVFPVPIDQCIDG GGSENLYFQGSREGCASRCTKYNAELEKCEARVMSMSNTEEDCEQELEDLLH CLDHCHSQGSSELYENKPRRPYIL |
| SEQ ID NO: 361 | METDTLLLWVLLLWVPGSTGDYKDEHHHHHHGGSQDSTSDLIPAPPLSKVPL QQNFQDNQFQGKWYVVGLAGNAILREDKDPQKMYATIYELKEDKSYNVTSV LFRKKKCDYWIRTFVPGSQPGEFTLGNIKSYPGLTSYLVRVVSTNYNQHAMVF FKKVSQNREYFKITLYGRTKELTSELKENFIRFSKSLGLPENHIVFPVPIDQCIDG GGSENLYFQGSREGCASRCMKYNDELEKCEARMMSMSNTEEDCEQELEDLL YCLDHCHSQGSSELYENKPRRPYIL |

Exemplary peptide-neurotensin constructs are set forth in SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, and SEQ ID NO: 359 - SEQ ID NO: 361. The peptides of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, and SEQ ID NO: 356 - SEQ ID NO: 358 could further be fused to neurotensin. Each of said peptides or peptide-NT fusions may include a peptide of SEQ ID NO: 1, SEQ ID NO: 2, or SEQ ID NO: 32 (or SEQ ID NO: 36, SEQ ID NO: 37, or SEQ ID NO: 67 with no N-terminal GS), and each of which can further include an expression tag or sequence to improve protein expression, secretion, and/or purification, such as the genetic fusion expression tag siderocalin. In some embodiments, the TfR-binding peptides of the present disclosure are conjugated to, linked to, or fused to anti-cancer drugs that can include an EGFRvIII inhibitor, Copiktra (duvelisib), Erleada (apalutamide), Libtayo (cemiplimab-rwlc), Lorbrena (lorlatinib), Lumoxiti (moxetumomab pasudotox-tdf), Lutathera (lutetium Lu 177 dotatate), Talzenna (talazoparib), Vizimpro (dacomitinib), Aliqopa (copanlisib), Alunbrig (brigatinib), Bavencio (avelumab), Besponsa (inotuzumab ozogamicin), Calquence (acalabrutinib), IDHIFA (enasidenib), Imfinzi (durvalumab), Kisqali (ribociclib), Kymriah (tisagenlecleucel), Nerlynx (neratinib), Rydapt (midostaurin),Vyxeos (daunorubicin and cytarabine), Xermelo (telotristat ethyl), Yescarta (axicabtagene ciloleucel), Zejula (niraparib), Cabometyx (cabozantinib), Keytruda (pembrolizumab), Lartruvo (olaratumab), Lenvima (lenvatinib), Opdivo (nivolumab), Rubraca (rucaparib), Sustol (granisetron), Syndros (dronabinol oral solution), Tecentriq (atezolizumab), Venclexta (venetoclax), Alecensa (alectinib), Cotellic (cobimetinib), Darzalex (daratumumab), Empliciti (elotuzumab), Farydak (panobinostat), Ibrance (palbociclib), Imlygic (talimogene laherparepvec), Lenvima (lenvatinib), Lonsurf (trifluridine and tipiracil), Ninlaro (ixazomib), Odomzo (sonidegib), Onivyde (irinotecan liposome injection), Portrazza (necitumumab), Tagrisso (osimertinib), Unituxin (dinutuximab), Varubi (rolapitant), Vistogard (uridine triacetate), Yondelis (trabectedin), Akynzeo (netupitant and palonosetron), Beleodaq (belinostat), Blincyto (blinatumomab), Cyramza (ramucirumab), Imbruvica (ibrutinib), Lynparza (olaparib), Zydelig (idelalisib), Zykadia (ceritinib), Gazyva (obinutuzumab), Gilotrif (afatinib), Imbruvica (ibrutinib), Kadcyla (ado-trastuzumab emtansine), Mekinist (trametinib), Pomalyst (pomalidomide), Revlimid (lenalidomide), Stivarga (regorafenib), Tafinlar (dabrafenib), Valchlor (mechlorethamine) gel, Xgeva (denosumab), Xofigo (radium Ra 223 dichloride), Abraxane (paclitaxel protein-bound particles for injectable suspension), Afinitor (everolimus), Afinitor (everolimus), Bosulif (bosutinib), Cometriq (cabozantinib), Erivedge (vismodegib), Iclusig (ponatinib), Inlyta (axitinib), Kyprolis (carfilzomib), Marqibo (vinCRIStine sulfate LIPOSOME injection), Neutroval (tbo-filgrastim), Perjeta (pertuzumab), Picato (ingenol mebutate) gel, Stivarga (regorafenib), Subsys (fentanyl sublingual spray), Synribo (omacetaxine mepesuccinate), Votrient (pazopanib), Xtandi (enzalutamide), Zaltrap (ziv-aflibercept), Abstral (fentanyl sublingual tablets), Adcetris (brentuximab vedotin), Afinitor (everolimus), Erwinaze (asparaginase Erwinia chrysanthemi), Lazanda (fentanyl citrate) nasal spray, Sutent (sunitinib malate), Sylatron (peginterferon alfa-2b), Vandetanib (vandetanib), Xalkori (crizotinib), Yervoy (ipilimumab), Zelboraf (vemurafenib), Zytiga (abiraterone acetate), Halaven (eribulin mesylate), Herceptin (trastuzumab), Jevtana (cabazitaxel), Provenge (sipuleucel-T), Xgeva (denosumab), Zuplenz (ondansetron oral soluble film), Afinitor (everolimus), Arzerra (ofatumumab), Avastin (bevacizumab), Cervarix [Human Papillomavirus Bivalent (Types 16 and 18) Vaccine, Recombinant, Elitek (rasburicase), Folotyn (pralatrexate injection), Istodax (romidepsin), Onsolis (fentanyl buccal), Votrient (pazopanib), Degarelix (degarelix for injection), Fusilev (levoleucovorin); Mozobil (plerixafor injection), Sancuso (granisetron), Treanda (bendamustine hydrochloride), Evista (raloxifene hydrochloride), Hycamtin (topotecan hydrochloride), Ixempra (ixabepilone), Tasigna (nilotinib hydrochloride monohydrate), Torisel (temsirolimus), Tykerb (lapatinib), Gardasil (quadrivalent human papillomavirus (types 6, 11, 16, 18) recombinant vaccine), Sprycel (dasatinib), Sutent (sunitinib), Vectibix (panitumumab), Arranon (nelarabine), Nexavar (sorafenib), Alimta (pemetrexed for injection), Avastin (bevacizumab); Clolar (clofarabine), Erbitux (cetuximab), Sensipar (cinacalcet), Tarceva (erlotinib, OSI 774), Aloxi (palonosetron), Emend (aprepitant), Iressa (gefitinib); Plenaxis (abarelix for injectable suspension); Premarin (conjugated estrogens); UroXatral (alfuzosin HCl extended-release tablets); Velcade (bortezomib); Eligard (leuprolide acetate); Eloxatin (oxaliplatin/5-fluorouracil/leucovorin); Faslodex (fulvestrant); Gleevec (imatinib mesylate); Neulasta; SecreFlo (secretin); Zevalin (ibritumomab tiuxetan); Zometa (zoledronic acid); Campath; Femara (letrozole); Gleevec (imatinib mesylate); Kytril (granisetron) solution; Trelstar LA (triptorelin pamoate); Xeloda; Zometa (zoledronic acid); Mylotarg (gemtuzumab ozogamicin); Trelstar Depot (triptorelin pamoate);Trisenox (arsenic trioxide); Viadur (leuprolide acetate implant); Aromasin Tablets; Busulflex; Doxil (doxorubicin HCl liposome injection); Ellence; Ethyol (amifostine); Temodar; UVADEX Sterile Solution; Zofran; Actiq; Anzemet; Camptosar; Gemzar (gemcitabine HCL); Herceptin; Inform HER-2/neu breast cancer test; Neupogen; Nolvadex; Photofrin; Proleukin; Sclerosol Intrapleural Aerosol; Valstar; Xeloda; Zofran; Anzemet; Bromfenac; Femara (letrozole); Gliadel Wafer (polifeprosan 20 with carmustine implant); Intron A (interferon alfa-2b, recombinant); Kytril (granisetron) tablets; Lupron Depot (leuprolide acetate for depot suspension); Miraluma test; Neumega; Quadramet (Samarium Sm 153 Lexidronam Injection); Rituxan; Taxol; Anexsia; Aredia (pamidronate disodium for injection); Arimidex (anastrozole); Campostar; Elliotts B Solution (buffered intrathecal electrolyte/dextrose injection); Eulexin (flutamide); Gemzar (gemcitabine HCL); Hycamtin (topotecan hydrochloride); Kadian; Leukine (sargramostim); Lupron Depot (leuprolide acetate for depot suspension); Photodynamic Therapy; Taxotere (Docetaxel); Visipaque (iodixanol); Ethyol (amifostine); Intron A (Interferon alfa-2b, recombinant), and Leukine (sargramostim).In some embodiments, the TfR-binding peptides of the present disclosure are conjugated to, linked to, or fused to Abemaciclib, Acalabrutinib, Afatinib, Alectinib, Axitinib, Baricitinib, Binimetinib, Bosutinib, Brigatinib, Cabozantinib, Ceritinib, Cobimetinib, Crizotinib, Dabrafenib, dacomitinib, Dasatinib, encorafenib, Erlotinib, Everolimus, Fostamatinib, Gefitinib, Ibrutinib, Imatinib, Lapatinib, Lenvatinib, Lorlatinib, Midostaurin, Neratinib, Nilotinib, Nintedanib, Osimertinib, Palbociclib, Pazopanib, Ponatinib, Regorafenib, Ribociclib, Ruxolitinib, Sirolimus, Sorafenib, Sunitinib, Temsirolimus, Tofacitinib, Trametinib, Vandetanib, Vemurafenib, an IL15, a fused IL15/IL15Ra, an IFNgamma, and anti-CD3 agents, or fragment(s) thereof.

In some embodiments, the TfR-binding peptides as described herein can be used to treat and/or prevent an infection including infections of the central nervous system. In some embodiments, the TfR-binding peptides of the present disclosure are conjugated to, linked to, or fused to an anti-infective agent. Anti-infective agents can include antibiotic, antiviral, and antifungal agents. Anti-infective agent that can be used include artemisinin, Aemcolo (rifamycin), Biktarvy (bictegravir/emtricitabine/tenofovir alafenamide), Nuzyra (omadacycline) , Trogarzo (ibalizumab-uiyk), Xofluza (baloxavir marboxil), Baxdela (delafloxacin) tablets and injection, Benznidazole, Giapreza (angiotensin II), Heplisav-B [Hepatitis B Vaccine (Recombinant), Adjuvanted] , Juluca (dolutegravir and rilpivirine), KedRab [Rabies Immune Globulin (Human)], Mavyret (glecaprevir and pibrentasvir), Prevymis (letermovir), Shingrix (Zoster Vaccine Recombinant, Adjuvanted), Solosec (secnidazole), Vabomere (meropenem and vaborbactam), Xepi (ozenoxacin), Anthim (obiltoxaximab), Descovy (emtricitabine and tenofovir alafenamide), Epclusa (sofosbuvir and velpatasvir), Odefsey (emtricitabine, rilpivirine, and tenofovir alafenamide), Vaxchora (Cholera Vaccine, Live, Oral), Vemlidy (tenofovir alafenamide), Zepatier (elbasvir and grazoprevir), Zinplava (bezlotoxumab), Avycaz (ceftazidime-avibactam), Bexsero (Meningococcal Group B Vaccine), Cresemba (isavuconazonium sulfate), Daklinza (daclatasvir), Evotaz (atazanavir and cobicistat), Fluad (trivalent influenza vaccine), Genvoya (elvitegravir, cobicistat, emtricitabine, and tenofovir alafenamide), Prezcobix (darunavir and cobicistat), Technivie, (ombitasvir, paritaprevir and ritonavir), Dalvance (dalbavancin), Harvoni (ledipasvir and sofosbuvir), Impavido (miltefosine), Kerydin (tavaborole), Orbactiv (oritavancin), Rapivab (peramivir injection), Sivextro (tedizolid phosphate), Triumeq (abacavir, dolutegravir, and lamivudine), Zerbaxa (ceftolozane + tazobactam), Flublok (seasonal influenza vaccine), Luzu (luliconazole) Cream 1%, Olysio (simeprevir), Sitavig (acyclovir) buccal tablets, Sovaldi (sofosbuvir), VariZIG, Varicella Zoster Immune Globulin (Human), Abthrax (raxibacumab), Afinitor (everolimus), Cystaran (cysteamine hydrochloride), Dymista (azelastine hydrochloride and fluticasone propionate), Flucelvax, Influenza Virus Vaccine, Jetrea (ocriplasmin), Linzess (linaclotide), Mytesi (crofelemer), Sirturo (bedaquiline), Sklice (ivermectin) lotion, Stribild (elvitegravir, cobicistat, emtricitabine, tenofovir disoproxil fumarate), Tudorza Pressair (aclidinium bromide inhalation powder), Afinitor (everolimus), Complera (emtricitabine/rilpivirine/tenofovir disoproxil fumarate), Dificid (fidaxomicin), Edurant (rilpivirine), Eylea (aflibercept), Firazyr (icatibant), Gralise (gabapentin), Incivek (telaprevir), Victrelis (boceprevir), Egrifta (tesamorelin for injection), Menveo (meningitis vaccine), Zymaxid (gatifloxacin ophthalmic solution), Afinitor (everolimus), Bepreve (bepotastine besilate ophthalmic solution), Hiberix (Haemophilus b Conjugate Vaccine, Tetanus Toxoid Conjugate), Vibativ (telavancin), Aptivus (tipranavir), Astepro (azelastine hydrochloride nasal spray), Intelence (etravirine), Patanase (olopatadine hydrochloride), Viread (tenofovir disoproxil fumarate), Isentress (raltegravir), Selzentry (maraviroc), Veramyst (fluticasone furoate), Xyzal (levocetirizine dihydrochloride), Eraxis (anidulafungin), Noxafil (posaconazole), Prezista (darunavir), Tyzeka (telbivudine), Veregen (kunecatechins), Aptivus (tipranavir), Baraclude (entecavir), FluMist ( Influenza Virus Vaccine), Fuzeon (enfuvirtide), Lexiva (fosamprenavir calcium), Reyataz (atazanavir sulfate), Botox Cosmetic (botulinum toxin type A), Clarinex, Hepsera (adefovir dipivoxil), Pediarix Vaccine, Pegasys (peginterferon alfa-2a), Sustiva, Vfend (voriconazole), Peg-Intron (peginterferon alfa-2b), Rebetol (ribavirin), Spectracef, Tavist (clemastine fumarate), Twinrix, Valcyte (valganciclovir HCl), Viread (tenofovir disoproxil fumarate), Xigris (drotrecogin alfa [activated]), ABREVA (docosanol), Cefazolin and Dextrose USP, Children's Motrin Cold, REBETRON (TM) Combination Therapy, Synagis, Viroptic, Aldara (imiquimod), Ceftin (cefuroxime axetil), Combivir, Famvir (famciclovir), Floxin otic, Fortovase, INFERGEN (interferon alfacon-1), Intron A (interferon alfa-2b, recombinant), Taxol, Timentin, Trovan, VIRACEPT (nelfinavir mesylate), Zerit (stavudine), AK-Con-A (naphazoline ophthalmic), Allegra (fexofenadine hydrochloride), Atrovent (ipratropium bromide), Augmentin (amoxicillin/clavulanate), Crixivan (Indinavir sulfate), Elmiron (pentosan polysulfate sodium), Havrix, IvyBlock, Leukine (sargramostim), Merrem I.V. (meropenem), Tavist (clemastine fumarate), Videx (didanosine), Viramune (nevirapine), Zithromax (azithromycin), Cedax (ceftibuten), Clarithromycin (Biaxin), Epivir (lamivudine), Invirase (saquinavir), Valtrex (valacyclovir HCl), Zerit (stavudine), Zyrtec (cetirizine HCl), or a functional fragment thereof.

In some embodiments, the TfR-binding peptides as described herein can be used to treat and/or prevent an inflammation including inflammation of the central nervous system and the gastrointestinal (GI) tract. In some embodiments, the TfR-binding peptides of the present disclosure are conjugated to, linked to, or fused to an anti-inflammatory agent. Anti-inflammatory agents can include steroidal and nonsteroidal anti-inflammatory agents. Nonsteroidal anti-inflammatory agents can include aspirin, celecoxib, diclofenac, diflunisal, etodolac, ibuprofen, indomethacin, ketoprofen, ketorolac, nabumetone, naproxen, oxaprozin, piroxicam, salsalate, sulindac, tolmetin, piroxicam, flurbiprofen, and fenoprofen, Anti-inflammatory drugs that can be used in combiantion with the herein disclosed methods and compositions can include corticosteroids and aminosalicylates including but not limited to mesalamine, balsalazide, and olsalazine, In various cases, anti-inflammatory drugs include tumor necrosis factor (TNF)-alpha inhibitors such as infliximab, adalimumab, and golimumab, and biological drug (e.g., antibodies, fragments or derivatives thereof) targeting other biological targets including natalizumab, vedolizumab, and ustekinumab.

As compared to antibody-drug conjugates (e.g., Adcetris, Kadcyla, Mylotarg) or transferrin, in some aspects the peptide conjugated to, linked to, or fused to an active agent as described herein can exhibit better penetration of solid tumors or of the CNS due to its smaller size. In certain aspects, the peptide conjugated to, linked to, or fused to an active agent as described herein can carry different or higher doses of active agents as compared to antibody-drug conjugates. In some embodiments, the peptide as described conjugated to, linked to, or fused to an active agent as described herein can deliver higher doses of an active agent to skeletal muscle tissue compared to antibody-drug conjugates. Thus, treatment of muscular dystrophy, SBMA, or muscle-derived sarcomas can be of particular interest using the peptides of the present disclosure. In some aspects, the TfR-binding peptides as described herein can exhibit significantly lower plasma half-lives compared to antibodies or antibody-drug conjugates, and thus can allow for a more rapid peripheral clearance (can be advantageous e.g., when radiolabeled conjugates are used providing significantly lower organ dosimetries). In still other aspects, the peptide conjugated to, linked to, or fused to an active agent as described herein can have better site specific delivery of defined drug ratio as compared to antibody-drug conjugates. In other aspects, the peptide can be amenable to solvation in organic solvents (in addition to water), which can allow more synthetic routes for solvation and conjugation of a drug (which often has low aqueous solubility) and higher conjugation yields, higher ratios of drug conjugated to, linked to, or fused to peptide (versus an antibody), and/or reduce aggregate/high molecular weight species formation during conjugation. Additionally, a unique amino acid residue(s) can be introduced into the peptide via a residue that is not otherwise present in the short sequence or via inclusion of a non-natural amino acid, allowing site specific conjugation to the peptide.

The peptides or peptide constructs of the present disclosure can also be conjugated to, linked to, or fused to other moieties that can serve other roles, such as providing an affinity handle (e.g., biotin) for retrieval of the peptides from tissues or fluids. For example, peptides or peptide constructs of the present disclosure can also be conjugated to, linked to, or fused to biotin. In addition to extension of half-life, biotin can also act as an affinity handle for retrieval of peptides or peptide constructs from tissues or other locations. In some embodiments, fluorescent biotin conjugates that can act both as a detectable label and an affinity handle can be used. Non limiting examples of commercially available fluorescent biotin conjugates can include Atto 425-Biotin, Atto 488-Biotin, Atto 520-Biotin, Atto-550 Biotin, Atto 565-Biotin, Atto 590-Biotin, Atto 610-Biotin, Atto 620-Biotin, Atto 655-Biotin, Atto 680-Biotin, Atto 700-Biotin, Atto 725-Biotin, Atto 740-Biotin, fluorescein biotin, biotin-4-fluorescein, biotin-(5-fluorescein) conjugate, and biotin-B-phycoerythrin, Alexa fluor 488 biocytin, Alexa flour 546, Alexa Fluor 549, lucifer yellow cadaverine biotin-X, Lucifer yellow biocytin, Oregon green 488 biocytin, biotin-rhodamine and tetramethylrhodamine biocytin. In some other examples, the conjugates can include chemiluminescent compounds, colloidal metals, luminescent compounds, enzymes, radioisotopes, and paramagnetic labels. In some embodiments, the peptide described herein can also be attached to another molecule. For example, the peptide sequence also can be attached to another active agent (e.g., small molecule, peptide, polypeptide, polynucleotide, antibody, aptamer, cytokine, growth factor, neurotransmitter, an active fragment or modification of any of the preceding agents, fluorophore, radioisotope, radionuclide chelator, acyl adduct, chemical linker, or sugar). In some embodiments, the peptide can be conjugated to, linked to, or fused with, or covalently or non-covalently linked to an active agent.

In some embodiments, peptides or peptide constructs of the present disclosure can also be conjugated to, linked to, or fused to other affinity handles. Other affinity handles may include genetic fusion affinity handles. Genetic fusion affinity handles may include 6xHis (HHHHHH (SEQ ID NO: 345); immobilized metal affinity column purification possible), FLAG (DYKDDDDK (SEQ ID NO: 346); anti-FLAG immunoprecipitation), "shorty" FLAG (DYKDE (SEQ ID NO: 347); anti-FLAG immunoprecipitation), hemagglutinin (YPYDVPDYA (SEQ ID NO: 348); anti-HA immunoprecipitation), and streptavidin binding peptide (DVEAWLGAR (SEQ ID NO: 349); streptavidin-mediated precipitation). In some embodiments, peptides or peptide constructs of the present disclosure can also be conjugated to, linked to, or fused to an expression tag or sequence to improve protein expression and/or purification. Such expression tags may include genetic fusion expression tags. Genetic fusion expression tags may include siderocalin (SEQ ID NO: 351, METDTLLLWVLLLWVPGSTGDYKDEHHHHHHGGSQDSTSDLIPAPPLSKVPLQQNFQD NQFQGKWYVVGLAGNAILREDKDPQKMYATIYELKEDKSYNVTSVLFRKKKCDYWIR TFVPGSQPGEFTLGNIKSYPGLTSYLVRVVSTNYNQHAMVFFKKVSQNREYFKITLYGRT KELTSELKENFIRFSKSLGLPENHIVFPVPIDQCIDGGGSENLYFQ ). Exemplary siderocalin peptide fusions include human soluble transferrin fused to siderocalin (METDTLLLWVLLLWVPGSTGDYKDEHHHHHHGGSQDSTSDLIPAPPLSKVPLQQNFQD NQFQGKWYVVGLAGNAILREDKDPQKMYATIYELKEDKSYNVTSVLFRKKKCDYWIR TFVPGSQPGEFTLGNIKSYPGLTSYLVRVVSTNYNQHAMVFFKKVSQNREYFKITLYGRT KELTSELKENFIRFSKSLGLPENHIVFPVPIDQCIDGGGSENLYFQGSRLYWDDLKRKLSE KLDSTDFTSTIKLLNENSYVPREAGSQKDENLALYVENQFREFKLSKVWRDQHFVKIQV KDSAQNSVIIVDKNGRLVYLVENPGGYVAYSKAATVTGKLVHANFGTKKDFEDLYTPV NGSIVIVRAGKITFAEKVANAESLNAIGVLIYMDQTKFPIVNAELSFFGHAHLGTGDPYTP GFPSFNHTQFPPSRSSGLPNIPVQTISRAAAEKLFGNMEGDCPSDWKTDSTCRMVTSESK NVKLTVSNVLKEIKILNIFGVIKGFVEPDHYVVVGAQRDAWGPGAAKSGVGTALLLKLA QMFSDMVLKDGFQPSRSIIFASWSAGDFGSVGATEWLEGYLSSLHLKAFTYINLDKAVL GTSNFKVSASPLLYTLIEKTMQNVKHPVTGQFLYQDSNWASKVEKLTLDNAAFPFLAYS GIPAVSFCFCEDTDYPYLGTTMDTYKELIERIPELNKVARAAAEVAGQFVIKLTHDVELN LDYERYNSQLLSFVRDLNQYRADIKEMGLSLQWLYSARGDFFRATSRLTTDFGNAEKT DRFVMKKLNDRVMRVEYHFLSPYVSPKESPFRHVFWGSGSHTLPALLENLKLRKQNNG AFNETLFRNQLALATWTIQGAANALSGDVWDIDNEFGGGSHHHHHHGGGSLNDIFEAQ KIEWHE; SEQ ID NO: 352) and various peptides of the present disclosure fused to siderocalin, such as 
METDTLLLWVLLLWVPGSTGDYKDEHHHHHHGGSQDSTSDLIPAPPLSKVPLQQNFQD
NQFQGKWYVVGLAGNAILREDKDPQKMYATIYELKEDKSYNVTSVLFRKKKCDYWIR
 
TFVPGSQPGEFTLGNIKSYPGLTSYLVRVVSTNYNQHAMVFFKKVSQNREYFKITLYGRT
KELTSELKENFIRFSKSLGLPENHIVFPVPIDQCIDGGGSENLYFQGSREGCASRCTKYNA
ELEKCEARVSSMSNTEETCVQELFDLLHCVDHCVSQ) (SEQ ID NO: 356),
 
METDTLLLWVLLLWVPGSTGDYKDEHHHHHHGGSQDSTSDLIPAPPLSKVPLQQNFQD
NQFQGKWYVVGLAGNAILREDKDPQKMYATIYELKEDKSYNVTSVLFRKKKCDYWIR
TFVPGSQPGEFTLGNIKSYPGLTSYLVRVVSTNYNQHAMVFFKKVSQNREYFKITLYGRT
KELTSELKENFIRFSKSLGLPENHIVFPVPIDQCIDGGGSENLYFQGSREGCASRCTKYNA
ELEKCEARVMSMSNTEEDCEQELEDLLHCLDHCHSQ (SEQ ID NO: 357),
 and (METDTLLLWVLLLWVPGSTGDYKDEHHHHHHGGSQDSTSDLIPAPPLSKVPLQQNFQD NQFQGKWYVVGLAGNAILREDKDPQKMYATIYELKEDKSYNVTSVLFRKKKCDYWIR TFVPGSQPGEFTLGNIKSYPGLTSYLVRVVSTNYNQHAMVFFKKVSQNREYFKITLYGRT KELTSELKENFIRFSKSLGLPENHIVFPVPIDQCIDGGGSENLYFQGSREGCASRCMKYND ELEKCEARMMSMSNTEEDCEQELEDLLYCLDHCHSQ (SEQ ID NO: 358).. Notably CDP-NT peptide constructs can be expressed with N-terminal siderocalin (e.g., SEQ ID NO: 359 - SEQ ID NO: 361).

In some embodiments, peptides or peptide constructs of the present disclosure can also be conjugated to, linked to, or fused to other moieties that can serve other roles, such as providing an affinity handle (e.g., biotin) for recruiting a non-conjugated therapeutic to or into the tissue that is targeted such as a tumor. In some cases, the therapeutic is a small molecule, peptide, antibody, or cellular therapeutic (e.g., a CAR T cell or another autologous or allogeneic engineered cell). For example, CAR T cells engineered with an affinity label (e.g., avidin) can be recruited to tissues that are targeted by the TfR-binding peptides that comprise a biotin moiety. In addition, biotin- (or other affinity labeled)-TfR-binding peptides as described herein can be administered (e.g., co-administered) with a variety of different avidin-tagged drugs, such as small molecules, peptides, or proteins that can provide pharmacologic activity in the brain, a tumor, or other tissues that the TfR-binding peptides target or accumulate in.

Additionally, more than one peptide sequence derived from a toxin or knotted venom protein can be present on, conjugated to, linked to, or fused with a particular peptide. A peptide can be incorporated into a biomolecule by various techniques. A peptide can be incorporated by a chemical transformation, such as the formation of a covalent bond, such as an amide bond. A peptide can be incorporated, for example, by solid phase or solution phase peptide synthesis. A peptide can be incorporated by preparing a nucleic acid sequence encoding the biomolecule, wherein the nucleic acid sequence includes a subsequence that encodes the peptide. The subsequence can be in addition to the sequence that encodes the biomolecule, or can substitute for a subsequence of the sequence that encodes the biomolecule.

### Detectable Agent Peptide Conjugates

A peptide can be conjugated to, linked to, or fused to an agent used in imaging, research, therapeutics, theranostics, pharmaceuticals, chemotherapy, chelation therapy, targeted drug delivery, and radiotherapy. In some embodiments, a peptide is conjugated to, linked to, or fused with detectable agents, such as a fluorophore, a near-infrared dye, a contrast agent, a nanoparticle, a metal-containing nanoparticle, a metal chelate, an X-ray contrast agent, a PET agent, a metal, a radioisotope, a dye, radionuclide chelator, or another suitable material that can be used in imaging.

In some embodiments, 1, 2, 3, 4, 5, 6, 7, 8, 9, or 10 detectable agents can be linked to a peptide. Non-limiting examples of radioisotopes include alpha emitters, beta emitters, positron emitters, and gamma emitters. In some embodiments, the metal or radioisotope is selected from the group consisting of actinium, americium, bismuth, cadmium, cesium, cobalt, europium, gadolinium, iridium, lead, lutetium, manganese, palladium, polonium, radium, ruthenium, samarium, strontium, technetium, thallium, and yttrium. In some embodiments, the metal is actinium, bismuth, lead, radium, strontium, samarium, or yttrium. In some embodiments, the radioisotope is actinium-225 or lead-212. In some embodiments, the near-infrared dyes are not easily quenched by biological tissues and fluids. In some embodiments, the fluorophore is a fluorescent agent emitting electromagnetic radiation at a wavelength between 650 nm and 4000 nm, such emissions being used to detect such agent. Non-limiting examples of fluorescent dyes that could be used as a conjugating molecule in the present disclosure include DyLight-680, DyLight-750, VivoTag-750, DyLight-800, IRDye-800, VivoTag-680, Cy5.5, or indocyanine green (ICG). In some embodiments, near infrared dyes often include cyanine dyes (e.g., Cy7, Cy5.5, and Cy5). Additional non-limiting examples of fluorescent dyes for use as a conjugating molecule in the present disclosure include acradine orange or yellow, Alexa Fluors (e.g., Alexa Fluor 790, 750, 700, 680, 660, and 647) and any derivative thereof, 7-actinomycin D, 8-anilinonaphthalene-1-sulfonic acid, ATTO dye and any derivative thereof, auramine-rhodamine stain and any derivative thereof, bensantrhone, bimane, 9-10-bis(phenylethynyl)anthracene, 5,12 - bis(phenylethynyl)naththacene, bisbenzimide, brainbow, calcein, carbodyfluorescein and any derivative thereof, 1-chloro-9,10-bis(phenylethynyl)anthracene and any derivative thereof, DAPI, DiOC6, DyLight Fluors and any derivative thereof, epicocconone, ethidium bromide, FlAsH-EDT2, Fluo dye and any derivative thereof, FluoProbe and any derivative thereof, Fluorescein and any derivative thereof, Fura and any derivative thereof, GelGreen and any derivative thereof, GelRed and any derivative thereof, fluorescent proteins and any derivative thereof, m isoform proteins and any derivative thereof such as for example mCherry, hetamethine dye and any derivative thereof, hoeschst stain, iminocoumarin, indian yellow, indo-1 and any derivative thereof, laurdan, lucifer yellow and any derivative thereof, luciferin and any derivative thereof, luciferase and any derivative thereof, mercocyanine and any derivative thereof, nile dyes and any derivative thereof, perylene, phloxine, phyco dye and any derivative thereof, propium iodide, pyranine, rhodamine and any derivative thereof, ribogreen, RoGFP, rubrene, stilbene and any derivative thereof, sulforhodamine and any derivative thereof, SYBR and any derivative thereof, synapto-pHluorin, tetraphenyl butadiene, tetrasodium tris, Texas Red, Titan Yellow, TSQ, umbelliferone, violanthrone, yellow fluroescent protein and YOYO-1. Other Suitable fluorescent dyes include, but are not limited to, fluorescein and fluorescein dyes (e.g., fluorescein isothiocyanine or FITC, naphthofluorescein, 4', 5'-dichloro-2',7' -dimethoxyfluorescein, 6-carboxyfluorescein or FAM, etc.), carbocyanine, merocyanine, styryl dyes, oxonol dyes, phycoerythrin, erythrosin, eosin, rhodamine dyes (e.g., carboxytetramethyl-rhodamine or TAMRA, carboxyrhodamine 6G, carboxy-X-rhodamine (ROX), lissamine rhodamine B, rhodamine 6G, rhodamine Green, rhodamine Red, tetramethylrhodamine (TMR), etc.), coumarin and coumarin dyes (e.g., methoxycoumarin, dialkylaminocoumarin, hydroxycoumarin, aminomethylcoumarin (AMCA), etc.), Oregon Green Dyes (e.g., Oregon Green 488, Oregon Green 500, Oregon Green 514, etc.), Texas Red, Texas Red-X, SPECTRUM RED, SPECTRUM GREEN, cyanine dyes (e.g., CY-3, Cy-5, CY-3.5, CY-5.5, etc.), ALEXA FLUOR dyes (e.g., ALEXA FLUOR 350, ALEXA FLUOR 488, ALEXA FLUOR 532, ALEXA FLUOR 546, ALEXA FLUOR 568, ALEXA FLUOR 594, ALEXA FLUOR 633, ALEXA FLUOR 660, ALEXA FLUOR 680, etc.), BODIPY dyes (e.g., BODIPY FL, BODIPY R6G, BODIPY TMR, BODIPY TR, BODIPY 530/550, BODIPY 558/568, BODIPY 564/570, BODIPY 576/589, BODIPY 581/591, BODIPY 630/650, BODIPY 650/665, etc.), IRDyes (e.g., IRD40, IRD 700, IRD 800, etc.), and the like. Additional suitable detectable agents are described in PCT/US14/56177. Non-limiting examples of radioisotopes include alpha emitters, beta emitters, positron emitters, and gamma emitters. In some embodiments, the metal or radioisotope is selected from the group consisting of actinium, americium, bismuth, cadmium, cesium, cobalt, europium, gadolinium, iridium, lead, lutetium, manganese, palladium, polonium, radium, ruthenium, samarium, strontium, technetium, thallium, and yttrium. In some embodiments, the metal is actinium, bismuth, lead, radium, strontium, samarium, or yttrium. In some embodiments, the radioisotope is actinium-225 or lead-212. Additionally, the following radionuclides can be used for diagnosis and/or therapy: carbon (e.g., ¹¹C or ¹⁴C), nitrogen (e.g., ¹³N), fluorine (e.g., ¹⁸F), gallium (e.g., ⁶⁷Ga or ⁶⁸Ga), copper (e.g., ⁶⁴Cu or ⁶⁷Cu), zirconium (e.g., ⁸⁹Zr), lutetium (e.g., ¹⁷⁷Lu).

Peptides can be conjugated to, linked to, or fused to a radiosensitizer or photosensitizer. Examples of radiosensitizers include but are not limited to: ABT-263, ABT-199, WEHI-539, paclitaxel, carboplatin, cisplatin, oxaliplatin, gemcitabine, etanidazole, misonidazole, tirapazamine, and nucleic acid base derivatives (e.g., halogenated purines or pyrimidines, such as 5-fluorodeoxyuridine). Examples of photosensitizers can include but are not limited to: fluorescent molecules or beads that generate heat when illuminated, nanoparticles, porphyrins and porphyrin derivatives (e.g., chlorins, bacteriochlorins, isobacteriochlorins, phthalocyanines, and naphthalocyanines), metalloporphyrins, metallophthalocyanines, angelicins, chalcogenapyrrillium dyes, chlorophylls, coumarins, flavins and related compounds such as alloxazine and riboflavin, fullerenes, pheophorbides, pyropheophorbides, cyanines (e.g., merocyanine 540), pheophytins, sapphyrins, texaphyrins, purpurins, porphycenes, phenothiaziniums, methylene blue derivatives, naphthalimides, nile blue derivatives, quinones, perylenequinones (e.g., hypericins, hypocrellins, and cercosporins), psoralens, quinones, retinoids, rhodamines, thiophenes, verdins, xanthene dyes (e.g., eosins, erythrosins, rose bengals), dimeric and oligomeric forms of porphyrins, and prodrugs such as 5-aminolevulinic acid. Advantageously, this approach can allow for highly specific targeting of diseased cells (e.g., cancer cells) using both a therapeutic agent (e.g., drug) and electromagnetic energy (e.g., radiation or light) concurrently. In some embodiments, the peptide is conjugated to, linked to, fused with, or covalently or non-covalently linked to the agent, e.g., directly or via a linker. Exemplary linkers suitable for use with the embodiments herein are discussed in further detail below.

In some aspects of the present disclosure, the radionuclide is attached to a peptide or peptide construct as described herein using a chelator. Exemplary chelator moieties can include 2,2',2"-(3-(4-(3-(1-(4-(1,2,4,5-tetrazin-3-yl)phenyl)-1-oxo-5,8,11,14,17,20,23-heptaoxa-2-azapentacosan-25-yl)thioureido)benzyl)-1,4,7-triazonane-2,5,8-triyl)triacetic acid; 2,2',2"-(3-(4-(3-(1-(4-(1,2,4,5-tetrazin-3-yl)phenyl)-1-oxo-5,8,11,14,17,20,23,26,29,32,35-undecaoxa-2-azaheptatriacontan-37-yl)thioureido)benzyl)-1,4,7-triazonane-2,5,8-triyl)triacetic acid; 2,2'-(7-(4-(3-(1-(4-(1,2,4,5-tetrazin-3-yl)phenyl)-1-oxo-5,8,11,14,17,20,23,26,29,32,35-undecaoxa-2-azaheptatriacontan-37-yl)thioureido)benzyl)-1,4,7-triazonane-1,4-diyl)diacetic acid; 2,2',2"-(3-(4-(3-(1-(4-(1,2,4,5-tetrazin-3-yl)phenyl)-3,7-dioxo-11,14,17,20,23,26,29-heptaoxa-2,8-diazahentriacontan-31-yl)thioureido)benzyl)-1,4,7-triazonane-2,5,8-triyl)triacetic acid; 2,2',2"-(3-(4-(3-(1-(4-(1,2,4,5-tetrazin-3-yl)phenyl)-3,7-dioxo-11,14,17,20,23,26,29,32,35,38,41-undecaoxa-2,8-diazatritetracontan-43-yl)thioureido)benzyl)-1,4,7-triazonane-2,5,8-triyl)triacetic acid; 2,2',2"-(3-(4-(3-(25,28-dioxo-28-((6-(6-(pyridin-2-yl)-1,2,4,5-tetrazin-3-yl)pyridin-3-yl)amino)-3,6,9,12,15,18,21-heptaoxa-24-azaoctacosyl)thioureido)benzyl)-1,4,7-triazonane-2,5,8-triyl)triacetic acid; 2,2',2"-(3-(4-(3-(37,40-dioxo-40-((6-(6-(pyridin-2-yl)-1,2,4,5-tetrazin-3-yl)pyridin-3-yl)amino)-3,6,9,12,15,18,21,24,27,30,33-undecaoxa-36-azatetracontyl)thioureido)benzyl)-1,4,7-triazonane-2,5,8-triyl)triacetic acid; 2,2',2"-(3-(4-(1-(4-(6-methyl-1,2,4,5-tetrazin-3-yl)phenyl)-3-oxo-6,9,12,15,18,21,24-heptaoxa-2-azaheptacosan-27-amido)benzyl)-1,4,7-triazonane-2,5,8-triyl)triacetic acid; 2,2',2"-(2-(4-(1-(4-(6-methyl-1,2,4,5-tetrazin-3-yl)phenoxy)-3,6,9,12,15,18,21,24,27,30,33-undecaoxahexatriacontan-36-amido)benzyl)-1,4,7-triazonane-1,4,7-triyl)triacetic acid; 2,2',2"-(3-(4-(3-(5-amino-6-((4-(6-methyl-1,2,4,5-tetrazin-3-yl)benzyl)amino)-6-oxohexyl)thioureido)benzyl)-1,4,7-triazonane-2,5,8-triyl)triacetic acid; 2,2'-(7-(4-(3-(5-amino-6-((4-6-methyl-1,2,4,5-tetrazin-3-yl)benzyl)amino)-6-oxohexyl)thioureido)benzyl)-1,4,7-triazonane-1,4-diyl)diacetic acid; 2,2',2"-(3-(4-(3-(5-amino-6-((5-amino-6-((4-(6-methyl-1,2,4,5-tetrazin-3-yl)benzyl)amino)-6-oxohexyl)amino)-6-oxohexyl)thioureido)benzyl)-1,4,7-triazonane-2,5,8-triyl)triacetic acid; and 2,2',2"-(3-(4-(3-(5-amino-6-((5-amino-6-((5-amino-6-((4-(6-methyl-1,2,4,5-tetrazin-3-yl)benzyl)amino)-6-oxohexyl)amino)-6-oxohexyl)amino)-6-oxohexyl)thioureido)benzyl)-1,4,7-triazonane-2,5,8-triyl)triacetic acid.

### Linkers

Peptides according to the present disclosure can be attached to a cargo molecule (e.g., a therapeutically active agent or a detectable agent), such as a small molecule, another peptide, a protein, an antibody, an antibody fragment, an aptamer, polypeptide, polynucleotide, a fluorophore, a radioisotope, a radionuclide chelator, a polymer, a biopolymer, a fatty acid, an acyl adduct, a chemical linker, or sugar or other active agent or detectable agent described herein through a linker, or directly in the absence of a linker. In case a linker is absent, a cargo molecule is conjugated, linked, or fused directly to the N-terminus and/or the C-terminus of a peptide. In cases where a linker is present, the linker can be a cleavable or stable linker is used. Cleavable linkers can be used for *in vivo* delivery of a cargo molecule, as a linker can be cleaved upon entry in a tissue, cell, endosome, or a nucleus. Stable linkers can be used for delivery of a cargo molecule that is active when conjugated or is degraded such as by catabolism. As described herein, a linker can also be used to covalently attach a TfR-binding peptide to another a cargo moiety having a separate function, such a targeting, cytotoxic, therapeutic, homing, imaging, or diagnostic functions.

In some embodiments, a cargo molecule can be conjugated to, linked to, or fused to the N-terminus and/or the C-terminus of a peptide to create an active agent or detectable agent peptide construct. In some embodiments, the TfR-targeting peptide can be attached at the N-terminus, an internal lysine, glutamic acid, or aspartic acid residue, or the C-terminus of another functional peptide or protein via a linker. In some embodiments, the peptide can be attached to another functional peptide or protein via a side chain, such as the side chain of a lysine, serine, threonine, cysteine, tyrosine, aspartic acid, a non-natural amino acid residue, or glutamic acid residue. As used herein, molecules can be linked, attached, or fused to each other via an amide bond, an ester bond, an ether bond, a carbamate bond, a carbonate bond, a carbon-nitrogen bond, a triazole, a macrocycle, an oxime bond, a thioester bond, a thioether bond a hydrazone bond, an azo bond, a carbon-carbon single, double, or triple bond, a disulfide bond, a two carbon bridge between two cysteines, a three carbon bridge between two cysteines, or a thioether bond. In some embodiments, the peptide comprises one or more non-natural amino acid, wherein the non-natural amino acid can be an insertion, appendage, or substitution for another amino acid. In some embodiments, similar regions of the disclosed peptide(s) itself (such as a terminus of the amino acid sequence, an amino acid side chain, such as the side chain of a lysine, serine, threonine, cysteine, tyrosine, aspartic acid, a non-natural amino acid residue, or glutamic acid residue, via an amide bond, an ester bond, an ether bond, a carbamate bond, a carbon-nitrogen bond, a triazole, a macrocycle, an oxime bond, a hydrazone bond, a carbon-carbon single, double, or triple bond, a disulfide bond, a thioether bond, or other linker as described herein) can be used to link other molecules. In some embodiments, the peptide is attached to a terminus of the amino acid sequence of a larger polypeptide or peptide molecule, or is attached to a side chain, such as the side chain of a lysine, serine, threonine, cysteine, tyrosine, aspartic acid, a non-natural amino acid residue, or glutamic acid residue.

Attachment via a linker can involve incorporation of a linker moiety between the other molecule (e.g., an active agent and/or a detectable agent) and the peptide. The peptide and the other molecule can both be covalently attached to the linker. The linker can be cleavable, stable, self-immolating, hydrophilic, or hydrophobic. The linker can have bulky side groups or chains that sterically limit access of enzymes, water, or other chemicals to the linking group. The linker can have at least two functional groups, one bonded to the other molecule, and one bonded to the peptide, and a linking portion between the two functional groups. Some example linkers are described in Jain, N., Pharm Res. 32(11): 3526-40 (2015), Doronina, S.O., Bioconj Chem. 19(10): 1960-3 (2008), Pillow, T.H., J Med Chem. 57(19): 7890-9 (2014), Dorywalksa, M., Bioconj Chem. 26(4): 650-9 (2015), Kellogg, B.A., Bioconj Chem. 22(4): 717-27 (2011), and Zhao, R.Y., J Med Chem. 54(10): 3606-23 (2011).

Non-limiting examples of the functional groups for attachment can include functional groups capable of forming, for example, an amide bond, an ester bond, an ether bond, a carbonate bond, a carbamate bond, a carbon-nitrogen bond, a triazole, a macrocycle, an oxime bond, a hydrazone bond, a carbon-carbon single, double, or triple bond, a disulfide bond or a thioether bond. Non-limiting examples of functional groups capable of forming such bonds can include amino groups; carboxyl groups; hydroxyl groups; aldehyde groups; azide groups; alkyne and alkene groups; ketones; hydrazides; hydrazines; acid halides such as acid fluorides, chlorides, bromides, and iodides; acid anhydrides, including symmetrical, mixed, and cyclic anhydrides; carbonates; carbonyl functionalities bonded to leaving groups such as cyano, succinimidyl, and *N*-hydroxysuccinimidyl; maleimides; linkers containing maleimide groups that are designed to hydrolyze; maleimidocaproyl; MCC ([N-maleimidomethyl]cyclohexane-1-carboxylate); N-ethylmaleimide; maleimide alkane; mc-vc-PABC; DUBA (DuocarmycinhydroxyBenzamide-Azaindole linker); SMCC Succinimidyl-4-(N-maleimidomethyl) cyclohexane- 1-carboxylate; SPDP (N-succinimidyl-3-(2-pyridyldithio) propionate); SPDB N-succinimidyl-4-(2-pyridyldithio) butanoate; sulfo-SPDB N-succinimidyl-4-(2-pyridyldithio) -2-sulfo butanoate; SPP N-succinimidyl 4-(2-pyridyldithio)pentanoate; a dithiopyridylmaleimide (DTM); a hydroxylamine, a vinyl-halo group; haloacetamido groups; bromoacetamido; hydroxyl groups; sulfhydryl groups; and molecules possessing, for example, alkyl, alkenyl, alkynyl, allylic, or benzylic leaving groups, such as halides, mesylates, tosylates, triflates, epoxides, phosphate esters, sulfate esters, and besylates.

Non-limiting examples of the linking portion can include alkylene, alkenylene, alkynylene, polyether, such as polyethylene glycol (PEG), hydroxy carboxylic acids, polyester, polyamide, polyamino acids, polypeptides, cleavable peptides, Val-Cit, Phe-Lys, Val-Lys, Val-Ala, other peptide linkers as given in Doronina et al., 2008, linkers cleavable by beta glucuronidase, linkers cleavable by a cathepsin or by cathepsin B, D, E, H, L, S, C, K, O, F, V, X, or W, Val-Cit-p-aminobenzyloxycarbonyl, glucuronide-MABC, valine-citrulline, aminobenzylcarbamates, D-amino acids, and polyamine, any of which being unsubstituted or substituted with any number of substituents, such as halogens, hydroxyl groups, sulfhydryl groups, amino groups, nitro groups, nitroso groups, cyano groups, azido groups, sulfoxide groups, sulfone groups, sulfonamide groups, carboxyl groups, carboxaldehyde groups, imine groups, alkyl groups, halo-alkyl groups, alkenyl groups, halo-alkenyl groups, alkynyl groups, halo-alkynyl groups, alkoxy groups, aryl groups, aryloxy groups, aralkyl groups, arylalkoxy groups, heterocyclyl groups, acyl groups, acyloxy groups, carbamate groups, amide groups, urethane groups, epoxides, charged groups, zwitterionic groups, and ester groups. Other non-limiting examples of reactions to link, fuse, or conjugate molecules together include click chemistry, copper-free click chemistry, HIPS ligation, Staudinger ligation, and hydrazine-iso-Pictet-Spengler.

In some embodiments, a linker for linking a repressor domain to a dCas9 can be about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, or 50 amino acid residues in length. A linker can be about 10 amino acid residues in length. A linker can be about 11 amino acid residues in length. A linker can be about 12 amino acid residues in length. A linker can be about 13 amino acid residues in length. A linker can be about 14 amino acid residues in length. A linker can be about 15 amino acid residues in length. A linker can be about 16 amino acid residues in length. A linker can be about 17 amino acid residues in length. A linker can be about 18 amino acid residues in length. A linker can be about 19 amino acid residues in length. A linker can be about 20 amino acid residues in length. A linker can be about 21 amino acid residues in length. A linker can be about 22 amino acid residues in length. A linker can be about 23 amino acid residues in length. A linker can be about 24 amino acid residues in length. A linker can be about 25 amino acid residues in length. A linker can be about 26 amino acid residues in length. A linker can be about 27 amino acid residues in length. A linker can be about 28 amino acid residues in length. A linker can be about 29 amino acid residues in length. A linker can be about 30 amino acid residues in length.

In some embodiments, a linker of the present disclosure can comprise a cleavable or stable linker moiety. In some embodiments, cleavable linkers of the present disclosure can include, for example, protease cleavable peptide linkers, nuclease sensitive nucleic acid linkers, lipase sensitive lipid linkers, glycosidase sensitive carbohydrate linkers, pH sensitive linkers, hypoxia sensitive linkers, photo-cleavable linkers, heat-labile linkers, enzyme cleavable linkers (e.g., esterase cleavable linker), ultrasound-sensitive linkers, and X-ray cleavable linkers. In some embodiments, the linker is not a cleavable linker.

Non-limiting examples of linkers include: 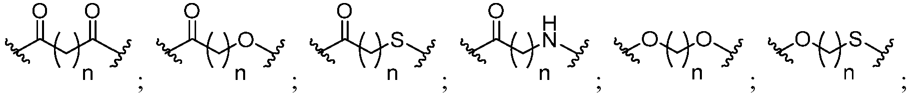 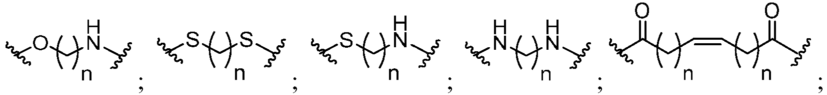 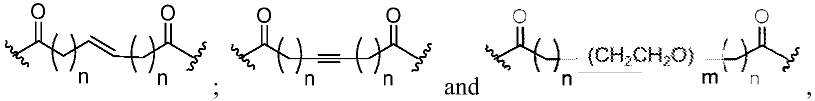 wherein each n is independently 0 to about 1,000; 1 to about 1,000; 0 to about 500; 1 to about 500; 0 to about 250; 1 to about 250; 0 to about 200; 1 to about 200; 0 to about 150; 1 to about 150; 0 to about 100; 1 to about 100; 0 to about 50; 1 to about 50; 0 to about 40; 1 to about 40; 0 to about 30; 1 to about 30; 0 to about 25; 1 to about 25; 0 to about 20; 1 to about 20; 0 to about 15; 1 to about 15; 0 to about 10; 1 to about 10; 0 to about 5; or 1 to about 5. In some embodiments, each n is independently 0, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50. In some embodiments, m is 1 to about 1,000; 1 to about 500; 1 to about 250; 1 to about 200; 1 to about 150; 1 to about 100; 1 to about 50; 1 to about 40; 1 to about 30; 1 to about 25; 1 to about 20; 1 to about 15; 1 to about 10; or 1 to about 5. In some embodiments, m is 0, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50, or any linker as disclosed in Jain, N., Pharm Res. 32(11): 3526-40 (2015) or Ducry, L., Antibody Drug Conjugates (2013).

In some cases, a linker can comprise a cyclic group, such as an organic nonaromatic or aromatic ring, optionally with 3-10 carbons in the ring, optionally built from a carboxylic acid, 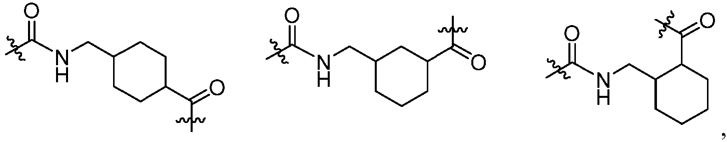 for example trans-4-(aminomethyl) cyclohexane carboxylic acid, 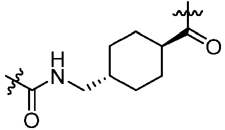 or a substituted analog or a stereoisomer thereof. This linker can optionally be used to form a carbamate linkage. In some cases, a carbamate linkage can be more resistant to cleavage, such as by hydrolysis, enzymes such as esterases, or other chemical reactions, than an ester linkage.

In some cases, a linker can comprise a cyclic carboxylic acid, for example a cyclic dicarboxylic acid, for example one of the following groups: 1,4-cyclohexane dicarboxylic acid, 1,2-cyclohexane dicarboxylic acid, or 1,3-cyclohexane dicarboxylic acid, 1,1-cyclopentanediacetic acid, 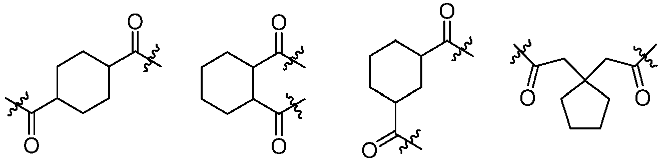 or a substituted analog or a stereoisomer thereof. For example, the linker can comprise one of the following groups. 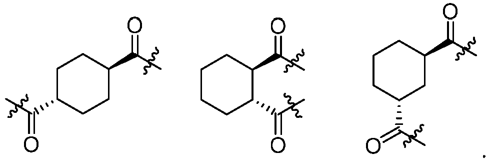 In some instances, the linker can optionally be used to form an ester linkage. In some cases, a cyclic ester linkage can be more sterically resistant to cleavage, such as by hydrolysis by water, enzymes such as esterases, or other chemical reactions, than a noncyclic or linear ester linkage.

In some cases, a linker can comprise an aromatic dicarboxylic acid, for example terephthalic acid, isophthalic acid, phthalic acid 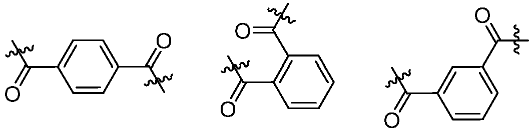 or a substituted analog thereof.

In some cases, a linker can comprise a natural or non-natural amino acid, for example cysteine, 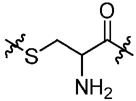 or a substituted analog or a stereoisomer thereof. In some instances, a linker can comprise alanine (A, Ala); arginine (R, Arg); asparagine (N, Asn); aspartic acid (D, Asp); glutamic acid (E, Glu); glutamine (Q, Gln); glycine (G, Gly); histidine (H, His); isoleucine (I, Ile); leucine (L, Leu); lysine (K, Lys); methionine (M, Met); phenylalanine (F, Phe); proline (P, Pro); serine (S, Ser); threonine (T, Thr); tryptophan (W, Trp); tyrosine (Y, Tyr); valine (V, Val); or any plurality or combination thereof. In some embodiments, the non-natural amino acid can comprise one or more functional groups, e.g., alkene or alkyne, that can be used as functional handles.

In some cases, a linker can comprise one of the following groups: 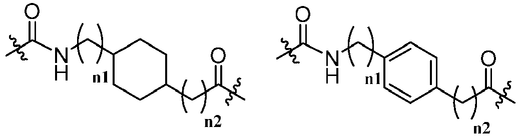 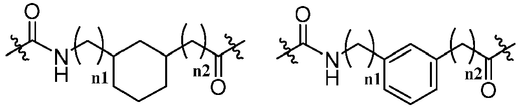 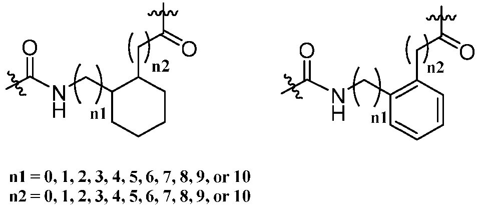 or a substituted analog or a stereoisomer thereof. In some instances, the linker is selected from one of the following groups: 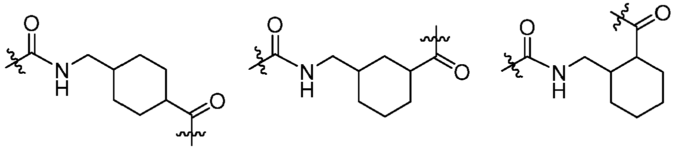 or a substituted analog or a stereoisomer thereof.

In some cases, a linker can comprise one of the following groups: 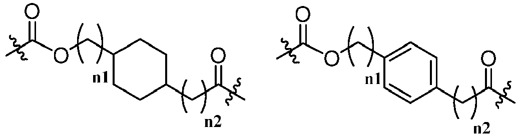 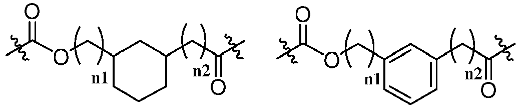 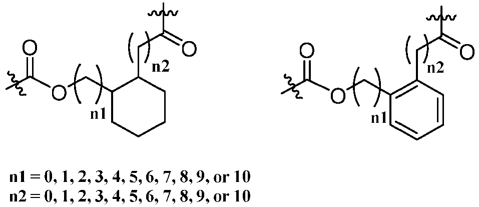 or a substituted analog or a stereoisomer thereof. In some instances, the linker is selected from one of the following groups: 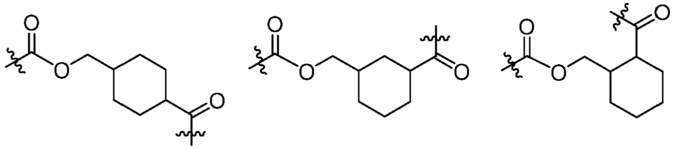 or a substituted analog or a stereoisomer thereof.

In some cases, a substituted analog or a stereoisomer is a structural analog of a compound disclosed herein, for which one or more hydrogen atoms of the compound can be substituted by one or more groups of halo (e.g., Cl, F, Br), alkyl (e.g., methyl, ethyl, propyl), alkenyl, alkynyl, arylalkyl, cycloalkyl, cycloalkylalkyl, heteroalkyl, heterocycloalkyl, or any combination thereof. In some cases, a stereoisomer can be an enantiomer, a diastereomer, a cis or trans stereoisomer, a E or Z stereoisomer, or a R or S stereoisomer.

Non-limiting examples of linear linkers include; 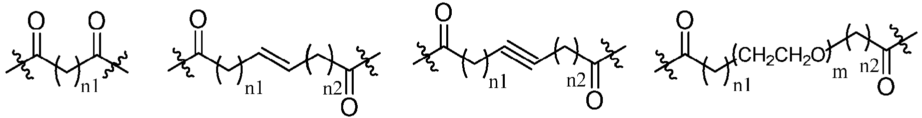 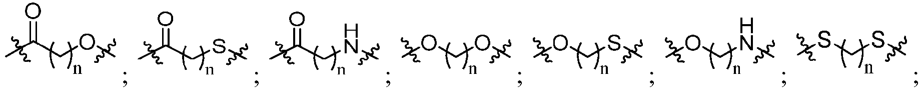 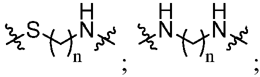 wherein each n1, or n2 or m is independently 0 to about 1,000; 1 to about 1,000; 0 to about 500; 1 to about 500; 0 to about 250; 1 to about 250; 0 to about 200; 1 to about 200; 0 to about 150; 1 to about 150; 0 to about 100; 1 to about 100; 0 to about 50; 1 to about 50; 0 to about 40; 1 to about 40; 0 to about 30; 1 to about 30; 0 to about 25; 1 to about 25; 0 to about 20; 1 to about 20; 0 to about 15; 1 to about 15; 0 to about 10; 1 to about 10; 0 to about 5; or 1 to about 5. In some embodiments, each n is independently 0, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50. In some embodiments, m is 1 to about 1,000; 1 to about 500; 1 to about 250; 1 to about 200; 1 to about 150; 1 to about 100; 1 to about 50; 1 to about 40; 1 to about 30; 1 to about 25; 1 to about 20; 1 to about 15; 1 to about 10; or 1 to about 5. In some embodiments, m is 0, about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, or about 50. In some instances, the linker can comprise a linear dicarboxylic acid, e.g., one of the following groups: succinic acid, 2,3-dimethylsuccinic acid, glutaric acid, adipic acid, 2,5-dimethyladipic acid, 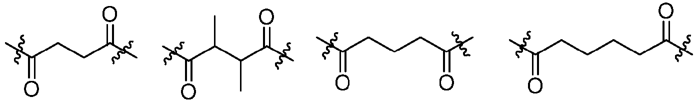 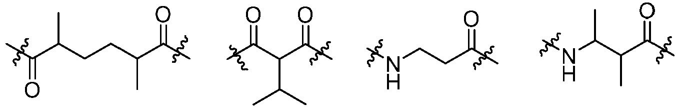 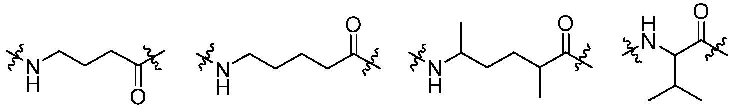 or a substituted analog or a stereoisomer thereof. In some cases, the linker can be used to form a carbamate linkage. In some embodiments, the carbamate linkage can be more resistant to cleavage, such as by hydrolysis, enzymes such as esterases, or other chemical reactions, than an ester linkage. In some cases, the linker can be used to form a linear ester linkage. In some embodiments, the linear ester linkage can be more susceptible to cleavage, such as by hydrolysis, enzymes such as esterases, or other chemical reactions, than a cyclic ester or carbamate linkage. Side chains such as methyl groups on the linear ester linkage can optionally make the linkage less susceptible to cleavage than without the side chains.

In some cases a linker can be a succinic linker, and a drug can be attached to a peptide via an ester bond or an amide bond with two methylene carbons in between. In other cases, a linker can be any linker with both a hydroxyl group and a carboxylic acid, such as hydroxy hexanoic acid or lactic acid.

In some cases, a drug can be attached to a peptide using any one or more of the linkers shown below in **TABLE 7.**

**TABLE 7 - Exemplary Linkers for Use in Peptide-Drug Conjugates**

| **Compound number** | **Chemical structure** | **Linker is based on:** |
|---|---|---|
| **1** | 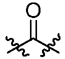 | Carbonic acid |
| **2** | 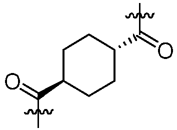 | *trans-1,4-*cyclohexanedicarboxylic acid |
| **3** | 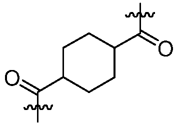 | 1,4-cyclohexanedicarboxylic acid |
| **4** | 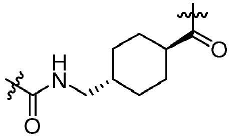 | *trans-*1-aminomethylamine-cyclohexane-4-carboxylic acid (carbamate) |
| **5** | 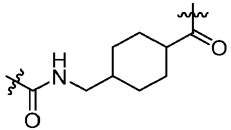 | 1-aminomethylamine-cyclohexane-4-carboxylic acid |
| **6** | 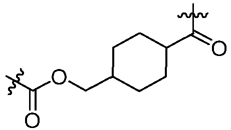 | 4-(carboxyoxy)-methylcyclohexane-1-carboxylic acid |
| **7** | 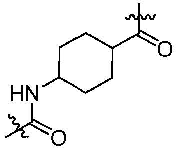 | 4-(carboxyamino)-cyclohexane-1-carboxylic acid |
| **8** | 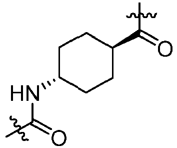 | *trans*-4-(carboxyamino)-cyclohexane-1-carboxylic acid |
| **9** | 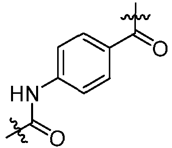 | 4-(carboxyamino)benzoic acid |
| **10** | 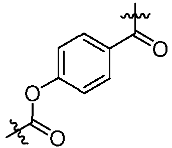 | 4-(carboxyoxy)benzoic acid |
| **11** | 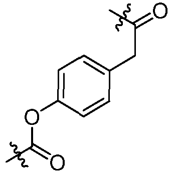 | 2-(4-(carboxyoxy)phenyl)acetic acid |
| **12** | 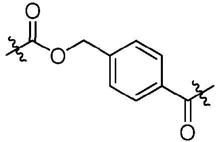 | 4-((carboxyoxy)methyl)benzoic acid |
| **13** | 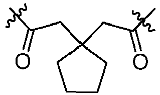 | 2,2'-(cyclopentane-1,1-diyl)diacetic acid |
| **14** | 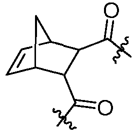 | cis-5-norborene-endo-2,3-dicarboxylic acid |
| **15** | 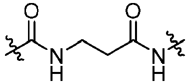 | (3-amino-3-oxypropyl)carbamic acid |
| **16** | 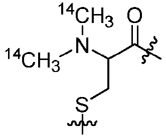 | [¹⁴C]-Cysteine |
| **17** | 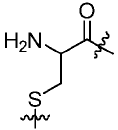 | Cysteine |
| **18** | 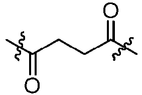 | Succinic acid |
| **19** | 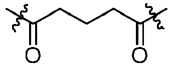 | Glutaric acid |
| **20** | 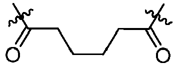 | Adipic acid |
| **21** | 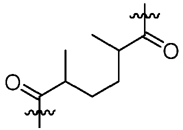 | 2,5-dimethyladipic acid (DMA) |
| **22** | 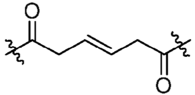 | *trans*-β-hydromuconic acid |

In some cases, a drug molecule is attached to a linker wherein a nucleophilic functional group (e.g., a hydroxyl group) of the drug molecule acts as the nucleophile and replaces a leaving group on the linker moiety, thereby attaching the drug to the linker.

In other cases, a drug molecule is attached to a linker wherein a nucleophilic functional group (e.g., thiol group, amine group, etc) of the linker replaces a leaving group on the drug molecule, thereby attaching the drug to the linker. Such leaving group (or functional group that may be converted into a leaving group) may be a primary alcohol to form a thioether bond, thereby attaching the drug to the linker. A primary alcohol can be converted into a leaving group such as a mesylate, a tosylate, or a nosylate in order to accelerate the nucleophilic substitution reaction.

The peptide-drug conjugates of the present disclosure can comprise a drug, a linker, and/or a peptide of the present disclosure. A general connectivity between these three components can be drug-linker-peptide, such that the linker is attached to both the drug and the peptide. In many cases, the peptide is attached to a linker via an amide bond. Amide bonds can be relatively stable (e.g., *in vivo)* compared to other bonds described herein, such as esters, carbonates, etc. The amide bond between the peptide and the linker may thus provide advantageous properties due to its *in vivo* stability if the drug is sought to be cleaved from a peptide-drug-conjugate without the linker being attached to the drug after such in vivo cleavage. Thus, in various cases, a drug is attached to the linker-peptide moiety via linkages such as ester, carbonate, carbamate, etc., wherein the peptide is attached to the linker via an amide bond. This can allow for selective cleavage of the drug-linker bond (as opposed to the linker-peptide bond) allowing the drug to be released without a linker moiety attached to it after cleavage. The use of such different drug-linker bonds or linkages can allow the modulation of drug release *in vivo,* e.g., in order to achieve a therapeutic function while minimizing off-target effects (e.g., reduction in drug release during circulation).

The linker can be a cleavable or a stable linker. The use of a cleavable linker permits release of the conjugated moiety (e.g., a therapeutic agent) from the peptide, e.g., after targeting to the target tissue or cell or subcellular compartment. In some cases the linker is enzyme cleavable, e.g., a valine-citrulline linker that can be cleavable by cathepsin, or an ester linker that can be cleavable by esterase. In some embodiments, the linker contains a self-immolating portion. In other embodiments, the linker includes one or more cleavage sites for a specific protease, such as a cleavage site for matrix metalloproteases (MMPs), thrombin, urokinase-type plasminogen activator, or cathepsin (e.g., cathepsin K).

Thus, in some cases, a peptide-active agent conjugate of the present disclosure can comprise one or more, about two or more, about three or more, about five or more, about ten or more, or about 15 or more amino acids that can form an amino acid sequence cleavable by an enzyme. Such enzymes can include proteinases. A peptide-drug conjugate can comprise an amino acid sequence that can be cleaved by a Cathepsin, a Chymotrypsin, an Elastase, a Subtilisin, a Thrombin I, or a Urokinas, or any combination thereof.

Alternatively or in combination, the cleavable linker can be cleaved, dissociated, or broken by other mechanisms, such as via pH, reduction, or hydrolysis. Hydrolysis can occur directly due to water reaction, or be facilitated by an enzyme, or be facilitated by presence of other chemical species. A hydrolytically labile linker, (amongst other cleavable linkers described herein) can be advantageous in terms of releasing active agents from the peptide. For example, an active agent in a conjugate form with the peptide may not be active, but upon release from the conjugate after targeting to the target tissue or cell or subcellular compartment, the active agent is active. The cleaved active agent may retain the chemical structure of the active agent before cleavage, or may be modified. In some embodiments, a stable linker may optionally not cleave in buffer over extended periods of time (e.g., hours, days, or weeks). In some embodiments, a stable linker may optionally not cleave in body fluids such as plasma or synovial fluid over extended periods of time (e.g., hours, days, or weeks). In some embodiments, a stable linker optionally may cleave, such as after exposure to enzymes, reactive oxygen species, other chemicals or enzymes that may be present in cells (such as macrophages), cellular compartments (such as endosomes and lysosomes), inflamed areas of the body (such as inflamed joints), or tissues or body compartments. In some embodiments, a stable linker may optionally not cleave in vivo but present an active agent that is still active when conjugated to, linked to, or fused to the peptide.

The rate of hydrolysis of the linker (e.g., a linker of a peptide conjugate) can be tuned. For example, the rate of hydrolysis of linkers with unhindered esters may be faster compared to the hydrolysis of linkers with bulky groups next an ester carbonyl. A bulky group can be a methyl group, an ethyl group, a phenyl group, a ring, or an isopropyl group, or any group that provides steric bulk. In another example, the rate of hydrolysis can be faster with hydrophilic groups, such as alcohols, acids, or ethers, or near an ester carbonyl. In another example, hydrophobic groups present as side chains or by having a longer hydrocarbon linker can slow cleavage of the ester. In some embodiments, cleavage of a carbamate group can also be tuned by hindrance, hydrophobicity, and the like. In another example, using a less labile linker, such as a carbamate rather than an ester, can slow the cleavage rate of the linker. In some cases, the steric bulk can be provided by the drug itself, such as by ketorolac when conjugated via its carboxylic acid. The rate of hydrolysis of the linker can be tuned according to the residency time of the conjugate in the target tissue or cell or subcellular compartment, according to how quickly the peptide accumulates in the target tissue or cell or subcellular compartment, or according to the desired time frame for exposure to the active agent in the target tissue or cell or subcellular compartment. For example, when a peptide is cleared from the target tissue or cell or subcellular compartment relatively quickly, the linker can be tuned to rapidly hydrolyze. In contrast, for example, when a peptide has a longer residence time in the target tissue or cell or subcellular compartment, a slower hydrolysis rate can allow for extended delivery of an active agent. This can be important when the peptide is used to deliver a drug to the target tissue or cell or subcellular compartment (e.g., a tumor cell or a tumor tissue). "Programmed hydrolysis in designing paclitaxel prodrug for nanocarrier assembly" Sci Rep 2015, 5, 12023 Fu et al., provides an example of modified hydrolysis rates. In some embodiments, rates of cleavage can vary by species, body compartment, and disease state. For instance, cleavage by esterases may be more rapid in rat or mouse plasma than in human plasma, such as due to different levels of carboxyesterases. In some embodiments, a linker may be tuned for different cleavage rates for similar cleavage rates in different species.

In some cases a linker can be a succinic linker, and a drug can be attached to a peptide via an ester bond or an amide bond with two methylene carbons in between. In other cases, a linker can be any linker with both a hydroxyl group and a carboxylic acid, such as hydroxy hexanoic acid or lactic acid.

In some embodiments, the linker can release the active agent in an unmodified form. In other embodiments, the active agent can be released with chemical modification. In still other embodiments, catabolism can release the active agent still linked to parts of the linker and/or peptide.

The linker can be a stable linker or a cleavable linker. In some embodiments, the stable linker can slowly release the conjugated moiety by an exchange of the conjugated moiety onto the free thiols on serum albumin. In some embodiments, the use of a cleavable linker can permit release of the conjugated moiety (e.g., a therapeutic agent) from the peptide, e.g., after administration to a subject in need thereof. In other embodiments, the use of a cleavable linker can permit the release of the conjugated therapeutic from the peptide. In some cases the linker is enzyme cleavable, e.g., a valine-citrulline linker. In some embodiments, the linker contains a self-immolating portion. In other embodiments, the linker includes one or more cleavage sites for a specific protease, such as a cleavage site for matrix metalloproteases (MMPs), thrombin, cathepsins, peptidases, or beta-glucuronidase. Alternatively or in combination, the linker is cleavable by other mechanisms, such as via pH, reduction, or hydrolysis.

The rate of hydrolysis or reduction of the linker can be fine-tuned or modified depending on an application. For example, the rate of hydrolysis of linkers with unhindered esters can be faster compared to the hydrolysis of linkers with bulky groups next to an ester carbonyl. A bulky group can be a methyl group, an ethyl group, a phenyl group, a ring, or an isopropyl group, or any group that provides steric bulk. In some cases, the steric bulk can be provided by the drug itself, such as by ketorolac when conjugated, linked, or fused via its carboxylic acid. The rate of hydrolysis of the linker can be tuned according to the residency time of the conjugate or fusion in the target location. For example, when a peptide is cleared from a tumor, or the brain, relatively quickly, the linker can be tuned to rapidly hydrolyze. When a peptide has a longer residence time in the target location, a slower hydrolysis rate would allow for extended delivery of an active agent.

The rate of hydrolysis of the linker (e.g., a linker of a peptide conjugate) can be measured. Such measurements can include determining free active agent in plasma, or synovial fluid, or other fluid or tissue of a subject *in vivo* and/or by incubating a linker or a peptide conjugate comprising a linker of the present disclosure with a buffer (e.g., PBS) or blood plasma from a subject (e.g., rat plasma, human plasma, etc.) or synovial fluid or other fluids or tissues *ex vivo.* The methods for measuring hydrolysis rates can include taking samples during incubation or after administration and determine free active agent, free peptide, or any other parameter indicate of hydrolysis, including also measuring total peptide, total active agent, or conjugated active agent-peptide. The results of such measurements can then be used to determine a hydrolysis half-life of a given linker or peptide conjugate comprising the linker. A hydrolysis half-life of a linker can differ depending on the plasma or fluid or species or other conditions used to determine such half-life. This can be due to certain enzymes or other compounds present in a certain plasma (e.g., rat plasma). For instance, different fluids (such as plasma or synovial fluid) can contain different amounts of enzymes such as esterases, and these levels of these compounds can also vary depending on species (such as rat versus human) as well as disease state (such as normal versus arthritic).

The conjugates of the present disclosure can be described as having a modular structure comprising various components, wherein each of the components (e.g., peptide, linker, active agent and/or detectable agent) can be selected dependently or independently of any other component. For example, a conjugate for use in the treatment of pain can comprise a TfR-binding peptide of the present disclosure
, a linker (e.g., any linker described in **TABLE 7)** and an active agent (e.g., an NSAID pain reliever, ibuprofen, or a molecule to treat disorders of the CNS or of cancer). The linker, for example, can be selected and/or modified to achieve a certain active agent release (e.g., a certain release rate) via a certain mechanism (e.g., via hydrolysis, such as enzyme and/or pH-dependent hydrolysis) at the target site (e.g., in the brain) and/or to minimize systemic exposure to the active agent. During the testing of a conjugate any one or more of the components of the conjugate can be modified and/or altered to achieve certain *in vivo* properties of the conjugate, e.g., pharmacokinetic (e.g., clearance time, bioavailability, uptake and retention in various organs) and/or pharmacodynamic (e.g., target engagement) properties. Thus, the conjugates of the present disclosure can be modulated to prevent, treat, and/or diagnose a variety of diseases and conditions, while reducing side effects (e.g., side effects that occur if such active agents are administered alone (i.e., not conjugated to a peptide)).

In some embodiments, the non-natural amino acid can comprise one or more functional groups, e.g., alkene or alkyne, that can be used as functional handles. For example, a multiple bond of such functional groups can be used to add one or more molecules to the conjugate. The one or more molecules can be added using various synthetic strategies, some of which may include addition and/or substitution chemistries. For example, an addition reaction using a multiple bond can comprise the use of hydrobromic acid, wherein the bromine can act as a leaving group and thus be substituted with various moieties, e.g., active agents, detectable agents, agents that can modify or alter the pharmacokinetic (e.g., plasma half-life, retention and/or uptake in central nervous system (CNS) or elsewhere) and/or pharmacodynamic (e.g., hydrolysis rate such as an enzymatic hydrolysis rate) properties of the conjugate.

In some embodiments, a conjugate as described herein comprises one or more non-natural amino acid and/or one or more linkers. Such one or more non-natural amino acid and/or one or more linkers can comprise one or more functional groups, e.g., alkene or alkyne (e.g., non-terminal alkenes and alkynes), which can be used as functional handles. For example, a multiple bond of such functional groups can be used to add one or more molecules to the conjugate. The one or more molecules can be added using various synthetic strategies, some of which may include addition and/or substitution chemistries, cycloadditions, etc. For example, an addition reaction using a multiple bond can comprise the use of hydrogen bromide (e.g., via hydrohalogenation reactions), wherein the bromide substituent, once attached, can act as a leaving group and thus be substituted with various moieties comprising a nucleophilic functional groups, e.g., active agents, detectable agents, agents. As another example, a multiple bond can be used as a functional handle in a cycloaddition reaction. Cycloaddition reactions can comprise 1,3-dipolar cycloadditions, [2+2]-cycloadditions (e.g., photocatalyzed), Diels-Alder reactions, Huisgen cycloadditions, nitrone-olefin cycloadditions, etc. Such cycloaddition reactions can be used to attached various functional groups, functional moieties, active agents, detectable agents, and so forth to the conjugate. For example, a 1,3-dipolar cycloaddition reaction can be used to attach a molecule to a conjugate, wherein the molecule comprises a 1,3-dipole that can react with, e.g., an alkyne to form a 5-membered ring, thereby attaching said molecule to the conjugate.

The addition of such agents or molecules (e.g., via nucleophilic or electrophilic addition followed by nucleophilic substitution) can have various application. For example, attaching such molecule or agent can modify or alter the pharmacokinetic (e.g., plasma half-life, retention and/or uptake in CNS or biodistribution) and/or pharmacodynamic (e.g., hydrolysis rate such as an enzymatic hydrolysis rate) properties of the conjugate. Attaching such molecule or agent can also alter (e.g., increase) the depot effect of a conjugate, or provide functionality for in vivo tracking, e.g., using fluorescence or other types of detectable agents.

In some embodiments, a conjugate of the present disclosure can comprise a linker comprising one or more of the following groups: 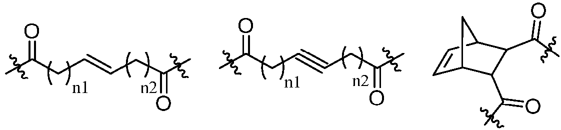 or a substituted analog or a stereoisomer thereof, wherein each n1 and n2 is independently a value from 1 to 10. Such a group can be used as a handle to attach one or more molecules to a conjugate, e.g., to alter the pharmacokinetic (e.g., plasma half-life, retention and/or uptake in central nervous system (CNS) or elsewhere) and/or pharmacodyna via nucleophilic or electrophilic addition followed by nucleophilic substitution mic properties of the conjugate. Functionalization of such a group can occur using one or more multiple bonds (e.g., double bonds, triple bonds, etc.) of the groups. Such functionalization can comprise addition and/or substitution chemistries. For example, a functional group of a linker, such as a double bond, can be converted into a single bond (e.g., via an addition reaction such as a nucleophilic addition reaction), wherein one or both of the carbon atoms of the newly formed single bond can have a leaving group (e.g., a bromine) attached to them. Such a leaving group can then be used (e.g., via nucleophilic substitution reaction) to attach a specific molecule (e.g., an active agent, a detectable agent, etc.) to that carbon atom(s) of the linker.

As another example, a multiple bond can be used as a functional handle in a cycloaddition reaction. Cycloaddition reactions can comprise 1,3-dipolar cycloadditions, [2+2]-cycloadditions (e.g., photocatalyzed), Diels-Alder reactions, Huisgen cycloadditions, nitrone-olefin cycloadditions, etc. Such cycloaddition reactions can be used to attached various functional groups, functional moieties, active agents, detectable agents, and so forth to the conjugate. For example, a 1,3-dipoalr cycloaddition reaction can be used to attach a molecule to a conjugate, wherein the molecule comprises a 1,3-dipole that can react with, e.g., an alkyne to form a 5-membered ring, thereby attaching said molecule (e.g., active agent, detectable agent, etc.) to the conjugate. In some cases, molecules may be attached to a conjugate to e.g., modulate the half-life, increase the depot effect, or provide new functionality of a conjugate, such as fluorescence for tracking.

### Pharmacokinetics of Peptides

The pharmacokinetics of any of the peptides of the present disclosure can be determined after administration of the peptide via different routes of administration. For example, the pharmacokinetic parameters of a peptide of this disclosure can be quantified after intravenous, subcutaneous, intramuscular, rectal, aerosol, parenteral, ophthalmic, pulmonary, transdermal, vaginal, optic, nasal, oral, sublingual, inhalation, dermal, intrathecal, intranasal, peritoneal, buccal, synovial, intratumoral, or topical administration. Peptides of the present disclosure can be analyzed by using tracking agents such as radiolabels or fluorophores. For example, radiolabeled peptides of this disclosure can be administered via various routes of administration. Peptide concentration or dose recovery in various biological samples such as plasma, urine, feces, any organ, skin, muscle, and other tissues can be determined using a range of methods including HPLC, fluorescence detection techniques (TECAN quantification, flow cytometry, iVIS), or liquid scintillation counting.

The methods and compositions described herein relate to pharmacokinetics of peptide administration via any route to a subject. Pharmacokinetics can be described using methods and models, for example, compartmental models or noncompartmental methods. Compartmental models include but are not limited to monocompartmental model, the two compartmental model, the multicompartmental model or the like. Models are often divided into different compartments and can be described by the corresponding scheme. For example, one scheme is the absorption, distribution, metabolism and excretion (ADME) scheme. For another example, another scheme is the liberation, absorption, distribution, metabolism and excretion (LADME) scheme. In some aspects, metabolism and excretion can be grouped into one compartment referred to as the elimination compartment. For example, liberation includes liberation of the active portion of the composition from the delivery system, absorption includes absorption of the active portion of the composition by the subject, distribution includes distribution of the composition through the blood plasma and to different tissues, metabolism, which includes metabolism or inactivation of the composition and finally excretion, which includes excretion or elimination of the composition or the products of metabolism of the composition. Compositions administered intravenously to a subject can be subject to multiphasic pharmacokinetic profiles, which can include but are not limited to aspects of tissue distribution and metabolism/excretion. As such, the decrease in plasma or serum concentration of the composition is often biphasic, including, for example an alpha phase and a beta phase, occasionally a gamma, delta or other phase is observed.

Pharmacokinetics includes determining at least one parameter associated with administration of a peptide to a subject. In some aspects, parameters include at least the dose (D), dosing interval (τ), area under curve (AUC), maximum concentration (Cₘₐₓ), minimum concentration reached before a subsequent dose is administered (Cₘᵢₙ), minimum time (Tₘᵢₙ), maximum time to reach Cmax (Tₘₐₓ), volume of distribution (V_{d}), steady-state volume of distribution (Vₛₛ), back-extrapolated concentration at time 0 (C₀), steady state concentration (Cₛₛ), elimination rate constant (kₑ), infusion rate (kᵢₙ), clearance (CL), bioavailability (f), fluctuation (%PTF) and elimination half-life (t_{1/2}).

In certain embodiments, the peptides or peptide constructs of the claims
exhibit optimal pharmacokinetic parameters after oral administration. In other embodiments, the peptides or peptide constructs of the claims
exhibit optimal pharmacokinetic parameters after any route of administration, such as oral administration, inhalation, intranasal administration, topical administration, intravenous administration, subcutaneous administration, intra-articular administration, intramuscular administration, intraperitoneal administration, intra-synovial, or any combination thereof.

In some embodiments, any peptide or peptide construct of the claims
exhibits an average Tₘₐₓ of 0.5 - 12 hours, or 1-48 hours at which the Cₘₐₓ is reached, an average bioavailability in serum of 0.1% - 10% in the subject after administering the peptide to the subject by an oral route, an average bioavailability in serum of less than 0.1% after oral administration to a subject for delivery to the GI tract, an average bioavailability in serum of 10-100% after parenteral administration, an average t_{½} of 0.1 hours - 168 hours, or 0.25 hours - 48 hours in a subject after administering the peptide to the subject, an average clearance (CL) of 0.5-100 L/hour or 0.5 - 50 L/hour of the peptide after administering the peptide to a subject, an average volume of distribution (V_{d}) of 200 - 20,000 mL in the subject after systemically administering the peptide to the subject, or optionally no systemic uptake, any combination thereof.

### Peptide Stability

A peptide of the present disclosure can be stable in various biological or physiological conditions, such as the pH or reducing environments inside a cell, in the cytosol, in a cell nucleus, or endosome or a tumor. For example, any peptide or peptide construct of the claims
can exhibit resistance to reducing agents, proteases, oxidative conditions, or acidic conditions.

In some cases, biologic molecules (such as peptides and proteins) can provide therapeutic functions, but such therapeutic functions are decreased or impeded by instability caused by the in vivo environment. (Moroz et al. Adv Drug Deliv Rev 101:108-21 (2016), Mitragotri et al. Nat Rev Drug Discov 13(9):655-72 (2014), Bruno et al. Ther Deliv (11):1443-67 (2013), Sinha et al. Crit Rev Ther Drug Carrier Syst. 24(1):63-92 (2007), Hamman et al. BioDrugs 19(3):165-77 (2005)). For instance, the GI tract can contain a region of low pH (e.g. pH ~1), a reducing environment, or a protease-rich environment that can degrade peptides and proteins. Proteolytic activity in other areas of the body, such as the mouth, eye, lung, intranasal cavity, joint, skin, vaginal tract, mucous membranes, and serum, can also be an obstacle to the delivery of functionally active peptides and polypeptides. Additionally, the half-life of peptides in serum can be very short, in part due to proteases, such that the peptide can be degraded too quickly to have a lasting therapeutic effect when administering reasonable dosing regimens. Likewise, proteolytic activity in cellular compartments such as lysosomes and reduction activity in lysosomes and the cytosol can degrade peptides and proteins such that they may be unable to provide a therapeutic function on intracellular targets. Therefore, peptides that are resistant to reducing agents, proteases, and low pH may be able to provide enhanced therapeutic effects or enhance the therapeutic efficacy of co-formulated or conjugated, linked, or fused active agents in vivo.

Additionally, oral delivery of drugs can be desirable in order to target certain areas of the body (e.g., disease in the GI tract such as colon cancer, irritable bowel disorder, infections, metabolic disorders, and constipation) despite the obstacles to the delivery of functionally active peptides and polypeptides presented by this method of administration. For example, oral delivery of drugs can increase compliance by providing a dosage form that is more convenient for patients to take as compared to parenteral delivery. Oral delivery can be useful in treatment regimens that have a large therapeutic window. Therefore, peptides that are resistant to reducing agents, proteases, and low pH can allow for oral delivery of peptides without nullifying their therapeutic function.

**Peptide Resistance to Reducing Agents.** Peptides of this disclosure can contain one or more cysteines, which can participate in disulfide bridges that can be integral to preserving the folded state of the peptide. Exposure of peptides to biological environments with reducing agents can result in unfolding of the peptide and loss of functionality and bioactivity. For example, glutathione (GSH) is a reducing agent that can be present in many areas of the body and in cells, and can reduce disulfide bonds. As another example, a peptide can become reduced during trafficking of a peptide across the gastrointestinal epithelium after oral administration. A peptide can become reduced upon exposure to various parts of the GI tract. The GI tract can be a reducing environment, which can inhibit the ability of therapeutic molecules with disulfide bonds to have optimal therapeutic efficacy, due to reduction of the disulfide bonds. A peptide can also be reduced upon entry into a cell, such as after internalization by endosomes or lysosomes or into the cytosol, or other cellular compartments. Reduction of the disulfide bonds and unfolding of the peptide can lead to loss of functionality or affect key pharmacokinetic parameters such as bioavailability, peak plasma concentration, bioactivity, and half-life. Reduction of the disulfide bonds can also lead to loss of functionality due to increased susceptibility of the peptide to subsequent degradation by proteases, resulting in rapid loss of intact peptide after administration. In some embodiments, a peptide that is resistant to reduction can remain intact and can impart a functional activity for a longer period of time in various compartments of the body and in cells, as compared to a peptide that is more readily reduced.

In certain embodiments, the peptides of this disclosure can be analyzed for the characteristic of resistance to reducing agents to identify stable peptides. In some embodiments, the peptides of this disclosure can remain intact after being exposed to different molarities of reducing agents such as 0.00001 M - 0.0001 M, 0.0001 M - 0.001 M, 0.001 M - 0.01 M, 0.01 M - 0.05 M, 0.05 M - 0.1 M, for 15 minutes or more. In some embodiments, the reducing agent used to determine peptide stability can be dithiothreitol (DTT), Tris(2-carboxyethyl)phosphine HCl (TCEP), 2-Mercaptoethanol, (reduced) glutathione (GSH), or any combination thereof. In some embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to a reducing agent. In some embodiments, peptides are completely resistant to GSH reducing conditions and are partially resistant to degradation in DTT reducing conditions. In some embodiments, peptides described herein can withstand or are resistant to degradation in physiological reducing conditions.

**Peptide Resistance to Proteases.** The stability of peptides of this disclosure can be determined by resistance to degradation by proteases. Proteases, also referred to as peptidases or proteinases, are enzymes that can degrade peptides and proteins by breaking bonds between adjacent amino acids. Families of proteases with specificity for targeting specific amino acids can include serine proteases, cysteine proteases, threonine proteases, aspartic proteases, glutamic proteases, and asparagine proteases. Additionally, metalloproteases, matrix metalloproteases, elastase, carboxypeptidases, Cytochrome P450 enzymes, and cathepsins can also digest peptides and proteins. Proteases can be present at high concentration in blood, in mucous membranes, lungs, skin, the GI tract, the mouth, nose, eye, and in compartments of the cell. Misregulation of proteases can also be present in various diseases such as rheumatoid arthritis and other immune disorders. Degradation by proteases can reduce bioavailability, biodistribution, half-life, and bioactivity of therapeutic molecules such that they are unable to perform their therapeutic function. In some embodiments, peptides that are resistant to proteases can better provide therapeutic activity at reasonably tolerated concentrations in vivo.

In some embodiments, peptides of this disclosure can resist degradation by any class of protease. In certain embodiments, peptides of this disclosure resist degradation by pepsin (which can be found in the stomach), trypsin (which can be found in the duodenum), serum proteases, or any combination thereof. In some embodiments, the proteases used to determine peptide stability can be pepsin, trypsin, chymotrypsin, or any combination thereof. In certain embodiments, peptides of this disclosure can resist degradation by lung proteases (e.g., serine, cysteinyl, and aspartyl proteases, metalloproteases , neutrophil elastase, alpha-1 antitrypsin, secretory leucoprotease inhibitor, and elafin), or any combination thereof. In some embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to a protease.

**Peptide Stability in Acidic Conditions.** Peptides of this disclosure can be administered in biological environments that are acidic. For example, after oral administration, peptides can experience acidic environmental conditions in the gastric fluids of the stomach and gastrointestinal (GI) tract. The pH of the stomach can range from about1-4 and the pH of the GI tract ranges from acidic to normal physiological pH descending from the upper GI tract to the colon. In addition, the vagina, late endosomes, and lysosomes can also have acidic pH values, such as less than pH 7. These acidic conditions can lead to denaturation of peptides and proteins into unfolded states. Unfolding of peptides and proteins can lead to increased susceptibility to subsequent digestion by other enzymes as well as loss of biological activity of the peptide. In certain embodiments, the peptides of this disclosure can resist denaturation and degradation in acidic conditions and in buffers, which simulate acidic conditions. In certain embodiments, peptides of this disclosure can resist denaturation or degradation in buffer with a pH less than 1, a pH less than 2, a pH less than 3, a pH less than 4, a pH less than 5, a pH less than 6, a pH less than 7, or a pH less than 8. In some embodiments, peptides of this disclosure remain intact at a pH of 1-3. In certain embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to a buffer with a pH less than 1, a pH less than 2, a pH less than 3, a pH less than 4, a pH less than 5, a pH less than 6, a pH less than 7, or a pH less than 8. In other embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to a buffer with a pH of 1-3. In other embodiments, the peptides of this disclosure can be resistant to denaturation or degradation in simulated gastric fluid (pH 1-2). In some embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to simulated gastric fluid. In some embodiments, low pH solutions such as simulated gastric fluid can be used to determine peptide stability.

In some embodiments, the peptides described herein are resistant to degradation in vivo, in the serum of a subject, or inside a cell. In some embodiments, the peptides are stable at physiological pH ranges, such as about pH 7, about pH 7.5, between about pH 5 to 7.5, between about 6.5 to 7.5, between about pH 5 to 8, or between about pH 5 to 7. In some embodiments, the peptides described herein are stable in acidic conditions, such as less than or equal to about pH 5, less than or equal to about pH 3, or within a range from about 3 to about 5. In some embodiments, the peptides are stable in conditions of an endosome or lysosome, or inside a nucleus.

**Peptide Stability at High Temperatures.** Peptides of this disclosure can be administered in biological environments with high temperatures. For example, after oral administration, peptides can experience high temperatures in the body. Body temperature can range from 36°C to 40°C. High temperatures can lead to denaturation of peptides and proteins into unfolded states. Unfolding of peptides and proteins can lead to increased susceptibility to subsequent digestion by other enzymes as well as loss of biological activity of the peptide. In some embodiments, a peptide of this disclosure can remain intact at temperatures from 25°C to 100°C. High temperatures can lead to faster degradation of peptides. Stability at a higher temperature can allow for storage of the peptide in tropical environments or areas where access to refrigeration is limited. In certain embodiments, 5%-100% of the peptide can remain intact after exposure to 25°C for 6 months to 5 years. 5%-100% of a peptide can remain intact after exposure to 70°C for 15 minutes to 1 hour. 5%-100% of a peptide can remain intact after exposure to 100°C for 15 minutes to 1 hour. In other embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to 25°C for at least 6 months to 5 years. In other embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to 70°C for 15 minutes to 1 hour. In other embodiments, at least 5%-10%, at least 10%-20%, at least 20%-30%, at least 30%-40%, at least 40%-50%, at least 50%-60%, at least 60%-70%, at least 70%-80%, at least 80%-90%, or at least 90%-100% of the peptide remains intact after exposure to 100°C for 15 minutes to 1 hour.

### Methods

**Peptide Production and Purification.** In some embodiments, amino acid sequences that are part of a peptide library are synthesized using expression vectors or solid phase or solution phase peptide synthesis methods. For example, TfR-binding peptides can be cloned into a secreted, soluble protein production/expression vector and subsequently be purified. Peptide purification methods include, but are not limited to, affinity purification columns, ion exchange (cation and/or anion column chromatography), reversed-phase chromatography, hydrophobic interaction chromatography, and size exclusion column chromatography. SDS-PAGE followed by Coomassie staining and reverse phase high-pressure liquid chromatography (HPLC) can be used to analyze a sample of the purified protein. Protein concentrations were determined by UV spectral absorption (e.g., absorbance at 280 nm) and/or amino acid analysis. In some embodiments, the peptides may be produced and purified using the methods described in Crook ZR et al. Methods Mol Biol. 2020; 2070:363-396.

**Surface Display Vector Construction.** In various embodiments, surface display vectors, SDGF and SDPR (a variant of the SDGF vector, but with all basic and aromatic residues within the stalk removed to prevent trypsin/chymotrypsin cleavage, and with a 6xHis tag (SEQ ID NO: 345) added to the C-terminus of the peptide) based on the original Daedalus vector (Bandaranayake et al., 2011), can be used to clone and express recombinant peptides as described herein. Peptides can be cloned or expressed with a modified coding sequence and without an IRES-GFP. In some cases, the coding sequence of the vector comprises: GFP; the transmembrane domain of the single pass Type II transmembrane protein FASLG (human Fas ligand); a poly-GGGS spacer (SEQ ID NO: 103); a 9-residue bovine rhodopsin antigen; a poly-GGGS spacer (SEQ ID NO: 103); a TEV-cleavage site, including a GS-linker; the displayed peptide; and for SDPR only, a 6xHis tag (SEQ ID NO: 345). In some cases, the coding sequence includes a sequence encoding siderocalin at the N-terminal of the peptide (e.g., SEQ ID NO: 352, or SEQ ID NO: 356 - SEQ ID NO: 361). As with the original Daedalus vector, the entire construct, in some embodiments, is cloned with lentiviral LTRs, and introduced to cells by transient transfection or transduction. Peptide sequences can be inserted between unique BamHI and NotI cut sites for SDGF vector, or between unique BamHI and AgeI cut sites for SDPR. In either SDGF or SDPR construct, a number of standard cloning strategies can be used to insert a desired coding sequence, including restriction digestion (BamHI and NotI, NEB; T4 DNA Ligase, Invitrogen), In-Fusion (Clontech), or Gibson Assembly (NEB), with all transformations using Stellar chemically competent cells (Clontech), or electrocompetent cells (NEB 10-beta cells), transformed by electroporation. DNA can be PCR-amplified using exemplary primers such as: SDGF Forward primer: TGTACTTCCAGGGAGGATCC (SEQ ID NO: 127); SDGF Reverse primer: AATGGTGATGAGCGGCC (SEQ ID NO: 128).

The use of both magnetic and flow sorting may allow for about 10⁹ cells to be screened. One-eighth of the number of cells to be screened, or >1.2 x 10⁸ cells, may be transduced to allow for the 3 days for optimal construct expression and an approximately 24-hour doubling time. To test a limited number of peptide scaffolds, library diversity can be achieved through saturation mutagenesis (e.g., NNK). However, if a more diverse scaffold library is preferred, the library may be reduced to about 10,000 members, ordered as an oligonucleotide pool, and further diversified it by error-prone PCR mutagenesis. For libraries diversified by error-prone PCR mutagenesis, the total library size (the number of library variants) may be heavily dependent on the degree of mutagenesis imposed. Since stop codons and alteration of the cysteine topology can render a peptide nonfunctional. A lower mutagenesis rates (1-1.5 per peptide) may reduce complications from cysteine topology disruption in libraries comprising CDPs, but the mutagenesis rate may be adjusted for each library.

An exemplary method for library design for optimized cloning into SDGF may comprise the steps of: 1) Determining the desired peptide library. The peptide library may be identified from cysteine-rich sequences in existing databases (e.g., Uniprot) or may be derived from *in silico* docking simulations using protein design software (e.g., Rosetta); 2) Converting the peptide sequences to cloning-ready oligonucleotides. Converting the peptide sequences to cloning-ready oligonucleotides may comprise the steps of: (a) Reverse-translating the peptide sequences, using human codon-optimization; (b) Incorporating uniform flanking sequences to facilitate PCR amplification and cloning; (c) If NGS is to be employed for library characterization, verifying that each library member contains a unique sequence within the entirety of the NGS read. Silent mutations may be introduced to ensure unique mapping is possible; and (d) Ordering the finalized library.

**Cloning Pooled Oligonucleotide Libraries into SDGF.** An exemplary method for cloning pooled oligonucleotide libraries into SDGF may comprise the steps of: 1) resuspending the pooled oligonucleotide library in 50 µL molecular biology-grade water or Tris-EDTA buffer; 2) preparing four reactions to PCR amplify the library, running the four reactions at different cycle numbers (e.g., 14, 16, 18, and 20); 3) running all PCR products on an agarose gel and extracting the expected band from a lane lacking the inappropriate, high molecular weight amplicon smear above the desired product, which may be a result of PCR product cross-priming when primers are depleted, and purifying the bands using a gel extraction kit; 4) optionally, if further diversification is desired, performing mutagenesis by error-prone PCR using a Gene-Morph II Random Mutagenesis Kit and the SDGF Gibson primers using manufacturer-recommended reaction conditions, with the PCR product from the previous step as the template, and running on a gel and purify the desired band from a lane without a substantial cross-priming smear, as in step 3; 5) preparing 2 µg per library or 100 ng per singleton to be cloned of BamHI/NotI-digested SDGF backbone; 6) cloning the amplicon into SDGF, for example by incubate a Gibson reaction for 2 h at 50°C instead of the recommended 1 hour; 7) de-salting the reaction using a PCR purification kit, eluting in a reduced volume of 20 µL; 8) electroporating the library into competent *E. coli* and incubating at 37°C overnight, for example, using specialized electrocompetent cells, plating electroporated cells onto a large plate (25 x 25 cm) of LB agar + carbenicillin, taking care to spread completely and allowing to dry completely before inversion and overnight 37 °C incubation with control petri dishes containing serial dilutions of the library to establish library colony forming units (CFUs); 9) counting the control plates to establish CFUs and, if CFUs are sufficient, scraping the plate(s) by adding 25-50 mL LB media plus carbenicillin (100 µg/mL) and scraping with a cell scraper, lifter, or spreader, repeating with more media and scraping until transfer is complete; 10) shaking bacteria in a final volume of 200 mL for 1 hour at 37°C, and then pelleting 50 mL aliquots (4000 x g for 20 min); and 11) freezing all but one pellet at -20°C, maxipreping the remaining pellet, and preferably sterile filtering the final DNA product prior to lentiviral production.

**Mammalian Surface Display.** Validation of peptides or single candidates for binding to TfR can be validated using a suspension of HEK-293 cells. In some embodiments, cells are transfected with 2.5 µg SDGF vector comprising a putative or candidate peptide and 3.5 µg polyethyleneimine to 2E6 cells in 1 mL media, in a 24-well suspension tissue culture dish. After incubation of cells, all pooled screening can be performed in 293T cells transduced with lentivirus (VSV-G coated, produced by standard methods in 293T cells), delivering the SDGF construct, at an MOI of approximately 1, or up to 5. Transduced or transfected suspension HEK-293 cells can be pelleted (500xg, 5 min) and resuspended at up to 8E6 cells/mL in Flow Buffer (PBS with 0.5% BSA and 2 mM EDTA) containing DAPI and target protein, with or without Alexa Fluor 647-conjugated streptavidin (ThermoFisher) in equimolar quantity to the target protein. All staining incubations took place at 4 C for 30 mins with mild agitation. For the low stringency protocol (initial assay validation and pooled screening), 200 nM target protein can be used, and streptavidin can be pre-mixed with target protein. Cells can be incubated, diluted 10-fold with Flow Buffer, pelleted (500xg, 5 min), and resuspended in fresh Flow Buffer (up to 6.5E6 cells/mL) before flow cytometry. For the medium stringency protocol (SSM maturation), only 20 nM target protein can be used, and cells can be incubated with target protein alone, diluted 10-fold with Flow Buffer, pelleted, and then resuspended with 20 nM streptavidin. Cells were incubated again, diluted 10-fold with Flow Buffer, pelleted (500xg, 5 min), and resuspended in fresh Flow Buffer (up to 6.5E6 cells/mL) before flow cytometry. The high stringency protocol is identical to the medium stringency protocol, except that an additional Flow Buffer-only wash step is included immediately after target protein staining. Initial screen was conducted with 200 nM TfR concentration, using a one-step staining protocol for tetravalent target avidity, as opposed to a two-step staining protocol for weaker, monovalent avidity. Subsequent affinity maturation used 20 nM one-step staining (first affinity maturation screen) and 8 nM two-step staining (second affinity maturation screen). Singleton variant validation and permutation test staining took place under the same conditions as the screen from which the variants were enriched and identified. For the transferrin competition staining assay, TfR levels were 8 nM with two-step staining, and transferrin levels were 10 µM. Transfected cells were incubated first with transferrin (10 µM), then TfR solution was added for the first staining step. Cells were pelleted and then resuspended in streptavidin solution for the second staining step. After this second incubation, cells were pelleted and analyzed by flow cytometry for streptavidin staining (representing TfR-binding). For the murine transferrin vs. human transferrin cross-reactivity testing, soluble peptides were labeled with AlexaFluor 647 dye via NHS-ester conjugation, and then stained at 50 nM on cells expressing either human or mouse TfR on their cell surface. No co-stain was necessary, but staining protocol is otherwise identical. In some cases, flow sorting is performed on a Beckton-Dickinson Aria II, while analysis is performed on a combination of Beckton-Dickson LSR II and Acea NovoCyte flow cytometers. Data analysis can be performed on FlowJo (FlowJo, LLC) and Excel (Microsoft).

**Site Saturation Mutagenesis (SSM) of Peptides.** SSM can be employed for affinity maturation of peptides of the present disclosure, for example, for those peptides that are identified in a the first mammalian surface display experiment. During each round of maturation, a library of every possible non-cysteine point variant can be constructed, and screened against TfR at higher stringency than first screen. Variants with improved binding can be enriched and identified by Sanger sequencing. Such enriched variant mutations can be combined with one another in various permutations (shown in the data) to identify a composite, improved binder.

**Next Generation Sequencing.** In some embodiments, screens for enrichment or depletion of variants affecting TfR-binding can be assessed by Illumina sequencing. Such sequencing method involves collecting cell pellets (1.5E6, 3 technical replicates) resuspended in 50 µL Terra Direct PCR Mix (from Clontech) and amplified for 16 cycles using the original cloning primers. Up to four aliquots can be diluted 16-fold into 60 µL Phusion DNA Polymerase reactions and amplified using distinct Illumina primers, containing adaptor sequences for flow cell adherence. Forward primers can include a 6 bp barcode for multiplexing. Illumina HiSeq 2500 in rapid mode can be used to run samples, which Bowtie2 software can be used for mapping, and Excel (Microsoft) and MATLAB (MathWorks) used for data analysis.

**His-Avi-TfR Production, Purification, and Biotinylation.** In some embodiments, in vitro assays are used to analyze candidate peptides for ability to bind TfR. In some cases, biotinylated, and His-Avi-TfR can be expressed in mammalian cells. Optionally, proteins can be biotinylated following purification (e.g., using BirA to biotinylate an encoded AviTag). In some embodiments, the expressed peptide is fused to siderocalin (SEQ ID NO: 351) at the N-terminus for increased stability during translation or secretion (e.g., SEQ ID NO: 352, or SEQ ID NO: 356 - SEQ ID NO: 361). Peptides as disclosed herein can be generated in mammalian cell culture using a published methodology. (A.D. Bandaranayke, C. Correnti, B.Y. Ryu, M. Brault, R.K. Strong, D. Rawlings. 2011. Daedalus: a robust, turnkey platform for rapid production of decigram quantities of active recombinant proteins in human cell lines using novel lentiviral vectors. Nucleic Acids Research. (39)21, e143). The peptide sequence was reverse-translated into DNA, synthesized, and cloned in-frame with siderocalin using standard molecular biology techniques. (M.R. Green, Joseph Sambrook. Molecular Cloning. 2012 Cold Spring Harbor Press.). The resulting construct was packaged into a lentivirus, transduced into HEK-293 cells, expanded, isolated by immobilized metal affinity chromatography (IMAC), cleaved with tobacco etch virus protease, and purified (e.g., separation of peptide and siderocalin carrier (SEQ ID NO: 351)) to homogeneity by size-exclusion chromatography using a gradient of acetonitrile and 0.1% TFA. Peptides expressed as siderocalin fusions can be treated with tobacco etch virus (TEV) protease to cleave siderocalin and leave an N-terminal GS. Thus, the sequences of this disclosure can optionally include an N-terminal GS. Peptides can be separated from the siderocalin carrier using chromatography, e.g., size exclusion chromatography (SEC) or reverse phase high performance liquid chromatography (RP-HPLC). Following purification, each peptide was lyophilized and stored frozen. Alternatively, purified proteins can be flash frozen in aqueous solution (e.g., PBS) with a cryoprotectant (e.g., 5% glycerol) and stored frozen.

**Fluorophore Conjugation.** In some embodiments, screening peptides or a library of peptides involves labeling of a protein of interest or a protein partner with a fluorophore or any other detectable moiety, such that detection of a signal from the fluorophore or detectable moiety is indicative of binding to the peptide. An example of a fluorophore that can be used is Alexa Fluor 647 NHS Ester (Life Technologies), which can be used to label a protein of interest as per manufacturer's protocol. Saturation is approximately 1 fluorophore per molecule, as determined by mass spectrometry. Free dye can be removed following labeling (e.g., by spin column dialysis).

**Surface Plasmon Resonance (SPR) Interaction Analyses.** In some embodiments, TfR-binding can be assessed using surface plasmon resonance (SPR). The peptides of the present disclosure can be tobacco etch virus (TEV)-cleavable and thus can be analyzed as independent, soluble proteins.

Various peptides of the present disclosure can be analyzed for binding affinity to TfR. Binding affinity can be analyzed by SPR experiments using captured biotinylated TfR, which can be performed at 25 °C on a Biacore T100 instrument (GE Healthcare) with Series S SA chips. HBS-EP+ (10 mM HEPES, pH 7.4, 150 mM NaCl, 3 mM EDTA, 0.05% surfactant P20) can be used as a running buffer in the experiments with 0.1 mg/mL bovine serum albumin (BSA). Soluble TfR-binding peptides can be evaluated for binding by incubation of a dilution series, in which the concentration range can be varied depending on the TfR-binding peptide being tested with 2 ug/ml TfR, capturing ~300 resonance units (RUs) of protein for SPR experiments.

In some cases, SPR is performed by injecting the peptides over two densities of captured and biotinylated hTfR and the resulting data can then be analyzed globally. Binding constants (e.g., K_{D} values) and on- and off-rates can be calculated for the interactions between TfR and the TfR-binding peptide.

In some embodiments, SPR is performed with purified apo-transferrin and holo-transferrin. Holo-transferrin can be produced from apo-transferrin by loading apo-transferrin with an iron solution (e.g., 0.4 equivalents of Fe(NTA)₂ added every 5 minutes until 3.2 equivalents have been added). Excess iron can be subsequently removed through buffer exchange (e.g., spin column dialysis into PBS).

**Circular Dichroism.** Protein secondary structures can be assessed using Circular Dichroism (CD). CD spectra can be measured with a Jasco J-720W spectropolarimeter using a 1.0mm path length cell. Protein samples in 10mM phosphate buffer (pH = 7.4) can be at 25-30 uM protein concentration. Samples can be analyzed at wavelength ranges of 260-190 nM. Data can be expressed in terms of relative ellipticity [θ]; (mdeg). To determine thermal stability of proteins, samples can be subjected to incremental increase in temperature at a ramp of 2°C/min from 20°C to 95°C. Stability and protein unfolding were monitored at 210 nm for α-helix secondary structure. Protein unfolding can also be monitored at 215 nm or 220 nm. Data can be expressed in terms of relative ellipticity [θ], reported in mdeg.

**Protease Resistance Testing.** Tumor environments, for instance, contain high concentrations of proteases. Furthermore, resistance to proteolysis (degradation or cleaving by proteases) reduces the likelihood of peptide degradation and subsequent immunogenicity, for example, in circulation. Resistance to proteolysis can further increase a peptide's serum half-life and the overall bioavailability of the peptide. Thus, it is advantageous for peptides of the present disclosure to be resistant to degradation by proteases.

To determine resistance to proteolytic digestion, various peptides of the present disclosure can be mixed with 50 U of porcine pepsin (Sigma-Aldrich P7012) in Simulated Gastric Fluid at pH 1.05, or 50 U of porcine trypsin (Sigma-Aldrich 6567) in PBS, incubated for 30 minutes at 37° C, and analyzed by reverse phase HPLC (RP-HPLC). Separately, the peptides can be mixed with 50 U of porcine pepsin (Sigma-Aldrich P7012) in Simulated Gastric Fluid43 at pH 1.05, or 50 U of porcine trypsin (Sigma-Aldrich 6567) in PBS, incubated for 30 m at 37° C, and analyzed by RP-HPLC. Control peptides can be incubated in PBS. All peptides can be incubated in nonreducing conditions.

In some embodiments, SDPR protease resistance testing can be performed having a sequence of SEQuencing-grade enzymes, including Trypsin and Chymotrypsin. Trypsin and trypsin inhibitor can be used for HPLC analysis.

**Peptide Stability testing under Reducing Conditions.** Peptides that can be designed or engineered to interact with one or more target proteins in the cell, such as proteins in the nucleus, can be exposed to reducing conditions in the cytosolic compartment of cells. Thus, it can be advantageous for peptides of this disclosure to display stability in reducing conditions. Stability of peptides of this disclosure can be tested in 10 mM dithiothreitol (DTT) and/or 10 mM glutathione (GSH). GSH can be a more physiologically relevant reducing agent for testing peptide stability in intracellular conditions. An example of a peptide-target protein interaction is a peptide binding to a TfR protein. Stability to the tested peptides can be evaluated using HPLC (or radio-HPLC when radiolabeled peptides are used), for example.

**Thermal Shift Assay.** Protein melting temperature (Tm) determination can be performed by monitoring protein unfolding using SYPRO Orange dye (Molecular Probes). In brief, 0.1 mg/mL protein sample in 20 µL total volume PBS buffer can be mixed with 2 µL of 10X SYPRO Orange dye. Dye intercalation into the hydrophobic protein core following protein unfolding was assayed using the C1000 Touch Thermal Cycler with CFX96 Deep Well Real-Time System (BioRad). Samples were heated from 20 °C to 95 °C with stepwise increments of 0.5 °C per minute and a 5 sec hold step for every point, followed by fluorescence reading. Tm were calculated by analyzing the derivatives of Relative florescence Units (RFU).

**Cross Reactivity of TfR-binding Peptides to Murine TfR.** Cross reactivity to human and murine Tf R of TfR-binding peptides as disclosed herein can be performed using cell surface binding assays. A reversal of the surface display paradigm is further described in **EXAMPLE 3.** 293F cells expressing either human or mouse TfR from their surface were stained with soluble TfR-binding peptides that were directly labeled with AlexaFluor 647 dye. Flow cytometry can subsequently be used for data analysis to assess human versus mouse reactive if TfR-binding peptides.

**Co-crystalizing the TfR-binding peptides with TfR and solving the crystal structure.** Co-crystalizing the TfR-binding peptides with TfR can be performed as follows. The peptide can be resuspended at a target concentration of approximately 5-15 mg/mL, in some cases 10 mg/mL. Higher concentrations of up to 80 mg/mL can be used for crystallization when, for example, a single peptide is present in the solution without any other components in it. Crystallization screening can be performed at room temperature by vapor diffusion, with 1:1 protein solution:reservoir solution sitting drops, set up using the Nextal JCSG+, PEGs, and (NH₄)₂SO₄ factorial suites (Qiagen) and sub-microliter robotics (TTP Labtech mosquito). Diffraction data can be collected from single crystals using a Rigaku MicroMax-007 HF home source or beamline 5.0.1 at the Advanced Light Source (Lawrence Berkley National Laboratory, Berkeley, CA). Initial phases can be determined either by molecular replacement (MR), using PHASER (McCoy, A.J., J Appl Crystallogr., 40(Pt 4): 658-674 (2007)) in the CCP4 program suite (Winn, M.D., Acta Crystallogr D Biol Crystallogr., 67(Pt 4): 235-42 (2011)) using homologous structures from the RCSB PDB (Berman, H.M., Nucleic Acids Res., 28(1): 235-42 (2000)) as search models, or sulfur single-wavelength anomalous diffraction (sSAD) (Liu, Q., Acta Crystallogr D Biol Crystallogr., 69(Pt 7): 1314-32 (2013)), using CuKalpha radiation to maximize the anomalous signal, and determining sulfur substructures with SHELX (Sheldrick, G.M., Acta Crystallogr D Biol Crystallogr., 66 (Pt 4): 479-85 (2010)). For sSAD phasing, Bijvoet pair measurement can be optimized by collecting data through 5° wedges with alternating phi rotations of 180°, in 1° oscillations. Data can be reduced and scaled with HKL2000 (Otwinowski, Z., Methods Enzymol., 276: 307-326 (1997)). Iterative cycles of model building and refinement can be performed with COOT (Emsely, P., Acta Crystallogr D Biol Crystallogr., 60(Pt 12 Pt 1): 2126-32 (2004)) and REFMAC (Murshudov, G.N., Acta Crystallogr D Biol Crystallogr., 53(Pt 3): 240-55 (1997)). Structure validation can be performed with MolProbity (Davis, I.W., Nucleic Acids Res., 35(Web Server issue): W375-83 (2007)).

**Whole-body autoradiography to elucidate biodistribution of the TfR-binding peptides in mice.** Whole body autoradiography (WBA) sagittal sectioning can be performed using the following steps. Each peptide can be radiolabeled by methylating lysines at the N-terminus as described further in EXAMPLE 14. As such, the peptide can contain methyl or dimethyl lysines and a methylated or dimethlyated amino terminus. A dose of 100 nmol radiolabeled peptide can be administered via tail vein injection in Female Harlan athymic nude mice with intact kidneys, weighing 20-25 g. Each radiolabeled peptide can be allowed to freely circulate within the animal for the described time period before the animals are euthanized and sectioned.

All mice of treatment cohorts can be dosed with 100 nmol radiolabeled peptide or peptide construct (~20 mg/kg). Control mice can be injected with saline or PBS. At the end of the dosing period, mice are frozen in a hexane/dry ice bath and then embedded in a frozen block of carboxymethylcellulose. Whole animal sagittal slices can be prepared that resulted in thin frozen sections for imaging. Thin frozen sections can be obtained using a microtome and allowed visualization of tissues such as brain, tumor, liver, kidney, lung, heart, spleen, pancreas, muscle, adipose, gall bladder, upper gastrointestinal tract, lower gastrointestinal tract, bone, bone marrow, reproductive tract, eye, cartilage, stomach, skin, spinal cord, bladder, salivary gland, and more. Sections are allowed to dessicate in a freezer prior to imaging.

For the autoradiography imaging, tape mounted thin sections were freeze dried and radioactive samples can be exposed to phosphoimager plates for about 7 days. These plates are developed and the signal (densitometry) from each organ was normalized to the signal found in the cardiac blood of each animal. A signal in tissue darker than the signal expected from blood in that tissue indicates accumulation in a region, tissue, structure, or cell.

Reported quantitation of peptide in tissue (nmol/g) was accomplished by referencing an in-block standard curve and the peptide's specific ¹⁴C activity.

**Serum and tissue quantitation of peptide levels over a time course.** Depending on the utilized radionuclide either ex vivo liquid scintillation counting (e.g., ¹⁴C) or gamma counting (e.g., all positron emitting nuclides) can be used to determine peptide levels of various organs at specific time points as well as peptide behavior over time such as for blood half-life and organ uptake and clearance. All mice of treatment cohorts can be dosed with 100 nmol radiolabeled peptide or peptide construct (~20 mg/kg). Control mice can be injected with saline or PBS. Each radiolabeled peptidecan be allowed to freely circulate within the animal for the described time period before the animals were euthanized and sectioned. Collected organs including kidney, liver, brain, spleen, muscle, and skin can be homogenized or weighed before or after counting. Internal reference standards and control samples can also be counted. The peptide uptake data can be visualized as amount of peptide per gram tissue, or as percent injected dose per gram (or per mole or per volume).

**General determination of peptide uptake in cells or tissues.** The uptake of a peptide or peptide construct as disclosed herein can be determined in a specific cell, cell population, tissue, or organ either *ex vivo* or *in vivo.* In the same way, the efficacy of cargo or payload delivery can be determined *ex vivo* or *in vivo,* for example, in cases where the cargo molecule comprises a detectable agent. Ex *vivo* analyses include organ harvest and fixation (e.g., using 4% formaldehyde) of harvested tissue prior to analyses. Tissue samples can be analyzed using a variety of analytical methods including microscopy, spectroscopy, flow cytometry, polymerase chain reaction (PCR), and via measurements of ultrasound, electromagnetic radiation (e.g., UV/VIS, X-ray) or radioactivity. For example, tissue uptake can be determined by measuring luminescence or bioluminescence of a cell, cell population, tissue, or organ sample, or by measuring radioactivity of a cell, cell population, tissue, or organ sample and by calculating uptake values such as percent injected dose per gram (or per mole or per volume). In addition, the acquisition of nuclear images visualizing the biodistribution at a specific point in time (e.g., static imaging) or over a time course (e.g. dynamic imaging) can be performed using various techniques (e.g., PET or SPECT) and appropriately radiolabeled peptides.

**MTR BBB transcytosis model, and capillary depletion quantitation of parenchyma/capillary distribution.** Mice (strain CD-1, N=1 per time point) can be anesthetized, and the left jugular vein and right carotid arteries can be surgically exposed. Labeled peptides (e.g., 0.25-1 µCi in about 200 µL volume; specific activities were 22-40 Ci/mol for SEQ ID NO: 1 and 127-150 Ci/mol for SEQ ID NO: 32) can be injected into the jugular vein. After a given period time within a time course (0-30 minutes), arterial blood can be collected, followed by decapitation and brain collection. Brains and serum can be tested via liquid scintillation counting for ¹⁴C levels, and the data can be incorporated into a multiple time regression mathematic model to quantify CNS accumulation rate. To determine whether this CNS accumulation is limited to capillaries or actually represents vascular transcytosis, a separate set of animals (N=3/peptide) can be euthanized 5 minutes after dosage, and brain homogenates are subjected to centrifugation through a dextran density gradient. This can allow separation of capillaries from parenchyma, and the two tissues can be subjected separately to scintillation counting to quantitate ¹⁴C signal. Normalized signal ratios can be an indicator of BBB penetration; for example, SEQ ID NO: 1 can have a substantially higher signal in the parenchyma than the capillaries, interpreted as evidence of BBB transcytosis.

**Methods for NT fusions and activation of the neuronal CRE reporter mouse.** Transgenic mice expressing firefly luciferase under the control of a cyclic AMP response element (CRE) can be used. Under conditions of elevated cyclic AMP (cAMP) or other mechanisms that activate the transcription factor CRE Binding Protein 1 (CREB), cells in these mice can express luciferase, which can be measured in whole animal luminescence instrumentation (IVIS imager) after IV dosage with a luciferin formulated for animal use. Mice can be validated to express the transgene under proper response element control by dosing these mice with forskolin and rolipram, two drugs that stimulate neuronal cAMP production and inhibit its degradation (respectively). Animals dosed with forskolin and rolipram can then be tested for luciferase expression 4 hours later as described herein. High levels of luminescence from the animal's brain can be typical in these control experiments. Animals that validate in this assay can be given about 1 week to return to steady state (low) luciferase expression, and can then be tested with neurotensin or TfR binding proteins fused with neurotensin. Neurotensin receptor expression can be limited to subsets of cells in the CNS, including the frontal cortex. Upon activation, a signal transduction pathway can be activated that culminates in CREB phosphorylation and CRE-mediated transcription. Its ligand, neurotensin, is a neuropeptide that cannot cross the BBB. Therefore, IV dosage of a fusion protein comprising neurotensin and a TfR binding peptide can demonstrate BBB penetration if CRE-driven luciferase is induced in the brains of these mice.

Peptide constructs, which are NT fusions include any sequence having at least 90%, at least 92%, at least 93%, at least 94%, at least 95%, at least 96%, at least 97%, at least 98%, at least 99%, or 100% sequence identity with any one of SEQ ID NO: 33 - SEQ ID NO: 35, SEQ ID NO: 68 - SEQ ID NO:70, SEQ ID NO: 168 - SEQ ID NO: 170, SEQ ID NO: 203 - SEQ ID NO: 205, or SEQ ID NO: 359 - SEQ ID NO: 361.

### Methods of Manufacture

Various expression vector/host systems can be utilized for the recombinant expression of peptides described herein. Non-limiting examples of such systems include microorganisms such as bacteria transformed with recombinant bacteriophage DNA, plasmid DNA or cosmid DNA expression vectors containing a nucleic acid sequence encoding peptides, peptide constructs, or peptide fusion proteins/chimeric proteins described herein, yeast transformed with recombinant yeast expression vectors containing the aforementioned nucleic acid sequence, insect cell systems infected with recombinant virus expression vectors (e.g., baculovirus) containing the aforementioned nucleic acid sequence, plant cell systems infected with recombinant virus expression vectors (e.g., cauliflower mosaic virus (CaMV), tobacco mosaic virus (TMV)), or transformed with recombinant plasmid expression vectors (e.g., Ti plasmid) containing the aforementioned nucleic acid sequence, or animal cell systems infected with recombinant virus expression vectors (e.g., adenovirus, vaccinia virus, lentivirus) including cell lines engineered to contain multiple copies of the aforementioned nucleic acid sequence, either stably amplified (e.g., CHO/dhfr, CHO/glutamine synthetase) or unstably amplified in double-minute chromosomes (e.g., murine cell lines). Disulfide bond formation and folding of the peptide could occur during expression or after expression or both.

A host cell can be adapted to express one or more peptides described herein. The host cells can be prokaryotic, eukaryotic, or insect cells. In some cases, host cells are capable of modulating the expression of the inserted sequences, or modifying and processing the gene or protein product in the specific fashion desired. For example, expression from certain promoters can be elevated in the presence of certain inducers (e.g., zinc and cadmium ions for metallothionine promoters). In some cases, modifications (e.g., phosphorylation) and processing (e.g., cleavage) of peptide products can be important for the function of the peptide. Host cells can have characteristic and specific mechanisms for the post-translational processing and modification of a peptide. In some cases, the host cells used to express the peptides secrete minimal amounts of proteolytic enzymes.

In the case of cell- or viral-based samples, organisms can be treated prior to purification to preserve and/or release a target polypeptide. In some embodiments, the cells are fixed using a fixing agent. In some embodiments, the cells are lysed. The cellular material can be treated in a manner that does not disrupt a significant proportion of cells, but which removes proteins from the surface of the cellular material, and/or from the interstices between cells. For example, cellular material can be soaked in a liquid buffer, or, in the case of plant material, can be subjected to a vacuum, in order to remove proteins located in the intercellular spaces and/or in the plant cell wall. If the cellular material is a microorganism, proteins can be extracted from the microorganism culture medium. Alternatively, the peptides can be packed in inclusion bodies. The inclusion bodies can further be separated from the cellular components in the medium. In some embodiments, the cells are not disrupted. A cellular or viral peptide that is presented by a cell or virus can be used for the attachment and/or purification of intact cells or viral particles. In addition to recombinant systems, peptides can also be synthesized in a cell-free system prior to extraction using a variety of known techniques employed in protein and peptide synthesis.

In some cases, a host cell produces a peptide that has an attachment point for a cargo molecule (e.g., a therapeutic agent). An attachment point could comprise a lysine residue, an N-terminus, a cysteine residue, a cysteine disulfide bond, a glutamic acid or aspartic acid residue, a C-terminus, or a non-natural amino acid. The peptide could also be produced synthetically, such as by solid-phase peptide synthesis, or solution-phase peptide synthesis. Peptide synthesis can be performed by fluorenylmethyloxycarbonyl (Fmoc) chemistry or by butyloxycarbonyl (Boc) chemistry. The peptide could be folded (formation of disulfide bonds) during synthesis or after synthesis or both. Peptide fragments could be produced synthetically or recombinantly. Peptide fragments can be then be joined together enzymatically or synthetically.

In other aspects, the peptides of the present disclosure can be prepared by conventional solid phase chemical synthesis techniques, for example according to the Fmoc solid phase peptide synthesis method ("Fmoc solid phase peptide synthesis, a practical approach," edited by W. C. Chan and P. D. White, Oxford University Press, 2000).

In some embodiments, the peptides of this disclosure can be more stable during manufacturing. For example, peptides of this disclosure can be more stable during recombinant expression and purification, resulting in lower rates of degradation by proteases that are present in the manufacturing process, a higher purity of peptide, a higher yield of peptide, or any combination thereof. In some embodiments, the peptides can also be more stable to degradation at high temperatures and low temperatures during manufacturing, storage, and distribution. For example, in some embodiments peptides of this disclosure can be stable at 25 °C. In other embodiments, peptides of this disclosure can be stable at 70 °C or higher than 70 °C. In some embodiments, peptides of this disclosure can be stable at 100 °C or higher than 100 °C.

### Pharmaceutical Compositions

A pharmaceutical composition of the disclosure can be a combination of any peptide as described herein with other chemical components, such as carriers, stabilizers, diluents, dispersing agents, suspending agents, thickening agents, antioxidants, solubilizers, buffers, osmolytes, salts, surfactants, amino acids, encapsulating agents, bulking agents, cryoprotectants, and/or excipients. The pharmaceutical composition facilitates administration of a peptide described herein to an organism. In some cases, the pharmaceutical composition comprises factors that extend half-life of the peptide and/or help the peptide to penetrate the target cells.

Pharmaceutical compositions can be administered in therapeutically-effective amounts as pharmaceutical compositions by various forms and routes including, for example, intravenous, subcutaneous, intramuscular, rectal, aerosol, parenteral, ophthalmic, pulmonary, transdermal, vaginal, optic, nasal, oral, sublingual, inhalation, dermal, intrathecal, intratumoral, intranasal, and topical administration. A pharmaceutical composition can be administered in a local or systemic manner, for example, via injection of the peptide described herein directly into an organ, optionally in a depot.

Parenteral injections can be formulated for bolus injection or continuous infusion. The pharmaceutical compositions can be in a form suitable for parenteral injection as a sterile suspension, solution or emulsion in oily or aqueous vehicles, and can contain formulatory agents such as suspending, stabilizing and/or dispersing agents. Pharmaceutical formulations for parenteral administration include aqueous solutions of a peptide described herein in water-soluble form. Suspensions of peptide-antibody complexes described herein can be prepared as oily injection suspensions. Suitable lipophilic solvents or vehicles include fatty oils such as sesame oil, or synthetic fatty acid esters, such as ethyl oleate or triglycerides, or liposomes. Aqueous injection suspensions can contain substances which increase the viscosity of the suspension, such as sodium carboxymethyl cellulose, sorbitol, or dextran. The suspension can also contain suitable stabilizers or agents which increase the solubility and/or reduce the aggregation of such peptide-antibody complexes described herein to allow for the preparation of highly concentrated solutions.

Formulations or approaches to increase cell penetration or a peptide as describe herein can include, but not limited to, using high dosage of a peptide as described herein, such as up to 10 µM; conjugating or fusing a Tat peptide or a cell-penetrating peptide, or a variant or derivative thereof to a peptide; co-delivering a Tat peptide or a cell-penetrating peptide, or a variant or derivative thereof with a peptide; conjugating or fusing an Arg patch to a peptide; or co-delivering a peptide with an Arg patch peptide, such as up to 10 µM. In some embodiments, protein transfection agents, direct cytosolic expression of the peptide, or electrophoration of the peptide can be used to increase cell penetration. In some embodiments, other excipients can be formulated with a peptide in order to increase the cell penetration of the peptide, such as those approaches described in "Protein and Peptide Drug Delivery: Oral Approaches" Indian J. Pharm. Sci., Shaji and Patole, v70(3) 269-277, 2008. Any combination of these formulations or approaches can be used to increase cell penetration of a peptide as described herein.

Alternatively, the peptide described herein can be lyophilized or in powder form for reconstitution with a suitable vehicle, e.g., sterile pyrogen-free water, before use. In some embodiments, a purified peptide is administered intravenously. A peptide described herein can be administered to a subject in order to home, target, migrate to, or be directed to a CNS cell, a brain cell, a cancerous cell, or a tumor. In some embodiments, a peptide can be conjugated to, linked to, or fused to another peptide that provides a targeting function to a specific target cell type in the central nervous system or across the blood brain barrier. Exemplary target cells include a CNS cell, erythrocyte, an erythrocyte precursor cell, an immune cell, a stem cell, a muscle cell, a brain cell, a thyroid cell, a parathyroid cell, an adrenal gland cell, a bone marrow cell, an appendix cell, a lymph node cell, a tonsil cell, a spleen cell, a muscle cell, a liver cell, a gallbladder cell, a pancreas cell, a cell of the gastrointestinal tract, a glandular cell, a kidney cell, a urinary bladder cell, an endothelial cell, an epithelial cell, a choroid plexus epithelial cell, a neuron, a glial cell, an astrocyte, or a cell associated with a nervous system.

A peptide of the disclosure can be applied directly to an organ, or an organ tissue or cells, such as brain or brain tissue or cells, during a surgical procedure. The recombinant peptide described herein can be administered topically and can be formulated into a variety of topically administrable compositions, such as solutions, suspensions, lotions, gels, pastes, medicated sticks, balms, creams, and ointments. Such pharmaceutical compositions can contain solubilizers, stabilizers, tonicity enhancing agents, buffers and preservatives.

In practicing the methods of treatment or use provided herein, therapeutically-effective amounts of a peptide described herein can be administered in pharmaceutical compositions to a subject suffering from a condition that affects the immune system. In some embodiments, the subject is a mammal such as a human or a primate. A therapeutically-effective amount can vary widely depending on the severity of the disease, the age and relative health of the subject, the potency of the compounds used, and other factors.

In some embodiments, a peptide is cloned into a viral or non-viral expression vector. Such expression vector can be packaged in a viral particle, a virion, or a non-viral carrier or delivery mechanism, which is administered to patients in the form of gene therapy. In other embodiments, patient cells are extracted and modified to express a peptide capable of binding TfR *ex vivo* before the modified cells are returned back to the patient in the form of a cell-based therapy, such that the modified cells will express the peptide once transplanted back in the patient.

Pharmaceutical compositions can be formulated using one or more physiologically-acceptable carriers comprising excipients and auxiliaries, which facilitate processing of the active compounds into preparations that can be used pharmaceutically. Formulation can be modified depending upon the route of administration chosen. Pharmaceutical compositions comprising a peptide described herein can be manufactured, for example, by expressing the peptide in a recombinant system, purifying the peptide, lyophilizing the peptide, mixing, dissolving, granulating, dragee-making, levigating, emulsifying, encapsulating, entrapping, or compression processes. The pharmaceutical compositions can include at least one pharmaceutically acceptable carrier, diluent, or excipient and compounds described herein as free-base or pharmaceutically-acceptable salt form.

Methods for the preparation of peptide described herein comprising the compounds described herein include formulating peptide described herein with one or more inert, pharmaceutically-acceptable excipients or carriers to form a solid, semi-solid, or liquid composition. Solid compositions include, for example, powders, tablets, dispersible granules, capsules, cachets, and suppositories. These compositions can also contain minor amounts of nontoxic, auxiliary substances, such as wetting or emulsifying agents, pH buffering agents, and other pharmaceutically-acceptable additives.

Non-limiting examples of pharmaceutically-acceptable excipients can be found, for example, in Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999),

Pharmaceutical compositions can also include permeation or absorption enhancers (Aungst et al. AAPS J. 14(1):10-8. (2012) and Moroz et al. Adv Drug Deliv Rev 101:108-21. (2016)). Permeation enhancers can facilitate uptake of molecules from the GI tract into systemic circulation. Permeation enhancers can include salts of medium chain fatty acids, sodium caprate, sodium caprylate, N-(8-[2-hydroxybenzoyl]amino)caprylic acid (SNAC), N-(5-chlorosalicyloyl)-8-aminocaprylic acid (5-CNAC), hydrophilic aromatic alcohols such as phenoxyethanol, benzyl alcohol, and phenyl alcohol, chitosan, alkyl glycosides, dodecyl-2-N,N-dimethylamino propionate (DDAIPP), chelators of divalent cations including EDTA, EGTA, and citric acid, sodium alkyl sulfate, sodium salicylate, lecithin-based, or bile salt-derived agents such as deoxycholates.

Compositions can also include protease inhibitors including soy bean trypsin inhibitor, aprotinin, sodium glycocholate, camostat mesilate, vacitracin, or cyclopentadecalactone.

### Peptides for use in Treatments

Any reference to a method of treatment identified in this specification refers to the composition as defined in the claims for use in the identified method. In some embodiments, the method includes administering an effective amount of a peptide as described herein to a subject in need thereof.

**In** one embodiment, the method includes administering an effective amount of a peptide as described herein to a subject in need thereof.

TfR can be expressed in various tissues such as the brain, the stomach, the liver, of the gall bladder. Hence, the TfR-binding peptides of the present disclosure can be used in the diagnosis and treatment of disease and conditions associated with various tissues and organs. For example, drug delivery to these tissues and organs can be improved by using the herein described peptides and peptide constructs carrying a diagnostic and/or therapeutic payload.

The term "effective amount," as used herein, refers to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. Compositions containing such agents or compounds can be administered for prophylactic, enhancing, and/or therapeutic treatments. An appropriate "effective" amount in any individual case can be determined using techniques, such as a dose escalation study.

The methods, compositions, and kits of this disclosure can comprise a method to prevent, treat, arrest, reverse, or ameliorate the symptoms of a condition. The treatment can comprise treating a subject (e.g., an individual, a domestic animal, a wild animal, or a lab animal afflicted with a disease or condition) with a peptide of the disclosure. The disease can be a cancer or tumor. In treating the disease, the peptide can contact the tumor or cancerous cells. The subject can be a human. Subjects can be humans; non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. A subject can be of any age. Subjects can be, for example, elderly adults, adults, adolescents, pre-adolescents, children, toddlers, infants, and fetuses in utero.

Treatment can be provided to the subject before clinical onset of disease. Treatment can be provided to the subject after clinical onset of disease. Treatment can be provided to the subject after 1 day, 1 week, 6 months, 12 months, or 2 years or more after clinical onset of the disease. Treatment can be provided to the subject for more than 1 day, 1 week, 1 month, 6 months, 12 months, 2 years or more after clinical onset of disease. Treatment can be provided to the subject for less than 1 day, 1 week, 1 month, 6 months, 12 months, or 2 years after clinical onset of the disease. Treatment can also include treating a human in a clinical trial. A treatment can comprise administering to a subject a pharmaceutical composition, such as one or more of the pharmaceutical compositions described throughout the disclosure. A treatment can comprise a once daily dosing. A treatment can comprise delivering a peptide of the disclosure to a subject, either intravenously, subcutaneously, intramuscularly, by inhalation, dermally, topically, by intra-articular injection, orally, sublingually, intrathecally, transdermally, intranasally, via a peritoneal route, directly into a tumor e.g., injection directly into a tumor, directly into the brain, e.g., via and intracerebral ventricle route, or directly onto a joint, e.g. via topical, intra-articular injection route. A treatment can comprise administering a peptide-active agent complex to a subject, either intravenously, subcutaneously, intramuscularly, by inhalation, by intra-articular injection, dermally, topically, orally, intrathecally, transdermally, intransally, parenterally, orally, via a peritoneal route, nasally, sublingually, or directly onto cancerous tissues.

Binding of the herein described peptides and peptide constructs (e.g., peptide conjugates or recombinantly produced peptide constructs) to TfR and subsequent transport across a cell layer or barrier such as the BBB (e.g., via vesicular transcytosis) or a cell membrane (e.g., via endocytosis such as receptor-mediated endocytosis) or accumulation in a tissue can have implications in a number of diseases, conditions, or disorders such as those associated with cell growth, cell proliferation, angiogenesis, organogenesis, tumor progression, and/or metastasis, cell death (e.g., senescence), neurodegeneration, and pain in various cells, tissues and organs, particularly those cells tissues or organs that express and/or overexpress TfR. In some embodiments, peptide constructs (e.g., peptide-NT constructs) interact with dopamine-signaling neurons to reduce pain. Compositions comprising any one of the peptides disclosed herein that bind TfR, or a pharmaceutical composition thereof, can be used in a method of treating, preventing, or diagnosing a cancer, tumor progression, dysregulated cell growth in a variety of cancers including ovarian cancer, colon cancer, glioblastoma, astrocytoma, glioma, medulloblastoma, ependymoma, choroid plexus carcinoma, midline glioma, diffuse intrinsic pontine glioma, and lung cancer, cancer located in the bone or bone marrow, glioblastoma, astrocytoma, glioma, medulloblastoma, ependymoma, choroid plexus carcinoma, midline glioma, and diffuse intrinsic pontine glioma (DIPG), breast cancer, liver cancer, colon cancer, brain cancer, spleen cancer, cancers of the salivary gland, kidney cancer, muscle cancers, bone marrow cell cancers, or skin cancer, genitourinary cancer, osteosarcoma, muscle-derived sarcoma, melanoma, head and neck cancer, a neuroblastoma, or a CMYC-overexpressing cancer. In some embodiments, the TfR-binding peptides of the present disclosure enable delivery to tumor cells, bone marrow cells (e.g., erythroid precursor cells), spleen cells, immune cells, cancers of the salivary gland, or muscle cells. The TfR-binding peptides of the present disclosure can be used in combination with drug used for the treatment or prevention of neurological and CNS-related disorder (e.g., neurodegenerative diseases). Additional diseases, disorders, or conditions can include multiple myeloma, plastic anemia, myelodysplasia, and related bone marrow failure syndromes, myeloproliferative diseases, acute and chronic myeloid leukemia, malignancies of lymphoid cells, hematologic malignancies, plasma cell disorders, skeletal muscle disorder, myopathy, muscular dystrophy (e.g., Becker muscular dystrophy, Duchenne muscular dystrophy, Emery-Dreifuss muscular dystrophy, Facioscapulohumoeral muscular dystrophy, Myotonia congentia, and myotonic dystrophy), and chronic obstructive pulmonary disorder. In some embodiments, compositions/peptides disclosed herein are used to treat dysregulated cell growth, cancer, tumor, and/or metastasis associated with any of the following cell, tissue, or organ types: brain, lung, liver, spleen, kidney, lymph node, small intestine, blood cell, bone marrow cell, hematopoietic stem cell, pancreatic, colon, stomach, cervix, breast, endometrial, muscular, connective, prostate, testicle, ovarian, skin, head and neck, esophageal, oral tissue, and bone marrow. In further embodiments, compositions/peptides disclosed herein are used to treat nay of the following: sarcoma, osteosarcoma, muscle-derived sarcoma, hepatocellular carcinoma, malignant mesothelioma, schwannoma, meningioma, renal carcinoma, cholangiocarcinoma, bile duct hamartoma, soft tissue carcinoma, myeloma, multiple myeloma, leukemia, lymphoma, ovarian carcinoma, colonic adenoma, T cell acute lymphoblastic leukemia, gastrointestinal hyperplasia, fibrosarcoma, pancreatic ductal metaplasia, squamous cell carcinoma, kaposis sarcoma, and HIV-induced non-Hodgkin's lymphoma.

In various embodiments, the present disclosure provides methods and compositions that enable TfR-mediated transport across cellular layers (e.g., endothelial cells or epithelial cells) or cell membranes. In addition to the BBB, various other cells, tissues, and organs express TfR. Single cells expressing TfR can include hepatocytes, erythrocytes and erythrocyte precursors in bone marrow, immune cells, stem cells, and rapidly dividing cells. Tissues and organs expressing TfR can include the brain (e.g., cerebral cortex, hippocampus, caudate, cerebellum), endocrine tissues (e.g., thyroid, parathyroid, and adrenal glands), bone marrow and immune system (e.g., appendix, lymph node, tonsil, spleen), muscle tissues (e.g., heart, skeletal, and smooth muscle), liver, gallbladder, pancreas, gastrointestinal tract (e.g., oral mucosa, esophagus, stomach, duodenum, small intestine, colon, rectum), kidney, urinary bladder, female tissues (e.g., fallopian tube, breast, vagina, cervix, endometrium, ovary, and placenta), adipose and soft tissue, and skin. Thus, the TfR-binding peptides of the present disclosure can be used to target these cells, tissues, and organs and deliver an active agent to these cells, tissues, and organs via, for example, TfR-mediated transcytosis (e.g., across cellular barrier such as the BBB) or TfR-mediated endocytosis (e.g., across cell membranes into cells).

In various embodiments, the present disclosure provides methods and compositions that enable TfR-mediated transport and delivery to tumor cells expressing TfR. Cancers overexpressing TfR can include ovarian cancer, colon cancer, lung cancer, cancer located in the bone or bone marrow, glioblastoma, astrocytoma, glioma, medulloblastoma, ependymoma, choroid plexus carcinoma, midline glioma, diffuse intrinsic pontine glioma (DIPG), breast cancer, liver cancer, colon cancer, brain cancer, spleen cancer, cancers of the salivary gland, kidney cancer, muscle cancers, bone marrow cell cancers, skin cancer, genitourinary cancer, osteosarcoma, muscle-derived sarcoma, melanoma, head and neck cancer, neuroblastoma, prostate cancer, bladder cancer, acute lymphocytic leukemia, acute myeloid leukemia, chronic lymphocytic leukemia, chronic myeloid leukemia, Hodgkin lymphoma, Non-Hodgkin lymphoma, or a CMYC-overexpressing cancer. Brain cancers include but are not limited to glioblastoma, astrocytoma, glioma, medulloblastoma, ependymoma, choroid plexus carcinoma, midline glioma, and diffuse intrinsic pontine glioma.

TfR-binding peptide constructs that can be used to prevent and/or treat a cancer are those comprising a TfR binding peptide and an active agent with anti-tumor activity such as an IL15, a fusion of IL15/IL15Ra, IFNgamma, and anti-CD3 agents. Examples of TfR-binding peptide constructs comprising an active agent with anti-tumor activity are those having an amino acid sequence set forth in any one of SEQ ID NO: 225 - SEQ ID NO: 332). An active agent with active tumor activity (e.g., IL15, a fusion of IL15/IL15Ra, IFNgamma, or anti-CD3 agents) may be fused to a TfR-binding peptide (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) to form a peptide-active agent construct.

Binding of the herein described peptides and peptide constructs (e.g., peptide conjugates or recombinantly produced peptide constructs) to TfR and subsequent transport across a cell layer or barrier such as the BBB (e.g., via TfR-mediated vesicular transcytosis) or a cell membrane (e.g., via TfR-mediated endocytosis) can have implications in a number of diseases, conditions, or disorders associated with chronic pain (e.g., headaches or migrane), neuropathic pain, obesity, insulin resistance, opioid addiction, or other neurologic or psychiatric disorders in a subject (e.g., a human). In some embodiments, peptide constructs (e.g., peptide-NT constructs) interact with dopamine-signaling neurons to reduce pain.

Binding of the herein described peptides and peptide constructs (e.g., peptide conjugates, fusion peptides, or recombinantly produced peptide constructs) to TfR and subsequent transport across a cell layer or barrier such as the BBB (e.g., via vesicular transcytosis) or a cell membrane (e.g., via endocytosis) can have implications in a number of diseases, conditions, or disorders associated with neurodegeneration. Neurodegenerative diseases that can treated, prevented, or diagnosed with the herein described TfR-binding peptides can include Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia. In some cases, the TfR-binding peptide can be used in combination with BACE inhibitors, galantamine, amantadine, benztropine, biperiden, bromocriptin, carbidopa, donepezil, entacapone, levodopa, pergolie, pramipexole, procyclidine, rivastigmine, ropinirole, selegiline, tacrine, tolcapone, or trihexyphenidyl to treat and/or prevent a neurodegenerative disease.

In some embodiments, modulation (e.g., inhibition) of ion channels such as Kv1.3 potassium channels using TfR-binding peptides and ion channel modulators such as Kv1.3 potassium channel inhibitor conjugates or peptide constructs can be used for treating or preventing inflammation in the brain. Kv1.3 potassium channels, for instance, can be highly expressed on microglia in the brain. Thus, neuropinflammatory and neurodegenerative diseases such as multiple sclerosis, Alzheimer's, Parkinson's, traumatic brain injury, radiation therapy toxicity and other neurodegenerative and neuroinflammatory diseases can be treated with TfR-binding peptides that are conjugated to, linked to, or fused to a Kv1.3 inhibitor. These diseases can be marked by upregulation of Kv1.3. In some cases, Kv1.3 inhibition can be used for treatment of psoriasis and other non-brain autoimmune diseases due to its effect on effector T cells.

In some embodiments, the TfR-binding peptides of the present disclosure can be used for the treatment and prevention of Crohn's disease or, more generally, inflammatory bowel diseases. In some embodiments, the TfR-binding peptides of the present disclosure show high uptake and retention in glandular cells of the intestine, which can express high amounts of TfR.

In some embodiments, the peptides or peptide constructs of the present disclosure (e.g., SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) are used to access and treat these disorders due to their enhanced stability in various biological environments, including low pH, protease-rich environments, acidic environments, reducing environments, or environments with varying temperatures.

In some embodiments, compositions comprise any one of the TfR-binding peptides disclosed herein and are capable of transporting one or more cargo molecules or active agents per TfR-binding peptides across cell layers or cell barriers such as endothelial layers (e.g., the BBB) or epithelial layers, or cell membranes. In some embodiments, the cargo molecule or active agent is an immunotherapeutic agent, a CTLA-4 targeting agent, a PD-1 targeting agent, a PDL-1 targeting agent, an IL15, a fusion of IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, or a neurotransmitter such as neurotensin.

### Peptide Kits

In one aspect, peptides described herein can be provided as a kit. In another embodiment, peptide constructs described herein can be provided as a kit. In another embodiment, a kit comprises amino acids encoding a peptide described herein, a vector, a host organism, and an instruction manual. In some embodiments, a kit includes written instructions on the use or administration of the peptides.

### EXAMPLES

The following examples are included to further describe some aspects of the present disclosure, and should not be used to limit the scope of the invention.

### EXAMPLE 1

### Manufacture of Peptides

This example describes the manufacture of the peptides and peptide constructs described herein (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361). Peptides derived from proteins were generated in mammalian cell culture using a published methodology. (A.D. Bandaranayke, C. Correnti, B.Y. Ryu, M. Brault, R.K. Strong, D. Rawlings. 2011. Daedalus: a robust, turnkey platform for rapid production of decigram quantities of active recombinant proteins in human cell lines using novel lentiviral vectors. Nucleic Acids Research. (39)21, e143).

The peptide sequence was reverse-translated into DNA, synthesized, and cloned in-frame with siderocalin using standard molecular biology techniques (M.R. Green, Joseph Sambrook. Molecular Cloning. 2012 Cold Spring Harbor Press). The resulting construct was packaged into a lentivirus, transduced into HEK-293 cells, expanded, isolated by immobilized metal affinity chromatography (IMAC), cleaved with tobacco etch virus (TEV) protease, and purified to homogeneity by reverse-phase chromatography. Following purification, each peptide was lyophilized and stored frozen.

### EXAMPLE 2

### Peptide Expression Using a Mammalian Expression System

This example describes expression of the peptides and peptide constructs using a mammalian expression system. Peptides were expressed according to the methods described in in Bandaranayake et al., Nucleic Acids Res. 2011 Nov; 39(21): e143. Peptides (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) were cleaved from siderocalin using tobacco etch virus protease and were purified by reversed-phase HPLC (RP-HPLC) in a gradient of acetonitrile and water with 0.1% TFA, and then aliquoted and lyophilized for later use. Molecular weight was verified by mass spectrometry.

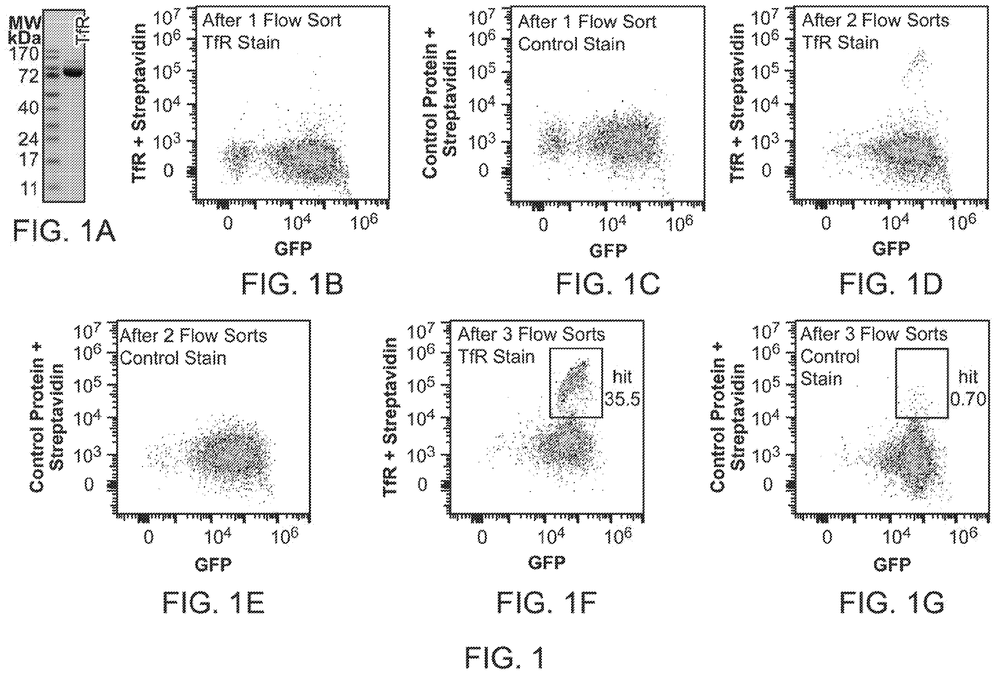

To optimize and validate the screening methodology and to identify TfR-binding peptides, transferrin receptor (TfR) ectodomain ("soluble TfR", SEQ ID NO: 353) was cloned into the Daedalus soluble protein production lentivector, and protein was purified from the growth media (a gel of soluble TfR is shown in **FIG. 1A**). The same strategy was used to produce and purify human apo-transferrin (residues 23-698, SEQ ID NO: 364, KTVRWCAVSEHEATKCQSFRDHMKSVIPSDGPSVACVKKASYLDCIRAIAANEADAVTL DAGLVYDAYLAPNNLKPVVAEFYGSKEDPQTFYYAVAVVKKDSGFQMNQLRGKKSCH TGLGRSAGWNIPIGLLYCDLPEPRKPLEKAVANFFSGSCAPCADGTDFPQLCQLCPGCGC STLNQYFGYSGAFKCLKDGAGDVAFVKHSTIFENLANKADRDQYELLCLDNTRKPVDE YKDCHLAQVPSHTVVARSMGGKEDLIWELLNQAQEHFGKDKSKEFQLFSSPHGKDLLF KDSAHGFLKVPPRMDAKMYLGYEYVTAIRNLREGTCPEAPTDECKPVKWCALSHHERL KCDEWSVNSVGKIECVSAETTEDCIAKIMNGEADAMSLDGGFVYIAGKCGLVPVLAEN YNKSDNCEDTPEAGYFAIAVVKKSASDLTWDNLKGKKSCHTAVGRTAGWNIPMGLLY NKINHCRFDEFFSEGCAPGSKKDSSLCKLCMGSGLNLCEPNNKEGYYGYTGAFRCLVEK GDVAFVKHQTVPQNTGGKNPDPWAKNLNEKDYELLCLDGTRKPVEEYANCHLARAPN HAVVTRKDKEACVHKILRQQQHLFGSNVTDCSGNFCLFRSETKDLLFRDDTVCLAKLH DRNTYEKYLGEEYVKAVGNLRKCSTSSLLEACTFRRP), and some of the material was iron-loaded to produce holo-transferrin. Both apo-transferrin and holo-transferrin were tested for binding to the soluble TfR ectodomain via surface plasmon resonance (FIG. 23A), where only holo-transferrin demonstrated any interaction with the immobilized TfR.

As further validation that the soluble TfR used in the screen represents the human, endogenous protein structure, interaction with the Machupo virus glycoprotein was tested (referred to as MaCV), which uses TfR to determine tropism and mediate cell entry. For this, MaCV was cloned into a mammalian surface display vector SDGF (FIG. 23B), and transfected suspension 293 Freestyle (293F) cells with SDGF-MaCV or a control protein (SDGF-Elafin, an inhibitor of elastase known to bind some native CDPs). Transfected cells were stained with 200 nM each biotinylated TfR (all TfR used in cell binding assays is biotinylated) and Alexa Fluor 647-labeled streptavidin, and then analyzed by flow cytometry (**FIG. 23C**). Cells transfected with MaCV were successfully stained with TfR, while SDGF-Elafin cells were not. Meanwhile, SDGF-MaCV cells incubated with 200 nM fluorescent elastase were not stained. This demonstrates both the specificity of TfR binding to its endogenous ligand, and the utility of SDGF as a means to identify novel TfR binding partners.

### EXAMPLE 3

### Expression and Purification of Functional Transferrin Receptor

Transferrin receptor (TfR) ectodomain (SEQ ID NO: 352, residues 121-760 of HsTfR by UniProt numbering), apo-transferrin (SEQ ID NO: 353 (human) and SEQ ID NO: 354 (mouse)), and all soluble CDPs were produced and purified as **EXAMPLE 2** and **EXAMPLE 3.** In brief, relevant sequences were cloned into the Daedalus vector, a lentivector driving secretion of proteins of interest. Proteins either contained their endogenous signal peptide or were fused at their N-terminus to siderocalin for stabilization during translation / secretion; siderocalin fusions were cleaved using TEV protease, leaving an N-terminal "GS" behind. VSV-G pseudotyped lentivirus was produced through standard methods in 293 cells. This virus was used to transduce 1×10⁷ suspension 293 Freestyle (293F, ThermoFisher R79007) cells at an approximate multiplicity of infection (MOI) of 10 in FreeStyle 293 Expression Medium (Invitrogen 12338018). The culture was then grown (suspension culture incubator at 125 RPM, 37°C, 80% humidity, 8% CO₂) and expanded over the course of 10-12 days (2 L final volume), after which media was collected and sterile filtered. Proteins were purified from media by batch Ni-NTA resin binding. CDPs were separated from the siderocalin carrier by TEV protease cleavage, and a final polish with either SEC (ÄKTA Pure, GE Healthcare) or RP-HPLC (Agilent model 1260, C18 column, 214 nm absorbance, with an in-line Agilent 6120 LC/MS) was performed (FIG. 1A). TfR and transferrin were frozen in PBS + 5% glycerol, while the CDP peptides, purified by RP-HPLC, were aliquoted and lyophilized in vials. Peptide quantitation was established by amino acid analysis before use *in vivo.*

Proper folding and function of purified TfR was verified using surface plasmon resonance. Binding of either apo-transferrin (ApoTf) or holo-transferrin (HoloTf) to the purified TfR was measured (**FIG. 23A**). Preferential binding of HoloTf over ApoTf is indicative of proper TfR function.

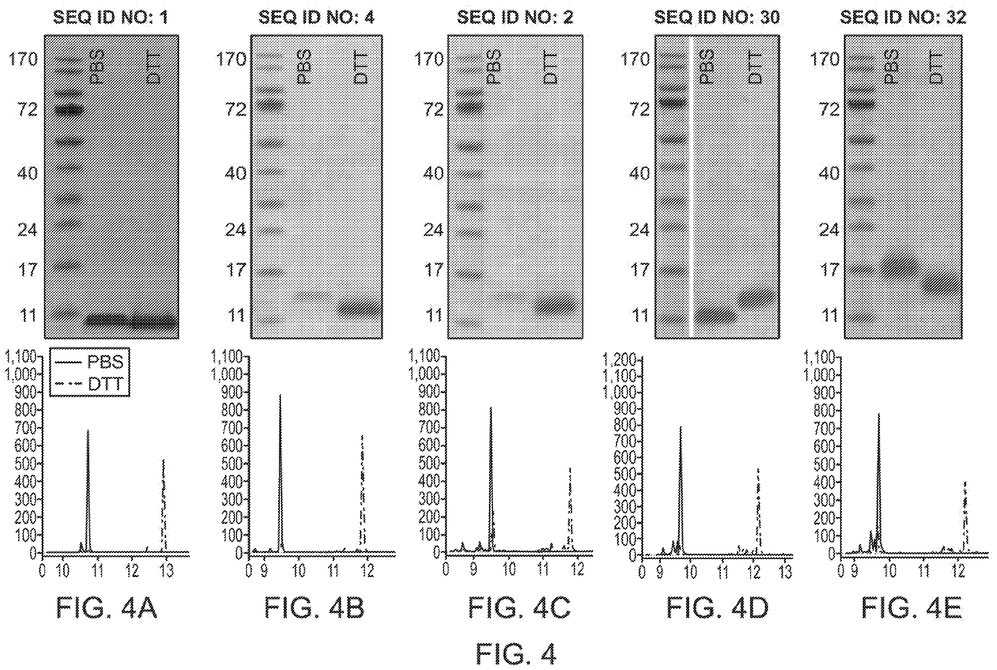

All proteins were analyzed by SDS-PAGE (NuPAGE 4-12% Bis-Tris, Thermo Fisher) and Coomassie staining (**FIG. 4** **and** **FIG. 27**). CDPs were run both non-reduced and reduced with 10 mM DTT. Proteolysis and reduction resistance testing was performed on the same RP-HPLC instrumentation as used for purification, after treatment with either 50 U of porcine pepsin (Sigma-Aldrich P7012) in simulated gastric fluid or 50 U of porcine trypsin (Sigma-Aldrich 6567) in PBS followed by incubation for 30 min at 37°C. Similarly, glutathione or DTT reduction involved treatment of the peptides with 10 mM glutathione or DTT in PBS for > 5 min prior to RP-HPLC analysis. CD spectra of a peptide of SEQ ID NO: 32 were measured with a Jasco J-720W spectropolarimeter (1 mm path length) in 20 mM phosphate buffer, pH 7.4, with or without 10 mM DTT at a concentration of 15-25 µM. Data are expressed in terms of relative ellipticity [θ] change at 210 nm, reported in mdeg.

Holo-transferrin was produced according to the method of Hamilton et al. In brief, a 10 mL solution of 4.5 mM Fe(NTA)₂, containing 4.5 mM FeCl₃ (Sigma Aldrich) and 9 mM nitrolotriacetic acid (NTA, Sigma Aldrich) in water, was titrated to pH 4.0 with HCl and NaOH. Apo-transferrin solution in PBS was loaded with Fe(NTA)₂, 0.4 equivalents at a time every 5 minutes, until 3.2 equivalents had been added. The solution was buffer exchanged into PBS using spin column dialysis (Amicon Ultra-4 centrifugal filter units, 10 kDa molecular weight cutoff) for >1,000-fold free Fe(NTA)₂ clearance.

Alexa Fluor 647 (Thermo Fisher A37573) CDP labeling took place in PBS buffer with a molar ratio of ~1.5 NHS-ester dye : 1 CDP for > 1 hour, after which free dye was removed by spin column dialysis (Amicon Ultra-4 centrifugal filter units, 3 kDa molecular weight cutoff) for >1,000-fold free dye clearance. Biotinylation of Avitagged TfR used the BirA-500 kit (Avidity) as per manufacturer's protocol, followed by a final buffer exchange into PBS containing 5% glycerol, and storage in small aliquots at -80°C.

### EXAMPLE 4

### Mammalian Surface Display of TfR-binding Peptides

This example describes mammalian surface display of TfR-binding peptides of the present disclosure including SEQ ID NO: 1 (SEQ ID NO: 1 is SEQ ID NO: 36 with an added N-terminal GS), SEQ ID NO: 2 (SEQ ID NO: 2 is SEQ ID NO: 37 with an added N-terminal GS), SEQ ID NO: 30 (SEQ ID NO: 30 is SEQ ID NO: 65 with an added N-terminal GS), and SEQ ID NO: 32 (SEQ ID NO: 32 is SEQ ID NO: 67 with an added N-terminal GS), and SEQ ID NO: 33 - SEQ ID NO: 35 (SEQ ID NO: 33 - SEQ ID NO: 35 are SEQ ID NO: 68 - SEQ ID NO: 70, respectively, with an added N-terminal GS). Screening for TfR-binding peptides was performed by transfecting or transducing mammalian cells to display candidate peptides (**FIG. 23B**) followed by screening against soluble human transferrin receptor ectodomain SEQ ID NO: 352 (200 nM, **FIG. 23C****,** **FIG. 1**). Mammalian cells had improved fidelity in folding disulfide crosslinked proteins, making them a suitable cell type for display of the peptides of the present disclosure.

Mammalian cell surface display screening was carried out as follows. The screening strategy used a surface display GFP FasL (SDGF) vector. In the vector, FasL-TM is the transmembrane domain of the FasL protein. More specifically, the designed peptides were cloned as a pool into SDGF, which were then made into lentivirus. 293F cells were transduced with this library at a multiplicity of infection of ~1, and after three days of growth, the pool of transduced cells are incubated with Alexa647-labeled TfR. For these experiments, fluorescent labeling was accomplished by co-staining TfR with fluorescent streptavidin or fluorescent anti-His antibodies. Soluble TfR contained both His tag and biotin label. Either Alexa Fluor 647 or iFluor 647 was used for the antibody/streptavidin fluorescence. A percentage of the highest-staining TfR-positive cells, from GFP and TfR double-positive cells, were sorted and expanded. At every expansion, a portion of the cells were collected, and at the end, the enriched peptides were identified by sequencing. Flow cytometry was used to assess gating criteria to identify GFP+ 293F cells expressing proteins on their surface via the SDGF peptide construct. Gating progresses using FSC-H vs SSC-H to gate out debris; FSC-H vs FSC-A to gate out doublets; FSC-H vs Pacific Blue H to gate out DAPI+ dead cells; and an optional FITC-H histogram to identify GFP+ cells. Once gated as such, Alexa Fluor 647 (a co-stain for detecting target binding) was used for sorting and analysis.

Screening was performed using a combination of magnetic sorting and flow sorting. Magnetic sorting was performed as follows: 2×10⁸ 293F cells were transduced with the SDGF CDP library at an MOI of ~1, and expanded until 3 days post-transduction. For initial screening, magnetic cell sorting was performed. 1×10⁹ transduced cells were resuspended in a binding buffer containing 200 nM biotinylated TfR, 2 mL anti-biotin MicroBeads (UltraPure, Miltenyi 130-105-637), and 21 mL Flow Buffer (PBS + 2 mM EDTA and 0.5% bovine serum albumin) in a final volume of 25 mL. Cells were incubated on ice with agitation (mild inversion every 2-5 min) for 30 mins, and were then diluted 10-fold to 250 mL with Flow Buffer, pelleted (500 x g, 5 mins), and resuspended to 40 mL with High BSA Flow Buffer (PBS containing 2 mM EDTA and 3% BSA). Cells were split into four 10 mL aliquots and run through a Miltenyi autoMACS^{®} Pro Separator using the "posseld" protocol and "quick rinses" after each sort. The running and wash buffers were High BSA Flow Buffer and PBS + 2 mM EDTA, respectively. Eluted cells were pooled, pelleted, and their CDP sequences PCR amplified (Terra^{™} PCR Direct Polymerase Mix (Takara 639271) for 16 cycles followed by Phusion). This sub-library was cloned into SDGF as above, made into lentivirus, and transduced into a new batch of 293F cells (1×10⁷ cells, MOI ~1) for flow sorting.

Flow sorting was performed as follows: Flow sorting took place using 2.4×10⁷ cells stained in 3 mL Flow Buffer with 200 nM TfR, 200 nM streptavidin Alexa Fluor 647 conjugate (ThermoFisher S21374), and 1 µg mL⁻¹ DAPI. Cells were diluted 4-fold to 12 mL with Flow Buffer, pelleted (500 x g, 5 mins), and resuspended in 3.6 mL Flow Buffer. Cells were sorted on a FACSAria II System (BD), gating based on FSC-A (medium), SSC-A (medium), DAPI-A (negative), GFP-A (positive), and APC-A (top 7% of GFP+) channels. After each flow sort, cells were cultured in FreeStyle Media starting at 0.5-1 mL in a suspension 24-well plate, shaking at 300 rpm, expanding to a final volume of 30 mL in a 125 mL baffled flask shaken at 125 RPM. At this point, cells were re-sorted as above. After the third flow sort, cells were expanded and frozen in 1.5×10⁶ cell pellets. Pellets were PCR amplified as above (Terra Direct PCR followed by InFusion), CDP inserts were subcloned into SDGF, transformed (Stellar competent cells), and colonies picked for miniprepping and sequencing of the cloned CDPs. Enriched variants were those that appeared in the sequence analysis more than once in ~100 picked colonies. All site-saturation mutagenesis (SSM) affinity maturation screening was done as above, with the following changes. 1) Staining was sequential; first with TfR, and then with an equimolar amount of dye-labeled streptavidin. 2) TfR / streptavidin concentrations were reduced to 20 nM for the first SSM maturation and to 8 nM for the second SSM maturation screen. After each SSM screen, enriched variants were studied to assemble a compound mutant (peptide of SEQ ID NO: 2 or SEQ ID NO: 32) that showed higher TfR staining than any of the variants containing either 1 or 2 of the individual mutations.

Flow cytometry plots in **FIG. 1** illustrate successive enrichment of cells that bind to TfR from pooled, high diversity library. **FIG. 1A** illustrates a Coomassie stained gel of transferrin receptor (TfR) protein. **FIG. 1B** illustrates a flow cytometry plot showing cells that bind to TfR after one flow sort. **FIG. 1C** illustrates a flow cytometry plot showing cells of a control stain after one flow sort. **FIG. 1D** illustrates a flow cytometry plot showing cells that bind to TfR after two flow sorts. **FIG. 1E** illustrates a flow cytometry plot showing cells of a control stain after two flow sorts. **FIG. 1F** illustrates a flow cytometry plot showing cells that bind to TfR after three flow sorts. **FIG. 1G** illustrates a flow cytometry plot showing cells of a control stain after three flow sorts.

Each flow sort represents growing the library of cells to >30 million cells, staining for TfR-binding, and flow sorting the top binders. Sorted binders were allowed to grow before the next flow sort was performed.

### EXAMPLE 5

### Identification of TfR-binding Peptides

This example describes identification of TfR-binding peptides using a mammalian surface display system, as described in **EXAMPLE 4.**

Using the mammalian surface display system of **EXAMPLE 4**, a single clonal peptide was identified having a sequence of SEQ ID NO: 1 (SEQ ID NO: 1 is SEQ ID NO: 36 with an added N-terminal GS). A library of oligonucleotides encoding 10,000 CDPs was amplified and mutagenized. The CDPs were 17-50 amino acids in length, with 4, 6, 8, or 10 cysteines. While there was some weighting of the library towards annotated knottins or defensins, the library contained CDPs from every domain / kingdom of life. This library was cloned into SDGF, made into lentivirus, and transduced into suspension 293F cells. The transduced cells were subjected to staining with TfR (200 nM) and co-stain over the course of one round of magnetic cell sorting and three rounds of flow sorting, each round enriching for cells stained with TfR. Binding was validated to specifically bind TfR in the surface display assay using 200 nM of soluble AF647-TfR, which was either biotinylated or attached to a His tag. Staining was carried out using a one-step staining protocol for tetravalent target avidity. A single TfR-binding CDP, designated SEQ ID NO: 1, was identified by DNA sequencing of the final enriched cell population. It represents a randomly mutated variant of cytochrome BC1 complex subunit 6 from the marine choanoflagellate *Monosiga brevicolis* (Uniprot ID: A9V0D7, DOI: 10.1093/nar/gku989), is 49 amino acids in length (six cysteines), and has a predicted molecular mass of 5.6 kDa. SEQ ID NO: 36 was then subjected to affinity maturation using site saturation mutagenesis (SSM), wherein a library is created containing every possible non-cysteine single amino acid substitution (43 non-Cys amino acids x 18 possible non-Cys substitutions = 775 variants, including SEQ ID NO: 1).

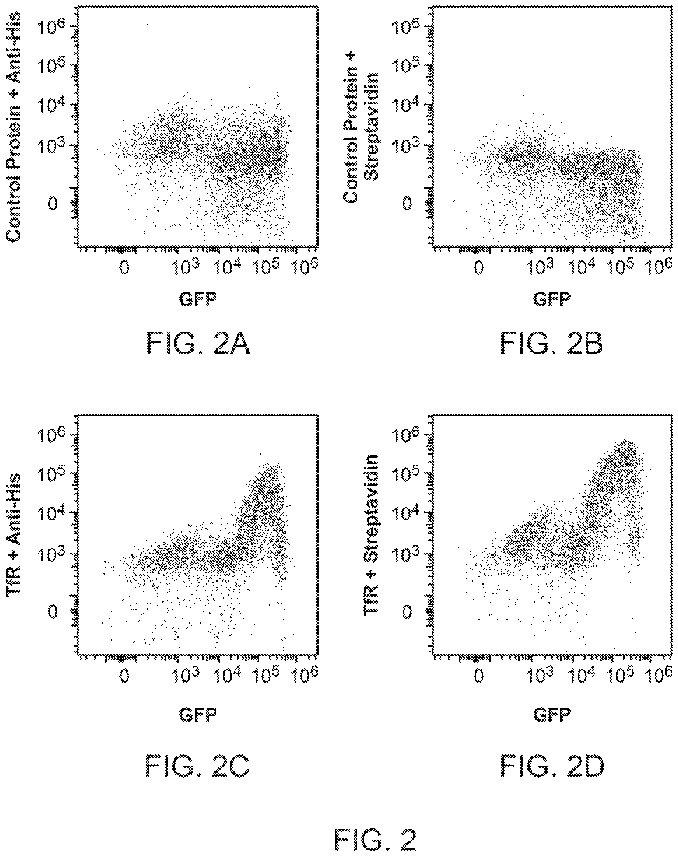

Flow cytometry plots in **FIG. 2** illustrate cells displaying TfR and the single clonal peptide. **FIG. 2A** illustrates a flow cytometry plot of cells expressing a peptide of SEQ ID NO: 1 (x-axis, GFP) and a control protein (y-axis, stained with a fluorescent anti-His antibody). **FIG. 2B** illustrates a flow cytometry plot of cells expressing a peptide of SEQ ID NO: 1 (x-axis, GFP) and a control protein (y-axis, stained with a fluorescent streptavidin). **FIG. 2C** illustrates a flow cytometry plot of cells expressing a peptide of SEQ ID NO: 1 (x-axis, GFP) and TfR (y-axis, stained with a fluorescent anti-His antibody). **FIG. 2D** illustrates a flow cytometry plot of cells expressing a peptide of SEQ ID NO: 1 (x-axis, GFP) and a TfR (y-axis, stained with a fluorescent streptavidin).

TfR staining was observed with cells expressing the identified clone, with no staining seen with a control protein. This staining was observed when either fluorescent streptavidin or anti-His antibody was the co-stain, demonstrating that the nature of the binding is dependent only on TfR, not the co-stain. Double-positive cells (upper right hand quadrant) indicate peptide-expressing cells that are bound to TfR.

An alternative method was also used to identify and optimize ("mature") TfR-binding peptides that are second and third generation binders using mammalian display screening. This library was screened with a modified staining protocol, using a lower concentration of target and co-stain (20 nM) and separate staining steps; the latter further increases stringency by eliminating the tetravalent avidity granted by streptavidin. Up to four rounds of flow sorting and enrichment were used to identify variants with improved TfR binding characteristics. Permutation of enriched variants identified an optimal mutant (SEQ ID NO: 2 (SEQ ID NO: 2 is SEQ ID NO: 37 with an added N-terminal GS)), and this process was repeated again (8 nM TfR and co-stain; otherwise, identical protocol) to generate SEQ ID NO: 32 (SEQ ID NO: 32 is SEQ ID NO: 67 with an added N-terminal GS). This twice-matured variant contains 14 point mutations from the original library member (GSREGCASHCTKYKAELEKCEARVSSRSNTEETCVQELFDFLHCVDHCVSQ, SEQ ID NO: 362). 4 point mutations from the parent sequence are found in SEQ ID NO: 1, while SEQ ID NO: 2 and SEQ ID NO: 32 contain 6 and 4 mutations, respectively, from the previous generation.

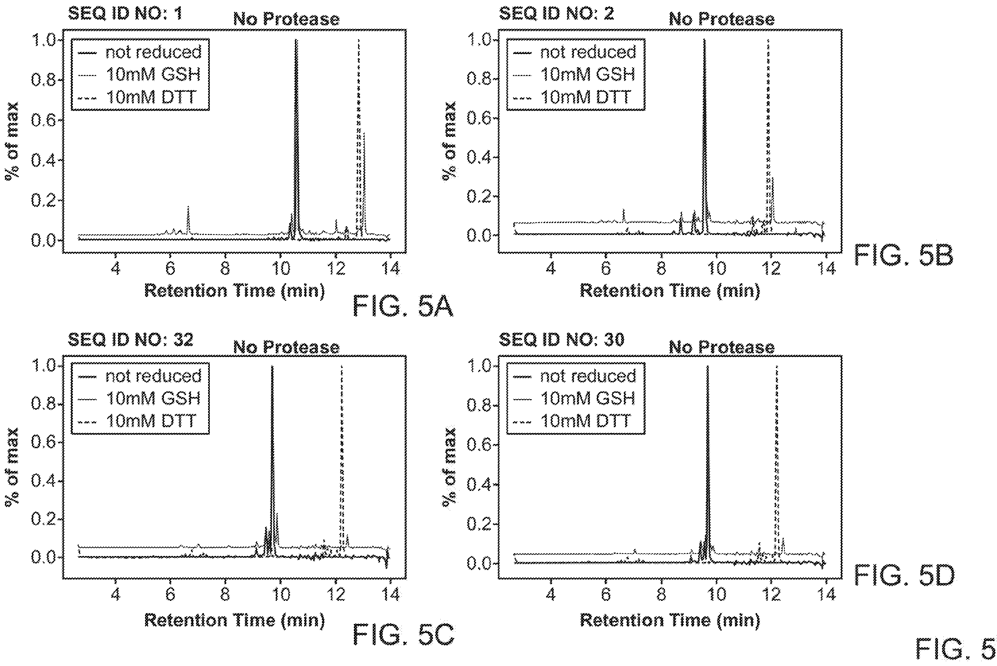

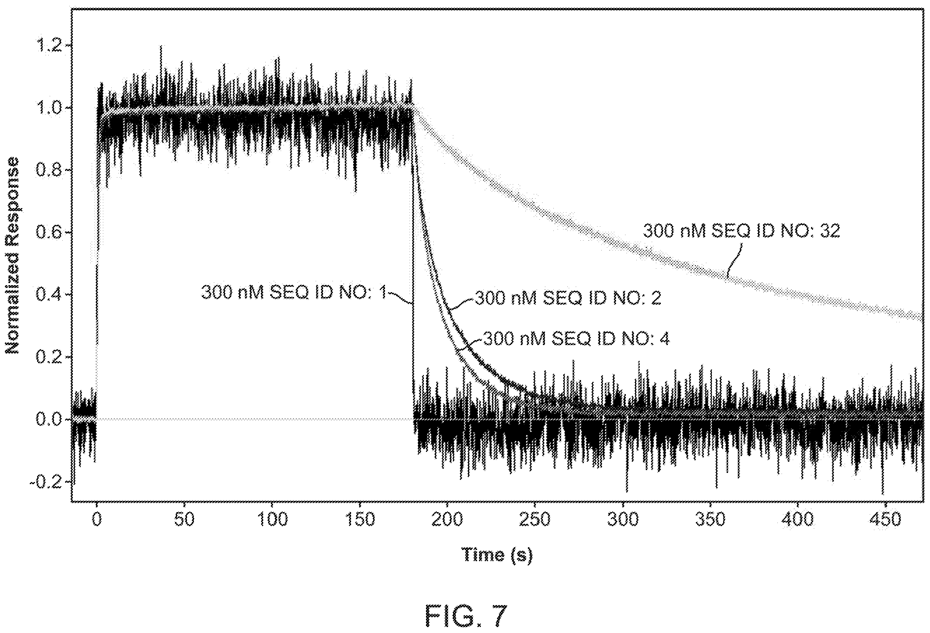

SEQ ID NO: 1 and its variants (e.g., SEQ ID NO: 2 and SEQ ID NO: 32) were produced as soluble peptides, and validated by reversed-phase HPLC (RP-HPLC), SDS-PAGE, and mass spectrometry (**FIG. 4A**, **FIG. 4C**, and **FIG. 4E**). Based on their masses of ~5-6 kDa, their slower-than-expected mobility in SDS-PAGE was a demonstration of the interesting electrophoretic mobility characteristics that some CDPs possess. All variants showed markedly different mobility upon DTT reduction (10 mM) in both SDS-PAGE and RP-HPLC, confirming disulfide bond stabilization. Their binding to TfR was verified by surface plasmon resonance (**FIG. 7**), also confirming increased affinity of matured variants (SEQ ID NO: 32 [*K_{D}* = 216 ±1 pM] > SEQ ID NO: 2 [*K_{D}* = 8.7 ± 0.4 nM] > SEQ ID NO: 1 [*K_{D}* not determined]). Peptides were further evaluated for stability under conditions of reduction, protease exposure, and heat (**FIG. 5**, **FIG. 6**, and **FIG. 26**). All variants demonstrated full or partial resistance to cellular reducing conditions (10 mM glutathione), while the affinity matured variants showed partial resistance to pepsin (though all were vulnerable to trypsin proteolysis). The intact, non-reduced peptide of SEQ ID NO: 32 protein demonstrates substantially improved heat tolerance to that of the DTT-reduced protein, with no substantial change in circular dichroism characteristics until well above common ambient temperatures (>50°C) and a failure to observe complete unfolding up to 95°C.

### EXAMPLE 6

### Site Saturation Mutagenesis of Peptides

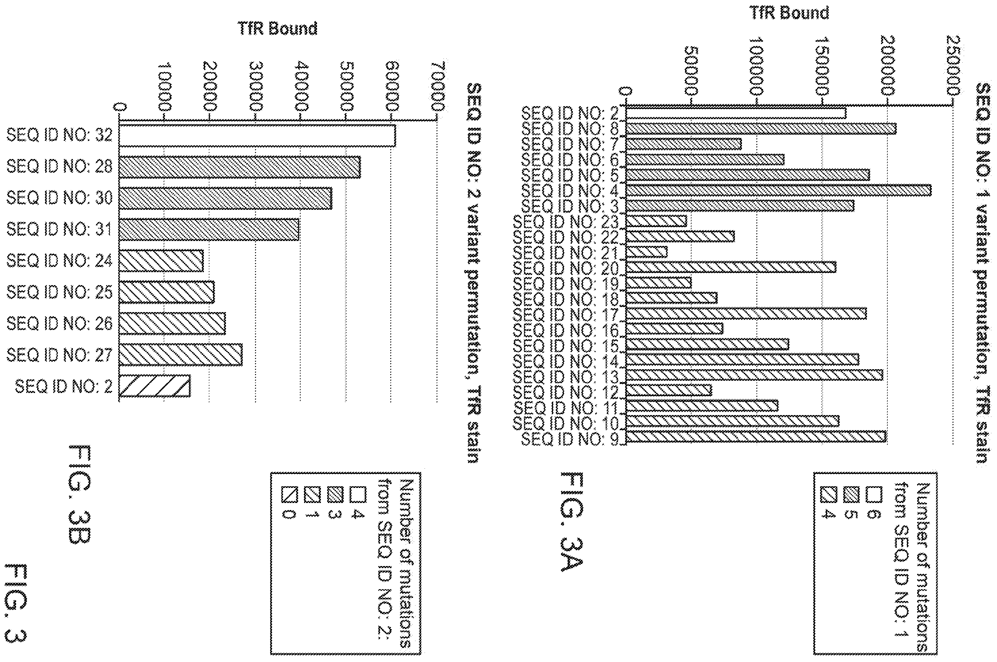

This example illustrates site saturation mutagenesis (SSM) of peptides of this disclosure to identify beneficial or deleterious mutations. Site saturation mutagenesis was performed on a peptide of SEQ ID NO: 1 (SEQ ID NO: 1 is SEQ ID NO: 36 with an added N-terminal GS, results in **FIG. 3A**) and a peptide of SEQ ID NO: 2 (SEQ ID NO: 2 is SEQ ID NO: 37 with an added N-terminal GS, results in **FIG. 3B**).

**FIG. 3** shows the TfR-binding capabilities of TfR-binding peptide variants identified during peptide maturation. SSM was employed for affinity maturation of the peptide having a sequence of SEQ ID NO: 1 as identified in the first mammalian surface display experiments (see e.g., **FIG. 1** and **FIG. 2**). During each round of maturation, a library of every possible non-cysteine point variants was constructed, and screened against TfR at higher stringency than first screen. Variants with improved binding were enriched and identified by Sanger sequencing. Such enriched variant mutations were combined with one another in various permutations (shown in the data) to identify a composite, improved binder. Two rounds of SSM were completed, yielding matured peptides comprising SEQ ID NO: 2, and SEQ ID NO: 32, respectively (SEQ ID NO: 32 is SEQ ID NO: 67 with an added N-terminal GS). TfR concentration in the first round of SSM was 20 nM and SSM was carried out using one-step staining. TfR concentration in the second round of SSM was 8 nM and SSM was carried out using two-step staining.

TfR-binding of mammalian cells expressing peptides of the SSM library was performed as described in **EXAMPLE 4** and **EXAMPLE 5,** but with a higher stringency protocol. The higher stringency protocol included a lower concentration of TfR (e.g., 20 nM).

**FIG. 3A** illustrates the results of a first site-saturation mutagenesis screen in SEQ ID NO: 1 (SEQ ID NO: 1 is SEQ ID NO: 36 with an added N-terminal GS), with some variants exhibiting improved binding activity to TfR such as peptides having a sequence of SEQ ID NO: 4, SEQ ID NO: 5, and SEQ ID NO: 8 (SEQ ID NO: 4, SEQ ID NO: 5, and SEQ ID NO: 8 are SEQ ID NO: 39, SEQ ID NO: 40, and SEQ ID NO: 43, respectively, with an added N-terminal GS). **FIG. 3B** show TfR-binding of variants identified during the second variant mutation.

The x-axis shows SEQ ID NOs of all variants and the y-axis shows the amount TfR bound in relative fluorescence units (RFUs) extrapolated from flow cytometry experiments.

### EXAMPLE 7

### TfR-binding of SSM-Generated TfR-binding Peptide Variants

This example demonstrates TfR-binding of site saturation mutagenesis (SSM)-generated TfR-binding peptide variants, as identified during SSM as described in **EXAMPLE 6**

In vivo BBB penetration experiments revealed that the TfR-binding capability of a peptide does not necessarily correspond with the capability of promoting vesicular transcytosis.

The six cysteines corresponding to residues C6, C10, C20, C34, C44, and C48, with reference to SEQ ID NO: 32 (C4, C8, C18, C32, C42, and C46, with reference to SEQ ID NO: 67), participate in disulfides, and thus contribute to peptide stability.

The surface interface residues corresponding to residues G5, A7, S8, N14, L17, E18, E21, L38, L42, L45, D46, H47, S50, Q51, with reference to SEQ ID NO: 32 (G3, A5, S6, N12, L15, E16, E19, L36, L40, L43, D44, H45, S48, Q49, with reference to SEQ ID NO: 67), that are present in all three generations of TfR-binding peptides likely contribute to TfR-binding. In some embodiments, the peptide or peptide construct of the present disclosure comprises at least one or more of these corresponding residues in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. Such peptides can accordingly be engineered with enhanced binding to TfR.

Hydrophilic surface-distal residues such as D, E, H, K, R, N, Q, S, or T likely contribute to peptide solubility corresponding to the following amino acid residues R3, E4, R9, K12, D14, E15, K19, R23, S26, S28, N29, T30, E31, E32, D33, E35, Q36, E37, E39, and D40, with reference to SEQ ID NO: 32 (R1, E3, R7, K10, D12, E13, K17, R21, S24, S26, N27, T28, E29, E30, D31, E33, Q34, E35, E37, and D38, with reference to SEQ ID NO: 67). In some embodiments, the peptide or peptide construct of the present disclosure comprises at least one or more of these corresponding residues in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. Such peptides can accordingly be engineered with enhanced solubility.

Higher binding affinity is associated with the presence of hydrophilic residues such as D, E, H, K, R, N, Q, S, or T as shown by improved binding from a mutation away from a nonpolar or hydrophobic residue such as A, M, I, L, V, F, W, or Y at the residues corresponding to D15, E35, E39, and H49, with reference to SEQ ID NO: 32 (D13, E33, E37, and H47, with reference to SEQ ID NO: 67). In some embodiments, the peptide or peptide construct of the present disclosure comprises at least one or more of these corresponding residues in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. Such peptides can accordingly be engineered with modified binding affinity.

Higher binding affinity to TfR is associated with nonpolar or hydrophobic residues such as A, M, I, L, V, F, W, or Y as shown by improved binding from a mutation away from a hydrophilic residue such as D, E, H, K, R, N, Q, S, or T at the amino acid residues corresponding to M11, M25, and M27, with reference to SEQ ID NO: 32 (M9, M23, and M25, with reference to SEQ ID NO: 67). In some embodiments, the peptide or peptide construct of the present disclosure comprises at least one or more of these corresponding residues in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. Such peptides can accordingly be engineered with modified binding affinity.

A higher TfR-binding affinity is associated with aliphatic residues such as A, M, I, L, or V as shown by improved binding from a mutation away from a large, aromatic residues such as F, W, or Y at the amino acid residue corresponding to L45 with reference to SEQ ID NO: 32 (L43 with reference to SEQ ID NO: 67). Substitutions of any one or more F, W, or Y in a peptide of the present disclosure to an aliphatic residue comprising A, M, I, L, or V can be sued to enhance the binding affinity of the peptide to TfR.

Any of peptides or peptide constructs of the present disclosure (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) can be modified at one or more of the corresponding residues described herein, to generate peptide variants with improved properties including enhanced stability and increased (or decreased) binding properties or modified TfR-binding affinity and increased (or decreased) transcytosis properties, including modified kₐ (association) and k_{d} (dissociation) rate constants.

Sequence alignments of certain TfR-binding peptides are shown in **TABLE 8.** Certain residues involved in the interaction with TfR are shown in bold. Surface interacting residues include but are not limited to those indicated.

**TABLE 8 - Corresponding Residues in TfR-Binding Peptides**

| **SEQ ID NO** | **Sequence** |
|---|---|
| SEQ ID NO: 1 | GSREGCA**S**RC**T**KY**N**AE**LE**KCEARVSSMSNTEETCVQE**L**FDLLHC**VD**HCVSQ |
| SEQ ID NO: 2 | GSREGCA**S**RC**T**KY**N**AE**LE**KCEARVMSMSNTEEDCEQE**L**EDLLHC**LD**HCHSQ |
| SEQ ID NO: 30 | GSREGCA**S**RC**M**KY**N**AE**LE**KCEARMMSMSNTEEDCEQE**L**EDLLYC**LD**HCHSQ |
| SEQ ID NO: 32 | GSREGCA**S**RC**M**KY**N**DE**LE**KCEARMMSMSNTEEDCEQE**L**EDLLYC**LD**HCHSQ |
| SEQ ID NO: 33 | GSREGCASRCTKYNAELEKCEARVSSMSNTEETCVQELFDLLHCVDHCVSQGSS ELYENKPRRPYIL |
| SEQ ID NO: 34 | GSREGCASRCTKYNAELEKCEARVMSMSNTEEDCEQELEDLLHCLDHCHSQGSS ELYENKPRRPYIL |
| SEQ ID NO: 35 | GSREGCASRCMKYNDELEKCEARMMSMSNTEEDCEQELEDLLYCLDHCHSQGSS ELYENKPRRPYIL |
| SEQ ID NO: 36 | REGCA**S**RC**T**KY**N**AE**LE**KCEARVSSMSNTEETCVQE**L**FDLLHC**VD**HCVSQ |
| SEQ ID NO: 37 | REGCA**S**RC**T**KY**N**AE**LE**KCEARVMSMSNTEEDCEQE**L**EDLLHC**LD**HCHSQ |
| SEQ ID NO: 65 | REGCA**S**RC**M**KY**N**AE**LE**KCEARMMSMSNTEEDCEQE**L**EDLLYC**LD**HCHSQ |
| SEQ ID NO: 67 | REGCA**S**RC**M**KY**N**DE**LE**KCEARMMSMSNTEEDCEQE**L**EDLLYC**LD**HCHSQ |
| SEQ ID NO: 68 | REGCASRCTKYNAELEKCEARVSSMSNTEETCVQELFDLLHCVDHCVSQG SSELYENKPRRPYIL |
| SEQ ID NO: 69 | REGCASRCTKYNAELEKCEARVMSMSNTEEDCEQELEDLLHCLDHCHSQG SSELYENKPRRPYIL |
| SEQ ID NO: 70 | REGCASRCMKYNDELEKCEARMMSMSNTEEDCEQELEDLLYCLDHCHSQG SSELYENKPRRPYIL |
| SEQ ID NO: 136 | GSREGCA**S**RC**T**RY**N**AE**LE**RCEARVSSMSNTEETCVQE**L**FDLLHC**VD**HCVSQ |
| SEQ ID NO: 137 | GSREGCA**S**RC**T**RY**N**AE**LE**RCEARVMSMSNTEEDCEQE**L**EDLLHC**LD**HCHSQ |
| SEQ ID NO: 165 | GSREGCA**S**RC**M**RY**N**AE**LE**RCEARMMSMSNTEEDCEQE**L**EDLLYC**LD**HCHSQ |
| SEQ ID NO: 167 | GSREGCA**S**RC**M**RY**N**DE**LE**RCEARMMSMSNTEEDCEQE**L**EDLLYC**LD**HCHSQ |
| SEQ ID NO: 168 | GSREGCASRCTRYNAELERCEARVSSMSNTEETCVQELFDLLHCVDHCVSQGSS ELYENRPRRPYIL |
| SEQ ID NO: 169 | GSREGCASRCTRYNAELERCEARVMSMSNTEEDCEQELEDLLHCLDHCHSQGSS ELYENRPRRPYIL |
| SEQ ID NO: 170 | GSREGCASRCMRYNDELERCEARMMSMSNTEEDCEQELEDLLYCLDHCHSQGSS ELYENRPRRPYIL |
| SEQ ID NO: 171 | REGCA**S**RC**T**RYNAE**LE**RCEARVSSMSNTEETCVQE**L**FDLLHC**VD**HCVSQ |
| SEQ ID NO: 172 | REGCA**S**RC**T**RYNAE**LE**RCEARVMSMSNTEEDCEQE**L**EDLLHC**LD**HCHSQ |
| SEQ ID NO: 200 | REGCA**S**RC**M**RYNAE**LE**RCEARMMSMSNTEEDCEQE**L**EDLLYC**LD**HCHSQ |
| SEQ ID NO: 202 | REGCA**S**RC**M**RYNDE**LE**RCEARMMSMSNTEEDCEQE**L**EDLLYC**LD**HCHSQ |
| SEQ ID NO: 203 | REGCASRCTRYNAELERCEARVSSMSNTEETCVQELFDLLHCVDHCVSQGSS ELYENRPRRPYIL |
| SEQ ID NO: 204 | REGCASRCTRYNAELERCEARVMSMSNTEEDCEQELEDLLHCLDHCHSQGSS ELYENRPRRPYIL |
| SEQ ID NO: 205 | REGCASRCMRYNDELERCEARMMSMSNTEEDCEQELEDLLYCLDHCHSQGSS ELYENRPRRPYIL |

### EXAMPLE 8

### Peptide Stability in Reducing Conditions

This example describes stability of designed or engineered peptides under reducing conditions. Peptides that are designed or engineered to interact with one or more target proteins in the cell, such as proteins in the nucleus or cytoplasm, are exposed to reducing conditions in the cytosolic compartment of cells. Thus, it is advantageous for peptides of this disclosure to display stability in reducing conditions. Stability of peptides of this disclosure was tested in 10 mM dithiothreitol (DTT) and 10 mM glutathione (GSH). DTT is a strong reducing agent that represents harsher conditions than what the peptides would be exposed to in biological environments (e.g., cells). GSH is a more physiologically relevant reducing agent for testing peptide stability in intracellular conditions. An example of a peptide-target protein interaction is a peptide binding to a TfR protein. This example describes stability of peptides under reducing conditions. SEQ ID NO: 1 and variants of SEQ ID NO: 1 were produced as soluble proteins and tested for their behavior in solution as well as their mobility upon disulfide reduction.

Stability of peptides was tested in 10 mM GSH (a biologically relevant reducing agent) and 10 mM DTT (stronger reducing agent than GSH). As shown in **FIG. 7****,** peptides having a sequence of SEQ ID NO: 1, SEQ ID NO: 4, SEQ ID NO: 2, SEQ ID NO: 30, and SEQ ID NO: 32 (SEQ ID NO: 1, SEQ ID NO: 4, SEQ ID NO: 2, SEQ ID NO: 30, and SEQ ID NO: 32 are SEQ ID NO: 36, SEQ ID NO: 39, SEQ ID NO: 37, SEQ ID NO: 65, and SEQ ID NO: 67, respectively, with an added N-terminal GS) were exposed to reducing conditions using DTT and compared to control experiments in which peptides were incubated in PBS. Peptide stability was measured by SDS-PAGE and HPLC. The data, presented in **FIG. 4A****-****FIG. 4E****,** show that peptides of SEQ ID NO: 2 and SEQ ID NO: 32 were most resistant to reduction with DTT, as evidenced by retention of the original peptide peak in HPLC chromatograms after exposure to DTT.

In a follow-up study, four peptides comprising SEQ ID NO: 1, SEQ ID NO: 2, SEQ ID NO: 30, and SEQ ID NO: 32, respectively, (SEQ ID NO: 1, SEQ ID NO: 2, SEQ ID NO: 30, and SEQ ID NO: 32 are SEQ ID NO: 36, SEQ ID NO: 37, SEQ ID NO: 65, and SEQ ID NO: 67, respectively, with an added N-terminal GS) were subjected to reducing conditions using in 10 mM DTT and 10 mM GSH, as shown in **FIG. 5****.** The overlaid HPLC chromatograms shown in **FIG. 5A****-****FIG. 5D** demonstrate that peptides having a sequence of SEQ ID NO: 30 and SEQ ID NO: 32 were almost fully resistant to GSH reduction, wherein the peptides having a sequence of SEQ ID NO: 1 and SEQ ID NO: 2 were only partially resistant to GSH reduction. GSH reduction is a more physiologically relevant reducing agent for testing stability in intracellular conditions, but all four peptides displayed reduction after exposure to DTT. These results demonstrated that SEQ ID NO: 1 and its variants exhibit disulfide driven structure and behavior.

### EXAMPLE 9

### Peptide Stability to Proteases

This example illustrates stability of peptides of this disclosure to proteases. Tumor environments, for instance, contain high concentrations of proteases. Furthermore, resistance to proteolysis (degradation or cleaving by proteases) reduces the likelihood of peptide degradation and subsequent immunogenicity, for example, in circulation. Resistance to proteolysis can further increase a peptide's serum half-life and the overall bioavailability of the peptide. Thus, it is advantageous for peptides of the present disclosure to be resistant to degradation by proteases.

To determine resistance to proteolytic digestion, peptides of SEQ ID NO: 1, SEQ ID NO: 2, SEQ ID NO: 30, and SEQ ID NO: 32 (SEQ ID NO: 1, SEQ ID NO: 2, SEQ ID NO: 30, and SEQ ID NO: 32 are SEQ ID NO: 36, SEQ ID NO: 37, SEQ ID NO: 65, and SEQ ID NO: 67, respectively, with an added N-terminal GS) were mixed with 50 U of porcine pepsin (Sigma-Aldrich P7012) in Simulated Gastric Fluid at pH 1.05, or 50 U of porcine trypsin (Sigma-Aldrich 6567) in PBS, incubated for 30 m at 37° C, and analyzed by reverse phase HPLC (RP-HPLC). Separately, the peptides were mixed with 50 U of porcine pepsin (Sigma-Aldrich P7012) in Simulated Gastric Fluid43 at pH 1.05, or 50 U of porcine trypsin (Sigma-Aldrich 6567) in PBS, incubated for 30 m at 37° C, and analyzed by RP-HPLC. Control peptides were incubated in PBS. All peptides were incubated in non-reducing conditions.

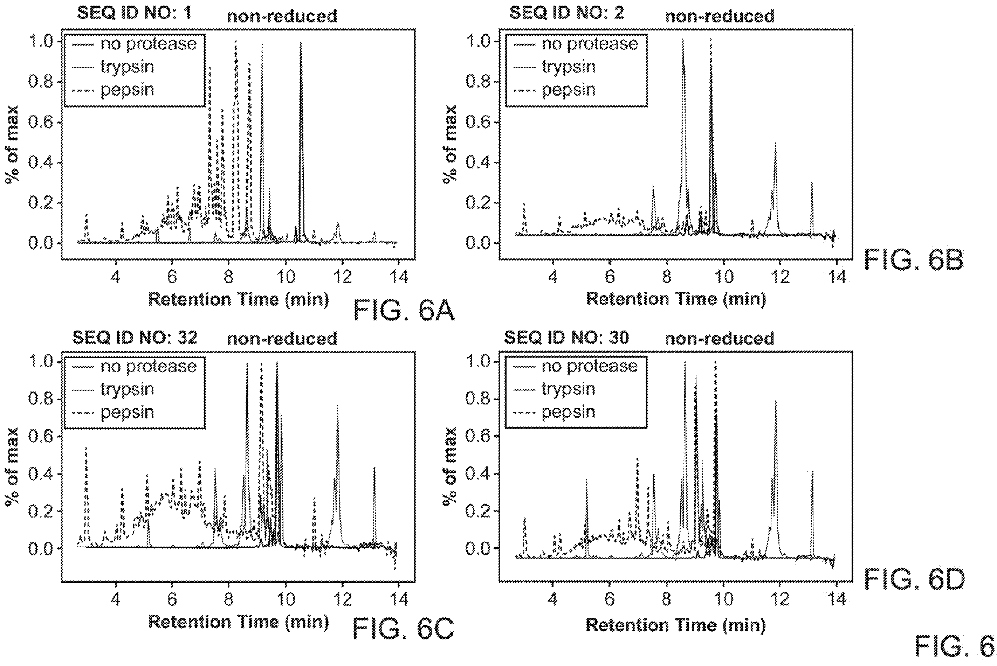

**FIG. 6** illustrates resistance of TfR-binding peptides to proteases, including pepsin and trypsin treatment. **FIG. 6** illustrates overlays (one per peptide tested) of reversed-phase HPLC traces of peptides subjected to either no treatment (red), trypsin (blue), or pepsin (green). **FIG. 6A** illustrates resistance to trypsin (blue) and pepsin (green) of the peptide having a sequence of SEQ ID NO: 1. **FIG. 6B** illustrates resistance to trypsin (blue) and pepsin (green) of the peptide having a sequence of SEQ ID NO: 2. **FIG. 6C** illustrates resistance to trypsin (blue) and pepsin (green) of the peptide having a sequence of SEQ IDNO: 32. **FIG. 6D** illustrates resistance to trypsin (blue) and pepsin (green) of the peptide having a sequence of SEQ ID NO: 30.

The peptides having a sequence of SEQ ID NO: 2, SEQ ID NO: 30, and SEQ ID NO: 32 were partly resistant to pepsin treatment, but not trypsin treatment under these conditions. The peptide having a sequence of SEQ ID NO: 1 was vulnerable to both proteases.

### EXAMPLE 10

### Surface Plasmon Resonance (SPR) Analysis of Peptide Binding Interactions

This example illustrates surface plasmon resonance (SPR) analysis of peptide binding interactions with TfR.

Various peptides of the present disclosure were analyzed for binding affinity to TfR. Briefly, binding affinity was analyzed by SPR experiments using captured biotinylated TfR, and which were performed at 25 °C on a Biacore T100 instrument (GE Healthcare) with Series S SA chips. HBS-EP+ (10 mM HEPES, pH 7.4, 150 mM NaCl, 3 mM EDTA, 0.05% surfactant P20) was used as a running buffer in the experiments with 0.1 mg/mL bovine serum albumin (BSA). Soluble TfR-binding peptides were evaluated for binding by incubation of a dilution series, in which the concentration range was varied depending on the TfR-binding peptide being tested with 2 ug/ml TfR, capturing ~300 resonance units (RUs) of protein for SPR experiments.

First, an allelic series of TfR-binding peptides with varying affinities was confirmed by SPR as shown in **FIG. 7****.** Peptides having a sequence of SEQ ID NO: 1, SEQ ID NO: 2, SEQ ID NO: 4, and SEQ ID NO: 32 (SEQ ID NO: 1, SEQ ID NO: 2, SEQ ID NO: 4, and SEQ ID NO: 32 are SEQ ID NO: 36, SEQ ID NO: 37, SEQ ID NO: 39, and SEQ ID NO: 67, respectively, with an added N-terminal GS) were tested at a concentration of 300 nM. The results confirmed that peptide variants from later rounds of affinity maturation exhibited different binding affinities for TfR. That is, the peptide from the latest round of affinity maturation having a sequence of SEQ ID NO: 32 showed the highest binding affinity for TfR, whereas SEQ ID NO: 1 showed the lowest binding affinity for human TfR (hTfR).

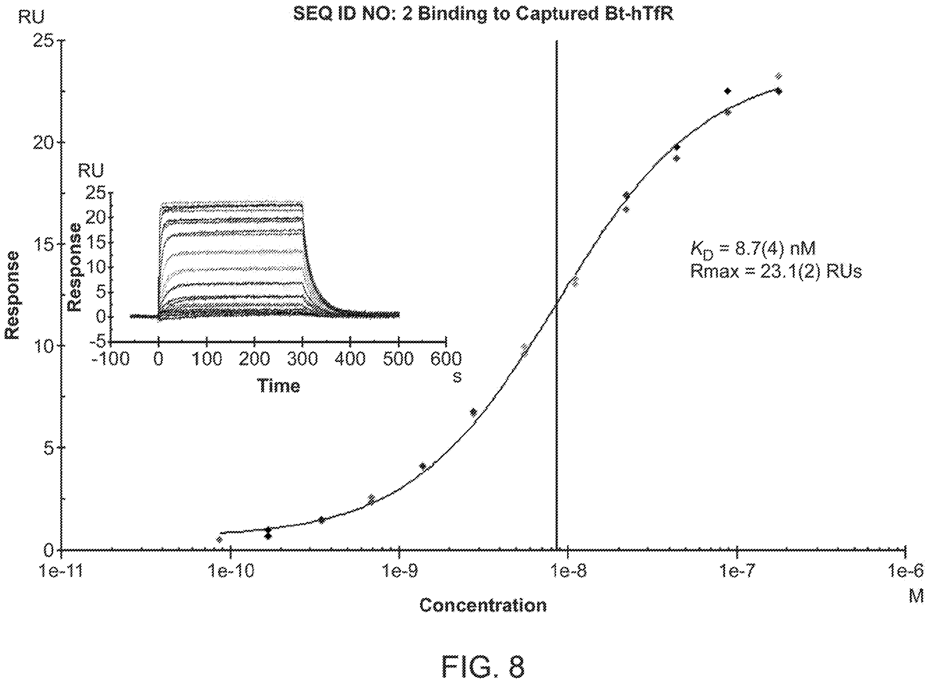

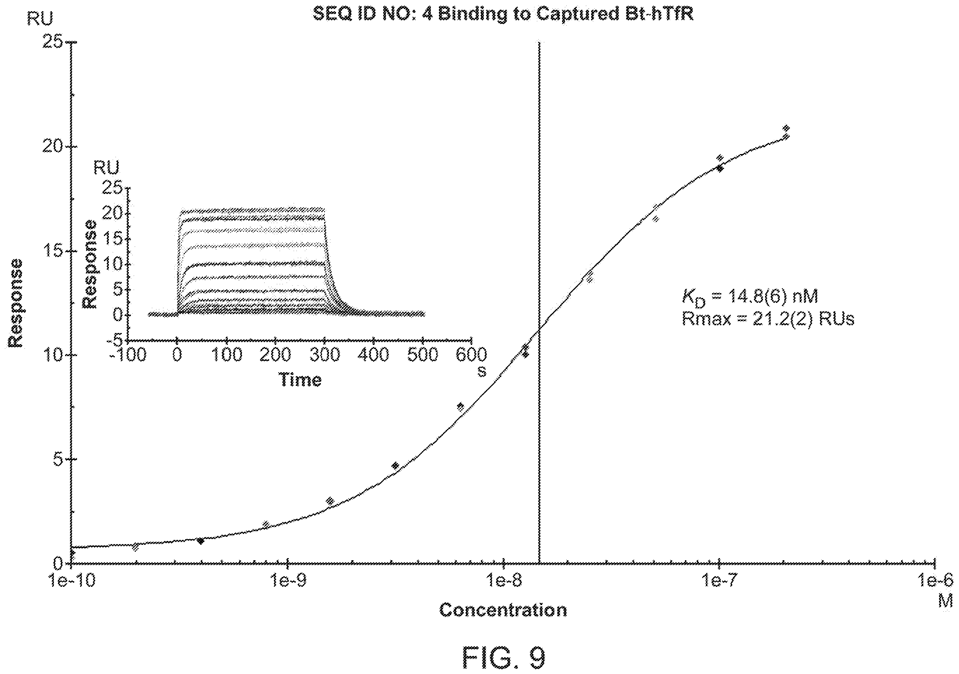

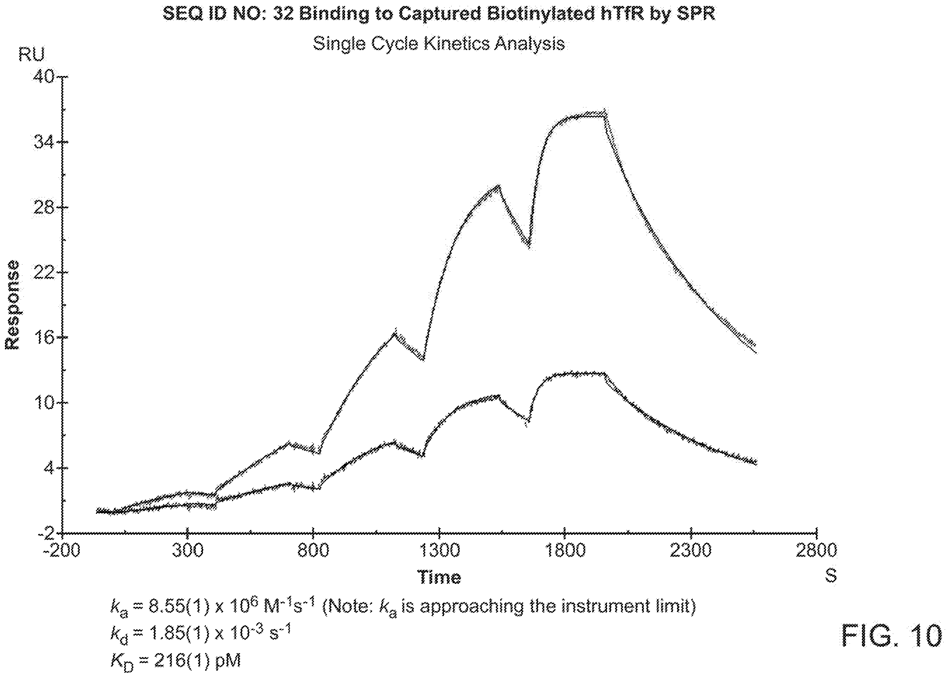

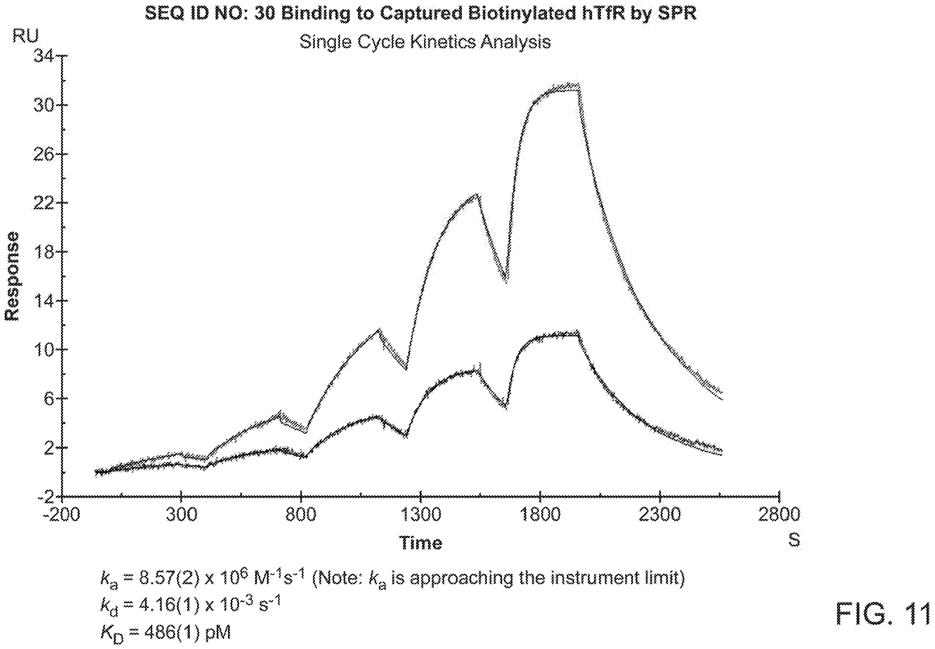

Next, the binding of four peptides having a sequence of SEQ ID NO: 2, SEQ ID NO: 4, SEQ ID NO: 30, and SEQ ID NO: 32, respectively, (SEQ ID NO: 2, SEQ ID NO: 4, SEQ ID NO: 30, and SEQ ID NO: 32 are SEQ ID NO: 37, SEQ ID NO: 39, SEQ ID NO: 65, and SEQ ID NO: 67, repsectively, with an added N-terminal GS) to captured and biotinylated hTfR was measured. **FIG. 8** illustrates a surface plasmon resonance (SPR) trace showing TfR-binding for varying concentrations of the peptide from 100 pM to 200 nM having a sequence of SEQ ID NO: 2. **FIG. 9** illustrates a surface plasmon resonance (SPR) trace showing TfR-binding for varying concentrations of the peptide from 100 pM to 200 nM having a sequence of SEQ ID NO: 4. **FIG. 10** illustrates binding and single cycle kinetics data of SEQ ID NO: 32 binding to captured biotinylated (Bt) hTfR by SPR. 5 concentrations of a peptide having a sequence of SEQ ID NO: 32 (0.037 nM, 0.11 nM, 0.33 nM, 1 nM, 3 nM) were injected over 2 densities of captured Bt-hTfR and analyzed globally. **FIG. 11** illustrates binding and single cycle kinetics data of SEQ ID NO: 30 binding to captured biotinylated hTfR by SPR. 5 concentrations of a peptide having a sequence of SEQ ID NO: 30 (0.037 nM, 0.11 nM, 0.33 nM, 1 nM, 3 nM) were injected over 2 densities of captured Bt-hTfR and analyzed globally.

The binding SEQ ID NO: 2 and SEQ ID NO: 4 was measured at serial dilutions between 100 pM and 200 nM, while SEQ ID NO: 30 and SEQ ID NO: 32 were tested at serial dilutions of 37 pM to 3 nM, to yield kinetic data. For the peptides of SEQ ID NO: 30 and SEQ ID NO: 32, the kinetic testing was performed by injecting the peptides over two densities (shown in **FIG. 10** and **FIG. 11** as red and green traces) of captured and biotinylated hTfR and data was analyzed globally.

**TABLE 9** below summarizes the data obtained from the analysis of the graphs shown in **FIG. 8** - **FIG. 11****.** For the peptide having a sequence of SEQ ID NO: 2 a *K*_{D} of 8.7 ± 4 nM and an Rₘₐₓ of 23.1 ± 2 RUs was determined. For the peptide having a sequence of SEQ ID NO: 4 a *K*_{D} of 14.8 ± 6 nM and an Rₘₐₓ of 21.2 ± 2 RUs was determined. For the peptide having a sequence of SEQ ID NO: 32 a *K*_{D} of 216 ± 1 pM was determined. For the peptide having a sequence of SEQ ID NO: 30 a *K*_{D} of 468 ± 1 pM was determined. Lower *K*_{D} values indicate a higher binding affinity. Rmax represents the maximum binding capacity of the peptide to hTfR. As shown in the table below, SEQ ID NO: 32 had the lowest *K*_{D}, indicating that it displayed the strongest binding to hTfR. An increased TfR-binding affinity can correspond to an improved transcytosis function. In some cases, an increased TfR-binding affinity can correspond to a reduced transcytosis function, wherein in some cases, an increased TfR-binding affinity does not correspond to a change in transcytosis function compared to the reference peptide. Without being bound to any theory, it is assumed that the ratio of Kₐ/K_{d} can affect the transcytosis function of a peptide, and thus modulation of Kₐ and/or K_{d} can be used to generate TfR-binding peptides with optimal TfR binding affinity and transcytosis function.

**TABLE 9 - SPR Analysis Results**

| **SEQ ID NO** | ***k*ₐ (M⁻¹ s⁻¹)** | ***k_{d}* (s⁻¹)** | ***K*_{D}** | **Analysis Method** |
|---|---|---|---|---|
| **SEQ ID NO: 1** | | | + | |
| **SEQ ID NO: 2** | -- | -- | 8.7(4) nM | Steady-state |
| **SEQ ID NO: 4** | -- | -- | 14.8(6) nM | Steady-state |
| **SEQ ID NO: 32** | 8.55(1) x 10⁶ | 1.85(1) x 10⁻³ | 216(1) pM | Single Cycle Kinetics |
| **SEQ ID NO: 30** | 8.57(2) x 10⁶ | 4.16(1) x 10⁻³ | 486(1) pM | Single Cycle Kinetics |

| | | | | |
|---|---|---|---|---|
| * reported *k*ₐ is approaching the limits that can be measured by the instrument | | | | |

### EXAMPLE 11

### Competitive Binding of Peptides to TfR in the presence of endogenous TfR binders

This example describes competitive binding of peptides of this disclosure to TfR in the presence of endogenous TfR binders Apo-transferrin (iron-free) and Holo-transferrin (iron-loaded).

HEK-293 suspension cells expressing SDGF-peptides having a sequence of SEQ ID NO: 2 and SEQ ID NO: 32 (SEQ ID NO: 2 and SEQ ID NO: 32 are SEQ ID NO: 37 and SEQ ID NO: 67, respectively, with an added N-terminal GS) were incubated with either TfR + PBS, TfR + Apo-transferrin (non-iron loaded) at 10 µM, or TfR + Holo-transferrin (iron-loaded) at 10 µM. Transfected cells were incubated with Holo-transferrin or Apo-transferrin followed by addition of a TfR solution containing 8 nM biotinylated TfR. Cells were pelleted and resuspended in an AF647-streptavidin solution. Cells were pelleted and analyzed by flow cytometry for streptavidin staining, which indicated TfR-binding.

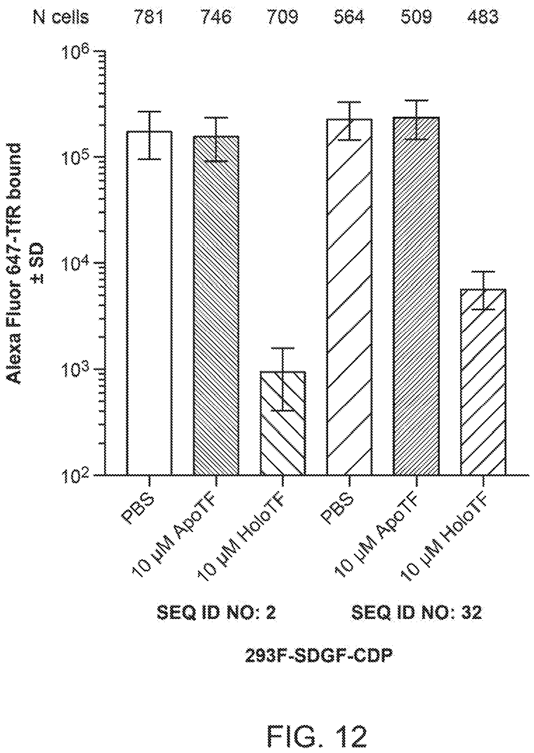

The data shown in **FIG. 12** demonstrate that the peptides having a sequence of SEQ ID NO: 2 and SEQ ID NO: 32 successfully competed with Holo-transferrin for TfR-binding (the y-axis shows relative fluorescence units derived from flow cytometry experiments). In the presence of Holo-transferrin, the third generation, SSM-generated peptide having a sequence of SEQ ID NO: 32 exhibited higher competitive binding for TfR than the first generation peptide having a sequence of SEQ ID NO: 2.

This data indicates that even in the presence of holotransferrin or apotransferrin, such as in the bloodstream, the peptides of this disclosure can still bind to TfR. This additionally confirmed that affinity maturation resulted in peptides having high binding affinity to TfR and which displayed competitive binding to TfR in comparison to endogenous binders.

### EXAMPLE 12

### Solved Crystal Structure of a Peptide of SEQ ID NO: 32

This example illustrates the solved crystal structure of peptides of SEQ ID NO: 32 and TfR. The TfR-SEQ ID NO: 32 complex was first purified by size exclusion chromatography prior to addition to crystal trays.

Briefly, lyophilized SEQ ID NO: 32 was reconstituted in 25 mM PIPES (pH = 7.0), 150 mM NaCl, and 1 mM EDTA at a concentration of at 11 mg/mL. Preliminary crystallization conditions were determined using the JCSG-plus sparse matrix screen (Molecular Dimensions) in a vapor equilibration format. Diffraction-quality crystals were obtained by vapor equilibration of drops comprising 1 µL protein solution plus 1 µL well solution over wells of 1 mL 9% w/w PEG (MW = 3350), 30 mM (NH₄)₂SO₄, 100 mM Bis-Tris (pH = 5.0) at ambient temperature. Crystals were cryopreserved for data collection by transferring into well solution plus 20% v/v glycerol. Diffraction data (dmin = 2.55 Å) were collected on an in-house Rigaku MicroMax-007HF rotating anode generator with Osmic Varimax optics and a Rigaku Saturn 944+ CCD detector.

To isolate stoichiometric complexes, purified SEQ ID NO: 32 and TfR were mixed at a 1.2:1 molar ratio, incubated, and fractionated by size exclusion chromatography in 25 mM PIPES, 150 mM NaCl, and 1 mM EDTA (pH = 7.0) on a Superose 6 10/300 column (GE Healthcare Life Sciences). The hTfR-peptide complex was resuspended at a target concentration of 10 mg/mL. Crystallization screening was performed at room temperature by vapor diffusion, with 1:1 protein solution:reservoir solution sitting drops, set up using the Nextal JCSG+, PEGs, and (NH₄)₂SO₄ factorial suites (Qiagen) and sub-microliter robotics (TTP Labtech mosquito). Diffraction-quality crystals were obtained by vapor equilibration of drops comprising 1 µL protein solution plus 1 µL well solution over wells of 1 mL 5% w/w PEG (Mr = 6000), 100 mM MES (pH = 6.5) at ambient temperature. Crystals were cryopreserved for data collection by transferring into well solution plus 15% v/v glycerol. Diffraction data (dmin = 1.85 Å) were collected from single crystals using a Rigaku MicroMax-007 HF home source or beamline 5.0.1 at the Advanced Light Source (Lawrence Berkley National Laboratory, Berkeley, CA) at a wavelength of 1.0000 Å.

Initial phases were determined either by molecular replacement (MR), using PHASER (McCoy, A.J., J Appl Crystallogr., 40(Pt 4): 658-674 (2007)) in the CCP4 program suite (Winn, M.D., Acta Crystallogr D Biol Crystallogr., 67(Pt 4): 235-42 (2011)) using homologous structures from the RCSB PDB (Berman, H.M., Nucleic Acids Res., 28(1): 235-42 (2000)) as search models, or sulfur single-wavelength anomalous diffraction (sSAD) (Liu, Q., Acta Crystallogr D Biol Crystallogr., 69(Pt 7): 1314-32 (2013)), using CuKalpha radiation to maximize the anomalous signal, and determining sulfur substructures with SHELX (Sheldrick, G.M., Acta Crystallogr D Biol Crystallogr., 66 (Pt 4): 479-85 (2010)). For sSAD phasing, Bijvoet pair measurement was optimized by collecting data through 5° wedges with alternating phi rotations of 180°, in 1° oscillations. Data were reduced and scaled with HKL2000 (Otwinowski, Z., Methods Enzymol., 276: 307-326 (1997)). Iterative cycles of model building and refinement were performed with COOT (Emsely, P., Acta Crystallogr D Biol Crystallogr., 60(Pt 12 Pt 1): 2126-32 (2004)) and REFMAC (Murshudov, G.N., Acta Crystallogr D Biol Crystallogr., 53(Pt 3): 240-55 (1997)). Structure validation was performed with MolProbity (Davis, I.W., Nucleic Acids Res., 35(Web Server issue): W375-83 (2007)). Twin refinement in REFMAC5 was needed to produce interpretable electron density maps for apo-SEQ ID NO: 32.

The crystal structure of SEQ ID NO: 32 was solved, both alone and bound to TfR (**FIG. 13** and **FIG. 14**). As shown by structural modeling, the CDP is an anti-parallel two-helix bundle. It contains three disulfide bonds with a simple hairpin topology (paired C4:C46, C8:C42, and C18:C32) (**FIG. 13C**). Upon complexing with TfR, the N- and C-terminal residues become ordered, making contacts to TfR. However, the helical core of the CDP (between and including the C8:C42 disulfide) is nearly identical between bound and unbound forms, with backbones aligning to an RMSD of 0.331 ±0.049 Å (N = 8 alignments). In both crystals, the loop connecting the helices is disordered. The cartoon representations of free and TfR-bound SEQ ID NO: 32 in **FIG. 13** are superpositions of all molecules of the asymmetric unit: four for free SEQ ID NO: 32, two for TfR-bound. Stable peptides that are resistant to protease degradation reduce any risk of immunogenicity upon dosing humans or other animals with peptides. Precise crystallographic knowledge of the TfR-binding pharmacophore of the peptides of this disclosure can allow sequence optimization to further limit any binding of peptide fragments to the major histocompatibility complex (MHC), and thus reduce any immunogenicity risk caused by repeat dosing of peptides. Immune recognition of foreign proteins requires fragmentation of the protein in antigen-presenting cells and binding of those fragments to MHC (HLA in humans). Some motifs are favored for fragment:MHC binding, while others are unfavorable. Knowing about the structure of the peptide, both bound and unbound, oen can design variants with reduced immunogenicity by making mutations that disrupt the binding of fragments to MHC while leaving crucial binding residues unaffected.

Binding to TfR is mediated by both polar and non-polar interactions; the latter are primarily Met and Leu residues fitting into small hydrophobic pockets at the interface (**FIG. 13D**). The distortion of the N-terminus results from forming three hydrogen bonds to TfR, while adjustment of the C-terminal helix between bound and unbound states accommodates sterics around H47 of SEQ ID NO: 32. The N-terminal GS stub, a remnant after TEV cleavage from the Siderocalin fusion partner, is also disordered. Overall, the affinity (*K*_{D} = 216 ± 1 pM) is quite strong considering the small buried surface area (1001 Å²); compared to the published affinities and buried surface areas of 113 heterodimeric complexes, only two of the 86 complexes with smaller buried surface areas demonstrated stronger affinity (coincidentally, one of them is a protease-inhibiting CDP and its target).

By comparison with the TfR:transferrin co-crystal structure, the SEQ ID NO: 32 footprint overlaps with that of transferrin, predicting competitive binding (**FIG. 13D**). This was confirmed in surface display flow cytometry binding assays in the presence of soluble apo- or holo-transferrin (**FIG. 12**). While this could reduce BBB penetration, owing to the high plasma levels of holo-transferrin (reportedly 20 µM or more), the highest affinity TfR-binding variant (SEQ ID NO: 32) has a similar off-rate (*k*_{d}) but a much faster on-rate (*k*ₐ) than that published for the TfR:transferrin interaction. It could be that, as soon as a vesicle bearing TfR to the apical cell surface fuses and exposes receptor to the bloodstream, SEQ ID NO: 32 is simply able to bind with the newly plasma-exposed TfR more rapidly than does transferrin. The half-life of SEQ ID NO: 32 release (about 10 minutes) is much longer than the reported endocytosis rate of transferrin (t½ = 3.5 minutes), and because TfR uptake is not ligand-dependent, SEQ ID NO: 32 might simply out-compete transferrin for its binding site and occupy it long enough for uptake. Most of the residues comprising the SEQ ID NO: 32 binding site on TfR are conserved between human and murine orthologs (**FIG. 13E**). This suggested cross-reactivity, which was confirmed in flow cytometry binding assays (**FIG. 13E** and **FIG. 15**) using surface displayed human or murine TfR (via SDGF) and dye-conjugated, soluble TfR-binding variants. This alternate strategy (soluble CDP, surface-bound target) was employed because soluble murine TfR ectodomain could not be produced, but was well behaved as a surface displayed protein.

**FIG. 13** illustrates co-crystallization of hTfR and a peptide having a sequence of SEQ ID NO: 32. **FIG. 13A** illustrates a first view of hTfR co-crystallized with a peptide having a sequence of SEQ ID NO: 32, showing the peptide molecule is on the opposite face of the hTtR, and confirming 2:2 binding stoichiometry between the bound peptide and hTfR. The linking sequence between the two helices, depicted as a solid, black coiled line, was not resolved in crystal structure and is based on structural modeling. **FIG. 13B** illustrates a zoomed in view of **FIG. 13A** and shows hTfR co-crystallized with a peptide having a sequence of SEQ ID NO: 32, showing numerous polar and non-polar interactions driving binding. Non-polar interactions are primarily Leu and Met, while polar interactions are a mix of several charged and non-charged residues. Amino acid residues involved in such interaction or binding to TfR correspond to residues G5, C6, A7, S8, C10, M11, N14, L17, E18, E21, L38, L41, L42, L45, D46, H47, H49, S50, Q51, with reference to SEQ ID NO: 32 (G3, C4, A5, S6, C8, M9, N12, L15, E16, E19, L36, L39, L40, L43, D44, H45, H47, S48, Q49, with reference to SEQ ID NO: 67). In some embodiments, the peptide or peptide construct of the present disclosure comprises at least one or more of these corresponding residues in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. Amino acid side chains either mutated from endogenous CDP or those that are part of the interface are shown as sticks. Interface-adjacent residues are shown in black, whereas interface-distal residues are shown in cyan. **FIG. 14** illustrates superimposition of the peptide having a sequence of SEQ ID NO: 32 and transferrin from published literature (RCSB PDB 1SUV) both bound to hTfR. Similarly, **FIG. 13E** illustrates the superimposition of the same peptide with a different published transferrin receptor (RCSB PDB 3S9L). The superimposition illustrates that transferrin and the peptide having a sequence of SEQ ID NO: 32 overlap in their binding sites on TfR (**FIG. 13E****,** left). The SEQ ID NO: 32 binding site, for both human (SEQ ID NO: 353) and murine (SEQ ID NO: 354) homology, shows that most of the binding surface on TfR shares identity or similarity between the two species (**FIG. 13E**, right). **FIG. 14A** illustrates a superimposition of transferrin (data taken from from published literature (RCSB PDB 1SUV)) and the peptide having a sequence of SEQ ID NO: 32 bound to TfR. **FIG. 14B** illustrates a zoomed in view of the peptide having a sequence of SEQ ID NO: 32 bound to TfR, indicating that, when compared to the binding site of endogenous transferrin in **FIG. 14A**, both the peptide having a sequence of SEQ ID NO: 32 and transferrin have overlapping binding sites on hTfR. Amino acid side chains either mutated from endogenous CDP or that are part of the interface are shown as sticks. Interface-adjacent residues are shown in black, whereas interface-distal residues are shown in cyan.

The solved crystal structures of complexes formed by hTfR and the TfR-binding peptide having a sequence of SEQ ID NO: 32 confirm that the binding site of SEQ ID NO: 32 to TfR overlaps with the binding site of transferrin to TfR.

Any of peptide or peptide construct of the present disclosure (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) can be modified at one or more of the corresponding residues identified in the crystal structure above, to generate peptide variants with improved properties including enhanced stability and increased (or decreased) binding properties or modified TfR-binding affinity, and/or increased (or decreased) transcytosis function.

### EXAMPLE 13

### Cross Reactivity of TfR-binding Peptides to Murine TfR

This example illustrates cross reactivity of TfR-binding peptides of the present disclosure to murine TfR in cell surface binding assays. A reversal of the surface display paradigm described in **EXAMPLE 3** was used. Briefly, 293F cells expressing either human or mouse TfR on their surface were stained with soluble TfR-binding peptides that were directly labeled with AlexaFluor 647 dye via NHS-ester conjugation. **FIG. 15A** and **FIG. 15B** show flow cytometry plots that verify human versus mouse TfR expression using species-specific antibodies. **FIG. 15C** and **FIG. 15D** demonstrate that the peptides effectively bind to both homologs. In a similar experiment, flow cytometry was used to demonstrate effective binding of SEQ ID NO: 1, SEQ ID NO: 2, SEQ ID NO: 32, and Anti-Tf antibodies (positive controls). SDGF surface expression of the human and murine TfR ectodomains (HsTfR and MmTfr, respectively) was confirmed by species-specific antibodies. Staining of these cells with Alexa Fluor 647-labeled CDP variants (e.g., SEQ ID NO: 1, SEQ ID NO:2, or SEQ ID NO: 3) demonstrated cross-reactivity and improved staining of higher affinity variants (**FIG. 13E**).

Any of peptide or peptide construct of the present disclosure (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) can be modified at one or more of the corresponding residues identified in the crystal structure above, to generate peptide variants with improved properties including enhanced stability and increased (or decreased) binding properties or modified TfR-binding affinity, and/or increased (or decreased) transcytosis function. Without being bound by any theory, the herein described data indicate that all amino acid residues of a TfR-binding peptide may be important for cross-reactivity.

Surface display flow cytometry was also used to verify the binding interface of SEQ ID NO: 32 on the transferrin receptor, as identified in the crystal structures from **EXAMPLE 12** (**FIG. 13** - **FIG. 14**). The TfR:SEQ ID NO: 32 interface overlaps with that of transferrin, predicting competitive binding (**FIG. 14A** and **FIG. 13E**). The highest affinity variant of the variant family of SEQ ID NO: 1 identified (the variant having a sequence of SEQ ID NO: 32) has a similar off-rate (k_{d}) but a much faster on-rate (kₐ) than that published for the TfR:transferrin interaction. The murine / human homology of the SEQ ID NO: 32 binding surface on TfR was also studied (**FIG. 14A** and **FIG. 13E**), which possesses few dissimilar residues compared to murine TfR. This suggested cross-reactivity, which was confirmed in flow cytometry binding assays (**FIG. 15**).

### EXAMPLE 14

### Whole Body Autoradiography of TfR-binding Peptides

This example illustrates the determination of biodistribution for the radiolabeled peptides having a sequence of SEQ ID NO: 1 and SEQ ID NO: 32 (SEQ ID NO: 1 and SEQ ID NO: 32 are SEQ ID NO: 36 and SEQ ID NO: 67, respectively, with an added N-terminal GS) acquired via autoradiography *in vivo.*

Each peptide was radiolabeled by methylating lysines and the N-terminus as described in **EXAMPLE 19.** As such, the peptide may contain methyl or dimethyl lysines and a methylated or dimethlyated amino terminus. A dose of 100 nmol radiolabeled peptide was administered via tail vein injection in Female Harlan athymic nude mice, weighing 20-25 g. ¹⁴C labeled CDPs were resuspended to 100 nmol in 100 µL PBS for intravenous injection into mice.

Each radiolabeled peptide was allowed to freely circulate within the animal for the described time period (30 minutes or 3 hours ) before the animals were euthanized and sectioned.

Whole body autoradiography (WBA) sagittal sectioning was performed as follows. At the end of the dosing period, mice were frozen in a hexane/dry ice bath and then embedded in a frozen block of carboxymethylcellulose. Frozen carcasses were allowed to off gas hexane overnight at -20°C prior to embedding in chilled 2% carboxymethylcellulose (Sigma Aldrich, C5013). Holes were then drilled into each block and radioactive ¹⁴C glycine controls (0.5 µCi mL⁻¹, American Radiolabeled Chemicals) in PBS + 0.5% BSA were pipetted in and allowed to freeze, prior to sectioning (40 µm) on a H/I Bright 8250 Cryostat (Hacker Instruments). Whole animal sagittal slices were prepared that resulted in thin frozen sections for imaging. Thin frozen sections were obtained using a microtome and allowed visualization of tissues such as brain, tumor, liver, kidney, lung, heart, spleen, pancreas, muscle, adipose, gall bladder, upper gastrointestinal tract, lower gastrointestinal tract, bone, bone marrow, reproductive tract, eye, cartilage, stomach, skin, spinal cord, bladder, salivary gland, and more. Sections were collected onto 4-inch wide tape (Scotch 821, ULINE) at 2-6 depths to sample all of the tissues of interest, and freeze dried in the cryostat for 2-3 days, after which they were mounted on sturdy paper and covered with cellophane. Mounted sections were exposed to storage phosphor plates (Raytest) along with a radioactive standard curve (146S-PL, American Radiolabeled Chemicals) for 7 days. Sections were allowed to desiccate in a freezer prior to imaging.

For the autoradiography imaging, tape mounted thin sections were freeze dried and radioactive samples were exposed to phophoimager plates for 7 days. These plates were developed and the signal (densitometry) from each organ was normalized to the signal found in the cardiac blood of each animal. A signal in tissue darker than the signal expected from blood in that tissue indicates accumulation in a region, tissue, structure, or cell. Scanning took place on a Raytest CR-35 imager at the "25 µm sensitive" setting. Quantitation was performed by background-adjusted densitometry using AIDA Image Analyzer v5.1 Whole Body Autoradiography Professional software (Raytest). For quantification, all region of interest measurements from a single tissue within a single section were averaged, adjusting for area. Blood values were assessed in the heart. Reporting values in nmol g⁻¹ tissue required calibration to the commercial standard strips co-exposed with each WBA sample set. The strips were themselves calibrated so that a known amount of radioactive counts per minute (CPM) per gram of tissue, determined by liquid scintillation counting (LSC), corresponded to the measured WBA densitometry at 11 standard strip concentration data points (linear range corresponding to 6.3 nCi g⁻¹ to 3.3 µCi g⁻¹). This calibration, combined with the peptides' known concentrations and specific activities (determined by liquid scintillation counting), permitted the conversion to nmol g⁻¹. For the quantitation shown in **FIG. 18****,** **FIG. 19****,** and **FIG. 33****,** total number of sections analyzed is indicated in the key; not every section contained every tissue, but every tissue quantitation represents 1-3 regions of interest per section over 1-4 sections per mouse from 3-10 mice. Of note, at the 100 nmol dose and average mouse weight of 25 g, 4 nmol/g represents "100% injected dose". Some tissues (spleen / kidney / liver) exceed this value because the peptide accumulates at the tissue beyond that expected by simple whole-body diffusion. This can indicate that the peptide preferentially accumulates in those tissues. Therefore, calculating a percent injected dose remaining in non-serum tissues can lead to uneven tissue distribution numbers, since there is no tissue-specific "time zero" amount.

**FIG. 16** shows autoradiography images of mice that were administered with TfR-binding peptides having a sequence of SEQ ID NO: 1 (**FIG. 16A**), SEQ ID NO: 2 (**FIG. 16B**), SEQ ID NO: 30 (**FIG. 16C**), and SEQ ID NO: 32 (**FIG. 16D**). Whole body autoradiography was used with IV-injected ¹⁴C-labeled peptides to assess biodistribution. Images were taken 3 hours after dose with 100 nmol drug (~20 mg/kg in ~25 g mice). The images demonstrate substantial accumulation in spleen, liver, kidney, and also high accumulation in muscle, bone marrow, and skin. CNS accumulation was less than other tissues but substantial compared to serum levels (>25% of serum). Background blood levels were found in brain to be 3% of the cardiac blood signal.

**FIG. 17** illustrates whole body autoradiography of a mouse injected intravenously with ¹⁴C-labeled peptides (SEQ ID NO: 1 and SEQ ID NO: 32) to assess biodistribution. Substantial accumulation in flank tumors was also seen. **FIG. 17A** illustrates white light images of mice injected intravenously with ¹⁴C-labeled SEQ ID NO: 1. **FIG. 17B** illustrates white light images of mice injected intravenously with ¹⁴C-labeled SEQ ID NO: 1. **FIG. 17C** illustrates whole body autoradiography of mice injected intravenously with ¹⁴C-labeled SEQ ID NO: 32. **FIG. 17D** illustrates whole body autoradiography of mice injected intravenously with ¹⁴C-labeled SEQ ID NO: 32. Notably, U87 tumor distribution of both peptides is complete as shown by significant levels of peptide throughout the flank tumor on the animal's back, even though the tumors appear poorly vascularized in the white light images to the right. The tumors were subcutaneous U87 brain tumors.

In addition to autoradiography, *ex vivo* scintillation counting was performed using the peptides having a sequence of SEQ ID NO: 1 and SEQ ID NO: 32 (**FIG. 18**, **FIG. 19**, and **FIG. 33**). **FIG. 18** and **FIG. 19** shows bar graphs of organ uptake data normalized to radiolabeled control samples in block and to peptide specific activity given in nmol/g levels, and only normalized to % of serum levels, respectively. Peptide specific activity given in nmol/g levels at both 30 min and 3 hour timepoints is show in **FIG. 33****.** **FIG. 18** illustrates the quantitation of peptide biodistribution and organ accumulation after a single 20 mg/kg IV dose of either a TfR-binding peptide having a sequence of SEQ ID NO: 1 or a peptide having a sequence of SEQ ID NO: 32. This dose achieved levels of >150 nM in the CNS, ~1 µM in tumor after 3 hours. **FIG. 19** illustrates the quantitation of peptide biodistribution and organ accumulation for the two peptides comprising SEQ ID NO: 1 and SEQ ID NO: 32, respectively. The values are shown as Percent of Blood Levels, which is a common BBB penetration metric (3% baseline). The data shows that for the TfR-binding peptides having a sequence of SEQ ID NO: 1 and SEQ ID NO: 32 biodistribution and organ accumulation for the peptides occurred in spleen, liver, kidney, skin, bones, and brain.

For the brain, the mice were not perfused prior to sectioning. Therefore, CNS capillaries can remain full of blood, and any peptide still in circulation can create a weak signal in the brain. However, it is generally accepted that CNS blood / capillaries occupy roughly 3% of the brain volume, and thus a signal of 3% vs blood may be considered equal to "no CNS accumulation." Any signal above 3% is considered evidence of peptide CNS accumulation, either stuck in capillary cells or actual BBB penetration. Thus, at >25% blood levels, both peptides demonstrate substantial CNS accumulation by this metric.

### EXAMPLE 15

### Quantification of Peptide Accumulation in Tissues by Scintillation Counting

This example illustrates quantification of peptide in serum in tissues by scintillation counting. Peptides were radiolabeled by the methods described in **EXAMPLE 19** and then administered intravenously to female Harlan athymic nude mice with intact kidneys. Radiolabeled peptides were administered intravenously at a dose of 100 nmol (14.7 µCi, 20 mg/kg). Mice were euthanized at various time points by CO₂ asphyxiation and tissues and biological fluids were collected including kidney, liver, brain, spleen, muscle, and skin. Solid tissues were homogenized prior to liquid scintillation counting. Quantitation of the amount of peptide in tissue (nmol/g) was performed by referencing peptide's molecular weight and specific ¹⁴C activity. **FIG. 20** shows quantification of tissue accumulation determined by scintillation counting after administration of a peptide having a sequence of SEQ ID NO: 32 (SEQ ID NO: 32 is SEQ ID NO: 67 with an added N-terminal GS). **FIG. 20A** illustrates scintillation counting over time in the kidneys. **FIG. 20B** illustrates scintillation counting over time in the liver. **FIG. 20C** illustrates scintillation counting over time in the brain (CNS). **FIG. 20D** illustrates scintillation counting over time in the spleen. **FIG. 20E** illustrates scintillation counting over time in the muscle. **FIG. 20F** illustrates scintillation counting over time in the skin. The data shows that for the peptide having a sequence of SEQ ID NO: 32 accumulation of peptides was confirmed in various tissues including spleen, liver, kidney, skin, bones, and brain. Over the time course of 64 hours, the amount of peptide having a sequence of SEQ ID NO: 32 significantly decreased in tissues such as kidney, liver, spleen, and muscle starting about 1-2 hours after administration, wherein the amount of peptide in the brain slightly decreased but then stabilized over the course of the experiment. SEQ ID NO: 32 demonstrated biphasic plasma elimination kinetics, with a first elimination half-life of 15.6 minutes. The highest levels are seen in the kidney, liver, and spleen; the kidney is responsible for excretion of small plasma solutes, while both hepatic and splenic tissues reportedly express high levels of TfR. The brain showed smaller but measurable levels.

Elimination of a peptide of SEQ ID NO: 32 was also tested in female Harlan athymic nude mice. SEQ ID NO: 32 was intravenously administered at a dose of 100 nmol (14.7 µCi, 20 mg/kg). **FIG. 21** illustrates a serum elimination plot, including two-phase elimination regression analysis showing fast (95%, 15.6 min t½) and slow (5%, 10.3 hr t½) phase kinetics in mice intravenously administered SEQ ID NO: 32.

This data demonstrates that the uptake and retention of these peptides in organs such as spleen, liver, kidney, skin, bones, and brain allows effective targeting of and delivery of active and/or detectable agents to these organs using the TfR-binding peptides of the present disclosure.

### EXAMPLE 16

### Multiple Time Regression and Capillary Depletion Analysis of Peptides

This example illustrates multiple time regression (MTR) and capillary depletion analysis of peptides of the present disclosure.

Mice (strain CD-1, N=1 per time point) were anesthetized and the left jugular vein and right carotid arteries were surgically exposed. Labeled peptides (0.45 µCi in 200 µL volume; specific activities were 32 Ci/mol for SEQ ID NO: 1 and 147 Ci/mol for SEQ ID NO: 32 (SEQ ID NO: 1 and SEQ ID NO: 32 are SEQ ID NO: 36 and SEQ ID NO: 67, respectively, with an added N-terminal GS), according to methods described in **EXAMPLE 19**) were injected into the jugular vein. After a given time within a time course (0-30 minutes), arterial blood was collected, followed by decapitation and brain collection. Brains and serum were tested via liquid scintillation counting for ¹⁴C levels, and the data was incorporated into a multiple time regression mathematic model to quantify CNS accumulation rate. To determine whether this CNS accumulation is limited to capillaries or actually represents vascular transcytosis, a separate set of animals (N=3/peptide) were euthanized 5 minutes after dosage, and brain homogenates were subject to centrifugation through a dextran density gradient. This allowed separation of capillaries from the parenchyma, and the two tissues were separately subjected to scintillation counting to quantify the ¹⁴C signal. Normalized signal ratios were an indicator of BBB penetration; for example, SEQ ID NO: 1 exhibited a substantially higher signal in the parenchyma than the capillaries, which demonstrated transcytosis across the BBB.

**FIG. 22** illustrates multiple time regression analysis and capillary depletion analysis of TfR-binding peptides having a sequence of SEQ ID NO: 1 and SEQ ID NO: 32. **FIG. 22A** illustrates multiple regression analysis of SEQ ID NO: 1 and SEQ ID NO: 32 in a single plot, wherein the y-axis indicates the brain to serum ratio. **FIG. 22B** illustrates parenchyma and capillary distribution of TfR-binding peptides having a sequence of SEQ ID NO: 1 and SEQ ID NO: 32, wherein the y-axis indicates the tissue-to-serum (T-to-S) ratio (µL/g), that is the ratio of radioactivity measured in the respective tissue (in this case, parenchyma or capillaries) to the radioactivity measured in circulating serum at the same time point.

The bar graph of **FIG. 22B****,** in particular, strongly suggests BBB transport and parenchyma access of a peptide of SEQ ID NO: 1, demonstrating the peptides of the present disclosure are in fact capable of promoting TfR-mediated transport across the BBB (e.g., vesicular transcytosis). Any peptide or peptide construct of the present disclosure (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) can be similarly capable of promoting TfR-mediated transport across the BBB (e.g., via vesicular transcytosis). The short time point for the capillary depletion experiment ( 30 mins) likely explains lack of obvious transcytosis of SEQ ID NO: 32 observed in this assay, but CRE-Luc reporter in mice was successfully induced (4 hr timepoint).

This data demonstrates that the peptides of the present disclosure enable TfR-mediated transport enabling the delivery of therapeutic and/or diagnostic agents into tissues expressing TfR such as brain (transcytosis via the BBB), tumors, bone and bone marrow, and immune cells. The transport can occur across cell layers such as endothelial cells (e.g., an intact BBB) or across cell membranes. Thus, the peptides of the present disclosure can be used as effective delivery vehicles for a variety of active agents into any cell, tissue, or organ that expresses TfR. In addition, this data demonstrates binding of the herein described peptides to murine TfR. Accessing and specifically targeting tumors behind an intact BBB may be a particular advantage (e.g., high unmet clinical need) of the herein described TfR-binding peptides compared to conventional methods.

### EXAMPLE 17

### Peptide Stability at High Temperatures

This example shows peptides of this disclosure are stable at higher temperatures.

Protein secondary structures were assessed using Circular Dichroism (CD). CD spectra were measured with a Jasco J-720W spectropolarimeter using a 1.0mm path length cell. Protein samples in 10mM phosphate buffer (pH = 7.4) were at 25-30 µM protein concentration. Samples were analyzed at a wavelength of 210 nm. Data are expressed in terms of relative ellipticity [θ]; (mdeg). To determine thermal stability of proteins, samples were subjected to incremental increase in temperature at a ramp of 2 °C/min from 20 °C to 95 °C. Stability and protein unfolding were monitored at 210 nm for α-helix secondary structure. Data were expressed in terms of relative ellipticity [θ], reported in mdeg.

The melting curve of SEQ ID NO: 32 is shown in **FIG. 27****.** Heat stability was tested under reducing (10 mM DTT) and non-reducing (NR) conditions. Vertical lines represent calculated melting points (~74°C for NR, ~38°C for 10 mM DTT treated) based on non-linear curve fitting (sigmoidal, variable slope, constrained bottom = 0; R² > 0.98 for both). Note that the NR sample fails to reach equilibrium up to 95°C, so it is likely that the protein is not fully "melted" at high temperature, but simply more disordered, particularly in the extended loop region. The difference in reduced and nonreduced samples illustrates the significant increase in melting temperature and structural robustness provided by the disulfide bonds.

### EXAMPLE 18

### Extension of Peptide Plasma Half-Life

This example demonstrates a method of extending the serum or plasma half-life of a peptide as disclosed herein. A peptide or peptide cosntruct having a sequence of any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361 is modified in order to increase its plasma half-life. Conjugation of the peptide to a near infrared dye, such as Cy5.5 is used to extend serum half-life of the peptide construct. Alternatively, conjugation of the peptide to an albumin binder, such as Albu-tag or a C14-C18 fatty acid, is used to extend plasma half-life. Optionally, plasma half-life is extended as a result of reduced immunogenicity by using minimal non-human protein sequences.

### EXAMPLE 19

### Peptide Radiolabeling with ¹⁴C

This example describes the radiolabeling of peptides with ¹⁴C.

Peptides of the present disclosure were radiolabeled by reductive methylation with ¹⁴C formaldehyde and sodium cyanoborohydride using standard techniques (such as those described in Jentoft et al. J Biol Chem. 254(11):4359-65. 1979). Briefly, peptides (10 mg) were dissolved in 3.2 mL water with 470 µL 10x PBS. 0.72 mCi (12.6 µM) ¹⁴C-formaldehyde (57 Ci mol⁻¹, Pharmaron) and sodium cyanoborohydride (to 100 mmol, in water) were added, followed by vortexing for 15 seconds. The reactions were incubated at RT overnight. 10 µL reaction was set aside in 1 mL water for analysis, after which the ¹⁴C methylated peptide was purified on a Strata-X reversed-phase column (30 mg, Phenomenex, washed with 3 mL methanol and 3 mL water). After loading, the column was washed with 4 mL water and eluted with 4 mL 2% formic acid in methanol. Labeled CDPs were dried in a blow-down evaporator (40°C under nitrogen stream) and stored at 18°C until use. Specific activity was determined by scintillation counting. Variant-specific activities of the variants of SEQ ID NO: 1 were as follows. SEQ ID NO: 1: 32 Ci/mol for 180 minute animals, 187 Ci/mol for 30 minute animals. SEQ ID NO: 2: 260 Ci/mol for 180 minute animals, 179 Ci/mol for 30 minute animals. SEQ ID NO: 32: 273 Ci/mol for 180 minute animals, 147 Ci/mol for 30 minute animals. The sequences were engineered to have the amino acids, "G" and "S" at the N terminus. *See* Methods in Enzymology V91:1983 p.570 and JBC 254(11):1979 p. 4359. An excess of formaldehyde was used to drive complete methylation (dimethylation of every free amine).

### EXAMPLE 20

### Peptide Detectable Agent Conjugates

This example describes the dye labeling of peptides. A peptide of the disclosure is expressed recombinantly or chemically synthesized, and then the N-terminus of the peptide is conjugated to, linked to, or fused to a detectable agent via an activated ester (e.g., NHS ester) in the presence of either DCC or EDC to produce a peptide-detectable agent conjugate. The detectable agent can be a cyanine dye, such as Cy5.5, Alexa fluorophore, or Alexa647.

The peptide detectable agent conjugates are administered to a subject. The subject can be a human or a non-human animal. After administration, the peptide detectable agent conjugates home to the kidneys. The subject, or a biopsy from the subject, is imaged to visualize localization of the peptide detectable agent conjugates to the kidney. In some aspects, diagnosis of renal disorders is based on the visualization of the peptide detectable agent conjugates in kidneys after administration.

### EXAMPLE 21

### Peptide Active Agent Conjugates

This example describes the peptide active agent conjugates. A peptide of the disclosure is expressed recombinantly or chemically synthesized, and then the N-terminus of the peptide is conjugated to, linked to, or fused to an active agent via an activated ester (e.g., NHS ester) in the presence of either DCC or EDC to produce a peptide-active agent conjugate.

The peptide active agent conjugates are administered to a subject in need thereof. The subject can be a human or a non-human animal. After administration, the peptide active agent conjugates transcytoses into an organ across an endothelial barrier via TfR-mediated transcytosis. The endothelial barrier is the blood brain barrier (BBB). The peptide active agent conjugate ameliorates the condition the subject is being treated for, for example brain cancer.

### EXAMPLE 22

### Mutation of Peptides to Improve Binding Affinity

This example illustrates mutation of peptides to decrease the off-rate and/or increase the on-rate of receptor binding.

A peptide of the present disclosure is mutated using Site Saturation Mutagenesis (SSM) or any random mutagenesis method to increase or decrease the off rate and/or increase the on rate, thereby increasing or decreasing the binding affinity. The peptide is selected from any one of the peptides shown in SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. The rate of delivery of the peptide to the target tissue, such as the brain parenchyma, is measured to determine the optimal binding affinity for the peptide for the given application.

### EXAMPLE 23

### Treatment of Cancer

This example illustrates treatment of cancer using peptides of the present disclosure.

A peptide of the present disclosure is recombinantly expressed or chemically synthesized and subsequently linked to a therapeutic molecule. The peptide or peptide constuct is administrated in a pharmaceutical composition to a subject in need thereof as a therapeutic for cancer. The peptide is selected from any one of the peptides or peptide constructs having a sequence of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. One or more peptides are administered to a subject. The subject can be a human or an animal. The pharmaceutical composition is administered intravenously, subcutaneously, intramuscularly, or orally. The cancer to be treated with a peptide-drug conjugate as described herein can be a brain cancer or a CNS-related cancer.

### EXAMPLE 24

### Combination Treatment of Cancer

This example illustrates combination treatment of cancer using peptides of the present disclosure. A peptide of the present disclosure is recombinantly expressed or chemically synthesized and used directly. The peptide is conjugated to, linked to, or fused to an active agent having anti-cancer activity. The peptide is administrated in a pharmaceutical composition to a subject in need thereof as a therapeutic for cancer. The peptide or peptide construct is selected from any one of the peptides having a sequence of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361.

One or more peptides are administered to a subject along with a treatment regimen of standard small molecule or chemotherapy, such as cisplatin, methotrexate, docetaxel, and etoposide, among others. The subject is a human or an animal. The pharmaceutical composition is administered intravenously, subcutaneously, intramuscularly, orally, or injected directly into the tumor microenvironment. The administered peptide-conjugates with standard small molecule therapy, treats a cancer condition in the subject. The cancer condition may be brain cancer, breast cancer, liver cancer, or colon cancer.

### EXAMPLE 25

### Imaging a Brain Condition with a TfR-binding Peptide

This example illustrates the imaging of a brain condition using a TfR-binding peptide or peptide construct disclosed herein (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) to transport detectable agent molecules attached to the peptides into the CNS.

A peptide of the present disclosure is conjugated to a detectable agent as described in **EXAMPLE 20.** The peptide detectable agent conjugate is administered to a subject in need thereof. The subject has a condition, such as brain cancer or Alzheimer's disease. The subject is a human or an animal. The peptide detectable agent conjugate is administered intravenously, subcutaneously, intramuscularly, orally, or injected directly into the tumor microenvironment.

The peptide is, optionally, further linked to an affinity moiety, for example, an affinity moiety such as F-18 for amyloid plaques. The peptide detectable agent crosses the BBB via TfR-peptide mediated transcytosis. Non-invasive imaging is performed to visualize the detectable agent in the brain, allowing for diagnosis or tracking of the condition in a subject.

### EXAMPLE 26

### Treatment of Brain Cancer with a TfR-binding Peptide

This example illustrates the treatment of a brain cancer using a TfR-binding peptide or peptide construct as described herein (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) to transport a therapeutic agent molecule attached to the peptide into the CNS and into tumor cells.

A peptide of the present disclosure is linked to an active agent as described in **EXAMPLE 21.** The active agent is temozolomide. The subject is a human or an animal. The peptide detectable agent conjugate is administered intravenously, subcutaneously, intramuscularly, orally, or injected directly into the tumor microenvironment. The peptide active agent conjugate is administered to a subject in need thereof. The subject has a brain cancer. Upon administration, the peptide active agent conjugate crosses the blood brain barrier (BBB) and accesses the brain parenchyma where the cancer is localized. The peptide active agent conjugate ameliorates and/or eradicates the brain cancer.

This data demonstrates that active agents that are generally prevented from accessing the brain (e.g., temozolomide that is a substrate of P-glycoprotein transporters that prevent crossing of endothelial cells of the BBB) can be transported across the BBB efficiently to prevent and/or treat diseases and conditions (e.g., cancers) located in the brain.

### EXAMPLE 27

### Cell-Penetrating Peptide Fusions

This example describes cell-penetrating peptide fusions. A TfR-binding peptide of the present disclosure is recombinantly expressed as a fusion to an additional cell penetrating peptide moiety. The TfR-binding peptide or peptide construct is selected fr any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. The cell penetrating peptide moiety is one or multiple Arg residues, such as an RRRRRRRR (SEQ ID NO: 73) sequence conjugated to, linked to, or fused at the N-terminus, or a Tat peptide with the sequence YGRKKRRQRRR (SEQ ID NO: 99) that is conjugated to, linked to, or fused to the N-terminus of any TfR-binding peptide of the present disclosure. Alternatively, the cell penetrating peptide moiety is selected from maurocaline, imperatoxin, hadrucalcin, hemicalcin, oplicalin-1, opicalcin-2, midkine (62-104), MCoTI-II, or chlorotoxin, which is fused to the N-terminus of any TfR-binding peptide of the present disclosure. Alternatively, the cell penetrating peptide moiety is selected from TAT such as CysTAT (SEQ ID NO: 71), S19-TAT (SEQ ID NO: 72), R8 (SEQ ID NO: 73), pAntp (SEQ ID NO: 74), Pas-TAT (SEQ ID NO: 75), Pas-R8 (SEQ ID NO: 76), Pas-FHV (SEQ ID NO: 77), Pas-pAntP (SEQ ID NO: 78), F2R4 (SEQ ID NO: 79), B55 (SEQ ID NO: 80), azurin (SEQ ID NO: 81), IMT-P8 (SEQ ID NO: 82), BR2 (SEQ ID NO: 83), OMOTAG1 (SEQ ID NO: 84), OMOTAG2 (SEQ ID NO: 85), pVEC (SEQ ID NO: 86), SynB3 (SEQ ID NO: 87), DPV1047 (SEQ ID NO: 88), C105Y (SEQ ID NO: 89), transportan (SEQ ID NO: 90), MTS (SEQ ID NO: 91), hLF (SEQ ID NO: 92), PFVYLI (SEQ ID NO: 93), or yBBR (SEQ ID NO: 94), which is fused to the N-terminus of any TfR-binding peptide of the present disclosure. Alternatively, the cell penetrating peptide moiety is fused to the N-terminus of any TfR-binding peptide of the present disclosure by a linker. The linker is selected from GGGSGGGSGGGS (SEQ ID NO: 113), KKYKPYVPVTTN (SEQ ID NO: 114) (linker from DkTx), or EPKSSDKTHT (SEQ ID NO: 115) (linker from human IgG3), or any other linker. Alternatively, the TfR-binding peptide, the cell penetrating peptide moiety, and, optionally, the linker are joined by other means. For example, the other means includes, but is not limited to, chemical conjugation at any location, fusion of the cell penetrating peptide moiety and/or the linker to the C-terminus of the TfR-binding peptide, co-formulation with liposomes, or other methods.

Cell-penetrating peptide fusions or conjugates are administered to a subject in need thereof. The subject is a human or animal and has a disease, such as a brain cancer or other brain condition. Upon administration, TfR-binding peptides promote transcytosis across the BBB and the cell-penetrating peptides promote crossing the cellular membranes to access intracellular compartments.

### EXAMPLE 28

### Peptide Fusions to Promote Nuclear Localization

This example describes peptide fusions to promote nuclear localization. A TfR-binding peptide of the present disclosure is recombinantly expressed or chemically synthesized. The peptide or peptide construct is selected from any sequence of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361. TfR-binding peptides are conjugated to, linked to, or fused to a nuclear localization signal, such as a four-residue sequence of K-K/R-X-K/R (SEQ ID NO: 129), wherein X can be any amino acid, or a variant thereof (Lange et al, J Biol Chem. 2007 Feb 23;282(8):5101-5). Peptide fusions are administered to a subject in need thereof. The subject is a human or animal and has a disease, such as a brain cancer or other brain condition. Upon administration, TfR-binding peptides promote transcytosis across the BBB and the nuclear localization signal promotes trafficking to the nucleus.

### EXAMPLE 29

### Treatment of Brain Cancer with an Immunotherapy Agent

This example illustrates treatment of brain cancer (e.g., a glioblastoma, an astrocytoma, a midline glioma, a DIPG, a medulloblastoma, a MYC- or MYCN-amplified brain tumor, or an ependymoma) using peptides or peptide constructs of the present disclosure (e.g., any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361) that are conjugated to, linked to, or fused to one or more immunotherapeutic agents such as a CTLA-4, a PD-1, or a PDL-1 targeting agent, or IL15, or a fused IL15/IL15Ra, IFNgamma, or anti-CD3 fusion polypeptides . In this experiment, the immunotherapeutic agent is pembrolizumab (anti-PD-1 antibody).

A TfR-binding peptide or peptide construct having a sequence of any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361 is expressed recombinantly or chemically synthesized. Upon purification using liquid chromatography (e.g., reversed-phase, ion exchange, or size-exclusion chromatograph), the peptide is then conjugated to pembrolizumab or a functional fragment thereof (e.g., a variable chain fragment) according to the method described in **EXAMPLE 21** (alternatively, the peptide can also be linked to, or fused (e.g., recombinantly expressed) to pembrolizumab (or any other immunotherapeutic agent) or a functional fragment thereof). The conjugate is purified and formulated for injection into a subject (e.g., a mouse or a human).

Following administration to a subject in need thereof, the peptide construct comprising a TfR-binding peptide conjugated to pembrolizumab or a functional fragment thereof, penetrates the BBB via vesicular transcytosis and accumulates in the CNS. Nuclear imaging (e.g., positron emission tomography, computed tomography (PET), or magnetic resonance imaging) and tissue or biopsy samples taken from the subject suffering from the brain cancer show that the peptide construct significantly reduces tumor burden in the subject. Whole body imaging (e.g., WBA or PET) shows that the peptide construct not only reduced the tumor size and sites of disease in the brain, but also at other location within the body of the subject (e.g., liver, lungs, and bone).

### EXAMPLE 30

### Treatment of Neuropathic Pain using Neurotensin-Containing Peptide Constructs

This example illustrates the treatment of neuropathic pain using a TfR-binding peptide fused to, expressed recombinantly with, chemically synthesized with, or otherwise conjugated to neurotensin (NT), as described herein, to transport neurotensin into the CNS.

A TfR-binding peptide having a sequence of any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358 is expressed recombinantly or chemically synthesized. The peptide is optionally purified using liquid chromatography (e.g., reversed-phase, ion exchange, or size-exclusion chromatograph) and further conjugated to NT according to the method described in **EXAMPLE 21.** An NT peptide having a sequence of any one of SEQ ID NO: 350, SEQ ID NO: 365 - SEQ ID NO: 371, or a functional fragement thereof is expressed recombinantly or chemically synthesized with a TfR-binding peptide to form a peptide-NT construct. Alternatively, a peptide is expressed recombinantly or chemically synthesized with neurotensin, or a functional fragment thereof, as a peptide-NT fusion (e.g., peptide-NT constructs shown in SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361) and optionally purified using liquid chromatography (e.g., reversed-phase, ion exchange, or size-exclusion chromatograph). A functional fragment of NT can be a fragment that is at least 8 amino acid residues, at least 10 amino acid residues, or at least 13 amino acid residues long. The purified NT conjugate or funsion peptide ("peptide construct") is formulated for injection into a subject (e.g., a mouse or a human).

Following administration, the peptide construct penetrates the BBB accumulates in the CNS. Symptoms of neuropathic pain are significantly reduced in the subject due to effective targeting of the NT moiety to NT receptors.

The TfR-binding peptides of the present disclosure provide therapeutically effective concentration of the NT receptor binding peptides in the CNS to effectively treat neuropathic pain.

### EXAMPLE 31

### Treatment or Management of Pain with a Peptide-NT Construct

This example describes a method for treating or managing pain. This method is used as a treatment for acute and/or chronic symptoms. A peptide of the disclosure is expressed and administered in a pharmaceutical composition to a patient as a therapeutic for pain as a result of injury or other condition as described herein. The peptide of the present disclosure is fused to neurotensin. The peptide is expressed recombinantly or chemically synthesized.

Any peptide disclosed herein (e.g., any one of SEQ ID NO: 1 - SEQ ID NO: 32, SEQ ID NO: 36 - SEQ ID NO: 67, SEQ ID NO: 137 - SEQ ID NO: 167, SEQ ID NO: 171 - SEQ ID NO: 202, or SEQ ID NO: 356 - SEQ ID NO: 358) is fused to NT to form a peptide-NT construct. An NT peptide having a sequence of any one of SEQ ID NO: 350, SEQ ID NO: 365 - SEQ ID NO: 371, or a functional fragement thereof is expressed recombinantly or chemically synthesized with a TfR-binding peptide to form a peptide-NT construct.

Alternatively, a peptide-NT construct disclosed herein (e.g., any one of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361) is used. Peptides are optionally mutated to maintain the TfR-binding function, but to add or increase neurotensin interaction with the neurotensin receptor. The peptide-NT construct is optionally purified using liquid chromatography (e.g., reversed-phase, ion exchange, or size-exclusion chromatograph). The peptide is formulated for administration and administered to the subject. Following administration, the peptide construct targets to the area, tissue, or system affected by pain. The peptide construct can modulate pain. Such pain modulation may operate by various mechanisms such as modulating inflammation, autoimmune responses, direct or indirect action on pain receptors, cell killing, or programmed cell death. One or more peptides are administered to a human or animal subcutaneously, intravenously, or orally, or are injected.

### EXAMPLE 32

### Treatment of Chronic Pain using Neurotensin-Containing Peptide Constructs

This example illustrates the treatment of neuropathic pain using a TfR-binding peptide as described herein (e.g., peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361) or peptides (e.g., SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) that can be fused to NT, to transport neurotensin (NT, SEQ ID NO: 350) into the CNS.

A TfR-binding peptide having a sequence of any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358, or a TfR-binding peptide fused to NT (e.g., any one of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361) is expressed recombinantly or can be chemically synthesized. Upon purification using liquid chromatography (e.g., reversed-phase, ion exchange, or size-exclusion chromatograph), the peptide can be optionally further conjugated to neurotensin (NT) or a functional fragment thereof, according to the method described in **EXAMPLE 21.** A functional fragment of NT can be a fragment that is at least 8 amino acid residues, at least 10 amino acid residues, or at least 13 amino acid residues long. An NT peptide having a sequence of any one of SEQ ID NO: 350, SEQ ID NO: 365 - SEQ ID NO: 371, or a functional fragement thereof is expressed recombinantly or chemically synthesized with a TfR-binding peptide to form a peptide-NT construct. Alternatively, the peptide is fused to neurotensin or a functional fragment thereof, (e.g., recombinantly expressed), for example as was used to make the peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361 or could be used to fuse NT to such as the peptides of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358, and then expressed recombinantly and purified as described herein or using other known methods.

The peptide-neurotensin fusion or conjugate is formulated for injection into a subject (e.g., a mouse or a human). Following administration of the conjugate, the peptide construct penetrates the BBB and accumulates in the CNS. Chronic pain is significantly reduced in the subject due to effective targeting of the NT moiety to NT receptors.

The peptide itself can modulate pain or it can be conjugated to an agent that modulates pain. Such pain modulation may operate by various mechanisms such as modulating inflammation, autoimmune responses, direct or indirect action on pain receptors, cell killing, or programmed cell death. Peptide-NT fusions can be used to treat chronic pain including from chronic injury, illness, or a chronic condition including familial episodic pain syndrome, erythromelalgia, or paroxysmal extreme pain disorder.

### EXAMPLE 33

### Treatment of Acute Pain using Neurotensin-Containing Peptide Constructs

This example illustrates the treatment of neuropathic pain using a TfR-binding peptide as described herein (e.g., peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361) or peptides (e.g., SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) that can be fused to NT, to transport neurotensin (NT, SEQ ID NO: 350) into the CNS.

A TfR-binding peptide having a sequence of any one of SEQ ID NO: 1 - SEQ ID NO: 70, SEQ ID NO: 136 - SEQ ID NO: 205, or SEQ ID NO: 356 - SEQ ID NO: 361, optionally fused to NT, is expressed recombinantly or can be chemically synthesized. Upon purification using liquid chromatography (e.g., reversed-phase, ion exchange, or size-exclusion chromatograph), the peptide can be further conjugated to neurotensin (NT) or a functional fragment thereof, according to the method described in **EXAMPLE 21.** A functional fragment of NT can be a fragment that is at least 8 amino acid residues, at least 10 amino acid residues, or at least 13 amino acid residues long. An NT peptide having a sequence of any one of SEQ ID NO: 350, SEQ ID NO: 365 - SEQ ID NO: 371, or a functional fragement thereof is expressed recombinantly or chemically synthesized with a TfR-binding peptide to form a peptide-NT construct. Alternatively, the peptide is fused to neurotensin or a functional fragment thereof, (e.g., recombinantly expressed), for example as was used to make the peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361 or could be used to fuse NT to such as the peptides of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358, and then expressed recombinantly and purified as described herein or using other known methods.

The peptide-neurotensin fusion or conjugate is formulated for injection into a subject (e.g., a mouse or a human). Following administration of the conjugate, the peptide construct penetrates the BBB and accumulates in the CNS. Acute pain is significantly reduced in the subject due to effective targeting of the NT moiety to NT receptors.

The peptide itself can modulate pain or it can be conjugated to an agent that modulates pain. Such pain modulation may operate by various mechanisms such as modulating inflammation, autoimmune responses, direct or indirect action on pain receptors, cell killing, or programmed cell death. Peptide-NT fusions can be used to treat acute pain including from acute injury, illness, or an acute condition, or from nociceptive pain, neuropathic pain, allodynia, phantom pain, visceral pain, breakthrough pain. Rapid pain relief within five minutes to an hour is optionally experienced by the subject, and pain relieve can last as long as over 3 hours.

### EXAMPLE 34

### Ibuprofen Peptide Conjugate

This example describes the conjugation of ibuprofen to a TfR-binding peptide as described herein (e.g., SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) using a PEG linker. A TfR-binding peptide is expressed recombinantly or can be chemically synthesized. The peptide is optionally purified using liquid chromatography (e.g., reversed-phase, ion exchange, or size-exclusion chromatograph), or other known methods. A peptide-ibuprofen conjugate is produced using ibuprofen and a PEG linker, which forms an ester bond that can hydrolyze as described in *"In vitro* and *in vivo* study of poly(ethylene glycol) conjugated ibuprofen to extend the duration of action," Scientia Pharmaceutica, 2011, 79:359-373, Nayak and Jain. Fischer esterification is used to conjugate ibuprofen with a short PEG, e.g., with triethylene glycol, to yield ibuprofen-ester-PEG-OH.

Following preparation of the PEG-ibuprofen conjugate as shown above, the hydroxyl moiety of PEG is activated with N,N'-disuccinimidyl carbonate (DSC) to form ibuprofen-ester-PEG-succinimidyl carbonate, which is then reacted with a lysine or the N-terminus of a peptide to form an ibuprofen-ester-PEG-peptide conjugate. The peptide-ibuprofen fusion or conjugate is formulated for injection into a subject (e.g., a mouse or a human). Following administration of the conjugate, the peptide construct penetrates the BBB via vesicular transcytosis and accumulates in the CNS. The conjugate can display anti-inflammatory activity, or free ibuprofen is released from the conjugate to provide anti-inflammatory activity. The free ibuprofen can result from hydrolysis that occurs after administration, such as hydrolysis at the ester bond.

The peptide itself can modulate pain or it can be conjugated to an agent that modulates pain (e.g., ibuprofen). Such pain modulation may operate by various mechanisms such as modulating inflammation, autoimmune responses, direct or indirect action on pain receptors, cell killing, or programmed cell death. Ibuprofen-peptide conjugates are administered to a subject in need thereof. The subject can be a human or a non-human animal.

### EXAMPLE 35

### Peptide Dalazatide Conjugates

This example describes a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to a Kᵥ1.3 potassium channel inhibitor for treatment of automimmune diseases. In this case, the Kᵥ1.3 inhibitor is dalazatide.

A peptide of the disclosure is expressed recombinantly or chemically synthesized, and then the N-terminus of the peptide is conjugated to, linked to, or fused to dalazatide via an activated ester (e.g., NHS ester) in the presence of either DCC or EDC to produce a TfR-binding peptide- dalazatide conjugate.

The peptide construct is administered to a subject in need thereof. The peptide construct is administered intravenously, subcutaneously, intramuscularly, or orally. The subject can be a human or a non-human animal. After administration, the peptide- Kᵥ1.3 inhibitor conjugate is delivered to the tissue or organ of interest via TfR-mediated transcytosis and/or TfR-mediated endocytosis. The peptide-dalazatide conjugate ameliorates the autoimmune disease or condition the subject is being treated for, for example those caused by self-reactive T lymphocytes.

This data demonstrates that the TfR-binding peptides of the present disclosure provide therapeutically effective concentrations of the Kᵥ1.3 inhibitor in the CNS to treat autoimmune disorders. The therapeutic activity is improved compared to treatment with the Kᵥ1.3 inhibitor alone.

### EXAMPLE 36

### Peptide 4SC-202 Conjugates

This example describes a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to a Kᵥ1.3 potassium channel inhibitor for treatment of automimmune diseases. In this case, the Kᵥ1.3 inhibitor is 4SC-202 (domatinostat tosylate).

A peptide of the disclosure is expressed recombinantly or chemically synthesized, and then the N-terminus of the peptide is conjugated to, linked to, or fused to 4SC-202 via, for example, an activated ester (e.g., NHS ester) in the presence of either DCC or EDC to produce a TfR-binding peptide-4SC-202 conjugate.

The peptide construct is administered to a subject in need thereof. The peptide construct is administered intravenously, subcutaneously, intramuscularly, or orally. The subject can be a human or a non-human animal. After administration, the peptide- Kᵥ1.3 inhibitor conjugate is delivered to the tissue or organ of interest via TfR-mediated transcytosis and/or TfR-mediated endocytosis. The peptide-4SC-202 penetrates the BBB via vesicular transcytosis and accumulates in the CNS. The peptide-4SC-202 conjugate ameliorates the autoimmune disease or condition the subject is being treated for, for example those caused by self-reactive T lymphocytes.

This data demonstrates that the TfR-binding peptides of the present disclosure provide therapeutically effective concentration of the Kᵥ1.3 inhibitor to treat autoimmune disorders. The therapeutic activity is improved compared to treatment with the Kᵥ1.3 inhibitor alone.

### EXAMPLE 37

### TfR-binding Peptide anti-Kᵥ1.3 Peptide Conjugates

This example describes a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to, linked to, or fused to a Kv1.3 potassium channel inhibitor for treatment of automimmune diseases. In this case, the Kv1.3 inhibitor is a peptide (e.g., Vm24 (SEQ ID NO: 378), ShK-170, ShK-186 (SEQ ID NO: 379), or ShK-192).

A peptide of the disclosure is expressed recombinantly or chemically synthesized, and then the N-terminus of the peptide is conjugated to, linked to, or fused to anti-Kᵥ1.3 peptide via, for example, an activated ester (e.g., NHS ester) in the presence of either DCC or EDC to produce a TfR-binding peptide-anti-Kᵥ1.3 peptide construct or fusion peptide (if recombinantly produced). The anti-Kᵥ1.3 peptide is Vm24 (SEQ ID NO: 378), ShK-170, ShK-186 (SEQ ID NO: 379), ShK-192, or another anti-Kᵥ1.3 peptide sequence.

The peptide construct is administered to a subject in need thereof. The peptide construct is administered intravenously, subcutaneously, intramuscularly, or orally. The subject can be a human or a non-human animal. After administration, the TfR-binding peptide-anti-Kᵥ1.3 peptide construct or fusion peptide is delivered to the tissue or organ of interest via TfR-mediated transcytosis and/or TfR-mediated endocytosis. The TfR-binding peptide-anti-Kᵥ1.3 peptide construct or peptide construct penetrates the BBB via vesicular transcytosis and accumulates in the CNS. The TfR-binding peptide-anti-Kᵥ1.3 peptide construct or peptide construct ameliorates the autoimmune disease or condition the subject is being treated for.

This demonstrates that the TfR-binding peptides of the present disclosure provide therapeutically effective concentration of the Kᵥ1.3 inhibitor to treat autoimmune disorders. The therapeutic activity is improved compared to treatment with the Kᵥ1.3 inhibitor alone.

### EXAMPLE 38

### TfR-binding Peptide Kᵥ1.3 Inhibitor Conjugates for Treatment of a Neurodegenerative Disease

This example describes a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to, linked to, or fused to an ion channel modulator such as a Kᵥ1.3 potassium channel inhibitor for treatment of neurodegenerative diseases (e.g., Alzheimer's disease, Amyotrophic lateral sclerosis, Friedreich's ataxia, Huntington's disease, Lewy body disease, Parkinson's disease, Spinal muscular atrophy, Motor neuron disease, Lyme disease, Ataxia-telangiectasia, Autosomal dominant cerebellar ataxia, Batten disease, Corticobasal syndrome, Creutzfeldt-Jakob disease, Fragile X-associated tremor/ataxia syndrome, Kufor-Rakeb syndrome, Machado-Joseph disease, multiple sclerosis, chronic traumatic encephalopathy, or frontotemporal dementia). Kᵥ1.3 inhibitors can include small molecules (e.g., dalazatide or 5-(4-Phenoxybutoxy)psoralen) and peptides (e.g., ShK-170, ShK-186, or ShK-192).

A peptide of the disclosure is expressed recombinantly or chemically synthesized, and then the N-terminus of the peptide is conjugated to, linked to, or fused to one or more Kᵥ1.3 inhibitors (e.g., dalazatide, 5-(4-Phenoxybutoxy)psoralen, ShK-170, ShK-186, or ShK-192) via, for example, an activated ester (e.g., NHS ester) in the presence of either DCC or EDC to produce a TfR-binding peptide- anti-Kᵥ1.3 peptide conjugate, fusion peptide, or peptide construct (if applicable). The peptide construct is administered to a subject in need thereof. The peptide construct is administered intravenously, subcutaneously, intramuscularly, or orally. The subject can be a human or a non-human animal. After administration, the TfR-binding peptide-Kᵥ1.3 inhibitor conjugate or peptide construct is delivered to the tissue or organ of interest via TfR-mediated transcytosis and/or TfR-mediated endocytosis. The TfR-binding peptide-Kᵥ1.3 inhibitor conjugate or peptide construct penetrates the BBB via vesicular transcytosis and accumulates in the CNS. The TfR-binding peptide-Kᵥ1.3 inhibitor conjugate or peptide construct ameliorates the neurodegenerative disease or condition the subject is being treated for.

This demonstrates that the TfR-binding peptides of the present disclosure provide therapeutically effective concentration of the Kᵥ1.3 inhibitor to treat neurodegenerative diseases. The therapeutic activity is improved compared to treatment with the Kᵥ1.3 inhibitor alone.

### EXAMPLE 39

### TfR-binding Peptide Verubecestat Conjugate

This example describes a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to verubecestat, a BACE inhibitor, for treatment of Alzheimer's Disease.

A peptide of the disclosure is expressed recombinantly or chemically synthesized, and then the N-terminus of the peptide is conjugated to, linked to, or fused to verubecestat via, for example, an activated ester (e.g., NHS ester) in the presence of either DCC or EDC to produce a TfR-binding peptide-verubecestat conjugate.

The peptide construct is administered to a subject in need thereof. The peptide construct is administered intravenously, subcutaneously, intramuscularly, or orally. The subject can be a human or a non-human animal. After administration, the TfR-binding peptide-verubecestat conjugate is delivered to the tissue or organ of interest via TfR-mediated transcytosis and/or TfR-mediated endocytosis. The TfR-binding peptide-verubecestat conjugate is efficiently transported across the BBB via TfR-mediated vesicular transcytosis and accumulates in the CNS. The TfR-binding peptide-verubecestat conjugate ameliorates the Alzheimer's Disease in the subject.

This demonstrates that the TfR-binding peptides of the present disclosure provide therapeutically effective concentration of the BACE inhibitor to treat Alzheimer's Disease. The therapeutic activity is improved compared to treatment with the BACE inhibitor alone.

### EXAMPLE 40

### Treatment of Brain Cancer with a Peptide Construct Comprising a non-BBB Penetrating Active Agent

This example illustrates the treatment of a brain cancer using a TfR-binding peptide construct comprising a TfR-binding peptide as described herein (e.g., a peptide having a sequence of any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) conjugated to, linked to, or fused to a non-BBB penetrating active agent (e.g., doxorubicin) to transport that agent into the CNS via TfR-mediated transcytosis to treat a brain tumor (e.g., glioblastoma, astrocytoma, midline glioma, DIPG, medulloblastoma, MYC- or MYCN-amplified brain tumors, or ependymoma) that would otherwise not be treatable or only show very limited efficacy with the drug alone.

A peptide of the disclosure is expressed recombinantly or chemically synthesized. The peptide of the present disclosure is conjugated to, linked to, or fused to an active agent as described in **EXAMPLE 21.** The peptide construct is administered intravenously, subcutaneously, intramuscularly, orally, or injected directly into a tumor microenvironment. The peptide construct is administered to a subject in need thereof. The subject is a human or an animal. The subject has a brain cancer. Upon administration, the peptide construct crosses the blood brain barrier (BBB) and accesses the region(s) of the brain where the cancer is localized. The peptide construct comprising the non-BBB penetrating agent ameliorates and/or eradicates the brain cancer.

This data demonstrates that the TfR-binding peptides of the present disclosure can be used to provide therapeutically effective concentrations of therapeutic agents (e.g., doxorubicin) in cells, tissues, or organs that may otherwise not be accessible for the therapeutic agents alone.

### EXAMPLE 41

### Targeting Bone Marrow using TfR-binding Peptides

This example describes targeting bone marrow using a TfR-binding peptide described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The TfR-binding peptides can be conjugated to, linked to, or fused to an active or detectable agent for treatment or diagnosis of disease, respectively.

The TfR-binding peptide or peptide construct of the disclosure is expressed recombinantly or chemically synthesized according to **EXAMPLE 1** and/or **EXAMPLE 2.** The TfR-binding peptide or peptide construct is administered to a subject in need thereof. The TfR-binding peptide or peptide construct is administered intravenously, subcutaneously, intramuscularly, orally, or injected directly into a tumor microenvironment. The subject is a human or an animal. After administration, the TfR-binding peptide or peptide construct is delivered to the bone marrow via TfR-mediated transcytosis and/or TfR-mediated endocytosis.

The presence of the TfR-binding peptide or peptide construct in the bone marrow of the subject is confirmed using tissue samples and/or non-invasive imaging, demonstrating that the TfR-binding peptides or peptide constructs of the present disclosure successfully target TfR-expressing cells, tissues, or organs such as the bone marrow as an important tissue for therapeutic intervention.

### EXAMPLE 42

### Targeting Erythroid cells using TfR-binding Peptides

This example describes targeting of erythroid cells or erythroid precursor cells using a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The TfR-binding peptides can be conjugated to, linked to, or fused to an active or detectable agent for treatment or diagnosis of disease, respectively.

The TfR-binding peptides or peptide constructs of the disclosure are expressed recombinantly or chemically synthesized according to **EXAMPLE 1** and/or **EXAMPLE 2.** The peptide or peptide construct is administered to a subject in need thereof. The peptide or peptide construct is administered intravenously, subcutaneously, intramuscularly, or orally. The subject is a human or an animal. After administration, the TfR-binding peptide or peptide construct is delivered to the erythroid cells or erythroid precursor cells via TfR-mediated transcytosis and/or TfR-mediated endocytosis.

The presence of the TfR-binding peptide or peptide construct in the erythroid cells or erythroid precursor cells of the subject is confirmed using tissue (e.g., bone marrow samples) or blood samples or via non-invasive imaging, demonstrating that the TfR-binding peptides and peptide constructs of the present disclosure successfully target TfR-expressing cells, tissues, or organs such as erythroid cells or erythroid precursor cells.

### EXAMPLE 43

### Targeting Muscle cells using TfR-binding Peptides

This example describes targeting of muscle cells using a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The TfR-binding peptides can be conjugated to, linked to, or fused to an active or detectable agent for treatment or diagnosis of disease, respectively.

The TfR-binding peptide or peptide construct of the disclosure is expressed recombinantly or chemically synthesized according to **EXAMPLE 1** and/or **EXAMPLE 2.** The TfR-binding peptide or peptide construct is administered to a subject in need thereof. The TfR-binding peptide or peptide construct is administered intravenously, subcutaneously, intramuscularly, or orally. After administration, the TfR-binding peptide or peptide construct is delivered to muscle cells or muscle tissue via TfR-mediated transcytosis and/or TfR-mediated endocytosis.

The presence of the TfR-binding peptide or peptide construct in the muscle cells or muscle tissue (particularly the uptake and retention in skeletal muscle tissue) of the subject is confirmed using tissue (e.g., muscle or skeletal muscle tissue samples) or blood samples or via non-invasive imaging, demonstrating that the TfR-binding peptides or peptide constructs of the present disclosure successfully target TfR-expressing cells, tissues, or organs such as muscle cells or muscle tissue.

### EXAMPLE 44

### Treatment of an Inflammatory Bowel Disease using TfR-binding Peptides

This example describes treatment of an inflammatory bowel disease using a TfR-binding peptide as described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The TfR-binding peptides is conjugated to, linked to, or fused to an active or detectable agent for treatment of an inflammatory bowel disease.

The TfR-binding peptide construct of the disclosure is expressed recombinantly or chemically synthesized according to **EXAMPLE 1** and/or **EXAMPLE 2.** The TfR-binding peptide construct is administered to a subject in need thereof. The TfR-binding peptide construct is administered intravenously, subcutaneously, intramuscularly, or orally. The subject is a human or an animal. After administration, the TfR-binding peptide construct is delivered to the intestines and accumulates in the glandular cells of the intestines.

The peptide construct comprising an active agent ameliorates and/or eradicates the inflammatory bowel disease. This demonstrates that the TfR-binding peptides of the present disclosure can be used to provide therapeutically effective concentrations within the intestinal tract for treatment of various diseases such as an inflammatory bowel disease.

### EXAMPLE 45

### TfR-binding Peptides for pH-dependent Endosomal Delivery

This example describes development and in vitro testing of TfR-binding peptides or peptide constructs capable of pH-dependent dissociation from TfR, for example, at endosomal pH (e.g., pH 5.4).

One or more additional histidine residues are introduced into the sequence of TfR-binding peptides (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) or peptide constructs (e.g., any one of SEQ ID NO: 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, SEQ ID NO: 225 - SEQ ID NO: 332, or SEQ ID NO: 359 - SEQ ID NO: 361). The resulting histidine-enriched TfR-binding peptides are evaluated for their TfR binding in comparative binding experiments at various pH levels or ranges. Peptides with high TfR binding affinity at physiological pH but a significantly reduced binding affinity at lower pH levels such as endosomal pH of 5.4 are selected for cellular binding, uptake, and intra-endosomal or intra-vesicular release experiments.

TfR-binding peptides with high endosomal delivery capabilities are identified and characterized. These results demonstrate that the TfR-binding peptides of the present disclosure can exhibit, or can be modified to exhibit pH-dependent TfR binding kinetics that allows intra-vesicular release of TfR-binding peptides and TfR-binding peptide constructs comprising one or more active agents for endosomal and/or intracellular delivery.

In order to improve the intracellular delivery functions, the TfR-binding peptides and TfR-binding peptide constructs as described herein are modified to comprise a motif that facilitates low-pH endosomal escape of the peptide or peptide construct.
Cellular uptake and release experiments demonstrate that the TfR-binding peptides and TfR-binding peptide constructs that comprise a motif for low-pH endosomal escape show are present in the cytosol at higher concentrations compared to peptides that do not comprise the motif for low-pH endosomal escape. This data demonstrates that the TfR-binding peptides and TfR-binding peptide constructs of the present disclosure can be successfully modified for enhanced intra-vesicular and intra-cellular delivery while retaining their TfR binding capabilities. These peptides can be used in combination with various therapeutic and/or compounds for treatment and/or diagnosis of diseases and conditions.

### EXAMPLE 46

### TfR-binding Peptides for Treatment of a Disease Located in Non-CNS Tissue

This example describes treatment of a disease that is located in a non-CNS tissue that expresses TfR using the TfR-binding peptide described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358).

The TfR-binding peptide construct of the disclosure is expressed recombinantly or chemically synthesized according to **EXAMPLE 1** and/or **EXAMPLE 2.** Optionally, an active agent used to treat the disease or condition can be conjugated to, linked to, or fused to the TfR-binding peptide to form a TfR-binding peptide construct. The active agent is, optionally, any NT disclosed herein (e.g., neurotensin (SEQ ID NO: 350) or a neurotensin variant (e.g., any one of SEQ ID NO: 365 - SEQ ID NO: 371)), or a functional fragment thereof, fused to a TfR-binding peptide (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The peptide-NT fusions may be any one of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361. The TfR-binding peptide construct is administered to a subject in need thereof. The TfR-binding peptide construct is administered intravenously, subcutaneously, intramuscularly, or orally. The subject is a human or an animal. After administration, the TfR-binding peptide construct accumulates in the non-CNS tissue that expresses TfR. The TfR-binding peptide construct ameliorates the disease or condition.

This data demonstrates that the TfR-binding peptides of the present disclosure effectively accumulate in TfR-expressing non-CNS tissue, and thus can be used to treat and/or prevent a disease or condition located in one or more of these tissues.

### EXAMPLE 47

### TfR-binding Peptides for Treatment of a Peripheral Cancer

This example describes treatment of a peripheral (e.g., non-CNS) cancer that expresses and/or overexpresses TfR using a TfR-binding peptide described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The peripheral cancer can be breast cancer, liver cancer, colon cancer, brain cancer, spleen cancer, cancers of the salivary gland, kidney cancer, muscle cancers, bone marrow cell cancers, or skin cancer, genitourinary cancer, osteosarcoma, muscle-derived sarcoma, melanoma, head and neck cancer, a neuroblastoma, a CMYC-overexpressing tumor.

The TfR-binding peptide construct of the disclosure is expressed recombinantly or chemically synthesized according to **EXAMPLE 1** and/or **EXAMPLE 2.** Optionally, the active agent used to treat the peripheral cancer can be conjugated to, linked to, or fused to the TfR-binding peptide to form a TfR-binding peptide construct. The active agent is, optionally, any NT disclosed herein (e.g., neurotensin (SEQ ID NO: 350) or a neurotensin variant (e.g., any one of SEQ ID NO: 365 - SEQ ID NO: 371)), or a functional fragment thereof, fused to a TfR-binding peptide (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). The peptide-NT fusions may be any one of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361. The TfR-binding peptide construct is administered to a subject in need thereof. The TfR-binding peptide construct is administered intravenously, subcutaneously, intramuscularly, orally, or intratumorally. The subject is a human or an animal. After administration, the TfR-binding peptide construct accumulates in the peripheral tumor tissue that expresses and/or overexpresses TfR. The TfR-binding peptide construct ameliorates the peripheral cancer.

This data demonstrates that the TfR-binding peptides of the present disclosure effectively accumulate in TfR-expressing and/or TfR-overexpress peripheral cancers, and thus can be used to treat and/or prevent a cancer located in one or more of these tissues.

### EXAMPLE 48

### TfR-binding Peptides for Treatment of a Spleen Cancer

This example describes treatment of a spleen cancer that expresses and/or overexpresses TfR using a TfR-binding peptide described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358).

The TfR-binding peptide construct of the disclosure is expressed recombinantly or chemically synthesized according to **EXAMPLE 1** and/or **EXAMPLE 2.** Optionally, the active agent used to treat the spleen cancer can be conjugated to, linked to, or fused to the TfR-binding peptide to form a TfR-binding peptide construct. The TfR-binding peptide construct is administered to a subject in need thereof. The TfR-binding peptide construct is administered intravenously, subcutaneously, intramuscularly, orally, or intratumorally. The subject is a human or an animal. After administration, the TfR-binding peptide construct accumulates in the spleen cancer as demonstrated by molecular imaging (e.g., CT, MRI) and/or the analysis of tissue sample. The TfR-binding peptide construct ameliorates the spleen cancer.

This data demonstrates that the TfR-binding peptides of the present disclosure effectively accumulate in TfR-expressing and/or TfR-overexpress cancers, such as spleen cancer, and thus can be used to treat and/or prevent a spleen cancer or other peripheral cancers.

### EXAMPLE 49

### TfR-binding Peptides for Treatment of a Bone Marrow Cancer

This example describes treatment of a cancer located in the bone marrow and that expresses and/or overexpresses TfR using a TfR-binding peptide described herein (e.g., any one of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358).

The TfR-binding peptide construct of the disclosure is expressed recombinantly or chemically synthesized according to **EXAMPLE 1** and/or **EXAMPLE 2.** Optionally, the active agent used to treat the bone marrow cancer can be conjugated to, linked to, or fused to the TfR-binding peptide to form a TfR-binding peptide construct. The TfR-binding peptide construct is administered to a subject in need thereof. The TfR-binding peptide construct is administered intravenously, subcutaneously, intramuscularly, orally, or intratumorally. The subject is a human or an animal. After administration, the TfR-binding peptide construct accumulates in the bone marrow cancer as demonstrated by molecular imaging (e.g., CT, MRI) and/or the analysis of tissue sample. The TfR-binding peptide construct ameliorates the bone marrow cancer.

This data demonstrates that the TfR-binding peptides of the present disclosure effectively accumulate in TfR-expressing and/or TfR-overexpress peripheral cancers, such as cancers of or located in the bone marrow, and thus can be used to treat and/or prevent a bone marrow cancer or other peripheral cancers.

### EXAMPLE 50

### Neurotensin Comprising Peptide Constructs, Delivery to CNS, and Activation of Neuronal CRE Reporter Mice

This example describes activation of neuronal CRE transporter mice using peptide constructs comprising one or more TfR-binding peptides as described herein. In this case, a fusion peptide comprising TfR-binding peptides and a neurotensin peptide was used. Peptides corresponding to SEQ ID NO: 1, SEQ ID NO: 2, and SEQ ID NO: 32 (SEQ ID NO: 1, SEQ ID NO: 2, and SEQ ID NO: 32 are SEQ ID NO: 36, SEQ ID NO: 37, and SEQ ID NO: 67, respectively, with an added N-terminal GS) were fused with neurotensin at the C-terminus of each peptide to produce the peptide-NT complexes SEQ ID NO: 33, SEQ ID NO: 34, and SEQ ID NO: 35, respectively (SEQ ID NO: 68, SEQ ID NO: 69, and SEQ ID NO: 70 with N-terminal GS, respectively). The downstream activity of neurotensin involves intracellular Ca²⁺ regulation and cAMP response element (CRE) driven transcriptional programs **(****FIG. 28A****),** and its modulation has been explored for suppression of chronic pain. CDP-NT peptide constructs **(****FIG. 27****)** that engage the neurotensin receptor were generated. SEQ ID NO: 33, SEQ ID NO: 34, and SEQ ID NO: 35 were expressed recombinantly in 293F cells as described in **EXAMPLE 1 - EXAMPLE 4** and purified. Purified peptide-NT fusions (e.g., SEQ ID NO: 33 - SEQ ID NO: 35) treated with DTT (a reducing agent) display a gel shift relative to the unreduced sample, suggesting that the recombinant NT peptides contain cystines **(****FIG. 27****).** Molecular weights of the purified peptides were verified using mass spectrometry.

Binding to the neurotensin receptor was demonstrated with a HEK-293 cell line expressing *NTSR1.* To demonstrate that the neurotensin extension on various proteins was functional, NTSR activity in HEK293 cells, or HEK293 cells transduced with a lentivector delivering human *NTSR1* (HEK293-NTSR1), was measured using the IP-One - Gq kit (CisBio 62IPAPEB, **FIG. 28B****).** Cells were grown in DMEM + 10% fetal bovine serum, removed from the plates with Accutase, pelleted, and suspended in Hanks Buffered Salt Solution at a density of 1.5X10⁶ cells per mL. HTFR reactions were set up in HTFR 96 well low volume plates (CisBio #66PL96025) according to the manufacturer's instructions. 10,000 cells (7 µL) were used per 25 µL reaction. The plate was incubated for 60 mins at 37°C. Then 3 µL IP1-d2 working solution was then added, followed by 3 µL Anti IP1-Cryptate working solution. After a 1 hour incubation at room temperature, the plate was scanned in a Perkin Elmer 2104 EnVision Multilabel Reader for fluorescence emission after excitement at 665 nm and 620 nm wavelengths. FRET ratio was calculated as10,000 x (Signal 665 nm / Signal 620 nm). In mammalian HEK-293 cells neurotensin (NT) receptor engagement showed IP₁ accumulation only in response to NT or NT peptide constructs (SEQ ID NO: 33 and SEQ ID NO: 35, as well as mTf-NT and NT, but not for SEQ ID NO: 1 or SEQ ID NO: 32, vehicle, or mTf), N = 3 wells for all except vehicle, which had N = 36 **(****FIG. 28B****).** Horizontal bar indicates sample mean.

Delivery of NT to the CNS was demonstrated with transgenic mice expressing firefly luciferase under the control of a cyclic AMP response element (CRE). Under conditions of elevated cyclic AMP (cAMP) or other mechanisms that activate the transcription factor CRE Binding Protein 1 (CREB), cells in these mice express luciferase, which are measured in whole animal luminescence instrumentation (IVIS imager) after IV dosage with a luciferin formulated for animal use. Neurotensin receptor expression was limited to subsets of cells in the CNS, including the frontal cortex. Upon activation, a signal transduction pathway is activated that culminates in CREB phosphorylation and CRE-mediated transcription. The endogenous ligand of the neurotensin receptor, neurotensin, is a neuropeptide that cannot cross the BBB by itself (demonstrated in **FIG. 32****,** mice dosed with 300 nmol NT or vehicle showed identical CRE-Luc-driven luminescence levels). Therefore, IV dosage of NT alone will not result in luciferase production in the brains of the CRE reporter mice. However, IV dosage of a fusion peptide comprising neurotensin and a TfR binding protein of the disclosure demonstrated BBB penetration as evinced by CRE-driven luciferase induction in the brains of the CRE reporter mice. All three of the of peptide-NT complexes (SEQ ID NO: 33, SEQ ID NO: 34, and SEQ ID NO: 35) tested exhibited delivery across the BBB into the brain, as determined by immunohistochemistry analysis, as well as functional engagement by NT on the NT receptor.

CRE-driven luciferase induction *in vivo* was verified by luciferin dosage four hours after administration of forskolin and rolipram **(****FIG. 31****),** potent CRE inducers via activation of adenylyl cyclase and inhibition of cAMP phosphodiesterase, respectively. CRE-luc GPCR reporter mice were administered 1.68 mg rolipram (Sigma Aldrich R6520) and 0.84 mg forskolin (Sigma Aldrich 344270) by intraperitoneal (IP) injection (200 µL in 17% DMSO solution), or TfR-binding peptide or other test and control peptides by intravenous tail vein injection; dosages were 100 nmol of TfR-binding peptides with or without NT (SEQ ID NO: 1, SEQ ID NO: 2, or SEQ ID NO: 32 - SEQ ID NO: 35) or 12 nmol transferrin or transferrin-NT (SEQ ID NO: 354 or SEQ ID NO: 355) in PBS. 4 hours after administration, mice were administered 100 µL of 30 mg/mL D-Luciferin (RediJect D-Luciferin Ultra, PerkinElmer 770505) by IP injection and anesthetized by isoflurane exposure after 10 minutes of unrestricted activity. Luminescence was measured in anesthetized mice on a Xenogen IVIS instrument with a 1 min exposure. For dosing with rolipram and forskolin as positive controls, high levels of luminescence from the animal's brain are seen in the miced dosed **(****FIG. 31****).** For the test peptides for comparison, identical regions of interest encompassing the entire brain were draw and quantified using LivingImage software (version 4.0, PerkinElmer, **FIG. 30****).** Mice were censored if their luminescence levels were > 4 SD from the mean / SD luminescence of the other mice in the cohort, or if their luciferin injections failed to hit the peritoneal cavity. For the former, this was interpreted as an incidental physiological response unrelated to drug treatment or NT receptor modulation. For the latter, this was identified by failure of a fluorescent dye (pre-formulated in the RediJect D-Luciferin Ultra, read on the ICG filter set over 5 seconds) to distribute evenly throughout the animal's abdomen; this indicates insufficient perfusion and incomplete exposure of the whole mouse to luciferin, rendering the animal's brain luminescence quantitation questionable. Animals that were validated in this assay were given 1 week to return to steady state (low) luciferase expression, and were then tested with neurotensin peptide or a fusion peptide comprising a TfR binding peptide fused to neurotensin.

Luminescence (via intraperitoneal luciferin dosage) was imaged either before ("Unstimulated") or four hours after intravenous administration of parent TfR-binding peptides(SEQ ID NO: 1, SEQ ID NO: 2, SEQ ID NO: 32, or SEQ ID NO: 354) or CDP-NT peptide constructs (SEQ ID NO: 33 - SEQ ID NO: 35), or the murine transferrin-NT construct(MRLAVGALLVCAVLGLCLADYKDEHHHHHHGLNDIFEAQKIEWHEGGGSVPDKTVK WCAVSEHENTKCISFRDHMKTVLPPDGPRLACVKKTSYPDCIKAISASEADAMTLDGGWVYDA GLTPNNLKPVAAEFYGSVEHPQTYYYAVAVVKKGTDFQLNQLEGKKSCHTGLGRSAGWVIPIGL LFCKLSEPRSPLEKAVSSFFSGSCVPCADPVAFPKLCQLCPGCGCSSTQPFFGYVGAFKCLKDGGG DVAFVKHTTIFEVLPEKADRDQYELLCLDNTRKPVDQYEDCYLARIPSHAVVARKNNGKEDLIW EILKVAQEHFGKGKSKDFQLFSSPLGKDLLFKDSAFGLLRVPPRMDYRLYLGHNYVTAIRNQQE GVCPEGSIDNSPVKWCALSHLERTKCDEWSIISEGKIECESAETTEDCIEKIVNGEADAMTLDGGH AYIAGQCGLVPVMAEYYESSNCAIPSQQGIFPKGYYAVAVVKASDTSITWNNLKGKKSCHTGVD RTAGWNIPMGMLYNRINHCKFDEFFSQGCAPGYEKNSTLCDLCIGPLKCAPNNKEEYNGYTGAF RCLVEKGDVAFVKHQTVLDNTEGKNPAEWAKNLKQEDFELLCPDGTRKPVKDFASCHLAQAPN HVVVSRKEKAARVKAVLTSQETLFGGSDCTGNFCLFKSTTKDLLFRDDTKCFVKLPEGTTPEKY LGAEYMQSVGNMRKCSTSRLLEACTFHKHGSSELYENKPRRPYIL, SEQ ID NO: 355), using matched cohorts **(****FIG. 30A****).** Transgenic Mice were first dosed with luciferin and luminescence was measured ("unstimulated"). Then the same mice were treated with either (i) the parent peptide (SEQ ID NO: 1, SEQ ID NO: 2, SEQ ID NO: 32, or SEQ ID NO: 354), and then dosed again with luciferin and luminescence was measured ("parent"), or (ii) the CDP-NT peptide constructs and murine transferrin-NT construct (SEQ ID NO: 355, SEQ ID NO: 33, SEQ ID NO: 34, SEQ ID NO: 35), and then dosed again with luciferin, and luminescence was measured ("NT fusion"). Quantitation is shown in **FIG. 30A****.** Horizontal bar indicates sample mean. Significance was determined using a T-test (unpaired, 2-tailed). (*: P < 0.05. **: P < 0.01. #: P < 0.0001.) Images for the SEQ ID NO: 32 cohort are show with pseudocolor luminescence in **FIG. 30B****.** The mice had a measurable basal level of CRE activity driving luciferase expression in the brain ("unstimulated" readings), and this was not increased by dosing of the parent peptides ("parent" readings). However, the level of CRE activity was significantly elevated in mice administered the peptide fusions comprising TfR-binding peptidesof this disclosure and NT, the CDP-NT peptide constructs of SEQ ID NO: 34 and SEQ ID NO: 35 **(****FIG. 30****).** The level of CRE activity was also elevated for mice treated with the CDP-NT peptide construct SEQ ID NO 33 and for the murine transferrin-NT fusion SEQ ID NO: 354, but was not statistically elevated as it was in the other CDP-NT peptide constructs (SEQ ID NO: 34 and SEQ ID NO: 35). Naked NT peptide alone, adminisetered at 300 nmol (three times the dose of the peptide-NT fusion constructs), fails to activate the reporter in the brain after intravenous administration **(****FIG. 32****),** as expected as it does not cross the BBB.Altogether, this demonstrates not only CNS accumulation, but BBB penetration and neuronal access of a molecule fused to SEQ ID NO: 1 variants, as well as engagement of neurotensin receptor by NT fusions of peptides of this disclosure.

The ability of peptides to deliver NT to areas of the CNS was confirmed by immunohistochemistry (IHC). By IHC staining, luciferase levels were visibly higher in mice treated with neurotensin variants of SEQ ID NO: 1, SEQ ID NO: 2, and SEQ ID NO: 32 (e.g., SEQ ID NO: 33 - SEQ ID NO: 35) **(****FIG. 29****),** particularly SEQ ID NO: 34 and SEQ ID NO: 35, whereas lower staining is visible in mice treated with Transferrin-NT (SEQ ID NO: 355) and much lower with mice that received no peptides (unstimulated). Formalin-fixed, paraffin-embedded brains were sectioned on a Microm HM 355S microtome at 6 µm and mounted on positively-charged Superfrost Plus slides (Fisher 12-550-15). Staining was automated on a Ventana Discovery Ultra IHC/ISH autostainer using kits and reagents designed for use on this instrument: EZ Prep deparaffinization reagents (Ventana 950-100), Protease 3 antigen retrieval kit (Ventana 760-2020), anti-rabbit HQ (Ventana 760-4815), anti-HQ-HRP (Ventana 760-4815), ChromoMap DAB kit (Ventana 760-159), hematoxylin (Ventana 760-2021), and bluing reagent (Ventana 760-2037), all at manufacturer's default settings. The primary anti-luciferase antibody was from Abcam (ab21176, concentration 1:350) and was diluted with Ventana antibody diluent with casein (Ventana 760-219). The sequence was as follows. Sections were deparaffinized at 69°C, and then antigen retrieval took place at 35°C for 4 mins. Slides were rinsed and warmed to 37°C prior to primary antibody addition by hand (1:350). Slides were incubated with the primary antibody for 28 minutes and rinsed. Subsequent antibody additions (followed by rinsing) were anti-rabbit HQ and then anti-HQ HRP, incubating 16 mins apiece. Slides were then DAB treated and counterstained with hematoxylin and bluing reagent (8 minutes apiece). Images in **FIG. 29** were mildly contrast-enhanced, with all image modifications performed identically across all images within a set without regard to treatment group. Immunohistochemistry showed enhanced luciferase expression in the cortex, striatum, and thalamus of mice 4 hours after CDP-NT administration. Scale bar (top left panel) is 100 µm **(****FIG. 29****).** All panels show the same magnification.

The peptide constructs of the present disclosure comprising a TfR binding peptide fused to neurotensin SEQ ID NO: 33 - SEQ ID NO: 35 induced CRE-driven luciferase in the brains of the CRE reporter mice, demonstrating that the TfR binding peptides of the present disclosure are capable of transporting active agents (e.g., peptides such as neurotensin) efficiently across the BBB that are otherwise not able to access the brain. Other peptide constructs of the present disclosure comprising a TfR binding peptide fused to neurotensin include SEQ ID NO: 68 - SEQ ID NO: 70, SEQ ID NO: 168 - SEQ ID NO: 170, and SEQ ID NO: 203- SEQ ID NO: 205 may also induce CRE-driven luciferase in the brains of CRE reporter mice.

Any peptide disclosed herein, (e.g., peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361, or peptides that can be fused to NT, such as the peptides of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) can be used fused to neurotensin to induce CRE-driven luciferase in the brains of the CRE reporter mice, demonstrating that the TfR binding peptides of the present disclosure are capable of transporting active agents (e.g., peptides such as neurotensin) efficiently across the BBB that are otherwise not able to access the brain.

This data demonstrates that the TfR-binding peptides of the present disclosure can provide therapeutically effective concentration of the NT receptor binding peptides in the CNS to effectively treat nociceptive or neuropathic pain. Any peptide disclosed herein (e.g., peptide-NT fusions of SEQ ID NO 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, or SEQ ID NO: 359 - SEQ ID NO: 361, or peptides that can be fused to NT, such as the peptides of SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358) can be used to transport neurotensin (NT) into the CNS. The peptides can be complexed to NT to form a CDP-NT peptide construct via recombinant fusion, chemical conjugation, or by other means.

### EXAMPLE 51

### Construction of the Cystine-Dense Peptide Library

This example describes the generation of the library used for screening peptides in **EXAMPLE 4** and **EXAMPLE 5.** The Uniprot database was screened for protein segments of 30-50 amino acids in length and containing 6, 8, or 10 cysteines. This list was narrowed with an emphasis on annotated knottins and defensins but otherwise with an eye on phylogenetic diversity, supplemented by a limited number of CDPs of interest from other sources, to a final library size of 10,000 CDPs. Oligonucleotides of the library containing appropriate flanking sequences for amplification and cloning were ordered (Twist Bioscience), amplified (Phusion^{®} High-Fidelity DNA Polymerase, NEB), and mutagenized (GeneMorph II Random Mutagenesis Kit, Agilent; 22 cycles of amplification) prior to Gibson Assembly cloning into BamHI / NotI digested SDGF vector (GenBank MF958494). The library was transformed into Stellar competent cells (Clontech), maxiprepped (QIAgen), and made into lentivirus (VSV-G pseudotyped, produced by standard methods in 293T cells using the envelope plasmid pMD2.G and the packaging plasmid psPAX). Singleton CDP candidates that required individual cloning were cloned from dsDNA (gBlock, Integrated DNA Technologies) into SDGF and tested for binding 2 days after transient transfection (2.5 µg plasmid transfected with 4 µg polyethyleneimine (Polysciences 23966-1) into 2×10⁶ 293F cells in 1 mL FreeStyle media, shaking in 24-well suspension plates as described above. Testing consisted of staining with TfR and dye-labeled streptavidin, either together or sequentially (TfR followed by pelleting and resuspension in streptavidin solution) in Flow Buffer with 1 µg mL⁻¹ DAPI, and then analyzed on an Acea NovoCyte flow cytometer.

### EXAMPLE 52

### Treatment or Management of Pain with a Peptide-Active Agent Construct

This example describes a method for treating or managing pain. This method is used as a treatment for acute and/or chronic symptoms. A peptide of the disclosure is expressed and administered in a pharmaceutical composition to a patient as a therapeutic for pain as a result of injury or other condition as described herein. The peptide of the present disclosure inhibits ion channels, such as Naᵥ 1.7. The peptide is expressed recombinantly or chemically synthesized, wherein the peptide selected from a TfR-binding peptide as described herein (e.g., SEQ ID NO: 1 - SEQ ID NO: 32, SEQ ID NO: 36 - SEQ ID NO: 67, SEQ ID NO: 136 - SEQ ID NO: 167, SEQ ID NO: 171 - SEQ ID NO: 202, or SEQ ID NO: 356 - SEQ ID NO: 358). Peptides are mutated to maintain the TfR-binding function, but to add or increase ion channel inhibition, such as to Naᵥ 1.7. Alternatively, rather than the peptide intereacting with ion channels such as Nav 1.7, following expression or synthesis, the peptide is conjugated to an active agent. In some aspects, the active agent is an NSAID pain reliever. In some aspects, the active agent is lidocaine. In some aspects, the active agent is a Naᵥ 1.7 ion channel inhibitor. The peptide-active agent conjugate is optionally purified using liquid chromatography (e.g., reversed-phase, ion exchange, or size-exclusion chromatograph). The peptide construct is formulated for administration and administered to the subject. Following administration, the peptide active-agent conjugate targets to the area, tissue, or system affected by pain. The peptide itself can be conjugated to an agent that modulates pain (e.g., an NSAID pain reliever). Such pain modulation may operate by various mechanisms such as modulating inflammation, autoimmune responses, direct or indirect action on pain receptors, cell killing, or programmed cell death. One or more peptides are administered to a human or animal subcutaneously, intravenously, or orally, or are injected.

### EXAMPLE 53

### Extension of Peptide Plasma Half-Life Using Serum Albumin-Binding Peptide Constructs

This example demonstrates a method of extending the serum or plasma half-life of a peptide using serum albumin-binding peptide constructs as disclosed herein. A peptide or peptide cosntruct having a sequence of any one of SEQ ID NO: 1 - SEQ ID NO: 70, SEQ ID NO: 136 - SEQ ID NO: 205, SEQ ID NO: 225 - SEQ ID NO: 332, or SEQ ID NO: 356 - SEQ ID NO: 361 is modified in order to increase its plasma half-life. The peptide and the serum half-life extending moietiy are fused recombinantly, chemically synthesized as a single fusion, separately recombantly expressed and conjugated, or separately chemically synthesized and conjugated. Fusing the peptide to a serum albumin-binding peptide extends the serum half-life of the peptide construct. The peptide or peptide construct is conjugated to a serum albumin-binding peptide, such as SA21 (SEQ ID NO: 376). Optionally, the peptide fused to SA21 has a sequence of any one of SEQ ID NO: 373 or SEQ ID NO: 375. Optionally, the peptide fused to SA21 is linked to SA21 via a peptide linker having a sequence of SEQ ID NO: 377. The linker having a sequence corresponding to SEQ ID NO: 377 links two separately functional CDPs to incporporate serum half-life extension function into the peptide or peptide construct. The linker having a sequence corresponding to SEQ ID NO: 377 enables SA21 to cyclize without steric impediment from either member of the peptide construct. Alternatively, conjugation of the peptide to an albumin binder, such as Albu-tag or a fatty acid, such as a C14-C18 fatty acid, is used to extend plasma half-life. Plasma half-life is also optionally extended as a result of reduced immunogenicity by using minimal non-human protein sequences.

### EXAMPLE 54

### Comparison of Dose Toxicity of a TfR-binding Peptide to a TfR-binding Antibody

This example describes the comparison of the dose toxicity of a TfR-binding peptide of this disclosure to anti-TfR antibody when administered to a murine subject. An anti-TfR antibody is administered to a subject at doses of 5 mg/kg, 25 mg/kg or 50 mg/kg, corresponding to molar doses per 25 g mouse mass of about 0.84 nmol, 4.2 nmol, and 8.4 nmol, respectively, as described in Couch, et al, 2013 (Couch et al, Sci Transl Med. 2013 May 1;5(183):183ra57). A TfR-binding peptide of this disclosure is administered to a subject at doses of about 31 mg/kg, corresponding to a molar concentration of about 100 nmol per 25 g mouse mass. Alternatively, a TfR-binding peptide of this disclosure is administered to a subject at doses of 0.84 nmol, 4.2 nmol and 8.4 nmol or 100 nmol. Subjects receiving 31 mg/kg, or about 100 nmol per 25 g mouse mass, of the TfR-binding peptide show effective pharmacodynamic and pharmacokinetic properties without signs of distress or toxicity over the course of at least 24 hours. When further fused to an active agent, such as neurotensin (NT), the TfR-binding peptides exhibits effective therapeutic efficacy, such as effective pharmacodynamic induction of CRE activity as assessed in CRE-luciferase model mice with no observed toxicity. Meanwhile subjects receiving 5 mg/kg, or about 0.84 nmol per 25 g mouse mass, of the anti-TfR antibody in bispecific format with a BACE inhibitory antibody show reduced therapeutic efficacy, such as reduced pharmacodynamic amyloid beta inhibition, as compared to the subjects receiving 25 or 50 mg/kg, or 4.2 or 0.4 nmol per 25 kg mouse mass. The 25 or 50 mg/kg, or 4.2 or 0.4 nmol per 25 kg mouse mass, doses of the anti-TfR antibody induce lethargy, distress, and hemolysis, or reduced reticulocyte count or other toxicities, within at least 30 minutes of administration. The results demonstrate that the therapeutic window (the dosage above which a therapeutic pharmacodynamic response is seen but below which toxicity is observed) is wider for peptide-based therapeutics than for antibody-based therapeutics. The results further demonstrate that TfR-binding peptide-based therapeutics show less off-target binding and lower immune response as compared to TfR-binding antibody-based therapeutics, due to the smaller protein lengths (approximately 50 amino acids) providing fewer epitopes for an adaptive immune response and smaller surface area.

### EXAMPLE 55

### Purification of a TfR-Binding Serum Albumin-Binding Peptide Fusion

This example describes the purification of a TfR-binding peptide fused to the serum albumin-binding peptide SA21. **FIG. 34** illustrates the purification of SA21 fusion peptides. SA21 was recombinantly expressed as a fusion peptide with a CDP and purified by HPLC. Peptides were purified fused to siderocalin ("Scn-CDP") and and cleaved to produce the cleaved SA21 fusion peptide ("CDP") and siderocalin ("Scn"). **FIG. 34A** shows purification of a peptide TfR-binding peptide fused to a serum albumin peptide (SA21) corresponding to SEQ ID NO: 373. Purity was verified by SDS-PAGE (left) and RP-HPLC (right) under DTT reducing ("R") or non-reducing ("NR") conditions. SDS-PAGE was also run on the uncleaved ("U") siderocalin-CDP fusion peptide. In the SDS-PAGE, distinct bands were seen corresponding to the siderocalin-CDP fusion ("Scn-CDP") in the uncleaved ("U") sample, as well as bands corresponding to the cleaved SA21 fusion ("CDP"), siderocalin alone ("Scn"), and the uncleaved fusion ("Scn-CDP") in the reduced ("R") and non-reduced ("NR") samples. The presence of a single peak in the unreduced RP-HPLC trace was indicative of a pure, undegraded sample. FIG. 34B shows purification of a peptide fused to SA21 corresponding to SEQ ID NO: 456. Purity was verified by SDS-PAGE (left) and RP-HPLC (right) under DTT reducing ("R") or non-reducing ("NR") conditions. SDS-PAGE was also run on the uncleaved ("U") siderocalin-CDP fusion peptide. In the SDS-PAGE, distinct bands were seen corresponding to the siderocalin-CDP fusion ("Scn-CDP") in the uncleaved ("U") sample, as well as bands corresponding to the cleaved SA21 fusion ("CDP"), siderocalin alone ("Scn"), and the uncleaved fusion ("Scn-CDP") in the reduced ("R") and non-reduced ("NR") samples. The presence of a single peak in the unreduced RP-HPLC trace was indicative of a pure, undegraded sample.

## Claims

1. A composition comprising an engineered transferrin-binding peptide, wherein the engineered transferrin-binding peptide comprises a sequence having:
a) at least 90% sequence identity with SEQ ID NO: 67, or a fragment thereof comprising at least 29 amino acid residues;
b) at least 90% sequence identity with any one of SEQ ID NO: 37, SEQ ID NO: 202, SEQ ID NO: 172, or SEQ ID NO: 171, or a fragment thereof comprising at least 29 amino acid residues;
c) at least 90% sequence identity with any one of SEQ ID NO: 32, SEQ ID NO: 2, SEQ ID NO: 167, SEQ ID NO: 137, or SEQ ID NO: 136, or a fragment thereof comprising at least 29 amino acid residues; or
d) at least 95% sequence identity with any one of SEQ ID NO: 36, SEQ ID NO: 1, or a fragment thereof comprising at least 29 amino acid residues.

2. The composition of claim 1, wherein the engineered transferrin-binding peptide comprises a sequence of:
a) any one of SEQ ID NO: 339 - SEQ ID NO: 344;
b) SEQ ID NO: 67;
c) SEQ ID NO: 37, SEQ ID NO: 202, SEQ ID NO: 172, SEQ ID NO: 36, or SEQ ID NO: 171; or
d) SEQ ID NO: 32, SEQ ID NO: 2, SEQ ID NO: 167, SEQ ID NO: 137, SEQ ID NO: 1, or SEQ ID NO: 136.

3. The composition of claim 1 or claim 2, wherein the engineered transferrin-binding peptide comprises:
a) at least four cysteine residues;
b) at least two disulfide bonds;
c) at least 43 amino acid residues; or
d) combinations thereof.

4. The composition of any one of claims 1-3, further comprising an active agent or a detectable agent conjugated to, linked to, or fused to, or grafted onto the engineered transferrin-binding peptide; optionally, wherein the active agent, the detectable agent, or both are linked to the engineered transferrin-binding peptide via a linker.

5. The composition of claim 4, wherein the active agent is an immunotherapeutic agent, a CTLA-4 targeting agent, a PD-1 targeting agent, a PDL-1 targeting agent, an IL15 agent, a fused IL-15/IL-15Ra complex agent, an IFNgamma agent, an anti-CD3 agent, an ion channel modulator, a Kv1.3 inhibitor, an auristatin, MMAE, a maytansinoid, DM1, DM4, doxorubicin, a calicheamicin, a platinum compound, cisplatin, a taxane, paclitaxel, SN-38, a BACE inhibitor, a Bcl-xL inhibitor, WEHI-539, venetoclax, ABT-199, navitoclax, AT-101, obatoclax, a pyrrolobenzodiazepine or pyrrolobenzodiazepine dimer, a dolastatin, a neurotransmitter, an antiinflammatory agent, an immune modulator, a tumor necrosis factor inhibitor, a tumor necrosis receptor antagonist, a checkpoint inhibitor, a CGRP receptor antagonist, a cytotoxic molecule, a tyrosine kinase inhibitor, an EGFRvIII inhibitor, or an oligonucleotide.

6. The composition of claim 5, wherein:
a) the Kv1.3 inhibitor is Vm24, ShK-170, ShK-186, or ShK-192;
b) the Vm24 has a sequence of SEQ ID NO: 378 or SEQ ID NO: 391; or wherein the Shk-185 has a sequence of SEQ ID NO: 379, SEQ ID NO: 390, or SEQ ID NO: 402; or
c) the neurotransmitter is neurotensin, a neurotensin peptide variant, or a fragment thereof comprising at least 8 amino acid residues, at least 10 amino acid residues, or at least 13 amino acid residues; optionally, wherein the neurotensin peptide variant comprises a sequence of any one of SEQ ID NO: 350 or SEQ ID NO: 365 - SEQ ID NO: 369.

7. The composition of any one of claims 4-6, wherein the detectable agent is a fluorophore, a near-infrared dye, a contrast agent, a nanoparticle, a metal-containing nanoparticle, a metal chelate, an X-ray contrast agent, a PET agent, a radionuclide, or a radionuclide chelator.

8. The composition of any one of claims 4-7, wherein the linker is selected from TABLE 7 or any one of SEQ ID NO: 103 - SEQ ID NO: 115, or SEQ ID NO: 125.

9. The composition of any one of claims 1-8, further comprising a half-life modifying agent, a cell-penetrating moiety, nuclear localization signal, or a combination thereof coupled to the engineered transferrin-binding peptide.

10. The composition of claim 9, wherein:
a) the half-life modifying agent comprises a polymer, a polyethylene glycol (PEG), a hydroxyethyl starch, polyvinyl alcohol, a water soluble polymer, a zwitterionic water soluble polymer, a water soluble poly(amino acid), a water soluble polymer of proline, alanine and serine, a water soluble polymer containing glycine, glutamic acid, and serine, an Fc region, a fatty acid, palmitic acid, a molecule that binds to albumin, or SA21;
b) the cell-penetrating moiety comprises a polycation, a polyorganic acid, an endosomal releasing polymers, poly(2-propylacrylic acid), poly(2-ethylacrylic acid), a Tat peptide, an Arg patch, a knotted peptide, CysTAT, S19-TAT, R8 (SEQ ID NO: 73), pAntp, Pas-TAT, Pas-R8 (SEQ ID NO: 76), Pas-FHV, Pas-pAntP, F2R4 (SEQ ID NO: 79), B55, aurein, IMT-P8, BR2, OMOTAG1, OMOTAG2, pVEC, SynB3, DPV1047, C105Y, Transportan, MTS, hLF, PFVYLI (SEQ ID NO: 93), maurocalcine, imperatoxin, hadrucalin, hemicalcin, opicalcin-1, opicalcin-2, midkine (62-104), MCoTI-II, chlorotoxin, DRI-TAT, cFΦR4 (SEQ ID NO: 96), R6W3 (SEQ ID NO: 421), myristate, yBBR, or any combination thereof;
c) the cell-penetrating moiety comprises a sequence of any one of SEQ ID NO: 71 - SEQ ID NO: 96, SEQ ID NO: 98 - SEQ ID NO: 101, SEQ ID NO: 116 - SEQ ID NO: 126, or SEQ ID NO: 403 - SEQ ID NO: 455; or
d) the nuclear localization signal comprises a sequence of any one of SEQ ID NO: 129 - SEQ ID NO: 135.

11. The composition of any one of claims 1-10 wherein the engineered transferrin-binding peptide comprises a sequence of any one of SEQ ID NO: 33 - SEQ ID NO: 35, SEQ ID NO: 68 - SEQ ID NO: 70, SEQ ID NO: 168 - SEQ ID NO: 170, SEQ ID NO: 203 - SEQ ID NO: 205, SEQ ID NO: 373, or SEQ ID NO: 375.

12. A composition of any one of claims 1-11 for use in a method of treating a condition in a subject in need thereof, the method comprising:
administering to the subject the composition;
delivering the composition to a cellular layer of the subject;
binding the engineered transferrin-binding peptide to a transferrin receptor (TfR); and
transporting the composition across the cellular layer, thereby treating the condition.

13. The composition for use of claim 12, wherein the condition is schizophrenia, drug abuse, Parkinson's disease, high blood pressure, an eating disorder, stroke, Alzheimer's disease, cancer, inflammation, pain, epilepsy, a mood disorder, an endocrine disorder, a CNS disorder, a neurodegenerative disorder, a neuroinflammatory disease, a brain autoimmune disease, amyotrophic lateral sclerosis, Huntington's disease, multiple sclerosis, a traumatic brain injury, Crohn's disease, an inflammatory bowel disease, or a muscle disease; optionally wherein
a) the pain is acute pain, chronic pain, or neuropathic pain; or
b) the cancer is brain cancer, a primary CNS tumor, glioblastoma, glioma, diffuse intrinsic pontine glioma, astrocytoma, medulloblastoma, breast cancer, lung cancer, head and neck cancer, liver cancer, kidney cancer, spleen cancer, prostate cancer, pancreatic cancer, skin cancer, colorectal cancer, colon cancer, squamous cell carcinoma, leukemia, lymphoma, or bone marrow cell cancer.

14. The composition for use of any one of claims 12-13, further comprising delivering an active agent across the cellular layer; optionally, wherein the cellular layer is a blood brain barrier.

15. The composition of claim 4, wherein the active agent is an anti-cancer drug_selected from the group consisting of an EGFRvIII inhibitor, Copiktra (duvelisib), Erleada (apalutamide), Libtayo (cemiplimab-rwlc), Lorbrena (lorlatinib), Lumoxiti (moxetumomab pasudotox-tdf), Lutathera (lutetium Lu 177 dotatate), Talzenna (talazoparib), Vizimpro (dacomitinib), Aliqopa (copanlisib), Alunbrig (brigatinib), Bavencio (avelumab), Besponsa (inotuzumab ozogamicin), Calquence (acalabrutinib), IDHIFA (enasidenib), Imfinzi (durvalumab), Kisqali (ribociclib), Kymriah (tisagenlecleucel), Nerlynx (neratinib), Rydapt (midostaurin),Vyxeos (daunorubicin and cytarabine), Xermelo (telotristat ethyl), Yescarta (axicabtagene ciloleucel), Zejula (niraparib), Cabometyx (cabozantinib), Keytruda (pembrolizumab), Lartruvo (olaratumab), Lenvima (lenvatinib), Opdivo (nivolumab), Rubraca (rucaparib), Sustol (granisetron), Syndros (dronabinol oral solution), Tecentriq (atezolizumab), Venclexta (venetoclax), Alecensa (alectinib), Cotellic (cobimetinib), Darzalex (daratumumab), Empliciti (elotuzumab), Farydak (panobinostat), Ibrance (palbociclib), Imlygic (talimogene laherparepvec), Lenvima (lenvatinib), Lonsurf (trifluridine and tipiracil), Ninlaro (ixazomib), Odomzo (sonidegib), Onivyde (irinotecan liposome injection), Portrazza (necitumumab), Tagrisso (osimertinib), Unituxin (dinutuximab), Varubi (rolapitant), Vistogard (uridine triacetate), Yondelis (trabectedin), Akynzeo (netupitant and palonosetron), Beleodaq (belinostat), Blincyto (blinatumomab), Cyramza (ramucirumab), Imbruvica (ibrutinib), Lynparza (olaparib), Zydelig (idelalisib), Zykadia (ceritinib), Gazyva (obinutuzumab), Gilotrif (afatinib), Imbruvica (ibrutinib), Kadcyla (ado-trastuzumab emtansine), Mekinist (trametinib), Pomalyst (pomalidomide), Revlimid (lenalidomide), Stivarga (regorafenib), Tafinlar (dabrafenib), Valchlor (mechlorethamine) gel, Xgeva (denosumab), Xofigo (radium Ra 223 dichloride), Abraxane (paclitaxel protein-bound particles for injectable suspension), Afinitor (everolimus), Afinitor (everolimus), Bosulif (bosutinib), Cometriq (cabozantinib), Erivedge (vismodegib), Iclusig (ponatinib), Inlyta (axitinib), Kyprolis (carfilzomib), Marqibo (vinCRIStine sulfate LIPOSOME injection), Neutroval (tbo-filgrastim), Perjeta (pertuzumab), Picato (ingenol mebutate) gel, Stivarga (regorafenib), Subsys (fentanyl sublingual spray), Synribo (omacetaxine mepesuccinate), Votrient (pazopanib), Xtandi (enzalutamide), Zaltrap (ziv-aflibercept), Abstral (fentanyl sublingual tablets), Adcetris (brentuximab vedotin), Afinitor (everolimus), Erwinaze (asparaginase Erwinia chrysanthemi), Lazanda (fentanyl citrate) nasal spray, Sutent (sunitinib malate), Sylatron (peginterferon alfa-2b), Vandetanib (vandetanib), Xalkori (crizotinib), Yervoy (ipilimumab), Zelboraf (vemurafenib), Zytiga (abiraterone acetate), Halaven (eribulin mesylate), Herceptin (trastuzumab), Jevtana (cabazitaxel), Provenge (sipuleucel-T), Xgeva (denosumab), Zuplenz (ondansetron oral soluble film), Afinitor (everolimus), Arzerra (ofatumumab), Avastin (bevacizumab), Cervarix [Human Papillomavirus Bivalent (Types 16 and 18) Vaccine, Recombinant, Elitek (rasburicase), Folotyn (pralatrexate injection), Istodax (romidepsin), Onsolis (fentanyl buccal), Votrient (pazopanib), Degarelix (degarelix for injection), Fusilev (levoleucovorin); Mozobil (plerixafor injection), Sancuso (granisetron), Treanda (bendamustine hydrochloride), Evista (raloxifene hydrochloride), Hycamtin (topotecan hydrochloride), Ixempra (ixabepilone), Tasigna (nilotinib hydrochloride monohydrate), Torisel (temsirolimus), Tykerb (lapatinib), Gardasil (quadrivalent human papillomavirus (types 6, 11, 16, 18) recombinant vaccine), Sprycel (dasatinib), Sutent (sunitinib), Vectibix (panitumumab), Arranon (nelarabine), Nexavar (sorafenib), Alimta (pemetrexed for injection), Avastin (bevacizumab); Clolar (clofarabine), Erbitux (cetuximab), Sensipar (cinacalcet), Tarceva (erlotinib, OSI 774), Aloxi (palonosetron), Emend (aprepitant), Iressa (gefitinib); Plenaxis (abarelix for injectable suspension); Premarin (conjugated estrogens); UroXatral (alfuzosin HCl extended-release tablets); Velcade (bortezomib); Eligard (leuprolide acetate); Eloxatin (oxaliplatin/5-fluorouracil/leucovorin); Faslodex (fulvestrant); Gleevec (imatinib mesylate); Neulasta; SecreFlo (secretin); Zevalin (ibritumomab tiuxetan); Zometa (zoledronic acid); Campath; Femara (letrozole); Gleevec (imatinib mesylate); Kytril (granisetron) solution; Trelstar LA (triptorelin pamoate); Xeloda; Zometa (zoledronic acid); Mylotarg (gemtuzumab ozogamicin); Trelstar Depot (triptorelin pamoate);Trisenox (arsenic trioxide); Viadur (leuprolide acetate implant); Aromasin Tablets; Busulflex; Doxil (doxorubicin HCl liposome injection); Ellence; Ethyol (amifostine); Temodar; UVADEX Sterile Solution; Zofran; Actiq; Anzemet; Camptosar; Gemzar (gemcitabine HCL); Herceptin; Inform HER-2/neu breast cancer test; Neupogen; Nolvadex; Photofrin; Proleukin; Sclerosol Intrapleural Aerosol; Valstar; Xeloda; Zofran; Anzemet; Bromfenac; Femara (letrozole); Gliadel Wafer (polifeprosan 20 with carmustine implant); Intron A (interferon alfa-2b, recombinant); Kytril (granisetron) tablets; Lupron Depot (leuprolide acetate for depot suspension); Miraluma test; Neumega; Quadramet (Samarium Sm 153 Lexidronam Injection); Rituxan; Taxol; Anexsia; Aredia (pamidronate disodium for injection); Arimidex (anastrozole); Campostar; Elliotts B Solution (buffered intrathecal electrolyte/dextrose injection); Eulexin (flutamide); Gemzar (gemcitabine HCL); Hycamtin (topotecan hydrochloride); Kadian; Leukine (sargramostim); Lupron Depot (leuprolide acetate for depot suspension); Photodynamic Therapy; Taxotere (Docetaxel); Visipaque (iodixanol); Ethyol (amifostine); Intron A (Interferon alfa-2b, recombinant), and Leukine (sargramostim).

## Patentansprüche

1. Zusammensetzung, umfassend ein gentechnisch verändertes Transferrin-bindendes Peptid, wobei das gentechnisch veränderte Transferrin-bindende Peptid eine Sequenz umfasst, die Folgendes aufweist:
a) eine Sequenzidentität mit SEQ ID NO: 67 von mindestens 90 %, oder ein Fragment davon, umfassend mindestens 29 Aminosäurereste;
b) eine Sequenzidentität mit einer beliebigen von SEQ ID NO: 37, SEQ ID NO: 202, SEQ ID NO: 172 oder SEQ ID NO: 171 von mindestens 90 %, oder ein Fragment davon, umfassend mindestens 29 Aminosäurereste;
c) eine Sequenzidentität mit einer beliebigen von SEQ ID NO: 32, SEQ ID NO: 2, SEQ ID NO: 167, SEQ ID NO: 137 oder SEQ ID NO: 136 von mindestens 90 %, oder ein Fragment davon, umfassend mindestens 29 Aminosäurereste; oder
d) eine Sequenzidentität mit einer beliebigen von SEQ ID NO: 36, SEQ ID NO: 1 von mindestens 95 %, oder ein Fragment davon, umfassend mindestens 29 Aminosäurereste.

2. Zusammensetzung nach Anspruch 1, wobei das gentechnisch veränderte Transferrin-bindende Peptid eine Sequenz umfasst von:
a) einer beliebigen von SEQ ID NO: 339-SEQ ID NO: 344;
b) SEQ ID NO: 67;
c) SEQ ID NO: 37, SEQ ID NO: 202, SEQ ID NO: 172, SEQ ID NO: 36 oder SEQ ID NO: 171; oder
d) SEQ ID NO: 32, SEQ ID NO: 2, SEQ ID NO: 167, SEQ ID NO: 137, SEQ ID NO: 1 oder SEQ ID NO: 136.

3. Zusammensetzung nach Anspruch 1 oder Anspruch 2, wobei das gentechnisch veränderte Transferrin-bindende Peptid Folgendes umfasst:
a) mindestens vier Cysteinreste;
b) mindestens zwei Disulfidbindungen;
c) mindestens 43 Aminosäurereste; oder
d) Kombinationen davon.

4. Zusammensetzung nach einem der Ansprüche 1-3, ferner umfassend einen Wirkstoff oder eine nachweisbare Substanz, der/die an das gentechnisch veränderte Transferrin-bindende Peptid konjugiert, gebunden, mit diesem fusioniert oder auf dieses gepfropft ist; wobei der Wirkstoff, das nachweisbare Mittel oder beide gegebenenfalls über einen Linker an das gentechnisch veränderte Transferrin-bindenden Peptid gebunden sind.

5. Zusammensetzung nach Anspruch 4, wobei der Wirkstoff eine immuntherapeutische Substanz, eine gegen CTLA-4 gerichtete Substanz, eine gegen PD-1 gerichtete Substanz, eine gegen PDL-1 gerichtete Substanz, eine IL15-Substanz, eine IL-15/IL-15Ra-Fusionskomplex-Substanz, eine IFNgamma-Substanz, ein Anti-CD3-Substanz, ein Ionenkanalmodulator, ein Kv1.3-Inhibitor, ein Auristatin, MMAE, ein Maytansinoid, DM1, DM4, Doxorubicin, ein Calicheamicin, eine Platinverbindung, ein Taxan, Paclitaxel, SN-38, ein BACE-Inhibitor, ein Bcl-xL-Inhibitor, WEHI-539, Venetoclax, ABT-199, Navitoclax, AT-101, Obatoclax, ein Pyrrolobenzodiazepin- oder Pyrrolobenzodiazepin-Dimer, ein Dolastatin, ein Neurotransmitter, eine antiinflammatorische Substanz, ein Immunmodulator, ein Tumornekrosefaktor-Inhibitor, ein Tumornekroserezeptor-Antagonist, ein Checkpoint-Inhibitor, ein CGRP-Rezeptorantagonist, ein zytotoxisches Molekül, ein Tyrosinkinase-Inhibitor, ein EGFRvIII-Inhibitor oder ein Oligonukleotid ist.

6. Zusammensetzung nach Anspruch 5, wobei:
a) der Kv1.3-Inhibitor Vm24, ShK-170, ShK-186 oder ShK-192 ist;
b) das Vm24 eine Sequenz von SEQ ID NO: 378 oder SEQ ID NO: 391 aufweist; oder wobei das Shk-185 eine Sequenz von SEQ ID NO: 379, SEQ ID NO: 390 oder SEQ ID NO: 402 aufweist; oder
c) der Neurotransmitter Neurotensin, eine Neurotensin-Peptidvariante oder ein Fragment davon ist, umfassend mindestens 8 Aminosäurereste, mindestens 10 Aminosäurereste oder mindestens 13 Aminosäurereste; wobei die Neurotensin-Peptidvariante gegebenenfalls eine Sequenz von einer beliebigen von SEQ ID NO: 350 oder SEQ ID NO: 365-SEQ ID NO: 369 umfasst.

7. Zusammensetzung nach einem der Ansprüche 4-6, wobei die nachweisbare Substanz ein Fluorophor, ein Nahinfrarotfarbstoff, ein Kontrastmittel, ein Nanopartikel, ein metallhaltiges Nanopartikel, ein Metallchelat, ein Röntgenkontrastmittel, eine PET-Substanz, ein Radionuklid oder ein Radionuklidchelator ist.

8. Zusammensetzung nach einem der Ansprüche 4-7, wobei der Linker ausgewählt ist aus Tabelle 7 oder einer von SEQ ID NO: 103-SEQ ID NO: 115 oder SEQ ID NO: 125.

9. Zusammensetzung nach einem der Ansprüche 1-8, ferner umfassend eine Halbwertszeit-modifizierende Substanz, eine zellpenetrierende Einheit, ein nukleares Lokalisierungssignal oder eine Kombination davon, gekoppelt an das gentechnisch veränderte Transferrin-bindende Peptid.

10. Zusammensetzung nach Anspruch 9, wobei:
a) die Halbwertszeit-modifizierende Substanz ein Polymer, ein Polyethylenglykol (PEG), eine Hydroxyethylstärke, Polyvinylalkohol, ein wasserlösliches Polymer, ein zwitterionisches wasserlösliches Polymer, eine wasserlösliche Poly(aminosäure), ein wasserlösliches Polymer aus Prolin, Alanin und Serin, ein wasserlösliches Polymer enthaltend Glycin, Glutaminsäure und Serin, eine Fc-Region, eine Fettsäure, Palmitinsäure, ein an Albumin bindendes Molekül oder SA21 umfasst;
b) die zellpenetrierende Einheit ein Polykation, eine polyorganische Säure, ein endosomal freisetzendes Polymer, Poly(2-propylacrylsäure), Poly(2-ethylacrylsäure), ein Tat-Peptid, ein Arg-Patch, ein geknotetes Peptid, CysTAT, S19-TAT, R8 (SEQ ID NO: 73), pAntp, Pas-TAT, Pas-R8 (SEQ ID NO: 76), Pas-FHV, Pas-pAntP, F2R4 (SEQ ID NO: 79), B55, Aurein, IMT-P8, BR2, OMOTAG1, OMOTAG2, pVEC, SynB3, DPV1047, C105Y, Transportan, MTS, hLF, PFVYLI (SEQ ID NO: 93), Maurocalcin, Imperatoxin, Hadrucalin, Hemicalcin, Opicalcin-1, Opicalcin-2, Midkin (62-104), MCoTI-II, Chlortoxin, DRI-TAT, cFΦR4 (SEQ ID NO: 96), R6W3 (SEQ ID NO: 421), Myristat, yBBR oder eine beliebige Kombination davon umfasst;
c) die zellpenetrierende Einheit eine Sequenz von einem beliebigen von SEQ ID NO: 71-SEQ ID NO: 96, SEQ ID NO: 98-SEQ ID NO: 101, SEQ ID NO: 116-SEQ ID NO: 126 oder SEQ ID NO: 403-SEQ ID NO: 455 umfasst; oder
d) das nukleare Lokalisierungssignal eine Sequenz einer beliebigen von SEQ ID NO: 129-SEQ ID NO: 135 umfasst.

11. Zusammensetzung nach einem der Ansprüche 1-10, wobei das gentechnisch veränderte Transferrin-bindende Peptid eine Sequenz von einer beliebigen von SEQ ID NO: 33-SEQ ID NO: 35, SEQ ID NO: 68-SEQ ID NO: 70, SEQ ID NO: 168-SEQ ID NO: 170, SEQ ID NO: 203-SEQ ID NO: 205, SEQ ID NO: 373 oder SEQ ID NO: 375 umfasst.

12. Zusammensetzung nach einem der Ansprüche 1-11 zur Verwendung in einem Verfahren zum Behandeln eines Zustands bei einem Subjekt, das dies benötigt, wobei das Verfahren Folgendes umfasst:
Verabreichen der Zusammensetzung an das Subjekt;
Abgeben der Zusammensetzung an eine zelluläre Schicht des Subjekts;
Binden des gentechnisch veränderten Transferrin-bindenden Peptids an einen Transferrin-Rezeptor (TfR); und
Transportieren der Zusammensetzung über die zelluläre Schicht, wodurch der Zustand behandelt wird.

13. Zusammensetzung zur Verwendung nach Anspruch 12, wobei der Zustand Schizophrenie, Drogenmissbrauch, Morbus Parkinson, Bluthochdruck, eine Essstörung, Schlaganfall, Morbus Alzheimer, Krebs, Entzündung, Schmerz, Epilepsie, eine affektive Störung, eine endokrine Störung, eine ZNS-Störung, eine neurodegenerative Störung, eine neuroinflammatorische Krankheit, eine Autoimmunkrankheit des Gehirns, amyotrophe Lateralsklerose, Chorea Huntington, multiple Sklerose, eine traumatische Hirnverletzung, Morbus Crohn, eine entzündliche Darmkrankheit oder eine Muskelkrankheit ist; wobei gegebenenfalls
a) der Schmerz akuter Schmerz, chronischer Schmerz oder neuropathischer Schmerz ist; oder
b) der Krebs Hirnkrebs, ein primärer ZNS-Tumor, Glioblastom, Gliom, diffuses intrinsisches Ponsgliom, Astrozytom, Medulloblastom, Brustkrebs, Lungenkrebs, Kopf- und Halskrebs, Leberkrebs, Nierenkrebs, Milzkrebs, Prostatakrebs, Bauchspeicheldrüsenkrebs, Hautkrebs, kolorektaler Krebs, Dickdarmkrebs, Plattenepithelkarzinom, Leukämie, Lymphom oder Knochenmarkkrebs ist.

14. Zusammensetzung zur Verwendung nach einem der Ansprüche 12-13, ferner umfassend Abgeben eines Wirkstoffs über die zelluläre Schicht; wobei die zelluläre Schicht gegebenenfalls eine Blut-Hirn-Schranke ist.

15. Zusammensetzung nach Anspruch 4, wobei der Wirkstoff ein Anti-Krebs-Medikament ist, ausgewählt aus der Gruppe, bestehend aus einem EGFRvIII-Inhibitor, Copiktra (Duvelisib), Erleada (Apalutamid), Libtayo (Cemiplimab-RWLC), Lorbrena (Lorlatinib), Lumoxiti (Moxetumomab-Pasudotox-TDF), Lutathera (Lutetium-Lu177-Dotatat), Talzenna (Talazoparib), Vizimpro (Dacomitinib), Aliqopa (Copanlisib), Alunbrig (Brigatinib), Bavencio (Avelumab), Besponsa (Inotuzumab ozogamicin), Calquence (Acalabrutinib), IDHIFA (Enasidenib), Imfinzi (Durvalumab), Kisqali (Ribociclib), Kymriah (Tisagenlecleucel), Nerlynx (Neratinib), Rydapt (Midostaurin),Vyxeos (Daunorubicin und Cytarabin), Xermelo (Telotristat-Ethyl), Yescarta (Axicabtagene ciloleucel), Zejula (Niraparib), Cabometyx (Cabozantinib), Keytruda (Pembrolizumab), Lartruvo (Olaratumab), Lenvima (Lenvatinib), Opdivo (Nivolumab), Rubraca (Rucaparib), Sustol (Granisetron), Syndros (Dronabinol, orale Lösung), Tecentriq (Atezolizumab), Venclexta (Venetoclax), Alecensa (Alectinib), Cotellic (Cobimetinib), Darzalex (Daratumumab), Empliciti (Elotuzumab), Farydak (Panobinostat), Ibrance (Palbociclib), Imlygic (Talimogen laherparepvec), Lenvima (Lenvatinib), Lonsurf (Trifluridin und Tipiracil), Ninlaro (Ixazomib), Odomzo (Sonidegib), Onivyde (Irinotecan, Liposominjektion), Portrazza (Necitumumab), Tagrisso (Osimertinib), Unituxin (Dinutuximab), Varubi (Rolapitant), Vistogard (Uridintriacetat), Yondelis (Trabectedin), Akynzeo (Netupitant und Palonosetron), Beleodaq (Belinostat), Blincyto (Blinatumomab), Cyramza (Ramucirumab), Imbruvica (Ibrutinib), Lynparza (Olaparib), Zydelig (Idelalisib), Zykadia (Ceritinib), Gazyva (Obinutuzumab), Gilotrif (Afatinib), Imbruvica (Ibrutinib), Kadcyla (Adotrastuzumab emtansin), Mekinist (Trametinib), Pomalyst (Pomalidomid), Revlimid (Lenalidomid), Stivarga (Regorafenib), Tafinlar (Dabrafenib), Valchlor (Mechlorethamin, Gel), Xgeva (Denosumab), Xofigo (Radium-Ra223-dichlorid), Abraxane (Paclitaxel, an Protein gebundene Partikel für eine injizierbare Suspension), Afinitor (Everolimus), Afinitor (Everolimus), Bosulif (Bosutinib), Cometriq (Cabozantinib), Erivedge (Vismodegib), Iclusig (Ponatinib), Inlyta (Axitinib), Kyprolis (Carfilzomib), Marqibo (Vincristinsulfat, Liposominjektion), Neutroval (TBO-Filgrastim), Perjeta (Pertuzumab), Picato (Ingenolmebutat, Gel), Stivarga (Regorafenib), Subsys (Fentanyl, sublinguales Spray), Synribo (Omacetaxin-Mepesuccinat), Votrient (Pazopanib), Xtandi (Enzalutamid), Zaltrap (ZIV-Aflibercept), Abstral (Fentanyl, Sublingualtabletten), Adcetris (Brentuximab vedotin), Afinitor (Everolimus), Erwinaze (Asparaginase Erwinia chrysanthemi), Lazanda (Fentanylcitrat, Nasenspray), Sutent (Sunitinibmalat), Sylatron (Peginterferon alfa-2b), Vandetanib (Vandetanib), Xalkori (Crizotinib), Yervoy (Ipilimumab), Zelboraf (Vemurafenib), Zytiga (Abirateronacetat), Halaven (Eribulinmesylat), Herceptin (Trastuzumab), Jevtana (Cabazitaxel), Provenge (Sipuleucel-T), Xgeva (Denosumab), Zuplenz (Ondansetron, oral löslicher Film), Afinitor (Everolimus), Arzerra (Ofatumumab), Avastin (Bevacizumab), Cervarix [bivalenter rekombinanter humaner Papillomavirus-Impfstoff (Typen 16 und 18)], Elitek (Rasburicase), Folotyn (Pralatrexat, Injektion), Istodax (Romidepsin), Onsolis (Fentanyl, buccal), Votrient (Pazopanib), Degarelix (Degarelix, zur Injektion), Fusilev (Levoleucovorin); Mozobil (Plerixafor, Injektion), Sancuso (Granisetron), Treanda (Bendamustin-Hydrochlorid), Evista (Raloxifen-Hydrochlorid), Hycamtin (Topotecan-Hydrochlorid), Ixempra (Ixabepilon), Tasigna (Nilotinib-Hydrochlorid-Monohydrat), Torisel (Temsirolimus), Tykerb (Lapatinib), Gardasil (quadrivalenter rekombinanter humaner Papillomavirus-Impfstoff (Typen 6, 11, 16, 18)), Sprycel (Dasatinib), Sutent (Sunitinib), Vectibix (Panitumumab), Arranon (Nelarabin), Nexavar (Sorafenib), Alimta (Pemetrexed, zur Injektion), Avastin (Bevacizumab); Clolar (Clofarabin), Erbitux (Cetuximab), Sensipar (Cinacalcet), Tarceva (Erlotinib, OSI 774), Aloxi (Palonosetron), Emend (Aprepitant), Iressa (Gefitinib); Plenaxis (Abarelix für eine injizierbare Suspension); Premarin (konjugierte Estrogene); UroXatral (Alfuzosin-HCl, Retardtabletten); Velcade (Bortezomib); Eligard (Leuprolidacetat); Eloxatin (Oxaliplatin/5-Fluoruracil/Leucovorin); Faslodex (Fulvestrant); Gleevec (Imatinibmesylat); Neulasta; SecreFlo (Secretin); Zevalin (Ibritumomab tiuxetan); Zometa (Zoledronsäure); Campath; Femara (Letrozol); Gleevec (Imatinibmesylat); Kytril (Granisetron, Lösung); Trelstar LA (Triptorelinpamoat); Xeloda; Zometa (Zoledronsäure); Mylotarg (Gemtuzumab ozogamicin); Trelstar Depot (Triptorelinpamoat); Trisenox (Arsentrioxid); Viadur (Leuprolidacetate, Implantat); Aromasin, Tabletten; Busulflex; Doxil (Doxorubicin-HCl, Liposominjektion); Ellence; Ethyol (Amifostin); Temodar; UVADEX, sterile Lösung; Zofran; Actiq; Anzemet; Camptosar; Gemzar (Gemcitabin-HCl); Herceptin; Inform HER-2/neu, Brustkrebstest; Neupogen; Nolvadex; Photofrin; Proleukin; Sclerosol, intrapleurales Aerosol; Valstar; Xeloda; Zofran; Anzemet; Bromfenac; Femara (Letrozol); Gliadel-Wafer (Polifeprosan 20 mit Carmustin-Implantat); Intron A (Interferon alfa-2b, rekombinant); Kytril (Granisetron, Tabletten); Lupron Depot (Leuprolidacetat, für eine Depotsuspension); Miraluma Test; Neumega; Quadramet (Samarium-Sm153-Lexidronam, Injektion); Rituxan; Taxol; Anexsia; Aredia (Pamidronat-Dinatrium, zur Injektion); Arimidex (Anastrozol); Campostar; Elliotts-B-Lösung (gepufferte intrathekale Elektrolyt/Dextrose-Injektion); Eulexin (Flutamid); Gemzar (Gemcitabin-HCl); Hycamtin (Topotecan-Hydrochlorid); Kadian; Leukine (Sargramostim); Lupron Depot (Leuprolidacetat, für eine Depotsuspension); photodynamische Therapie; Taxotere (Docetaxel); Visipaque (Iodixanol); Ethyol (Amifostin); Intron A (Interferon alfa-2b, rekombinant) und Leukine (Sargramostim).

## Revendications

1. Composition comprenant un peptide de liaison à la transferrine modifié, dans laquelle le peptide de liaison à la transferrine modifiée comprend une séquence présentant :
a) au moins 90 % d'identité de séquence avec SEQ ID NO : 67, ou un fragment de celle-ci comprenant au moins 29 résidus d'acides aminés ;
b) au moins 90 % d'identité de séquence avec l'une quelconque des SEQ ID NO : 37, SEQ ID NO : 202, SEQ ID NO : 172 ou SEQ ID NO : 171, ou un fragment de celle-ci comprenant au moins 29 résidus d'acides aminés ;
c) au moins 90 % d'identité de séquence avec l'une quelconque des SEQ ID NO : 32, SEQ ID NO : 2, SEQ ID NO : 167 ou SEQ ID NO : 137 ou SEQ ID NO :136, ou un fragment de celle-ci comprenant au moins 29 résidus d'acides aminés ; ou
d) au moins 95 % d'identité de séquence avec l'une quelconque des SEQ ID NO : 36, SEQ ID NO : 1, ou un fragment de celles-ci comprenant au moins 29 résidus d'acides aminés.

2. Composition selon la revendication 1, dans laquelle le peptide de liaison à la transferrine modifiée comprend une séquence de :
a) l'une quelconque des SEQ ID NO : 339 à SEQ ID NO : 344 ;
b) SEQ ID NO : 67 ;
c) SEQ ID NO : 37, SEQ ID NO : 202, SEQ ID NO : 172, SEQ ID NO : 36 ou SEQ ID NO : 171 ; ou
d) SEQ ID NO : 32, SEQ ID NO : 2, SEQ ID NO : 167, SEQ ID NO :137, SEQ ID NO : 1, ou SEQ ID NO : 136.

3. Composition selon la revendication 1 ou la revendication 2, dans laquelle le peptide de liaison à la transferrine modifiée comprend :
a) au moins quatre résidus de cystéine ;
b) au moins deux liaisons disulfures ;
c) au moins 43 résidus d'acides aminés ; ou
d) des combinaisons de ceux-ci.

4. Composition selon l'une quelconque des revendications 1 à 3, comprenant également un agent actif ou un agent détectable conjugué, lié, fusionné, ou greffé sur le peptide de liaison à la transferrine modifiée ; éventuellement, dans laquelle l'agent actif, l'agent détectable ou les deux sont liés au peptide de liaison à la transferrine modifiée par l'intermédiaire d'un lieur.

5. Composition selon la revendication 4, dans laquelle l'agent actif est un agent immunothérapeutique, un agent de ciblage CTLA-4, un agent de ciblage PD-1, un agent de ciblage PDL-1, un agent IL15, un agent complexe fusionné IL-15/IL-15Ra, un agent IFNgamma, un agent anti-CD3, un modulateur de canal ionique, un inhibiteur de Kv1.3, une auristatine, un MMAE, un maytansinoïde, le DM1, le DM4, la doxorubicine, une calicheamicine, un composé de platine, le cisplatine, un taxane, le paclitaxel, le SN-38, un inhibiteur de BACE, un inhibiteur de Bcl-xL, le WEHI-539, le vénétoclax, l'ABT-199, le navitoclax, l'AT-101, l'obatoclax, une pyrrolobenzodiazépine ou un dimère de pyrrolobenzodiazépine, une dolastatine, un neurotransmetteur, un agent anti-inflammatoire, un modulateur immunitaire, un inhibiteur du facteur de nécrose tumorale, un antagoniste du récepteur de nécrose tumorale, un inhibiteur de point de contrôle, un antagoniste du récepteur CGRP, une molécule cytotoxique, un inhibiteur de la tyrosine kinase, un inhibiteur de l'EGFRvIII ou un oligonucléotide.

6. Composition selon la revendication 5, dans laquelle :
a) l'inhibiteur Kv1.3 est Vm24, ShK-170, ShK-186 ou ShK-192 ;
b) le Vm24 a une séquence de SEQ ID NO : 378 ou SEQ ID NO : 391 ; ou dans lequel le Shk-185 a une séquence de SEQ ID NO : 379, SEQ ID NO : 390 ou SEQ ID NO : 402 ; ou
c) le neurotransmetteur est la neurotensine, un variant de peptide de neurotensine ou un fragment de celui-ci comprenant au moins 8 résidus d'acides aminés, au moins 10 résidus d'acides aminés ou au moins 13 résidus d'acides aminés ; éventuellement, dans laquelle le variant de peptide de neurotensine comprend une séquence de l'une quelconque des SEQ ID NO : 350 ou SEQ ID NO : 365 - SEQ ID NO : 369.

7. Composition selon l'une quelconque des revendications 4 à 6, dans laquelle l'agent détectable est un fluorophore, un colorant infrarouge proche, un agent de contraste, une nanoparticule, une nanoparticule contenant du métal, un chélate métallique, un agent de contraste à rayons X, un agent PET, un radionucléide ou un chélateur de radionucléide.

8. Composition selon l'une quelconque des revendications 4 à 7, dans laquelle le lieur est choisi dans le TABLEAU 7 ou l'une quelconque des SEQ ID NO : 103 - SEQ ID NO : 115, ou SEQ ID NO : 125.

9. Composition selon l'une quelconque des revendications 1 à 8, comprenant également un agent de modification de demi-vie, une fraction de pénétration cellulaire, un signal de localisation nucléaire ou une combinaison de ceux-ci couplée au peptide de liaison à la transferrine modifiée.

10. Composition selon la revendication 9, dans laquelle :
a) l'agent de modification de demi-vie comprend un polymère, un polyéthylène glycol (PEG), un amidon hydroxyéthylique, un alcool polyvinylique, un polymère hydrosoluble, un polymère hydrosoluble zwitterionique, un poly(acide aminé) hydrosoluble, un polymère hydrosoluble de proline, d'alanine et de sérine, un polymère hydrosoluble contenant de la glycine, de l'acide glutamique et de la sérine, une région Fc, un acide gras, de l'acide palmitique, une molécule qui se lie à l'albumine ou le SA21 ;
b) la fraction de pénétration cellulaire comprend une polycation, un acide polyorganique, des polymères libérant des endosomes, un poly(acide 2-propylacrylique), un poly(acide 2-éthylacrylique), un peptide Tat, un patch Arg, un peptide noué, le CysTAT, le S19-TAT, le R8 (SEQ ID NO : 73), le pAntp, le Pas-TAT, le Pas-R8 (SEQ ID NO : 76), le Pas-FHV, le Pas-pAntP, le F2R4 (SEQ ID NO : 79), le B55, l'auréine, l'IMT-P8, BR2, l'OMOTAG1, l'OMOTAG2, le pVEC, le SynB3, le DPV1047, le C105Y, le transportan, le MTS, le hLF, le PFVYLI (SEQ ID NO : 93), la maurocalcine, l'imperatoxine, l'hadrucaline, l'hémicalcine, l'opicalcine-1, l'opicalcine-2, le midkine (62-104), le MCoTI-II, le chlorotoxine, le DRI-TAT, le cFΦR4 (SEQ ID NO : 96), le R6W3 (SEQ ID NO : 421), le myristate, le yBBR, ou toute combinaison de ceux-ci ;
c) la fraction de pénétration cellulaire comprend une séquence de l'une quelconque des séquences SEQ ID NO : 71 - SEQ ID NO : 96, SEQ ID NO : 98 - SEQ ID NO : 101, SEQ ID NO : 116 - SEQ ID NO : 126, ou SEQ ID NO : 403 - SEQ ID NO : 455 ; ou
d) le signal de localisation nucléaire comprend une séquence de l'une quelconque des SEQ ID NO : 129 - SEQ ID NO : 135.

11. Composition selon l'une quelconque des revendications 1 à 10, dans laquelle le peptide de liaison à la transferrine modifiée comprend une séquence de l'une quelconque des séquences SEQ ID NO : 33 - SEQ ID NO : 35, SEQ ID NO : 68 - SEQ ID NO : 70, SEQ ID NO : 168 - SEQ ID NO : 170, SEQ ID NO : 203 - SEQ ID NO : 205, SEQ ID NO : 373 ou SEQ ID NO : 375.

12. Compositionselon l'une quelconque des revendications 1 à 11 destinée à être utilisée dans une méthode de traitement d'une affection chez un sujet dans le besoin, le procédé comprenant :
l'administration de la composition au sujet ;
la livraison de la composition à une couche cellulaire du sujet ;
la liaison du peptide de liaison à la transferrine modifiée à un récepteur de la transferrine (TfR) ; et
le transport de la composition à travers la couche cellulaire, traitant ainsi la maladie.

13. Composition destinée à être utilisée selon la revendication 12, dans laquelle l'état est la schizophrénie, la toxicomanie, la maladie de Parkinson, l'hypertension artérielle, un trouble de l'alimentation, un accident vasculaire cérébral, la maladie d'Alzheimer, le cancer, l'inflammation, la douleur, l'épilepsie, un trouble de l'humeur, un trouble endocrinien, un trouble du système nerveux central, un trouble neurodégénératif, une maladie neuro-inflammatoire, une maladie auto-immune cérébrale, la sclérose latérale amyotrophique, la maladie de Huntington, la sclérose en plaques, une lésion cérébrale traumatique, la maladie de Crohn, une maladie inflammatoire de l'intestin ou une maladie musculaire; éventuellement dans laquelle
a) la douleur est aiguë, chronique ou neuropathique ; ou
b) le cancer est un cancer du cerveau, une tumeur primaire du SNC, un glioblastome, un gliome, un gliome pontin intrinsèque diffus, un astrocytome, un médulloblastome, un cancer du sein, un cancer du poumon, un cancer de la tête et du cou, un cancer du foie, un cancer du rein, un cancer de la rate, un cancer de la prostate, un cancer du pancréas, un cancer de la peau, un cancer colorectal, un cancer du côlon, un carcinome squameux, une leucémie, un lymphome ou un cancer de la moelle osseuse.

14. Composition destinée à être utilisée selon l'une quelconque des revendications 12 à 13, comprenant également l'administration d'un agent actif à travers la couche cellulaire ; éventuellement, dans laquelle la couche cellulaire étant une barrière hémato-encéphalique.

15. Composition selon la revendication 4, dans laquelle l'agent actif est un médicament anticancéreux choisi dans le groupe constitué d'un inhibiteur de l'EGFRvIII, Copiktra (duvelisib), Erleada (apalutamide), Libtayo (cemiplimab-rwlc), Lorbrena (lorlatinib), Lumoxiti (moxetumomab pasudotox-tdf), Lutathera (lutetium Lu 177 dotatate), Talzenna (talazoparib), Vizimpro (dacomitinib), Aliqopa (copanlisib), Alunbrig (brigatinib), Bavencio (avélumab), Besponsa (inotuzumab ozogamicin), Calquence (acalabrutinib), IDHIFA (énasidenib), Imfinzi (durvalumab), Kisqali (ribociclib), Kymriah (tisagenlecleucel), Nerlynx (nératinib), Rydapt (midostaurine), Vyxeos (daunorubicine et cytarabine), Xermelo (éthyle de télotristat), Yescarta (axicabtagene ciloleucel), Zejula (niraparib), Cabometyx (cabozantinib), Keytruda (pembrolizumab), Lartruvo (olaratumab), Lenvima (lenvatinib), Opdivo (nivolumab), Rubraca (rucaparib), Sustol (granisétron), Syndros (solution buvable de dronabinol), Tecentriq (atézolizumab), Venclexta (vénétoclax), Alecensa (alectinib), Cotellic (cobimétinib) ), Darzalex (daratumumab), Empliciti (élotuzumab), Farydak (panobinostat), Ibrance (palbociclib), Imlygic (talimogène laherparepvec), Lenvima (lenvatinib), Lonsurf (trifluridine et tipiracil), Ninlaro (ixazomib), Odomzo (sonidégib), Onivyde (irinotécan) injection de liposomes), Portrazza (nécitumumab), Tagrisso (osimertinib), Unituxin (dinutuximab), Varubi (rolapitant), Vistogard (triacétate d'uridine), Yondelis (trabectédine), Akynzeo (nétupitant et palonosétron), Beleodaq (bélinostat), Blincyto (blinatumomab) ), Cyramza (ramucirumab), Imbruvica (ibrutinib), Lynparza (olaparib), Zydelig (idélalisib), Zykadia (céritinib), Gazyva (obinutuzumab), Gilotrif (afatinib), Imbruvica (ibrutinib), Kadcyla (ado-trastuzumab emtansine), Mekinist (tramétinib), alys Pomt (pomalidomide), Revlimid (lénalidomide), Stivarga (régorafénib), Tafinlar (dabrafenib), Valchlor (méchloréthamine) gel, Xgeva (dénosumab), Xofigo (dichlorure de radium Ra 223), Abraxane (particules liées aux protéines de paclitaxel pour suspension injectable), Afinitor (évérolimus), Afinitor (évérolimus), Bosulif (bosutinib), Cometriq (cabozantinib), Erivedge (vismodegib), Iclusig (ponatinib), Inlyta (axitinib), Kyprolis (carfilzomib), Marqibo (injection de LIPOSOME de sulfate de vinCRIStine), Neutroval (tbo-filgrastim), Perjeta (pertuzumab), Picato (mébutate d'ingénol) gel, Stivarga (régorafenib), Subsys (vaporisateur sublingual de fentanyl), Synribo (mépésuccinate d'omacétaxine), Votrient (pazopanib), Xtandi (enzalutamide), Zaltrap (ziv-aflibercept), Abstral (comprimés sublinguaux de fentanyl), Adcetris (brentuximab védotine), Afinitor (évérolimus), Erwinaze (asparaginase Erwinia chrysanthemi), Lazanda (citrate de fentanyl) en vaporisateur nasal, Sutent (malate de sunitinib), Sylatron (peginterféron alfa-2b), Vandetanib (vandétanib), Xalkori (crizotinib), Yervoy (ipilimumab), Zelboraf (vémurafénib), Zytiga (acétate d'abiratérone), Halaven (éribuline) mésylate) , Herceptin (trastuzumab), Jevtana (cabazitaxel), Provenge (sipuleucel-T), Xgeva (denosumab), Zuplenz (film soluble oral d'ondansétron), Afinitor (everolimus), Arzerra (ofatumumab), Avastin (bévacizumab), Cervarix [ Vaccin bivalent contre le virus du papillome humain (types 16 et 18), recombinant, Elitek (rasburicase), Folotyn (pralatrexate injectable), Istodax (romidepsine), Onsolis (fentanyl buccal), Votrient (pazopanib), Degarelix (degarelix pour injection), Fusilev (lévoleucovorine) ; Mozobil (injection de plérixafor), Sancuso (granisétron), Treanda (chlorhydrate de bendamustine), Evista (chlorhydrate de raloxifène), Hycamtin (chlorhydrate de topotécan), Ixempra (ixabépilone), Tasigna (chlorhydrate de nilotinib monohydraté), Torisel (temsirolimus), Tykerb (lapatinib), Gardasil (vaccin recombinant quadrivalent contre le virus du papillome humain (types 6, **11,** 16, 18)), Sprycel (dasatinib), Sutent (sunitinib), Vectibix (panitumumab), Arranon (nélarabine), Nexavar (sorafenib), Alimta (pemetrexed pour injection) ), Avastin (bévacizumab) ; Clolar (clofarabine), Erbitux (cétuximab), Sensipar (cinacalcet), Tarceva (erlotinib, OSI 774), Aloxi (palonosétron), Emend (aprépitant), Iressa (géfitinib) ; Plenaxis (abarélix pour suspension injectable) ; Premarin (œstrogènes conjugués) ) ; UroXatral (comprimés à libération prolongée d'alfuzosine HCl) ; Velcade (bortézomib) ; Eligard (acétate de leuprolide) ; Eloxatin (oxaliplatine/5-fluorouracile/leucovorine) ; Faslodex (fulvestrant) ; Gleevec (mésylate d'imatinib) ; Neulasta ; SecreFlo (sécrétine ) ; Zevalin (ibritumomab tiuxétan) ; Zometa (acide zolédronique) ; Campath ; Femara (létrozole) ; Gleevec (mésylate d'imatinib) ; solution de Kytril (granisétron) ; Trelstar LA (pamoate de triptoréline) ; Xeloda ; Zometa (acide zolédronique) ; Mylotarg (gemtuzumab ozogamicine) ; Trelstar Depot (pamoate de triptoréline) ;Trisenox (trioxyde d'arsenic) ; Viadur (acétate de leuprolide) implant) ; Comprimés d'Aromasin ; Busulflex ; Doxil (injection liposomale de doxorubicine HCl) ; Ellence ; Ethyol (amifostine) ; Temodar ; Solution stérile UVADEX ; Zofran ; Actiq ; Anzemet ; Camptosar ; Gemzar (gemcitabine HCL) ; Herceptin ; Inform HER-2/ test de cancer du sein ; Neupogen ; Nolvadex ; Photofrin ; Proleukin ; Sclerosol Intrapleural Aerosol ; Valstar ; Xeloda ; Zofran ; Anzemet ; Bromfénac ; Femara (létrozole) ; Gliadel Wafer (polyféprosan 20 avec implant de carmustine) ; Intron A (interféron alfa-2b, recombinant) ; comprimés de Kytril (granisétron) ; Lupron Depot (acétate de leuprolide pour suspension à effet retard) ; test Miraluma ; Neumega ; Quadramet (injection de samarium Sm 153 Lexidronam) ; Rituxan ; Taxol ; Anexsia ; Aredia (pamidronate disodique pour injection) ; Arimidex (anastrozole) ; Campostar ; Elliotts B Solution (injection intrathécale tamponnée d'électrolyte/dextrose) ; Eulexin (flutamide) ; Gemzar ( gemcitabine HCL) ; Hycamtin (chlorhydrate de topotécan) ; Kadian ; Leukine (sargramostim) ; Lupron Depot (acétate de leuprolide pour suspension dépôt) ; Thérapie photodynamique ; Taxotère (Docétaxel) ; Visipaque (iodixanol) ; Ethyol (amifostine) ; Intron A (Interféron alfa-2b, recombinant)et Leukine (sargramostim).
